(19) Europäisches Patentamt
European Patent Office
Office européen des brevets

(11) EP 0 900 023 B1

(12) EUROPÄISCHE PATENTSCHRIFT

(45) Veröffentlichungstag und Bekanntmachung des Hinweises auf die Patenterteilung:
**27.11.2002 Patentblatt 2002/48**

(21) Anmeldenummer: **97916445.6**

(22) Anmeldetag: **07.04.1997**

(51) Int Cl.⁷: **A01N 47/36**, C07D 239/47, C07D 251/16, C07D 239/52, C07D 239/42, C07D 251/46, C07D 239/48, C07D 251/22

(86) Internationale Anmeldenummer:
**PCT/EP97/01715**

(87) Internationale Veröffentlichungsnummer:
**WO 97/040690 (06.11.1997 Gazette 1997/47)**

(54) **SUBSTITUIERTE AMINOMETHYLPHENYLSULFONYLHARNSTOFFE, IHRE HERSTELLUNG UND VERWENDUNG ALS HERBIZIDE UND PFLANZENWACHSTUMSREGULATOREN**

SUBSTITUTED AMINOMETHYL PHENYL SULPHONYL UREAS, THEIR PRODUCTION AND THEIR USE AS HERBICIDES AND PLANT GROWTH REGULATORS

AMINOMETHYLPHENYLSULFONYLUREES SUBSTITUEES, LEUR PRODUCTION ET LEUR UTILISATION COMME HERBICIDES ET REGULATEURS DE LA CROISSANCE VEGETALE

(84) Benannte Vertragsstaaten:
**DE DK ES FR GB IT**

(30) Priorität: **25.04.1996 DE 19616445**

(43) Veröffentlichungstag der Anmeldung:
**10.03.1999 Patentblatt 1999/10**

(73) Patentinhaber: **Bayer CropScience GmbH**
**65929 Frankfurt/Main (DE)**

(72) Erfinder:
- **LORENZ, Klaus**
  **D-64331 Weiterstadt (DE)**
- **WILLMS, Lothar**
  **D-65719 Hofheim (DE)**
- **BAUER, Klaus**
  **D-63456 Hanau (DE)**
- **BIERINGER, Hermann**
  **D-65817 Eppstein (DE)**
- **ROSINGER, Christopher**
  **D-65179 Hofheim (DE)**

(56) Entgegenhaltungen:
**EP-A- 0 205 348**
**WO-A-89/10921**
**DE-A- 4 335 297**
**US-A- 4 927 453**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 900 023 B1

## Beschreibung

Es ist bekannt, daß einige Phenylsulfonylharnstoffe herbizide und pflanzenwachstumsregulierende Eigenschaften besitzen; vgl. US-A-4,786,314, US-A-4,927,453, WO 89/10921 und WO 95/10507 (= ZA 94/8063). Diese weisen jedoch bei ihrer Anwendung zum Teil Nachteile auf, wie beispielsweise eine hohe Persistenz oder unzureichende Selektivität in wichtigen Nutzpflanzenkulturen oder zu geringes Spektrum der kontrollierbaren Schadpflanzen.

Es wurden nun neue Phenysulfonylharnstoffe mit speziellen Resten am Phenylring gefunden, die mit Vorteilen als Herbizide und Pflanzenwachstumsregulatoren eingesetzt werden können.

Gegenstand der vorliegenden Erfindung sind Verbindungen der Formel (I) oder deren Salze,

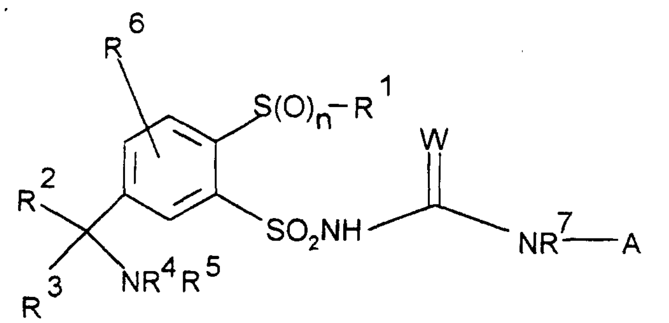

(I)

worin

| | |
|---|---|
| $R^1$ | $NR^8R^9$, einen Kohlenwasserstoffrest, der unsubstituiert oder substituiert ist und vorzugsweise inklusive Substituenten 1 bis 20 C-Atome enthält, |
| n | 0, 1 oder 2, ausgenommen n = 0 oder 1, wenn $R^1$ = $NR^8R^9$ bedeutet, |
| $R^2$, $R^3$ | unabhängig voneinander H oder (1-4)Alkyl, |
| $R^4$ | H, OH, Formyl, einen Rest der Formel R, R-O-, R-CO- oder R-$SO_2$-, wobei R einen Kohlenwasserstoffrest bedeutet, der unsubstituiert oder substituiert ist und vorzugsweise inklusive Substituenten 1 bis 20 C-Atomen aufweist, und |
| $R^5$ | einen Acylrest oder |
| $NR^4R^5$ | gemeinsam einen heterocyclischen Rest, der unsubstituiert oder substituiert ist und vorzugsweise inklusive Substituenten 2 bis 12 C-Atome enthält und insbesondere durch Substituenten des Heterocyclus am N-Atom der Gruppe $NR^4R^5$ einen elektronenziehenden Rest aufweist, |
| W | ein Sauerstoff- oder Schwefelatom, |
| $R^6$ | H, (1-4)Alkyl, (1-4)Alkoxy, (1-4)Alkylthio, [(1-4)Alkyl]-carbonyl, [(1-4)Alkoxy]-carbonyl, wobei jeder der letztgenannten Reste unsubstituiert oder durch einen oder mehrere Halogenatome substituiert ist, oder Halogen, $NO_2$, CN oder mono- oder disubstituiertes Amino, |
| $R^7$ | H oder (1-4)Alkyl, |
| $R^8$,$R^9$ | unabhängig voneinander H, (1-4)Alkyl, (1-4)Alkoxy, [(1-4)Alkyl]carbonyl oder (1-4) Alkylsulfonyl, |
| A | einen Rest der Formel |

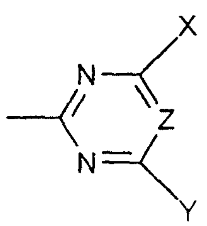 ,

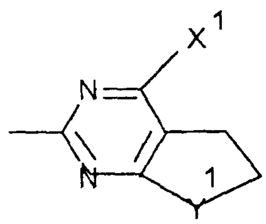 ,

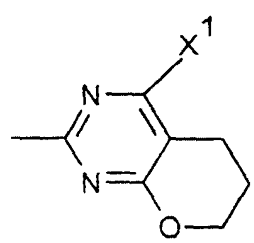 ,

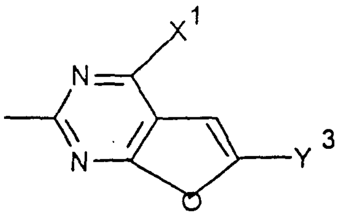,
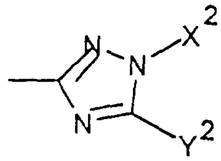,
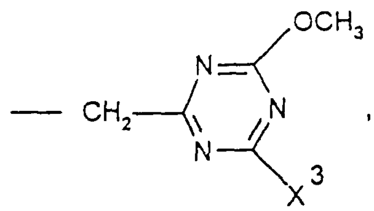,

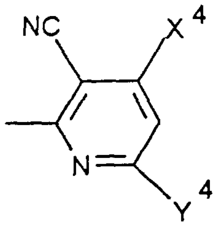
oder
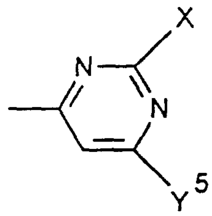

| | |
|---|---|
| einer der Reste X und Y | Wasserstoff, (1-3)Alkyl oder (1-3)Alkoxy, wobei die beiden letztgenannten Reste unsubstituiert oder ein- oder mehrfach durch Halogen oder einfach durch (1-3)Alkoxy substituiert sind, und |
| der andere der Reste X und Y | Wasserstoff, Halogen, (1-3)Alkyl, (1-3)Alkoxy oder (1-3)Alkylthio, wobei die drei letztgenannten alkylhaltigen Reste unsubstituiert oder ein- oder mehrfach durch Halogen oder ein- oder zweifach durch (1-3)Alkoxy oder (1-3)Alkylthio substituiert sind, oder einen Rest der Formel $NR^aR^b$, worin $R^a$, $R^b$ unabhängig voneinander H, (1-3)Alkyl oder Alkenyl mit bis zu 3 C-Atomen bedeuten, oder (3-6)Cycloalkyl, (2-4)Alkenyl, (2-4)Alkinyl, (3-4)-Alkenyloxy oder (3-4)Alkinyloxy, |
| Z | CH oder N, |
| $X^1$ | $CH_3$, $OCH_3$, $OC_2H_5$ oder $OCHF_2$, |
| $Y^1$ | -O- oder $-CH_2-$, |
| $X^2$ | $C_{H3}$, $C_2H_5$ oder $CH_2CF_3$, |
| $Y^2$ | $OCH_3$, $OC_2H_5$, $SCH_3$, $SCH_2CH_3$, $CH_3$ oder $C_2H_5$, |
| $X^3$ | $CH_3$ oder $OCH_3$, |
| $Y^3$ | H oder $CH_3$, |
| $X^4$ | $CH_3$, $OCH_3$, $OC_2H_5$, $CH_2OCH_3$ oder Cl, |
| $Y^4$ | $CH_3$, $OCH_3$, $OC_2H_5$ oder Cl und |
| $Y^5$ | $CH_3$, $C_2H_5$, $OCH_3$ oder Cl |

bedeuten.

Definitionen für allgemeine Reste mit Kohlenstoffatomen in Formel (I) enthalten oft Bereiche oder Einzelangaben für die Anzahl der möglichen Kohlenstoffatome. Die Bereichsangabe bzw. Zahl für die C-Atome wird der Bezeichnung für die allgemeine chemische Gruppe in Klammern vorangestellt; so bedeutet z.B. (1-4)Alkyl einen Alkylrest mit 1 bis 4 C-Atomen; oder (1-4)Haloalkyl bedeutet Haloalkyl mit 1 bis 4 C-Atomen im Alkylteil oder Alkylgerüst; (1) Alkyl ist gleichbedeutend mit Methyl; unter die allgemeine Definition unsubstituiertes (3)Alkyl fallen demnach n-Propyl und i-Propyl.

Die Verbindungen der Formel (I) können Salze bilden, bei denen der Wasserstoff der $-SO_2-NH$-Gruppe durch ein für die Landwirtschaft geeignetes Kation ersetzt wird. Diese Salze sind beispielsweise Metallsalze, insbesondere Alkalimetallsalze oder Erdalkalimetallsalze, insbesondere Natrium- und Kaliumsatze, oder auch Ammoniumsalze oder Salze mit organischen Aminen. Ebenso kann Salzbildung durch Anlagerung einer Säure an basischen Gruppen, wie z.B. Amino und Alkylamino, erfolgen. Geeignete Säuren hierfür sind starke anorganische und organische Säuren, beispielsweise HCl, HBr, $H_2SO_4$ oder $HNO_3$.

In Formel (I) und allen nachfolgenden Formeln können die Reste Alkyl, Alkoxy, Haloalkyl, Haloalkoxy, Alkylamino und Alkylthio sowie die entsprechenden ungesättigten und/oder substituierten Reste im Kohlenstoffgerüst jeweils geradkettig oder verzweigt sein. Wenn nicht speziell angegeben, sind bei diesen Resten die niederen Kohlenstoffgerüste, z.B. mit 1 bis 6 C-Atomen bzw. bei ungesättigten Gruppen mit 2 bis 6 C-Atomen, bevorzugt. Alkylreste, auch in den zusammengesetzten Bedeutungen wie Alkoxy, Haloalkyl usw., bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl, Heptyle, wie n-Heptyl, 1-Methylhexyl und 1,4-Dimethylpentyl; Alkenyl- und Alkinylreste haben die Bedeutung der den Alkylresten entsprechenden möglichen ungesättigten Reste; Alkenyl bedeutet z.B. Allyl, 1-Methylprop-2-en-1-yl, 2-Methyl-prop-2-en-1-yl, But-2-en-1-yl, But-3-en-1-yl, 1-Methyl-but-3-en-1-yl und 1-Methyl-but-2-en-1-yl; Alkinyl bedeutet z.B. Propargyl, But-2-in-1-yl, But-3-in-1-yl, 1-Methyl-but-3-in-1-yl.

Cycloalkyl bedeutet ein carbocyclisches, gesättigtes Ringsystem, z.B. mit 3-8 C-Atomen, z.B. Cyclopropyl, Cyclopentyl oder Cyclohexyl.

Alkenyl in der Form "(3-4)Alkenyl" oder "(3-6)Alkenyl" bedeutet vorzugsweise einen Alkenylrest mit 3 bis 4 bzw. 3 bis 6 C-Atomen, bei dem die Doppelbindung nicht an dem C-Atom liegt, das mit dem übrigen Molekülteil der Verbindung (I) verbunden ist ("yl"-Position). Entsprechendes gilt für (3-4)Alkinyl usw.

Halogen bedeutet beispielsweise Fluor, Chlor, Brom oder lod. Haloalkyl, -alkenyl und -alkinyl bedeuten durch Halogen, vorzugsweise durch Fluor, Chlor und/oder Brom, insbesondere durch Fluor oder Chlor, teilweise oder vollständig substituiertes Alkyl, Alkenyl bzw. Alkinyl, z.B. $CF_3$, $CHF_2$, $CH_2F$, $CF_3CF_2$, $CH_2FCHCl$, $CCl_3$, $CHCl_2$, $CH_2CH_2Cl$; Haloalkoxy ist z.B. $OCF_3$, $OCHF_2$, $OCH_2F$, $CF_3CF_2O$, $OCH_2CF_3$ und $OCH_2CH_2Cl$; entsprechendes gilt für Haloalkenyl und andere durch Halogen substituierte Reste.

Ein Kohlenwasserstoffrest ist ein geradkettiger, verzweigter oder cyclischer und gesättigter oder ungesättigter aliphatischer oder aromatischer Kohlenwasserstoffrest, z.B. Alkyl, Alkenyl, Alkinyl, Cycloalkyl, Cycloalkenyl oder Aryl; Aryl bedeutet dabei ein mono-, bi- oder polycyclisches aromatisches System, beispielsweise Phenyl, Naphthyl, Tetrahydronaphthyl, Indenyl, Indanyl, Pentalenyl, Fluorenyl und ähnliches, vorzugsweise Phenyl;
vorzugsweise bedeutet ein Kohlenwasserstoffrest Alkyl, Alkenyl oder Alkinyl mit bis zu 12 C-Atomen oder Cycloalkyl mit 3, 4, 5, 6 oder 7 Ringatomen oder Phenyl; entsprechendes gilt für einen Kohlenwasserstoffrest in einem Kohlenwasserstoffoxyrest.

Ein heterocyclischer Rest oder Ring (Heterocyclyl) kann gesättigt, ungesättigt oder heteroaromatisch sein; er enthält vorzugsweise ein oder mehrere Heteroeinheiten im Ring, vorzugsweise aus der Gruppe N, O, S, SO, $SO_2$; vorzugsweise ist er ein aliphatischer Heterocyclylrest mit 3 bis 7 Ringatomen oder ein heteroaromatischer Rest mit 5 oder 6 Ringatomen und enthält 1, 2 oder 3 Heteroeinheiten. Der heterocyclische Rest kann z.B. ein heteroaromatischer Rest oder Ring (Heteroaryl) sein, wie z.B. ein mono-, bi- oder polycyclisches aromatisches System, in dem mindestens 1 Ring ein oder mehrere Heteroatome enthält, beispielsweise Pyridyl, Pyrimidinyl, Pyridazinyl, Pyrazinyl, Thienyl, Thiazolyl, Oxazolyl, Furyl, Pyrrolyl, Pyrazolyl und Imidazolyl, oder ist ein partiell oder vollständig hydrierter Rest wie Oxiranyl, Pyrrolidyl, Piperidyl, Piperazinyl, Dioxolanyl, Morpholinyl, Tetrahydrofuryl. Als Substituenten für einen substituierten heterocyclischen Rest kommen die weiter unten genannten Substituenten in Frage, zusätzlich auch Oxo. Die Oxogruppe kann auch an den Heteroringatomen, die in verschiedenen Oxidationsstufen existieren können, z.B. bei N und S, auftreten.

Substituierte Reste, wie substituierte Kohlenwasserstoffreste, z.B. substituiertes Alkyl, Alkenyl, Alkinyl, Aryl, Phenyl und Benzyl, oder substituiertes Heterocyclyl oder Heteroaryl, bedeuten beispielsweise einen vom unsubstituierten Grundkörper abgeleiteten substituierten Rest, wobei die Substituenten beispielsweise einen oder mehrere, vorzugsweise 1, 2 oder 3 Reste aus der Gruppe Halogen, Alkoxy, Haloalkoxy, Alkylthio, Hydroxy, Amino, Nitro, Carboxy, Cyano, Azido, Alkoxycarbonyl, Alkylcarbonyl, Formyl, Carbamoyl, Mono- und Dialkylaminocarbonyl, substituiertes Amino, wie Acylamino, Mono- und Dialkylamino, und Alkylsulfinyl, Haloalkylsulfinyl, Alkylsulfonyl, Haloalkylsulfonyl und, im Falle cyclischer Reste, auch Alkyl und Haloalkyl sowie den genannten gesättigten kohlenwasserstoffhaltigen Resten entsprechende ungesättigte aliphatische Reste, wie Alkenyl, Alkinyl, Alkenyloxy, Alkinyloxy etc. bedeuten. Bei Resten mit C-Atomen sind solche mit 1 bis 4 C-Atomen, insbesondere 1 oder 2 C-Atomen, bevorzugt. Bevorzugt sind in der Regel Substituenten aus der Gruppe Halogen, z.B. Fluor und Chlor, (1-4)Alkyl, vorzugsweise Methyl oder Ethyl, (1-4) Haloalkyl, vorzugsweise Trifluormethyl, (1-4)Alkoxy, vorzugsweise Methoxy oder Ethoxy, (1-4)Haloalkoxy, Nitro und Cyano. Besonders bevorzugt sind dabei die Substituenten Methyl, Methoxy und Chlor.

Mono- oder disubstituiertes Amino bedeutet einen chemisch stabilen Rest aus der Gruppe der substituierten Aminoreste, welche beispielsweise durch einen bzw. zwei gleiche oder verschiedene Reste aus der Gruppe Alkyl, Alkoxy, Acyl und Aryl N-substituiert sind; vorzugsweise Monoalkylamino, Dialkylamino, Acylamino, Arylamino, N-Alkyl-N-arylamino sowie N-Heterocyclen; dabei sind Alkylreste mit 1 bis 4 C-Atomen bevorzugt; Aryl ist dabei vorzugsweise Phenyl oder substituiertes Phenyl; für Acyl gilt dabei die weiter unten genannte Definition, vorzugsweise (1-4) Alkanoyl. Entsprechenes gilt für substituiertes Hydroxylamino oder Hydrazino.

Gegebenenfalls substituiertes Phenyl ist vorzugsweise Phenyl, das unsubstituiert oder ein- oder mehrfach, vorzugsweise bis zu dreifach durch gleiche oder verschiedene Reste aus der Gruppe Halogen, (1-4)Alkyl, (1-4)Alkoxy, (1-4)Halogenalkyl, (1-4)Halogenalkoxy und Nitro substituiert ist, z.B. o-, m- und p-Tolyl, Dimethylphenyle, 2-, 3- und 4-Chlorphenyl, 2-, 3- und 4-Trifluor- und -Trichlorphenyl, 2,4-, 3,5-, 2,5- und 2,3-Dichlorphenyl, o-, m- und p-Methoxyphenyl. Ein Acylrest bedeutet den Rest einer organischen Säure, z.B. den Rest einer Carbonsäure und Reste davon abgeleiteter Säuren wie der Thiocarbonsäure, gegebenenfalls N-substituierten Iminocarbonsäuren oder den Rest von Kohlensäuremonoestern, gegebenenfalls N-substituierter Carbaminsäure,

Sulfonsäuren, Sulfinsäuren, Phosphonsäuren, Phosphinsäuren. Acyl bedeutet beispielsweise Formyl, Alkylcarbonyl wie [(1-4)-Alkyl]-carbonyl, Phenylcarbonyl, wobei der Phenylring substituiert sein kann, z.B. wie oben für Phenyl gezeigt, oder Alkyloxycarbonyl, Phenyloxycarbonyl, Benzyloxycarbonyl, Alkylsulfonyl, Alkylsulfinyl, N-Alkyl-1-iminoalkyl und andere Reste von organischen Säuren.

Gegenstand der Erfindung sind auch alle Stereoisomeren, die von Formel (I) umfaßt sind, und deren Gemische. Solche Verbindungen der Formel (I) enthalten ein oder mehrere asymmetrische C-Atome oder auch Doppelbindungen, die in der allgemeinen Formel (I) nicht gesondert angegeben sind. Die durch ihre spezifische Raumform definierten möglichen Stereoisomeren, wie Enantiomere, Diastereomere, Z- und E-Isomere sind alle von der Formel (I) umfaßt und können nach üblichen Methoden aus Gemischen der Stereoisomeren erhalten oder auch durch stereoselektive Reaktionen in Kombination mit dem Einsatz von stereochemisch reinen Ausgangsstoffen hergestellt werden.

Die vorstehenden Beispiele für Reste oder Restebereiche, die unter die allgemeinen Begriffe wie "Alkyl", "Acyl", "substituierte Reste" etc., fallen bedeuten keine vollständige Aufzählung. Die allgemeinen Begriffe umfassen insbesondere auch die weiter unten angeführten Definitionen für Restebereiche in Gruppen bevorzugter Verbindungen, insbesondere Restebereiche, welche spezifische Reste aus den Tabellenbeispielen umfassen.

Vor allem aus Gründen der höheren herbiziden Wirkung, besseren Selektivität und/oder besseren Herstellbarkeit sind erfindungsgemäße Verbindungen der Formel (I) oder deren Salze von besonderem Interesse, worin

$R^1$ $NR^8R^9$, (1-6)Alkyl, (2-6)Alkenyl, (2-6)Alkinyl, (3-6)Cycloalkyl, (3-6)Cycloalkenyl oder Phenyl, wobei jeder der letztgenannten 6 Reste unsubstituiert oder durch einen oder mehrere Reste aus der Gruppe Halogen, CN, (1-4)Alkoxy, (1-4)Haloalkoxy, (1-4)Alkoxy(1-4)alkoxy, (1-4)Alkylthio, (1-4)Alkylsulfinyl, (1-4)-Alkylsulfonyl, Formyl, [(1-4)Alkyl]-carbonyl, [(1-4)Alkoxy]carbonyl, [(1-4)Alkyl]-carbonyloxy und im Falle cyclischer Reste auch (1-4)Alkyl, (1-4)Haloalkyl und (1-4)Alkoxy-(1-4)alkyl substituiert ist,

n 0, 1 oder 2, ausgenommen n = 0 oder 1, wenn $R^1 = NR^8R^9$,

$R^2$, $R^3$ unabhängig voneinander H oder (1-4)Alkyl,

$R^4$ H, OH, Formyl, (1-6)Alkyl, (2-6)Alkenyl, (2-6)Alkinyl, (1-6)Alkoxy, (2-5)Alkenyloxy, (2-5)Alkinyloxy, [(1-6)Alkyl]-carbonyl, (1-6)Alkyl-sulfonyl, [(2-6)Alkenyl]-carbonyl, (2-6)Alkenylsulfonyl, [(2-6)Alkinyl]-carbonyl, (2-6)Alkinylsulfonyl, (3-6)Cycloalkyl, (3-6)Cycloalkenyl, [(3-6)Cycloalkyl]-carbonyl, (3-6)Cycloalkylsulfonyl, [(3-6)Cycloalkenyl]-carbonyl, (3-6)Cycloalkenylsulfonyl, wobei jeder der 18 letztgenannten Reste unsubstituiert oder durch einen oder mehrere Reste aus der Gruppe Halogen, (1-4) Alkoxy, (1-4)Alkylthio, (1-4)Alkylsulfinyl, (1-4)Alkyl-sulfonyl, [(1-4)Alkoxy]-carbonyl, [(1-4)Alkyl]-carbonyl, [(1-4)Alkyl]-carbonyloxy und CN und im Falle cyclischer Reste auch (1-4)Alkyl und (1-4)Haloalkyl substituiert ist, oder Phenylcarbonyl oder Phenylsulfonyl, wobei in den beiden letztgenannten Resten der Phenylring unsubstituiert oder durch einen oder mehrere Reste aus der Gruppe Halogen, CN, $NO_2$, (1-4)Alkyl, (1-4)Haloalkyl, (1-4)Alkoxy und (1-4)Haloalkoxy substituiert ist, und

$R^5$ CHO, [(1-6)Alkyl]-carbonyl, [(2-6)Alkenyl]-carbonyl, [(2-6)Alkinyl]-carbonyl, (1-6)Alkylsulfonyl, (2-6) Alkenylsulfonyl, (2-6)Alkinylsulfonyl, [(3-6)Cycloalkyl]-carbonyl, [(3-6)Cycloalkenyl]-carbonyl, (3-6) Cycloalkyl-sulfonyl oder (3-6)Cycloalkenylsulfonyl, wobei jeder der 10 letztgenannten Reste unsubstituiert oder durch einen oder mehrere Reste aus der Gruppe Halogen, (1-4)Alkoxy, (1-4)Alkylthio, (1-4)Alkylsulfinyl (1-4)Alkylsulfonyl, [(1-4)Alkyl]-carbonyl, [(1-4)Alkoxy]-carbonyl, [(1-4)Alkyl]-carbonyloxy und CN und im Falle cyclischer Reste auch (1-4)Alkyl und (1-4)Haloalkyl substituiert ist, oder Phenylcarbonyl oder Phenylsulfonyl, wobei jeder der beiden letztgenannten Reste im Phenylring unsubstituiert oder durch einen oder mehrere Reste aus der Gruppe Halogen, CN, $NO_2$, (1-4)Alkyl, (1-4) Haloalkyl, (1-4)Alkoxy und (1-4)Haloalkoxy substituiert ist, oder Mono- oder Di-[(1-4)alkyl]amino-sulfonyl, das im Alkylteil unsubstituiert oder durch einen oder mehrere Reste aus der Gruppe Halogen, (1-4)Alkoxy, (1-4)Alkylthio, (1-4)Alkyl-sulfinyl, [(1-4)Alkyl]-carbonyl, [(1-4)Alkyl]-carbonyloxy, [(1-4) Alkoxy]-carbonyl und CN substituiert ist oder eine Gruppe der Formel COCOR', worin R' = H, OH, (1-4)Alkoxy oder (1-4)Alkyl ist, oder eine Gruppe der Formel

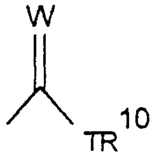 , 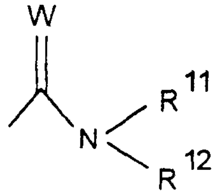 oder 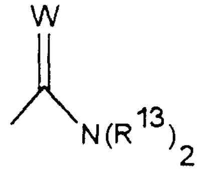 ,

oder

$R^4$ und $R^5$ gemeinsam eine Kette der Formel $(-CH_2)_{m1}$ $B^1$- oder $B^1$-$(CH_2)_{m2}B^2$, wobei einzelne Gruppen $CH_2$ durch Sauerstoffatome ersetzt sein können und wobei die Kette unsubstituiert oder durch einen oder mehrere (1-3)Alkylreste oder Halogen substituiert ist und m1 = 3, 4 oder 5 bzw. m2 = 2, 3 oder 4 sind, sowie

W O oder S,

$B^1$, $B^2$ unabhängig voneinander $SO_2$ oder CO,

T O oder S,

$R^6$ H, (1-4)Alkyl, (1-4)Alkoxy, (1-4)Alkylthio, [(1-4)Alkyl]-carbonyl oder [(1-4)Alkoxy]-carbonyl, wobei jeder der letztgenannten 5 Reste unsubstituiert oder im Alkylteil durch ein oder mehrere Halogenatome substituiert ist, oder Halogen, $NO_2$, CN oder Mono- oder Di-[(1-4)alkyl]-amino,

$R^7$ H oder (1-4)Alkyl, vorzugsweise H oder $CH_3$,

$R^8$ (1-4)Alkyl oder (1-4)Alkoxy, (3-6)Cycloalkyl, (3-6)Cycloalkenyl,

$R^9$ H oder (1-4)Alkyl,

$R^{10}$ (1-4)Alkyl, (3-4)Alkenyl oder (3-4)Alkinyl, wobei jeder der drei letztgenannten Reste unsubstituiert oder durch einen oder mehrere Reste aus der Gruppe Halogen, (1-4)Alkoxy, (1-4)Alkylthio, [(1-4) Alkyl]-carbonyl und [(1-4)Alkoxy]-carbonyl substituiert ist,

$R^{11}$, $R^{12}$ unabhängig voneinander H, (1-4)Alkyl, (3-4)Alkenyl oder (3-4)Alkinyl, wobei jeder der drei letztgenannten Reste unsubstituiert oder durch einen oder mehrere Reste aus der Gruppe Halogen, (1-4) Alkoxy, (1-4)Alkylthio, [(1-4)Alkyl]-carbonyl und [(1-4)Alkoxy]-carbonyl substituiert ist,

die Reste $R^{13}$ gemeinsam mit dem N-Atom einen heterocyclischen Ring mit 5 oder 6 Ringgliedern, der weitere Heteroatome aus der Gruppe N, O und S in den möglichen Oxidationsstufen enthalten kann und unsubstituiert oder durch (1-4)Alkyl oder die Oxogruppe substituiert ist oder benzokondensiert ist, bedeuten.

Weiterhin von Interesse sind erfindungsgemäße Verbindungen der Formel (I) und deren Salze, worin

$R^1$ $NR^8R^9$, (1-4)Alkyl, (3-6)Cycloalkyl oder Phenyl, wobei jeder der letztgenannten 3 Reste unsubstituiert oder durch einen oder mehrere Reste aus der Gruppe Halogen, CN, (1-4)Alkoxy, (1-4)Haloalkoxy, (1-4)Alkylthio, (1-4)-Alkylsulfonyl, Formyl, [(1-4) Alkoxy]carbonyl, [(1-4)Alkyl]-carbonyloxy und im Falle cyclischer Reste auch (1-4) Alkyl, (1-4)Haloalkyl und (1-4)Alkoxy(1-4)alkyl substituiert ist, oder (2-4)Alkenyl oder (2-4)Alkinyl,

n 0, 1 oder 2, ausgenommen n = 0 oder 1, wenn $R^1 = NR^8R^9$,

$R^2$, $R^3$ unabhängig voneinander H oder (1-4)Alkyl,

$R^4$ H, OH, Formyl, (1-4)Alkyl, (2-4)Alkenyl, (2-4)Alkinyl, (1-4)Alkoxy, [(1-4)Alkyl]-carbonyl, (1-4)Alkyl-sulfonyl, wobei jeder der 6 letztgenannten Reste unsubstituiert oder durch einen oder mehrere Reste aus der Gruppe Halogen und (1-4)Alkoxy substituiert ist, oder Phenylcarbonyl oder Phenylsulfonyl, wobei in den beiden letztgenannten Resten der Phenylring unsubstituiert oder durch einen oder mehrere Reste aus der Gruppe Halogen, (1-4)Alkyl, (1-4)Haloalkyl, (1-4)Alkoxy und (1-4)Haloalkoxy substituiert ist, und

$R^5$ CHO, [(1-4)Alkyl]-carbonyl, [(2-4)Alkenyl]-carbonyl, [(2-4)Alkinyl]-carbonyl, (1-4)Alkylsulfonyl, (2-4)Alkenylsulfonyl, (2-4)Alkinylsulfonyl, [(3-6)Cycloalkyl]-carbonyl, wobei jeder der 7 letztgenannten Reste unsubstituiert oder durch einen oder mehrere Reste aus der Gruppe Halogen, (1-4)Alkoxy, (1-4)Alkylthio, [(1-4)Alkoxy]-carbonyl, [(1-4)Alkyl]-carbonyloxy und CN und im Falle cyclischer Reste auch [(1-4)Alkyl] und (1-4)Haloalkyl substituiert ist, oder Phenylcarbonyl oder Phenylsulfonyl, wobei jeder der beiden letztgenannten Reste im Phenylring unsubstituiert oder durch einen oder mehrere Reste aus der Gruppe Halogen, CN, $NO_2$, (1-4)Alkyl, (1-4)Haloalkyl, (1-4) Alkoxy und (1-4)Haloalkoxy substituiert ist, oder Mono- oder Di-[(1-4)alkyl]aminosulfonyl, das im Alkylteil unsubstituiert oder durch einen oder mehrere Halogenatome substituiert ist, oder eine Gruppe der Formel COCOR', worin R' = H, OH, (1-4)Alkoxy oder (1-4)Alkyl ist, oder eine Gruppe der Formel

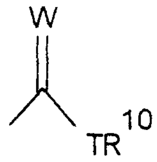 , 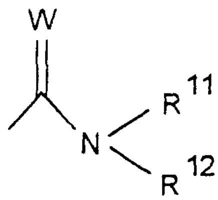 oder 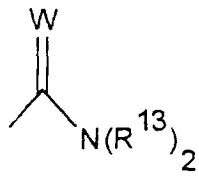 ,

oder

| | |
|---|---|
| $R^4$ und $R^5$ | x gemeinsam eine Kette der Formel $(-CH_2)_{m1}$ $B^1$- oder $B^1$-$(CH_2)_{m2}B^2$, wobei die Kette unsubstituiert oder durch einen oder mehrere (1-3)Alkylreste oder Halogen substituiert ist und m 1 = 3, 4 oder 5 bzw. m2 = 2, 3 oder 4 sind, sowie |
| W | O oder S, |
| $B^1$, $B^2$ | unabhängig voneinander $SO_2$ oder CO, |
| T | O oder S, |
| $R_6$ | H, (1-4)Alkyl, (1-4)Alkoxy oder Halogen, |
| $R^7$ | H oder $CH_3$, |
| $R^8$ | (1-4)Alkyl, |
| $R^9$ | H oder (1-4)Alkyl, |
| $R^{10}$ | (1-4)Alkyl, (1-4)Haloalkyl, (3-4)Alkenyl oder (3-4)Alkinyl, |
| $R^{11}$, $R^{12}$ | unabhängig voneinander H oder (1-4)Alkyl, eine Alkylenkette mit 4 oder 5 C-Atomen, |
| die Reste $R^{17}$ | gemeinsam eine Alkenylenkette mit 4 oder 5 C-Atomen, |
| A | einen Rest der Formel |

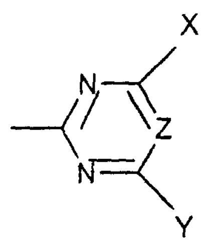

| | |
|---|---|
| einer der Reste X und Y | Halogen, (1-3)Alkyl, Halo(1-3)alkyl, (1-3)Alkoxy oder Halo(1-3)alkoxy und |
| der andere der Reste X und Y | (1-3)Alkyl, Halo(1-3)alkyl, (1-3)Alkoxy, Halo(1-3)alkoxy, Halogen, Mono- oder Di-[(1-3)Alkyl]-amino und |
| Z | CH oder N bedeuten. |

Bevorzugt sind erfindungsgemäße Verbindungen der Formel (I) und deren Salze, worin

| | |
|---|---|
| $R^1$ | Mono- oder Di-[(1-4)alkyl]-amino oder (1-4)Alkyl, vorzugsweise $CH_3$, $C_2H_5$, n-$C_3H_7$, i-$C_3H_7$ oder $N(CH_3)_2$, |
| n | die Zahl 2, |
| $R^2$, $R^3$ | jeweils Wasserstoff, |
| $R^4$ | H oder (1-4)Alkyl, |
| $R^5$ | CHO, [(1-4)Alkyl]-carbonyl, [(1-4)Haloalkyl]-carbonyl, (1-4)Alkylsulfonyl, (1-4)Haloalkylsulfonyl, [(1-4)Alkoxy]-carbonyl, Mono- oder Di-[(1-4)alkyl]-aminocarbonyl, Mono- oder Di-[(1-4)alkyl]-aminosulfonyl oder |
| $R^4$, $R^5$ | gemeinsam eine Kette der Formel $(-CH_2)_{m1}B^1$- oder -$B^1$-$(CH_2)_{m2}B^2$worin $B^1$ und $B^2$ unabhängig voneinander $SO_2$ oder CO bedeuten, |
| W, T | jeweils O, |
| $R^6$ | H und |
| $R^7$ | H oder $CH_3$ bedeuten. |

Gegenstand der vorliegenden Erfindung sind auch Verfahren zur Herstellung der Verbindungen der allgemeinen Formel (I) oder deren Salze, dadurch gekennzeichnet, daß man

a) eine Verbindung der Formel (II)

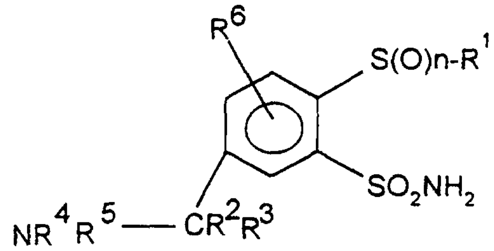

(II)

mit einem heterocyclischen Carbamat der Formel (III),

$$R^* - O - CO - NR^7 - A \qquad (III)$$

worin R* gegebenenfalls substituiertes Phenyl oder (1-4)Alkyl bedeutet, umsetzt oder

b) ein Arylsulfonylcarbamat der Formel (IV)

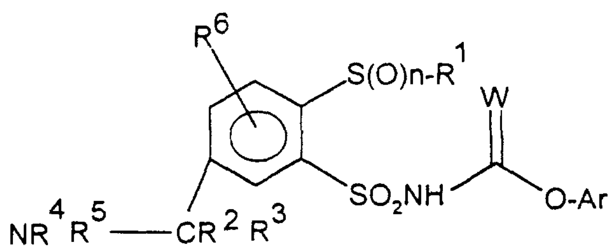

(IV) ,

worin Ar einen Arylrest, vorzugsweise gegebenenfalls substituiertes Phenyl, bedeutet mit einem Aminoheterocydus der Formel (V)

$$H - NR^7 - A \qquad (V)$$

umsetzt oder

c) ein Sulfonylisocyanat der Formel (VI)

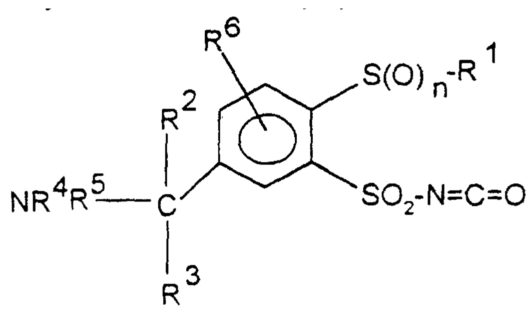

(VI)

mit einem Aminoheterocyclus der Formel $H\text{-}NR^7\text{-}A$ (V) umsetzt oder

d) in einer Eintopfreaktion zunächst einen Aminoheterocyclus der Formel $H\text{-}NR^7\text{-}A$ (V) in Gegenwart einer Base

mit Phosgen umsetzt und das gebildete Intermediat mit einem Phenylsulfonamid der Formel (II) umsetzt oder

e) ein Sulfonylchlorid der Formel (VII)

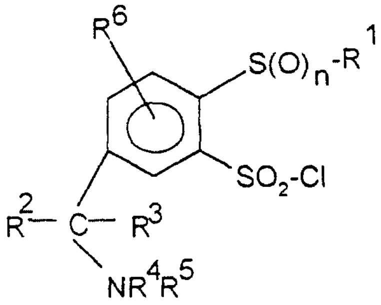

(VII)

mit einem Cyanat M-OCN, worin M = ein Kation,z.B. $NH_4$, Na oder K bedeutet, und mit einem Aminoheterocyclus der Formel H-$NR^7$-A (V) in Gegenwart einer Base umsetzt, oder

f) ein Sulfonamid der genannten Formel (II) mit einem (Thio-)Isocyanat der Formel (V')

$$W = C = N - A \qquad (V')$$

in Gegenwart einer Base umsetzt,

wobei in den Formeln (II)-(VII) und (V') die Reste bzw. Symbole $R^1$-$R^7$, A, W und n wie in Formel (I) definiert sind und wobei in den Varianten a) und c)-e) zunächst Verbindungen der Formel (I) mit W = O erhalten werden.

Die Umsetzung der Verbindungen der Formeln (II) und (III) erfolgt vorzugsweise basenkatalysiert in einem inerten organischen Lösungsmitteln, wie z.B. Dichlormethan, Acetonitril, Dioxan oder THF bei Temperaturen zwischen 0°C und dem Siedepunkt des Lösungsmittels. Als Base werden dabei beispielsweise organische Aminbasen, wie 1,8-Diazabicyclo[5.4.0]undec-7-3n (DBU), insbesondere bei R* = (subst.) Phenyl (vgl. EP-A-44807), oder Trimethylaluminium oder Triethylaluminium, letztere insbesondere bei R* = Alkyl (vgl. EP-A-166516) verwendet.

Die Sulfonamide (II) sind neue Verbindungen. Sie und ihre Herstellung sind ebenfalls Gegenstand dieser Erfindung.

Die Herstellung der Sulfonamide (II) kann in der Regel auf folgende Weise erfolgen: Ausgehend von gegebenenfalls substituierten 4-Chlor-3-nitrotoluolen (VIII) wird in den Fällen, wo $R^1$ ungleich $NR^8R^9$ ist, zunächst das Chlor durch Reaktion mit einem Mercaptan der Formel $R^1$-SH substitutiert. Reduktion der Nitrogruppe in (IX) zum Anilinderivat (X) und anschließende Diazotierung und Kupplung mit $SO_2$/CuCl (s. H. Meerwein et al., Chem. Ber. 90, 841-1178 (1957)) und Aminolyse mit tert-Butylamin liefert Sulfonamid (XI) (siehe Schema I).

Schema 1:

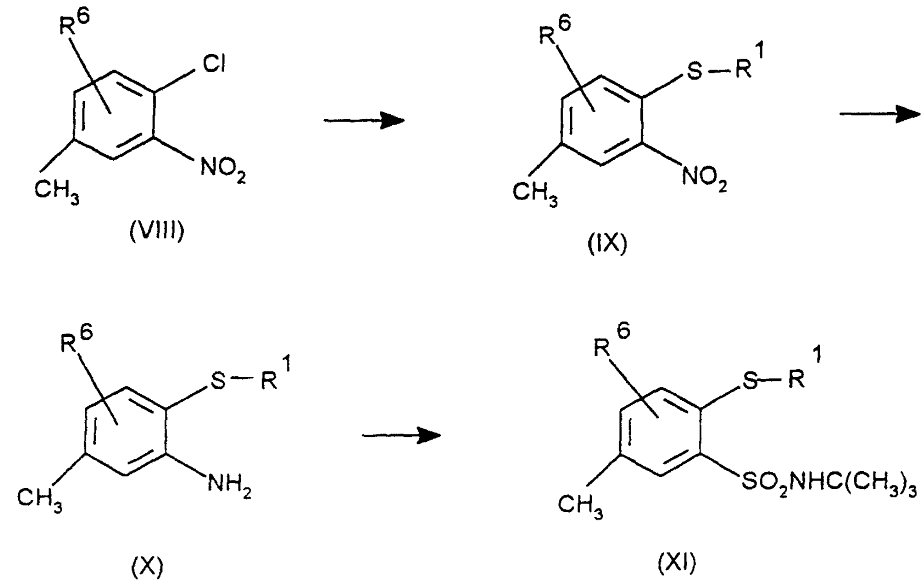

(VIII) (IX) (X) (XI)

Zur Herstellung der Sulfonamide (II) werden dann Verbindungen der Formel (XI) nach eventueller vorangehender Oxidation zu (XII) via Seitenkettenhalogenierung zu (XIII), anschließender Substitution des Halogenatoms in (XIII) mit Aminen oder gegen Azid mit nachfolgender Reduktion zu Benzylaminen (XIV), und weiterer Funktionalisierung der Aminogruppe sowie Abspaltung der tert.-Butylschutzgruppe analog bekannter Verfahrensweise (z.B. mit $CF_3COOH$) zu den Sulfonamiden (II) umgesetzt (siehe Schema 2).

Schema 2:

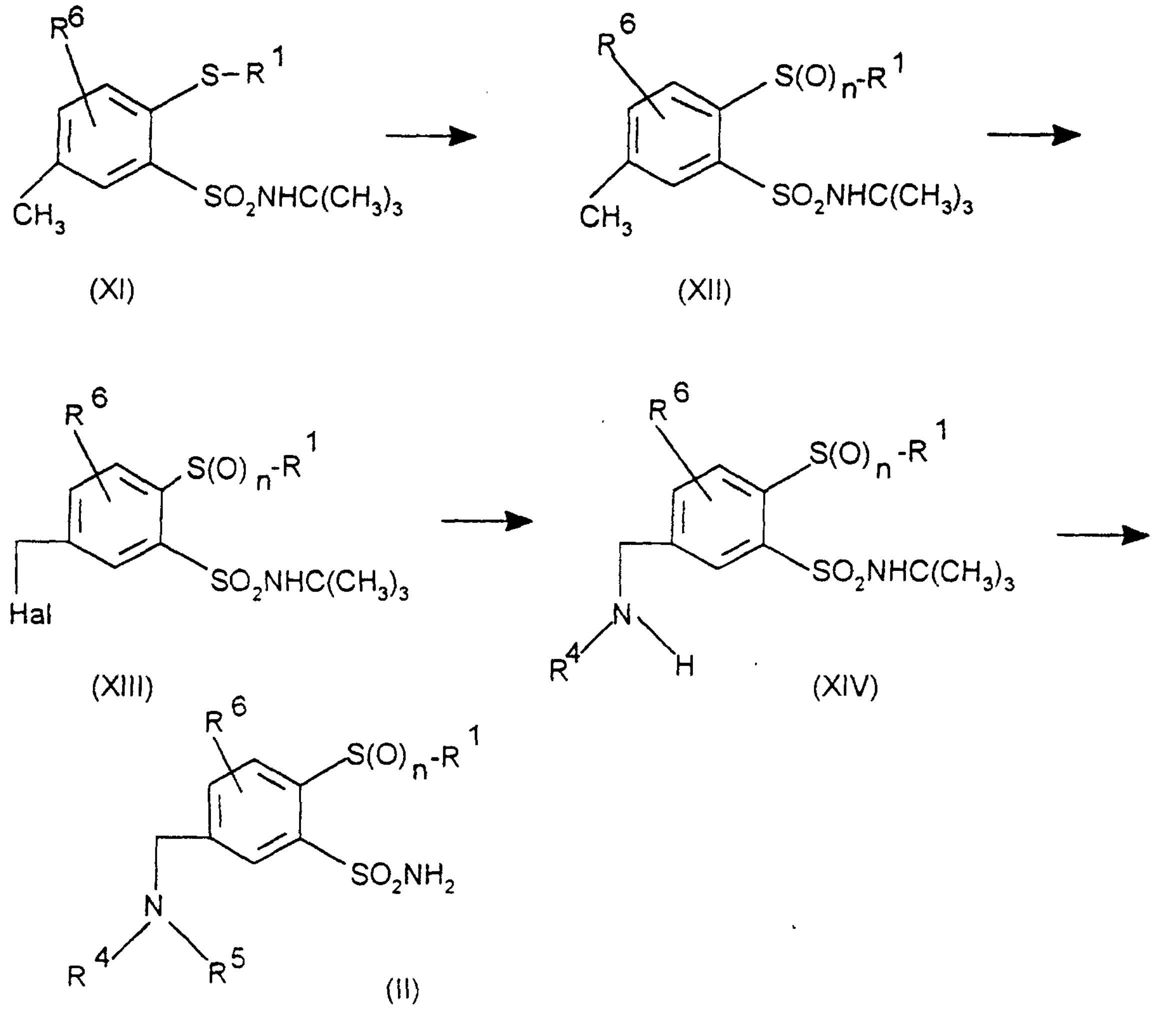

(XI)

(XII)

(XIII)

(XIV)

(II)

Für Verbindungen der Formel (II) mit $R^1 = NR^8R^9$ (siehe Schema 3) wird z.B. zunächst in gegebenenfalls substituierten 4-Chlor-3-nitrotoluolen (VIII) das Chlor mit Alkylmercaptanen bzw. bevorzugt mit Benzylmercaptanen zu (IX') ausgetauscht. Nach Oxidation und Aminolyse der intermediär erhaltenen Sulfochloride mit $HNR^8R^9$ erhält man Sulfonamide (XV), welche nach Reduktion zu (XVI) wie in Schema 1 und 2 für (X) beschrieben zu analogen Sulfonamiden (II) mit $R^1 = NR^8R^9$ umgesetzt werden.

Schema 3

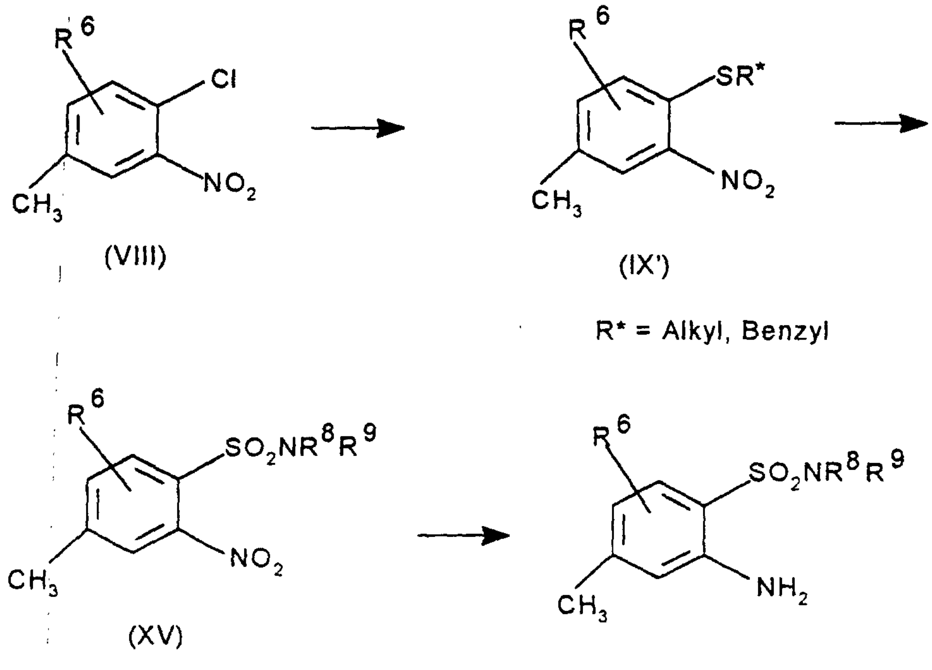

(VIII)

(IX')

R* = Alkyl, Benzyl

(XV)

(XVI)

Die Carbamate der Formel (III) können nach Methoden hergestellt werden, die in den südafrikanischen Patentanmeldungen 82/5671 und 82/5045 bzw. EP-A-70804 (US-A-4 480 101) oder RD 275056 beschrieben sind.

Die Umsetzung der Verbindungen (IV) mit den Aminoheterocyden (V) führt man vorzugsweise in inerten, aprotischen Lösungsmitteln wie z.B. Dioxan, Acetonitril oder Tetrahydrofuran bei Temperaturen zwischen 0°C und der Siedetemperatur des Lösungsmittels durch. Die benötigten Ausgangsmaterialien (V) sind literaturbekannt oder können nach literaturbekannten Verfahren hergestellt werden. Die Arylsulfonylcarbamate der Formel (IV) erhält man analog US-PS 4 684 393 oder US-PS-4 743 290.

Die Phenylsulfonylisocyanate der Formel (VI) lassen sich analog US-PS-4 481 029 herstellen und mit den Aminoheterocyclen (V) umsetzen.

Die Phosgenierung von Verbindungen der Formel (V) nach Variante d) kann vorzugsweise in Gegenwart von Basen, wie sterisch gehinderten organischen Aminbasen, beispielsweise Triethylamin, erfolgen. Die anschließende Umsetzung mit Verbindungen der Formel (II) nach Variante d) kann analog bekannten Verfahren durchgeführt werden (vgl. EP-A-232 067).

Die Sulfochloride (VII) können aus entsprechenden Sulfonsäuren erhalten werden, z.B. nach Standardmethoden wie der Umsetzung des Kaliumsalzes mit Phoshoroxychlorid oder Thionylchlorid in inerten Solventien wie Acetonitril und/oder Sulfolan oder in Substanz durch Erwärmen zum Rückfluß (vgl. Houben-Weyl-Klamann, "Methoden der organischen Chemie", 4. Aufl. Bd. 3 XI/2, S. 1067-1073, Thieme Verlag Stuttgart, 1985).

Die entsprechenden Sulfonsäuren sind aus entsprechenden Nitroverbindungen analog der Umsetzung von Verbindungen (IX) erhältlich.

Alternativ sind in Einzelfällen Sulfochloride (VII) durch Sulfonierung (+Chlorierung) oder Sulfochlorierung geeigneter substituierter Benzoesäureester herstellbar; Sulfochlorierung analog Houben-Weyl-Klamann, "Methoden der organischen Chemie", 4. Aufl. Bd. E XI/2, S. 1067 ff., Thieme Verlag Stuttgart, 1985; Houben-Weyl-Müller, "Methoden der organischen chemie", 4. Aufl. Bd. IX, s. 563ff., Thieme Verlag Stuttgart, 1955; Sulfonierung analog Houben-Weyl-Klamann, "Methoden der organischen Chemie, 4. Aufl. Bd. E XI/2, S.1055ff., Thieme Verlag Stuttgart, 19085; Houben-Weyl-Müller, "Methoden der organischen Chemie", 4. Aufl. Bd. IX, S. 435 ff., Thieme Verlag Stuttgart, 1955.

Die (Thio)-Isocyanate der Formel (V') sind nach literaturbekannten Verfahren erhältlich (EP-A-232067, EP-A-166516). Die Umsetzung der (Thio)-Isocyanate (V') mit Verbindungen (II) erfolgt bei -10°C bis 100°C, vorzugsweise 20 bis 100°C, in einem inerten aprotischen Lösungsmittel, wie z.B. Aceton oder Acetonitril, in Gegenwart einer geeigneten Base, z.B. $N(C_2H_5)_3$ oder $K_2CO_3$.

Die Salze der Verbindungen der Formel (I) werden vorzugsweise in inerten polaren Lösungsmitteln wie z.B. Wasser, Methanol oder Aceton bei Temperaturen von 0-100°C hergestellt. Geeignete Basen zur Herstellung der erfindungsgemäßen Salze sind beispielsweise Alkalicarbonate, wie Kaliumcarbonat, Alkali- und Erdafalkalihydroxide, z. B. NaOH oder KOH, oder Ammoniak oder Ethanolamin.

Mit den in den vorstehenden Verfahrensvarianten bezeichneten "inerten Lösungsmitteln" sind jeweils Lösungsmittel gemeint, die unter den jeweiligen Reaktionsbedingungen inert sind, jedoch nicht unter beliebigen Reaktionsbedingungen inert sein müssen.

Die erfindungsgemäßen Verbindungen der Formel (I) weisen eine ausgezeichnete herbizide Wirksamkeit gegen ein breites Spektrum wirtschaftlich wichtiger mono- und dikotyler Schadpflanzen auf. Auch schwer bekämpfbare perennierende Unkräuter, die aus Rhizomen, Wurzelstöcken oder anderen Dauerorganen austreiben, werden durch die Wirkstoffe gut erfaßt. Dabei ist es gleichgültig, ob die Substanzen im Vorsaat-, Vorauflauf- oder Nachauflaufverfahren ausgebracht werden.

Im einzelnen seien beispielhaft einige Vertreter der mono- und dikotylen Unkrautflora genannt, die durch die erfindungsgemäßen Verbindungen kontrolliert werden können, ohne daß durch die Nennung eine Beschränkung auf bestimmte Arten erfolgen soll.
Auf der Seite der monokotylen Unkrautarten werden z.B. Avena, Lolium, Alopecurus, Phalaris, Echinochloa, Digitaria, Setaria sowie Cyperusarten aus der annuellen Gruppe und auf seiten der perennierenden Spezies Agropyron, Cynodon, Imperata sowie Sorghum und auch ausdauernde Cyperusarten gut erfaßt.
Bei dikotylen Unkrautarten erstreckt sich das Wirkungsspektrum auf Arten wie z.B. Galium, Viola, Veronica, Lamium, Steliaria, Amaranthus, Sinapis, Ipomoea, Matricaria, Abutilon und Sida auf der annuellen Seite sowie Convolvulus, Cirsium, Rumex und Artemisia bei den perennierenden Unkräutern.

Unter den spezifischen Kulturbedingungen im Reis vorkommende Unkräuter wie z.B. Sagittaria, Alisma, Eleocharis, Scirpus und Cyperus werden von den erfindungsgemäßen Wirkstoffen ebenfalls hervorragend bekämpft.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt ebenfalls sehr rasch nach der Behandlung ein drastischer Wachstumsstop ein und die Unkrautpflanzen bleiben in dem zum Applikationszeitpunkt vorhandenen Wachstumsstadium stehen oder sterben nach einer gewissen Zeit ganz ab, so daß auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen wie z.B. Weizen, Gerste, Roggen, Reis, Mais, Zuckerrübe, Baumwolle und Soja nur unwesentlich oder gar nicht geschädigt. Die vorliegenden Verbindungen eignen sich aus diesen Gründen sehr gut zur selektiven Bekämpfung von unerwünschtem Pflanzenwuchs in landwirtschaftlichen Nutzpflanzungen.

Darüberhinaus weisen die erfindungsgemäßen Substanzen hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, die Verbindungen der Formel (I) enthalten.

Die Verbindungen der Formel (I) können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasserin-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streuund Bodenapplikation, Granulate (GR) in Form von Mikro-, Sprüh-, Aufzugs- und Adsorptionsgranufaten, wasserdispergierbare Granulate (WG), wasserlösliche Granulate (SG), ULV-Formulierungen, Mikrokapseln und Wachse.

Diese einzelnen Formulierungstypen sind im Prinzip bekannt und werden beispielsweise beschrieben in: Winnacker-Küchler, "Chemische Technologie", Band 7, C. Hauser Verlag München, 4. Aufl. 1986, Wade van Valkenburg, "Pesticide Formulations", Marcel Dekker, N.Y., 1973; K. Martens, "Spray Drying" Handbook, 3rd Ed. 1979, G. Goodwin

Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hauser Verlag München, 4. Aufl. 1986.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt. Zur Herstellung von Teller-, Fließbett-, Extruder- und Sprühgranulate siehe z.B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0,1 bis 99 Gew.-%, insbesondere 0,1 bis 95 Gew.-%, Wirkstoff der Formel (I).

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0,05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Als Kombinationspartner für die erfindungsgemäßen Wirkstoffe in Mischungsformulierungen oder im Tank-Mix sind beispielsweise bekannte Wirkstoffe einsetzbar, wie sie in z.B. aus Weed Research 26, 441-445 (1986), oder "The Pesticide Manual", 10th edition, The British Crop Protection Council and the Royal Soc. of Chemistry, 1994 und dort zitierter Literatur beschrieben sind. Als literaturbekannte Herbizide, die mit den Verbindungen der Formel (I) kombiniert werden können, sind z.B. folgende Wirkstoffe zu nennen (Anmerkung: Die Verbindungen sind entweder mit dem "common name" nach der International Organization for Standardization (ISO) oder mit dem chemischen Namen, ggf. zusammen mit einer üblichen Codenummer bezeichnet):
acetochlor; acifluorfen; aclonifen; AKH 7088, d.h. [[[1-[5-[2-Chloro-4-(trifluoromethyl)-phenoxy]-2-nitrophenyl]-2-methoxyethylidene]-amino]-oxy]-essigsäure und -essigsäuremethylester; alachlor; alloxydim; ametryn; amidosulfuron; amitrol; AMS, d.h. Ammoniumsulfamat; anilofos; asulam; atrazin; azimsulfurone (DPX-A8947); aziprotryn; barban; BAS 516 H, d.h. 5-Fluor-2-phenyl-4H-3,1-benzoxazin-4-on; benazolin; benfluralin; benfuresate; bensulfuron-methyl; bensulide; bentazone; benzofenap; benzofluor; benzoyiprop-ethyl; benzthiazuron; bialaphos; bifenox; bromacil; bromobutide; bromofenoxim; bromoxynil; bromuron; buminafos; busoxinone; butachlor; butamifos; butenachlor; buthidazole; butralin; butylate; cafenstrole (CH-900); carbetamide; cafentrazone (ICI-A0051); CDAA, d.h. 2-Chlor-N,N-di-2-propenylacetamid; CDEC, d.h. Diethyldithiocarbaminsäure-2-chlorallylester; chlomethoxyfen; chloramben; chlorazifop-butyl, chlormesulon (ICI-A0051); chlorbromuron; chlorbufam; chlorfenac; chlorflurecol-methyl; chloridazon; chlorimuron ethyl; chlornitrofen; chlorotoluron; chloroxuron; chlorpropham; chlorsulfuron; chlorthal-dimethyl; chlorthiamid; cinmethylin; cinosulfuron; clethodim; clodinafop und dessen Esterderivate (z.B. clodinafop-propargyl); clomazone; clomeprop; cloproxydim; clopyralid; cumyluron (JC 940); cyanazine; cycloate; cyclosulfamuron (AC 104); cycloxydim; cycluron; cyhalofop und dessen Esterderivate (z.B. Butylester, DEH-112); cyperquat; cyprazine; cyprazole; daimuron; 2,4-DB; dalapon; desmedipham; desmetryn; di-allate; dicamba; dichlobenil; dichlorprop; diclofop und dessen Ester wie didofop-methyl; diethatyl; difenoxuron; difenzoquat; diflufenican; dimefuron; dimethachlor; dimethametryn; dimethenamid (SAN-582H); dimethazone, clomazon; dimethipin; dimetrasulfuron, dinitramine; dinoseb; dinoterb; diphenamid; dipropetryn; diquat; dithiopyr; diuron; DNOC; eglinazine-ethyl; EL 77, d.h. 5-Cyano-1-(1,1-dimethylethyl)-N-methyl-1Hpyrazole-4-carboxamid; endothal; EPTC; esprocarb; ethalfluralin; ethametsulfuron-methyl; ethidimuron; ethiozin; ethofumesate; F5231, d.h. N-[2-Chlor-4-fluor-5-[4-(3-fluorpropyl)-4,5-dihydro-5-oxo-1 H-tetrazol-1-yl]-phenyl]-ethansulfonamid; ethoxyfen und dessen Ester (z.B. Ethylester, HN-252); etobenzanid (HW 52); fenoprop; fenoxan, fenoxaprop und fenoxaprop-P sowie deren Ester, z.B. fenoxaprop-P-ethyl und fenoxaprop-ethyl; fenoxydim; fenuron; flamprop-methyl; fiazasulfuron; fluazifop und fluazifop-P und deren Ester, z.B. fluazifop-butyl und fluazifop-P-butyl; fluchioralin; flumetsulam; flumeturon; flumiclorac und dessen Ester (z.B. Pentylester, S-23031); flumioxazin (S-482); flumipropyn; flupoxam (KNW-739); fluorodifen; fluoroglycofen-ethyl; flupropacil (UBIC-4243); fluridone; flurochloridone; fluroxypyr; flurtamone; fomesafen; fosamine; furyloxyfen; glufosinate; glyphosate; halosafen; halosulfuron und dessen Ester (z.B. Methylester, NC-319); haloxyfop und dessen Ester; haloxyfop-P (= R-haloxyfop) und dessen Ester; hexazinone; imazamethabenz-methyl; imazapyr; imazaquin und Salze wie das Ammoniumsalz; imazethamethapyr; imazethapyr; imazosulfuron; ioxynil; isocarbamid; isopropalin; isoproturon; isouron; isoxaben; isoxapyrifop; karbutilate; lactofen; lenacil; linuron; MCPA; MCPB; mecoprop; mefenacet; mefluidid; metamitron; metazachlor; methabenzthiazuron; metham; methazole; methoxyphenone; methyldymron; metabenzuron, methobenzuron; metobromuron; metolachlor; metosulam (XRD 511); metoxuron; metribuzin; metsulfuron-methyl; MH; molinate; monalide; monocarbamide dihydrogensulfate; monölinuron; monuron; MT 128, d.h. 6-Chlor-N-(3-chlor-2-propenyl)-5-methyl-N-phenyl-3-pyridazinamin; MT 5950, d.h. N-[3-Chlor-4-(1-methylethyl)-phenyl]-2-methylpentanamid; naproanilide; napropamide; naptalam; NC 310, d.h. 4-(2,4-dichlorbenzoyl)-1-methyl-5-benzyloxypyrazol; neburon; nicosulfuron; nipyraclophen; nitralin; nitrofen; nitrofluorfen; norflurazon; orbencarb; oryzalin; oxadiargyl (RP-020630); oxadiazon; oxyfluorfen; paraquat; pebulate; pendimethalin; perfluidone; phenisopham; phenmedipham; pidoram; piperophos; piributicarb; pirifenop-butyl; pretilachlor; primisulfuron-methyl; procyazine; prodiamine; profluralin; proglinazine-ethyl; prometon; prometryn; propachlor; propanil; propaquizafop und dessen Ester; propazine; propham; propisochlor; propyzamide; prosulfalin; prosulfocarb; prosulfuron (CGA-152005); prynachlor; pyrazolinate; pyrazon; pyrazosulfuron-ethyl; pyrazoxyfen; pyridate; pyrithiobac (KIH-2031); pyroxofop und dessen Ester (z.B. Propargylester); quinclorac; quinmerac; quinofop und dessen Esterderivate, quizalofop und quizalofop-P und deren Esterderivate z.B. quizalofop-ethyl; quizalofop-P-tefuryl und -ethyl; renriduron; rimsulfuron (DPX-E 9636); S 275, d.h. 2-[4-Chlor-2-fluor-5-(2-propynyloxy)-phenyl]-4,5,6,7-tetrahydro-2H-indazol; secbumeton; sethoxydim; siduron; simazine; simetryn; SN 106279, d.h. 2-[[7-[2-Chlor-4-(trifluormethyl)-phenoxy]-2-naphthalenyl]-oxy]-propansäure und methylester; sulfentrazon (FMC-97285, F-6285); sulfazuron; sulfometuron-methyl; sulfosate (ICI-A0224); TCA; tebutam (GCP-5544); tebuthiuron; terbacil; terbucarb; terbuchlor; terbumeton; terbuthylazine; terbutryn; TFH 450, d.h. N,N-Diethyl-3-[(2-ethyl-6-methylphenyl)-sulfonyl]-1 H-1,2,4-triazol-1-carboxamid; thenylchlor (NSK-850); thiazafluron; thiazopyr (Mon-13200); thidiazimin (SN-24085); thifensulfuron-methyl; thiobencarb; tiocarbazil; tralkoxydim; tri-allate; triasulfuron; triazofenamide; tribenuron-methyl; triclopyr; tridiphane; trietazine; trifluralin; triflusulfuron und Ester (z.B. Methylester, DPX-66037); trimeturon; tsitodef; vernolate; WL 110547, d.h. 5-Phenoxy-1-[3-(trifluormethyl)-phenyl]-1H-tetrazol; UBH-509; D-489; LS 82-556; KPP-300; NC-324; NC-330; KH-218; DPX-N8189; SC-0774; DOWCO-535; DK-8910; V-53482; PP-600; MBH-001; KIH-9201; ET-751; KIH-6127 und KIH-2023.

Zur Anwendung werden die in handelsüblicher Form vorliegenden Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken, z. B. zwischen 0,001 und 10,0 kg/ha oder mehr Aktivsubstanz, vorzugsweise liegt sie jedoch zwischen 0,005 und 5 kg/ha.

A. Chemische Beispiele

Abkürzungen: Die % Angaben und Mengenverhältnisse beziehen sich auf das Gewicht, wenn nicht näher spezifiziert.
i. Vak. = unter reduziertem Druck
h = Stunde(n)
DMF = Dimethylformamid
EtOH = $C_2H_5OH$

Beispiel A1

4-Methylthio-3-nitrotoluol

Zu einer Lösung von 171,6 g (1 mol) 4-Chlor-3-nitrotoluol in 500 ml DMF gibt man 138 g (1 mol) $K_2CO_3$, gast 1 Moläquivalent Methylmercaptan ein und rührt 3 h lang bei 50°C nach. Das Reaktionsgemisch gießt man auf reichlich Eiswasser, saugt vom gebildeten Feststoff ab, wäscht mit Wasser und trocknet; man erhält 146,3 g (80 %) 4-Methylthio-3-nitrotoluol vom Schmp. 71-73°C.

Beispiel A2

3-Amino-4-methylthio-toluol

Eine Lösung von 146 g (0,8 mol) 4-Methylthio-3-nitrotoluol in einem Gemisch von 1000 ml EtOH und 700 ml Eisessig wird auf 65-80°C erhitzt und im Verlauf von ca. 8 h portionsweise mit insgesamt 122,9 g (2,2 mol) Eisenpulver versetzt. Nach vollständigem Umsatz wird über $Na_2SO_4$ abgesaugt, gut mit EtOH nachgewaschen und i.Vak. eingeengt Man erhält 122 g (99 %) 3-Amino-4-methylthio-toluol als dunkelbraunes Öl.
$^1$H-NMR (300 MHz, $CDCl_3$):

$\delta$ = 2,24 (s, 3H, $SCH_3$)
2,30 (s, 3H, Ar-$CH_3$)
4,25 (bs, 2H, $NH_2$)
6,52 (m, 2H, Ar-H)
7,25 (d, 1H, Ar-H)

Beispiel A3

N-tert.-Butyl-2-methylthio-5-methylbenzolsulfonamid

In einer Suspension von 49 g (0,32 mol) 3-Amino-4-methylthiotoluol in 180 ml konz. HCl tropft man bei 0-10°C 24,3 g (0,352 mol) $NaNO_2$ gelöst in 50 ml $H_2O$ und rührt eine'halbe Stunde nach. Die erhaltene Diazoniumsalzsuspension wird portionsweise zu einer auf 30°C erwärmten Mischung von $SO_2$-gesättigtem Eisessig (450 ml), 9,5 g (0,096 mol) CuCl und $CH_2Cl_2$ (450 ml) gegeben. Nach vollst. Zugabe läßt man nach 1-2 h bei 30°C nachrühren, gießt den Ansatz auf Eiswasser, trennt die Phasen und extrahiert die Wasserphase noch zweimal mit $CH_2Cl_2$. Die vereinigten

org. Extrakte werden gewaschen, über $Na_2SO_4$ getrocknet und i.Vak. vollständig eingeengt.

Das erhaltene Rohprodukt (66,5 g, 0,28 mol) wird in 650 ml $CH_2Cl_2$ gelöst und bei 0°C mit 73 ml (0,7 mol, 2.5 equiv.) tert.-Butylamin versetzt.

Zur Aufarbeitung wird der Ansatz nach ca. 1h auf Eiswasser gegossen, die Phasen getrennt und die wäßrige Phase zweimal mit $CH_2Cl_2$ nachextrahiert. Die vereinigten org. Extrakte werden gewaschen, über $Na_2SO_4$ getrocknet und i.Vak. eingeengt. Der nach Digerieren des Rückstandes mit einem Gemisch von Diispropylether/Essigester erhaltene Feststoff wird abfiltriert und getrocknet. Man erhält 41,5 g (47,4 %) N-tert.-Butyl-2-methylthio-5-methylbenzolsulfonamid vom Schmp. 119-121°C.

Beispiel A4

N-tert-Butyl-2-methansulfonyl-5-methylbenzolsulfonamid

Eine Lösung von 41,5 g (0,152 mol) N-tert.-Butyl-2-methylthio-5-methylbenzolsulfonamid in 470 ml MeOH und 190 ml $H_2O$ wird auf 50°C erhitzt und im Verlauf von 7 h portionsweise mit 140,2 g (0,228 mol) Natriumperoxomonosulfat (®Oxone) versetzt. Nach vollst. Umsatz wird auf reichlich Eiswasser gegossen und vom ausgefallenen Produkt filtiert. Nach Trocknen erhält man 46,0 g (99 %) N-tert.-Butyl-2-methansulfonyl-5-methylbenzolsulfonamid vom Schmp. 129-133°C.

Beispiel A5

N-tert.-Butyl-5-brommethyl-2-methansulfonylbenzolsulfonamid

Eine Lösung von 46 g (0,152 mol) N-tert-Butyl-2-methansulfonyl-5-methylbenzolsulfonamid in 450 ml $CCl_4$ wird nach Zugabe von 27,0 g (0,152 mol) NBS (N-Bromsuccinimid) und 1 g AIBN (Azobisisobutyronitril) mit einer Tageslichtlampe 7h am Rückfluß erhitzt. Das Reaktionsgemisch wird danach filtriert und sukzessive mit $NaHSO_3$-Lösung, $NaHCO_3$-Lösung und Wasser gewaschen, über $MgSO_4$ getrocknet und i.Vak. vollständig eingeengt. Man erhält 51,2 g eines Rohgemisches, das zu 55 Gew.-% N-tert-Butyl-5-brommethyl-2-methansulfonylbenzolsulfonamid enthält.

$^1$H-NMR (300 MHz, $CDCl_3$):

$\delta$ = 1,25 (s, 9H, tert.Butyl)
3,40 (s, 3H, $SO_2CH_3$)
4,52 (s, 2H, $ArCH_2$)
6,26 (s, 1H, NH)
7,75 (dd, 1H, Ar-H, J = 2 Hz, 8 Hz)
8,25 (d, 1H, Ar-H, J = 8 Hz)
8,31 (d, 1H, Ar-H, J = 2 Hz)

Beispiel A6

N-tert.-Butyl-5-methylaminomethyl-2-methansulfonylbenzolsulfonamid

Zu einer Lösung von 59,5 ml (0,69 mol) einer 40 %igen wäßrigen MethylaminLösung in 150 ml THF gibt man bei 0°C 51 g N-tert.-Butyl-5-brommethyl-2-methansulfonyibenzolsulfonamid (55 Gew.-% ≙28,05 g = 0,669 mol) gelöst in 250 ml THF zu und läßt 2-3 h bei 0-5°C nachrühren. Zur Aufarbeitung wird vollständig eingeengt, der Rückstand in reichlich 2n HCl aufgenommen und mit Diethylether (2x 200 ml) extrahiert. Die saure wäßrige Phase wird mit 4 N NaOH auf pH 9 eingestellt und mit Essigester (3 x 200 ml) extrahiert. Die vereinigten Essigesterextrakte werden mit Wasser gewaschen, über $Na_2SO_4$ getrocknet und i.Vak. eingedampft. Es verbleiben 17 g (73,7 %) N-tert.-Butyl-5-methylaminomethyl-2-methansulfonylbenzolsulfonamid vom Schmp. 76-78°C.

Beispiel A7

N-tert-Butyl-5-(N-methyl-N-methansulfonyl-aminomethyl)-2-methansulfonylbenzolsulfonamid

Zu einer auf 0°C gekühlten Lösung von 1,67 g (5 mmol) N-tert.-Butyl-5-methylaminomethyl-2-methansulfonylbenzolsulfonamid und 0,73 ml (5,3 mmol) Triethylamin in 20 ml Dichlormethan tropft man 0,39 ml (5 mmol) Methansulfonylchlorid gelöst in 5 ml Dichlormethan. Nach 30 min bei Raumtemperatur wird die Reaktionslösung mit Wasser

gewaschen, getrocknet und i.Vak. vollständig eingeengt. Man erhält 1,85 g (89,7 %) N-tert.-Butyl-5-(N-methyl-N-methansulfonyl-aminomethyl)-2-methansulfonylbenzolsulfonamid als zähes Öl.
$^1$H-NMR (300 MHz, $CDCl_3$):

δ = 1,23 (s, 9H, tert-Butyl)
2,82 (s, 3H, $N-CH_3$)
2,94 (s, 3H, $N-SO_2CH_3$)
3,40 (s, 3H, $Ar-SO_2CH_3$)
4,46 (s, 2H, $Ar-CH_2$)
6,30 (s, 1H, NH)
7,83 (dd, 1 H, Ar-H, J = 2 Hz, 8 Hz)
8,20 (d, 1H, Ar-H, J = 2 Hz)
8,30 (d, 1H, Ar-H, J = 8 Hz)

Beispiel A8

5-(N-Methyl-N-methansulfonyl-aminomethyl)-2-methansulfonylbenzolsulfonamid

Eine Lösung von 1,85 g (4,5 mmol) N-tert-Butyl-5-(N-methyl-N-methansulfonylaminomethyl)-2-methansulfonylbenzolsulfonamid in 20 ml Trifluoressigsäure wird 8 h bei Raumtemperatur gerührt. Der Ansatz wird vollständig eingeengt, mit Toluol nachgedampft und der erhaltene Rückstand mit Essigester/Diisopropyl-ether kristallisiert. Man erhält 1,32 g (83 %) 5-(N-Methyl-N-methansulfonyl-amino-methyl)-2-methansulfonylbenzoisulfonamid vom Schmp. 165-169°C.

Beispiel A9

N-(4,6-Dimethoxypyrimidin-2-ylaminocarbonyl)-5-(N-methyl-N-methansulfonylaminomethyl)-2-methansulfonylbenzolsulfonamid

Zu einer Suspension von 1,33 g (3,7 mmol) 5-(N-Methyl-N-methansulfonyl-aminomethyl)-2-methansulfonylbenzolsulfonamid und 1,03 g (3,7 mmol) N-(4,6-Dimethoxypyrimidin-2-yl)carbaminsäurephenylester in 20 ml Acetonitril gibt man tropfenweise 0,56 ml (3,7 mmol) DBU. Nach 2 h wird mit Wasser und Diethylether verdünnt, mit HCl auf pH-1-2 angesäuert und das ausgefallene Produkt abfiltriert, mit Wasser und Diethylether nachgewaschen und getrocknet; Ausbeute: 1,78 g (89,5 %) N-(4,6-Dimethoxypyrinidin-2-ylaminocarbonyl)-5-(N-methyl-N-methansulfonyl-aminomethyl)-2-methan-sulfonylbenzolsulfonamid von Schmp. 205-210°C (unter Zers.).

Die in den nachfolgenden Tabellen aufgeführten erfindungsgemäßen Verbindungen werden gemäß bzw. analog den Beispielen A1 bis A8 oder den am allgemeinen Teil erwähnten Verfahren erhalten.

Abkürzungen zu den Tabellen:

Nr. = Beispielnummer, Beispielverbindung Nr.
Fp = Festpunkt, Schmelzpunkt
n-, i-, s-, t-$C_4H_9$ = n-, iso- sekundär-, tertiär-Butyl
n-$C_3H_7$ = n-Propyl
i-$C_3H_7$ = Isopropyl

Tabelle 1: Verbindung der Formel (Ia)

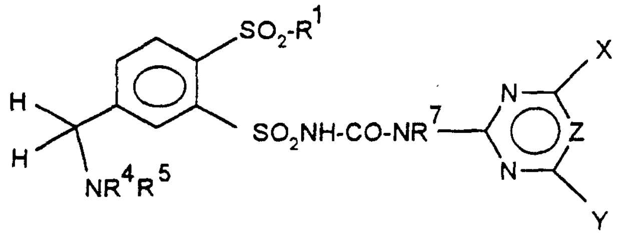

(Ia)

| Nr. | $R^1$ | $R^4$ | $R^5$ | $R^7$ | X | Y | Z | Fp. (°C) |
|---|---|---|---|---|---|---|---|---|
| 1 | $CH_3$ | H | CHO | H | $OCH_3$ | $OCH_3$ | CH | 142-148 |
| 2 | $CH_3$ | H | CHO | H | $OCH_3$ | $CH_3$ | CH | |
| 3 | $CH_3$ | H | CHO | H | $CH_3$ | $CH_3$ | CH | |
| 4 | $CH_3$ | H | CHO | H | $CH_3$ | $OC_2H_5$ | CH | |
| 5 | $CH_3$ | H | CHO | H | $C_2H_5$ | $OCH_3$ | CH | |
| 6 | $CH_3$ | H | CHO | H | $OC_2H_5$ | $OC_2H_5$ | CH | |
| 7 | $CH_3$ | H | CHO | H | $OCHF_2$ | $OCHF_2$ | CH | |
| 8 | $CH_3$ | H | CHO | H | $CH_3$ | $OCHF_2$ | CH | |
| 9 | $CH_3$ | H | CHO | H | Cl | $OCH_3$ | CH | |
| 10 | $CH_3$ | H | CHO | H | $OCH_3$ | $OCH_3$ | N | |
| 11 | $CH_3$ | H | CHO | H | $OCH_3$ | $CH_3$ | N | |
| 12 | $CH_3$ | H | CHO | H | $CH_3$ | $CH_3$ | N | |
| 13 | $CH_3$ | H | CHO | H | $N(CH_3)_2$ | $OCH_2CF_3$ | N | |
| 14 | $CH_3$ | H | CHO | H | $OCH_3$ | $CF_3$ | N | |
| 15 | $CH_3$ | H | CHO | H | $OC_2H_5$ | $NHCH_3$ | N | |
| 16 | $CH_3$ | H | CHO | $CH_3$ | $OCH_3$ | $OCH_3$ | CH | |
| 17 | $CH_3$ | H | CHO | $CH_3$ | $OCH_3$ | $CH_3$ | N | |
| 18 | $CH_3$ | H | $COCH_3$ | H | $OCH_3$ | $OCH_3$ | CH | 195-200 |
| 19 | $CH_3$ | H | $COCH_3$ | H | $OCH_3$ | $CH_3$ | CH | |
| 20 | $CH_3$ | H | $COCH_3$ | H | $CH_3$ | $CH_3$ | CH | |
| 21 | $CH_3$ | H | $COCH_3$ | H | $CH_3$ | $OC_2C_5$ | CH | |
| 22 | $CH_3$ | H | $COCH_3$ | H | $C_2H_5$ | $OCH_3$ | CH | |
| 23 | $CH_3$ | H | $COCH_3$ | H | $OC_2H_5$ | $OC_2H_5$ | CH | |
| 24 | $CH_3$ | H | $COCH_3$ | H | $OCHF_2$ | $OCHF_2$ | CH | |
| 25 | $CH_3$ | H | $COCH_3$ | H | $CH_3$ | $OCHF_2$ | CH | |
| 26 | $CH_3$ | H | $COCH_3$ | H | Cl | $OCH_3$ | CH | |
| 27 | $CH_3$ | H | $COCH_3$ | H | $OCH_3$ | $OCH_3$ | N | |

| Nr. | $R^1$ | $R^4$ | $R^5$ | $R^7$ | X | Y | Z | Fp. (°C) |
|---|---|---|---|---|---|---|---|---|
| 28 | $CH_3$ | H | $COCH_3$ | H | $OCH_3$ | $CH_3$ | N | |
| 29 | $CH_3$ | H | $COCH_3$ | H | $CH_3$ | $CH_3$ | N | |
| 30 | $CH_3$ | H | $COCH_3$ | H | $N(CH_3)_2$ | $OCH_2CF_3$ | N | |
| 31 | $CH_3$ | H | $COCH_3$ | H | $OCH_3$ | $CF_3$ | N | |
| 32 | $CH_3$ | H | $COCH_3$ | H | $OC_2H_5$ | $NHCH_3$ | N | |
| 33 | $CH_3$ | H | $COCH_3$ | $CH_3$ | $OCH_3$ | $OCH_3$ | CH | |
| 34 | $CH_3$ | H | $COCH_3$ | $CH_3$ | $OCH_3$ | $CH_3$ | N | |
| 35 | $CH_3$ | H | $COC_2H_5$ | H | $OCH_3$ | $OCH_3$ | CH | 196-200 |
| 36 | $CH_3$ | H | $COC_2H_5$ | H | $OCH_3$ | $CH_3$ | CH | |
| 37 | $CH_3$ | H | $COC_2H_5$ | H | $CH_3$ | $CH_3$ | CH | |
| 38 | $CH_3$ | H | $COC_2H_5$ | H | $CH_3$ | $OC_2H_5$ | CH | |
| 39 | $CH_3$ | H | $COC_2H_5$ | H | $C_2H_5$ | $OCH_3$ | CH | |
| 40 | $CH_3$ | H | $COC_2H_5$ | H | $OC_2H_5$ | $OC_2H_5$ | CH | |
| 41 | $CH_3$ | H | $COC_2H_5$ | H | $OCHF_2$ | $OCHF_2$ | CH | |
| 42 | $CH_3$ | H | $COC_2H_5$ | H | $CH_3$ | $OCHF_2$ | CH | |
| 43 | $CH_3$ | H | $COC_2H_5$ | H | Cl | $OCH_3$ | CH | |
| 44 | $CH_3$ | H | $COC_2H_5$ | H | $OCH_3$ | $OCH_3$ | N | |
| 45 | $CH_3$ | H | $COC_2H_5$ | H | $OCH_3$ | $CH_3$ | N | |
| 46 | $CH_3$ | H | $COC_2H_5$ | H | $CH_3$ | $CH_3$ | N | |
| 47 | $CH_3$ | H | $COC_2H_5$ | H | $N(CH_3)_2$ | $OCH_2CF_3$ | N | |
| 48 | $CH_3$ | H | $COC_2H_5$ | H | $OCH_3$ | $CF_3$ | N | |
| 49 | $CH_3$ | H | $COC_2H_5$ | H | $OC_2H_5$ | $NHCH_3$ | N | |
| 50 | $CH_3$ | H | $COC_2H_5$ | H | $OCH_3$ | $OCH_3$ | CH | |
| 51 | $CH_3$ | H | $COC_2H_5$ | H | $OCH_3$ | $CH_3$ | N | |
| 52 | $CH_3$ | H | $CO\text{-}n\text{-}C_3H_7$ | H | $OCH_3$ | $OCH_3$ | CH | |
| 53 | $CH_3$ | H | $CO\text{-}n\text{-}C_3H_7$ | H | $OCH_3$ | $CH_3$ | CH | |
| 54 | $CH_3$ | H | $CO\text{-}n\text{-}C_3H_7$ | H | $CH_3$ | $CH_3$ | CH | |
| 55 | $CH_3$ | H | $CO\text{-}n\text{-}C_3H_7$ | H | $CH_3$ | $OC_2H_5$ | CH | |
| 56 | $CH_3$ | H | $CO\text{-}n\text{-}C_3H_7$ | H | $C_2H_5$ | $OCH_3$ | CH | |
| 57 | $CH_3$ | H | $CO\text{-}n\text{-}C_3H_7$ | H | $OC_2H_5$ | $OC_2H_5$ | CH | |
| 58 | $CH_3$ | H | $CO\text{-}n\text{-}C_3H_7$ | H | $OCHF_2$ | $OCHF_2$ | CH | |
| 59 | $CH_3$ | H | $CO\text{-}n\text{-}C_3H_7$ | H | $CH_3$ | $OCHF_2$ | CH | |
| 60 | $CH_3$ | H | $CO\text{-}n\text{-}C_3H_7$ | H | Cl | $OCH_3$ | CH | |
| 61 | $CH_3$ | H | $CO\text{-}n\text{-}C_3H_7$ | H | $OCH_3$ | $OCH_3$ | N | |

| Nr. | $R^1$ | $R^4$ | $R^5$ | $R^7$ | X | Y | Z | Fp. (°C) |
|---|---|---|---|---|---|---|---|---|
| 62 | $CH_3$ | H | CO-n-$C_3H_7$ | H | $OCH_3$ | $CH_3$ | N | |
| 63 | $CH_3$ | H | CO-n-$C_3H_7$ | H | $CH_3$ | $CH_3$ | N | |
| 64 | $CH_3$ | H | CO-n-$C_3H_7$ | H | $N(CH_3)_2$ | $OCH_2CF_3$ | N | |
| 65 | $CH_3$ | H | CO-n-$C_3H_7$ | H | $OCH_3$ | $CF_3$ | N | |
| 66 | $CH_3$ | H | CO-n-$C_3H_7$ | H | $OC_2H_5$ | $NHCH_3$ | N | |
| 67 | $CH_3$ | H | CO-n-$C_3H_7$ | $CH_3$ | $OCH_3$ | $OCH_3$ | CH | |
| 68 | $CH_3$ | H | CO-n-$C_3H_7$ | $CH_3$ | $OCH_3$ | $CH_3$ | N | |
| 69 | $CH_3$ | H | CO-i-$C_3H_7$ | H | $OCH_3$ | $OCH_3$ | CH | 80-89 |
| 70 | $CH_3$ | H | CO-i-$C_3H_7$ | H | $OCH_3$ | $CH_3$ | CH | |
| 71 | $CH_3$ | H | CO-i-$C_3H_7$ | H | $CH_3$ | $CH_3$ | CH | |
| 72 | $CH_3$ | H | CO-i-$C_3H_7$ | H | $CH_3$ | $OC_2H_5$ | CH | |
| 73 | $CH_3$ | H | CO-i-$C_3H_7$ | H | $C_2H_5$ | $OCH_3$ | CH | |
| 74 | $CH_3$ | H | CO-i-$C_3H_7$ | H | $OC_2H_5$ | $OC_2H_5$ | CH | |
| 75 | $CH_3$ | H | CO-i-$C_3H_7$ | H | $OCHF_2$ | $OCHF_2$ | CH | |
| 76 | $CH_3$ | H | CO-i-$C_3H_7$ | H | $CH_3$ | $OCHF_2$ | CH | |
| 77 | $CH_3$ | H | CO-i-$C_3H_7$ | H | Cl | $OCH_3$ | CH | |
| 78 | $CH_3$ | H | CO-i-$C_3H_7$ | H | $OCH_3$ | $OCH_3$ | N | |
| 79 | $CH_3$ | H | CO-i-$C_3H_7$ | H | $OCH_3$ | $CH_3$ | N | |
| 80 | $CH_3$ | H | CO-i-$C_3H_7$ | H | $CH_3$ | $CH_3$ | N | |
| 81 | $CH_3$ | H | CO-i-$C_3H_7$ | H | $N(CH_3)_2$ | $OCH_2CF_3$ | N | |
| 82 | $CH_3$ | H | CO-i-$C_3H_7$ | H | $OCH_3$ | $CF_3$ | N | |
| 83 | $CH_3$ | H | CO-i-$C_3H_7$ | H | $OC_2H_5$ | $NHCH_3$ | N | |
| 84 | $CH_3$ | H | CO-i-$C_3H_7$ | $CH_3$ | $OCH_3$ | $OCH_3$ | CH | |
| 85 | $CH_3$ | H | CO-i-$C_3H_7$ | $CH_3$ | $OCH_3$ | $CH_3$ | N | |
| 86 | $CH_3$ | H | $COCF_3$ | H | $OCH_3$ | $OCH_3$ | CH | 138-141 |
| 87 | $CH_3$ | H | $COCF_3$ | H | $OCH_3$ | $CH_3$ | CH | |
| 88 | $CH_3$ | H | $COCF_3$ | H | $CH_3$ | $CH_3$ | CH | |
| 89 | $CH_3$ | H | $COCF_3$ | H | $CH_3$ | $OC_2H_5$ | CH | |
| 90 | $CH_3$ | H | $COCF_3$ | H | $C_2H_5$ | $OCH_3$ | CH | |
| 91 | $CH_3$ | H | $COCF_3$ | H | $OC_2H_5$ | $OC_2H_5$ | CH | |
| 92 | $CH_3$ | H | $COCF_3$ | H | $OCHF_2$ | $OCHF_2$ | CH | |
| 93 | $CH_3$ | H | $COCF_3$ | H | $CH_3$ | $OCHF_2$ | CH | |
| 94 | $CH_3$ | H | $COCF_3$ | H | Cl | $OCH_3$ | CH | |
| 95 | $CH_3$ | H | $COCF_3$ | H | $OCH_3$ | $OCH_3$ | N | |

| Nr. | $R^1$ | $R^4$ | $R^5$ | $R^7$ | X | Y | Z | Fp. (°C) |
|---|---|---|---|---|---|---|---|---|
| 96 | $CH_3$ | H | $COCF_3$ | H | $OCH_3$ | $CH_3$ | N | |
| 97 | $CH_3$ | H | $COCF_3$ | H | $CH_3$ | $CH_3$ | N | |
| 98 | $CH_3$ | H | $COCF_3$ | H | $N(CH_3)_2$ | $OCH_2CF_3$ | N | |
| 99 | $CH_3$ | H | $COCF_3$ | H | $OCH_3$ | $CF_3$ | N | |
| 100 | $CH_3$ | H | $COCF_3$ | H | $OC_2H_5$ | $NHCH_3$ | N | |
| 101 | $CH_3$ | H | $COCF_3$ | $CH_3$ | $OCH_3$ | $OCH_3$ | CH | |
| 102 | $CH_3$ | H | $COCF_3$ | $CH_3$ | $OCH_3$ | $CH_3$ | N | |
| 103 | $CH_3$ | H | $COOCH_3$ | H | $OCH_3$ | $OCH_3$ | CH | 150-154 |
| 104 | $CH_3$ | H | $COOCH_3$ | H | $OCH_3$ | $CH_3$ | CH | |
| 105 | $CH_3$ | H | $COOCH_3$ | H | $CH_3$ | $CH_3$ | CH | |
| 106 | $CH_3$ | H | $COOCH_3$ | H | $CH_3$ | $OC_2H_5$ | CH | |
| 107 | $CH_3$ | H | $COOCH_3$ | H | $C_2H_5$ | $OCH_3$ | CH | |
| 108 | $CH_3$ | H | $COOCH_3$ | H | $OC_2H_5$ | $OC_2H_5$ | CH | |
| 109 | $CH_3$ | H | $COOCH_3$ | H | $OCHF_2$ | $OCHF_2$ | CH | |
| 110 | $CH_3$ | H | $COOCH_3$ | H | $CH_3$ | $OCHF_2$ | CH | |
| 111 | $CH_3$ | H | $COOCH_3$ | H | Cl | $OCH_3$ | CH | |
| 112 | $CH_3$ | H | $COOCH_3$ | H | $OCH_3$ | $OCH_3$ | N | |
| 113 | $CH_3$ | H | $COOCH_3$ | H | $OCH_3$ | $CH_3$ | N | |
| 114 | $CH_3$ | H | $COOCH_3$ | H | $CH_3$ | $CH_3$ | N | |
| 115 | $CH_3$ | H | $COOCH_3$ | H | $N(CH_3)_2$ | $OCH_2CF_3$ | N | |
| 116 | $CH_3$ | H | $COOCH_3$ | H | $OCH_3$ | $CF_3$ | N | |
| 117 | $CH_3$ | H | $COOCH_3$ | H | $OC_2H_5$ | $NHCH_3$ | N | |
| 118 | $CH_3$ | H | $COOCH_3$ | $CH_3$ | $OCH_3$ | $OCH_3$ | CH | |
| 119 | $CH_3$ | H | $COOCH_3$ | $CH_3$ | $OCH_3$ | $CH_3$ | N | |
| 120 | $CH_3$ | H | $COOC_2H_5$ | H | $OCH_3$ | $OCH_3$ | CH | 151-156 |
| 121 | $CH_3$ | H | $COOC_2H_5$ | H | $OCH_3$ | $CH_3$ | CH | |
| 122 | $CH_3$ | H | $COOC_2H_5$ | H | $CH_3$ | $CH_3$ | CH | |
| 123 | $CH_3$ | H | $COOC_2H_5$ | H | $CH_3$ | $OC_2H_5$ | CH | |
| 124 | $CH_3$ | H | $COOC_2H_5$ | H | $C_2H_5$ | $OCH_3$ | CH | |
| 125 | $CH_3$ | H | $COOC_2H_5$ | H | $OC_2H_5$ | $OC_2H_5$ | CH | |
| 126 | $CH_3$ | H | $COOC_2H_5$ | H | $OCHF_2$ | $OCHF_2$ | CH | |
| 127 | $CH_3$ | H | $COOC_2H_5$ | H | $CH_3$ | $OCHF_2$ | CH | |
| 128 | $CH_3$ | H | $COOC_2H_5$ | H | Cl | $OCH_3$ | CH | |
| 129 | $CH_3$ | H | $COOC_2H_5$ | H | $OCH_3$ | $OCH_3$ | N | |

| Nr. | $R^1$ | $R^4$ | $R^5$ | $R^7$ | X | Y | Z | Fp. (°C) |
|---|---|---|---|---|---|---|---|---|
| 130 | $CH_3$ | H | $COOC_2H_5$ | H | $OCH_3$ | $CH_3$ | N | |
| 131 | $CH_3$ | H | $COOC_2H_5$ | H | $CH_3$ | $CH_3$ | N | |
| 132 | $CH_3$ | H | $COOC_2H_5$ | H | $N(CH_3)_2$ | $OCH_2CF_3$ | N | |
| 133 | $CH_3$ | H | $COOC_2H_5$ | H | $OCH_3$ | $CF_3$ | N | |
| 134 | $CH_3$ | H | $COOC_2H_5$ | H | $OC_2H_5$ | $NHCH_3$ | N | |
| 135 | $CH_3$ | H | $COOC_2H_5$ | $CH_3$ | $OCH_3$ | $OCH_3$ | CH | |
| 136 | $CH_3$ | H | $COOC_2H_5$ | $CH_3$ | $OCH_3$ | $CH_3$ | CH | |
| 137 | $CH_3$ | H | $COOC_2H_5$ | $CH_3$ | $OCH_3$ | $CH_3$ | N | |
| 138 | $CH_3$ | H | $SO_2CH_3$ | H | $OCH_3$ | $OCH_3$ | CH | 208-210 |
| 139 | $CH_3$ | H | $SO_2CH_3$ | H | $OCH_3$ | $CH_3$ | CH | |
| 140 | $CH_3$ | H | $SO_2CH_3$ | H | $CH_3$ | $CH_3$ | CH | |
| 141 | $CH_3$ | H | $SO_2CH_3$ | H | $CH_3$ | $OC_2H_5$ | CH | |
| 142 | $CH_3$ | H | $SO_2CH_3$ | H | $C_2H_5$ | $OCH_3$ | CH | |
| 143 | $CH_3$ | H | $SO_2CH_3$ | H | $OC_2H_5$ | $OC_2H_5$ | CH | |
| 144 | $CH_3$ | H | $SO_2CH_3$ | H | $OCHF_2$ | $OCHF_2$ | CH | |
| 145 | $CH_3$ | H | $SO_2CH_3$ | H | $CH_3$ | $OCHF_2$ | CH | |
| 146 | $CH_3$ | H | $SO_2CH_3$ | H | Cl | $OCH_3$ | CH | |
| 147 | $CH_3$ | H | $SO_2CH_3$ | H | $OCH_3$ | $OCH_3$ | N | |
| 148 | $CH_3$ | H | $SO_2CH_3$ | H | $OCH_3$ | $CH_3$ | N | |
| 149 | $CH_3$ | H | $SO_2CH_3$ | H | $CH_3$ | $CH_3$ | N | |
| 150 | $CH_3$ | H | $SO_2CH_3$ | H | $N(CH_3)_2$ | $OCH_2CF_3$ | N | |
| 151 | $CH_3$ | H | $SO_2CH_3$ | H | $OCH_3$ | $CF_3$ | N | |
| 152 | $CH_3$ | H | $SO_2CH_3$ | H | $OC_2H_5$ | $NHCH_3$ | N | |
| 153 | $CH_3$ | H | $SO_2CH_3$ | $CH_3$ | $OCH_3$ | $OCH_3$ | CH | |
| 154 | $CH_3$ | H | $SO_2CH_3$ | $CH_3$ | $OCH_3$ | $CH_3$ | N | |
| 155 | $CH_3$ | H | $C_2H_5SO_2$ | H | $OCH_3$ | $OCH_3$ | CH | 157-160 |
| 156 | $CH_3$ | H | $C_2H_5SO_2$ | H | $OCH_3$ | $CH_3$ | CH | |
| 157 | $CH_3$ | H | $C_2H_5SO_2$ | H | $CH_3$ | $CH_3$ | CH | |
| 158 | $CH_3$ | H | $C_2H_5SO_2$ | H | $CH_3$ | $OC_2H_5$ | CH | |
| 159 | $CH_3$ | H | $C_2H_5SO_2$ | H | $C_2H_5$ | $OCH_3$ | CH | |
| 160 | $CH_3$ | H | $C_2H_5SO_2$ | H | $OC_2H_5$ | $OC_2H_5$ | CH | |
| 161 | $CH_3$ | H | $C_2H_5SO_2$ | H | $OCHF_2$ | $OCHF_2$ | CH | |
| 162 | $CH_3$ | H | $C_2H_5SO_2$ | H | $CH_3$ | $OCHF_2$ | CH | |
| 163 | $CH_3$ | H | $C_2H_5SO_2$ | H | Cl | $OCH_3$ | CH | |

| Nr. | $R^1$ | $R^4$ | $R^5$ | $R^7$ | X | Y | Z | Fp. (°C) |
|---|---|---|---|---|---|---|---|---|
| 164 | $CH_3$ | H | $C_2H_5SO_2$ | H | $OCH_3$ | $OCH_3$ | N | |
| 165 | $CH_3$ | H | $C_2H_5SO_2$ | H | $OCH_3$ | $CH_3$ | N | |
| 166 | $CH_3$ | H | $C_2H_5SO_2$ | H | $CH_3$ | $CH_3$ | N | |
| 167 | $CH_3$ | H | $C_2H_5SO_2$ | H | $N(CH_3)_2$ | $OCH_2CF_3$ | N | |
| 168 | $CH_3$ | H | $C_2H_5SO_2$ | H | $OCH_3$ | $CF_3$ | N | |
| 169 | $CH_3$ | H | $C_2H_5SO_2$ | H | $OC_2H_5$ | $NHCH_3$ | N | |
| 170 | $CH_3$ | H | $C_2H_5SO_2$ | $CH_3$ | $OCH_3$ | $OCH_3$ | CH | |
| 171 | $CH_3$ | H | $C_2H_5SO_2$ | $CH_3$ | $OCH_3$ | $CH_3$ | N | |
| 172 | $CH_3$ | H | $n-C_3H_7SO_2$ | H | $OCH_3$ | $OCH_3$ | CH | |
| 173 | $CH_3$ | H | $n-C_3H_7SO_2$ | H | $OC_2H_5$ | $OCH_3$ | CH | |
| 174 | $CH_3$ | H | $n-C_3H_7SO_2$ | H | $OCH_3$ | $CH_3$ | CH | |
| 175 | $CH_3$ | H | $n-C_3H_7SO_2$ | H | $CH_3$ | $CH_3$ | CH | |
| 176 | $CH_3$ | H | $n-C_3H_7SO_2$ | H | $CH_3$ | $OC_2H_5$ | CH | |
| 177 | $CH_3$ | H | $n-C_3H_7SO_2$ | H | $C_2H_5$ | $OCH_3$ | CH | |
| 178 | $CH_3$ | H | $n-C_3H_7SO_2$ | H | $OC_2H_5$ | $OC_2H_5$ | CH | |
| 179 | $CH_3$ | H | $n-C_3H_7SO_2$ | H | $OCHF_2$ | $OCHF_2$ | CH | |
| 180 | $CH_3$ | H | $n-C_3H_7SO_2$ | H | $CH_3$ | $OCHF_2$ | CH | |
| 181 | $CH_3$ | H | $n-C_3H_7SO_2$ | H | Cl | $OCH_3$ | CH | |
| 182 | $CH_3$ | H | $n-C_3H_7SO_2$ | H | $OCH_3$ | $OCH_3$ | N | |
| 183 | $CH_3$ | H | $n-C_3H_7SO_2$ | H | $OCH_3$ | $CH_3$ | N | |
| 184 | $CH_3$ | H | $n-C_3H_7SO_2$ | H | $CH_3$ | $CH_3$ | N | |
| 185 | $CH_3$ | H | $n-C_3H_7SO_2$ | H | $N(CH_3)_2$ | $OCH_2CF_3$ | N | |
| 186 | $CH_3$ | H | $n-C_3H_7SO_2$ | H | $OCH_3$ | $CF_3$ | N | |
| 187 | $CH_3$ | H | $n-C_3H_7SO_2$ | H | $OC_2H_5$ | $NHCH_3$ | N | |
| 188 | $CH_3$ | H | $n-C_3H_7SO_2$ | $CH_3$ | $OCH_3$ | $OCH_3$ | CH | |
| 189 | $CH_3$ | H | $n-C_3H_7SO_2$ | $CH_3$ | $OCH_3$ | $CH_3$ | N | |
| 190 | $CH_3$ | H | $i-C_3H_7SO_2$ | H | $OCH_3$ | $OCH_3$ | CH | |
| 191 | $CH_3$ | H | $i-C_3H_7SO_2$ | H | $OCH_3$ | $CH_3$ | CH | |
| 192 | $CH_3$ | H | $i-C_3H_7SO_2$ | H | $CH_3$ | $CH_3$ | CH | |
| 193 | $CH_3$ | H | $i-C_3H_7SO_2$ | H | $CH_3$ | $OC_2H_5$ | CH | |
| 194 | $CH_3$ | H | $i-C_3H_7SO_2$ | H | $C_2H_5$ | $OCH_3$ | CH | |
| 195 | $CH_3$ | H | $i-C_3H_7SO_2$ | H | $OC_2H_5$ | $OC_2H_5$ | CH | |
| 196 | $CH_3$ | H | $i-C_3H_7SO_2$ | H | $OCHF_2$ | $OCHF_2$ | CH | |
| 197 | $CH_3$ | H | $i-C_3H_7SO_2$ | H | $CH_3$ | $OCHF_2$ | CH | |

| Nr. | $R^1$ | $R^4$ | $R^5$ | $R^7$ | X | Y | Z | Fp. (°C) |
|---|---|---|---|---|---|---|---|---|
| 198 | $CH_3$ | H | i-$C_3H_7SO_2$ | H | Cl | $OCH_3$ | CH | |
| 199 | $CH_3$ | H | i-$C_3H_7SO_2$ | H | $OCH_3$ | $OCH_3$ | N | |
| 200 | $CH_3$ | H | i-$C_3H_7SO_2$ | H | $OCH_3$ | $CH_3$ | N | |
| 201 | $CH_3$ | H | i-$C_3H_7SO_2$ | H | $CH_3$ | $CH_3$ | N | |
| 202 | $CH_3$ | H | i-$C_3H_7SO_2$ | H | $N(CH_3)_2$ | $OCH_2CF_3$ | N | |
| 203 | $CH_3$ | H | i-$C_3H_7SO_2$ | H | $OCH_3$ | $CF_3$ | N | |
| 204 | $CH_3$ | H | i-$C_3H_7SO_2$ | H | $OC_2H_5$ | $NHCH_3$ | N | |
| 205 | $CH_3$ | H | i-$C_3H_7SO_2$ | $CH_3$ | $OCH_3$ | $OCH_3$ | CH | |
| 206 | $CH_3$ | H | i-$C_3H_7SO_2$ | $CH_3$ | $OCH_3$ | $CH_3$ | N | |
| 207 | $CH_3$ | H | $ClCH_2CO$ | H | $OCH_3$ | $OCH_3$ | CH | |
| 208 | $CH_3$ | H | $ClCH_2CO$ | H | $OCH_3$ | $CH_3$ | CH | |
| 209 | $CH_3$ | H | $ClCH_2CO$ | H | $CH_3$ | $CH_3$ | CH | |
| 210 | $CH_3$ | H | $ClCH_2CO$ | H | Cl | $OCH_3$ | CH | |
| 211 | $CH_3$ | H | $ClCH_2CO$ | H | $OCH_3$ | $OCH_3$ | N | |
| 212 | $CH_3$ | H | $ClCH_2CO$ | H | $OCH_3$ | $CH_3$ | N | |
| 213 | $CH_3$ | H | $ClCH_2CO$ | H | $OCH_3$ | $CF_3$ | N | |
| 214 | $CH_3$ | H | $ClCH_2CO$ | $CH_3$ | $OCH_3$ | $OCH_3$ | CH | |
| 215 | $CH_3$ | H | $ClCH_2CO$ | $CH_3$ | $OCH_3$ | $CH_3$ | N | |
| 216 | $CH_3$ | H | $Cl_2CHCO$ | H | $OCH_3$ | $OCH_3$ | CH | |
| 217 | $CH_3$ | H | $Cl_2CHCO$ | H | $OCH_3$ | $CH_3$ | CH | |
| 218 | $CH_3$ | H | $Cl_2CHCO$ | H | $CH_3$ | $CH_3$ | CH | |
| 219 | $CH_3$ | H | $Cl_2CHCO$ | H | Cl | $OCH_3$ | CH | |
| 220 | $CH_3$ | H | $Cl_2CHCO$ | H | $OCH_3$ | $OCH_3$ | N | |
| 221 | $CH_3$ | H | $Cl_2CHCO$ | H | $OCH_3$ | $CH_3$ | N | |
| 222 | $CH_3$ | H | $Cl_2CHCO$ | H | $OCH_3$ | $CF_3$ | N | |
| 223 | $CH_3$ | H | $Cl_2CHCO$ | $CH_3$ | $OCH_3$ | $OCH_3$ | CH | |
| 224 | $CH_3$ | H | $Cl_2CHCO$ | $CH_3$ | $OCH_3$ | $CH_3$ | N | |
| 225 | $CH_3$ | H | $Cl_3CCO$ | H | $OCH_3$ | $OCH_3$ | CH | 125-130 |
| 226 | $CH_3$ | H | $Cl_3CCO$ | H | $OCH_3$ | $CH_3$ | CH | |
| 227 | $CH_3$ | H | $Cl_3CCO$ | H | $CH_3$ | $CH_3$ | CH | |
| 228 | $CH_3$ | H | $Cl_3CCO$ | H | Cl | $OCH_3$ | CH | |
| 229 | $CH_3$ | H | $Cl_3CCO$ | H | $OCH_3$ | $OCH_3$ | N | |
| 230 | $CH_3$ | H | $Cl_3CCO$ | H | $OCH_3$ | $CH_3$ | N | |
| 231 | $CH_3$ | H | $Cl_3CCO$ | H | $OCH_3$ | $CF_3$ | N | |

| Nr. | $R^1$ | $R^4$ | $R^5$ | $R^7$ | X | Y | Z | Fp. (°C) |
|---|---|---|---|---|---|---|---|---|
| 232 | $CH_3$ | H | $Cl_3CCO$ | $CH_3$ | $OCH_3$ | $OCH_3$ | CH | |
| 233 | $CH_3$ | H | $Cl_3CCO$ | $CH_3$ | $OCH_3$ | $CH_3$ | N | |
| 234 | $CH_3$ | H | $CH_3OCH_2CO$ | H | $OCH_3$ | $OCH_3$ | CH | |
| 235 | $CH_3$ | H | $CH_3OCH_2CO$ | H | $OCH_3$ | $CH_3$ | CH | |
| 236 | $CH_3$ | H | $CH_3OCH_2CO$ | H | $CH_3$ | $CH_3$ | CH | |
| 237 | $CH_3$ | H | $CH_3OCH_2CO$ | H | Cl | $OCH_3$ | CH | |
| 238 | $CH_3$ | H | $CH_3OCH_2CO$ | H | $OCH_3$ | $OCH_3$ | N | |
| 239 | $CH_3$ | H | $CH_3OCH_2CO$ | H | $OCH_3$ | $CH_3$ | N | |
| 240 | $CH_3$ | H | $CH_3OCH_2CO$ | H | $OCH_3$ | $CF_3$ | N | |
| 241 | $CH_3$ | H | $CH_3OCH_2CO$ | $CH_3$ | $OCH_3$ | $OCH_3$ | CH | |
| 242 | $CH_3$ | H | $CH_3OCH_2CO$ | $CH_3$ | $OCH_3$ | $CH_3$ | N | |
| 243 | $CH_3$ | H | $CH_2{=}CHCO$ | H | $OCH_3$ | $OCH_3$ | CH | |
| 244 | $CH_3$ | H | $CH_2{=}CHCO$ | H | $OCH_3$ | $CH_3$ | CH | |
| 245 | $CH_3$ | H | $CH_2{=}CHCO$ | H | $CH_3$ | $CH_3$ | CH | |
| 246 | $CH_3$ | H | $CH_2{=}CHCO$ | H | Cl | $OCH_3$ | CH | |
| 247 | $CH_3$ | H | $CH_2{=}CHCO$ | H | $OCH_3$ | $OCH_3$ | N | |
| 248 | $CH_3$ | H | $CH_2{=}CHCO$ | H | $OCH_3$ | $CH_3$ | N | |
| 249 | $CH_3$ | H | $CH_2{=}CHCO$ | H | $OCH_3$ | $CF_3$ | N | |
| 250 | $CH_3$ | H | $CH_2{=}CHCO$ | $CH_3$ | $OCH_3$ | $OCH_3$ | CH | |
| 251 | $CH_3$ | H | $CH_2{=}CHCO$ | $CH_3$ | $OCH_3$ | $CH_3$ | N | |
| 252 | $CH_3$ | H | $CH{\equiv}CCO$ | H | $OCH_3$ | $OCH_3$ | CH | |
| 253 | $CH_3$ | H | $CH{\equiv}CCO$ | H | $OCH_3$ | $CH_3$ | CH | |
| 254 | $CH_3$ | H | $CH{\equiv}CCO$ | H | $OCH_3$ | $CH_3$ | CH | |
| 255 | $CH_3$ | H | $CH{\equiv}CCO$ | H | Cl | $OCH_3$ | CH | |
| 256 | $CH_3$ | H | $CH{\equiv}CCO$ | H | $OCH_3$ | $OCH_3$ | N | |
| 257 | $CH_3$ | H | $CH{\equiv}CCO$ | H | $OCH_3$ | $CH_3$ | N | |
| 258 | $CH_3$ | H | $CH{\equiv}CCO$ | H | $OCH_3$ | $CF_3$ | N | |
| 259 | $CH_3$ | H | $CH{\equiv}CCO$ | $CH_3$ | $OCH_3$ | $OCH_3$ | CH | |
| 260 | $CH_3$ | H | $CH{\equiv}CCO$ | $CH_3$ | $OCH_3$ | $CH_3$ | N | |
| 261 | $CH_3$ | H | CO 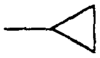 | H | $OCH_3$ | $OCH_3$ | CH | |
| 262 | $CH_3$ | H | CO 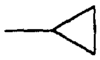 | H | $OCH_3$ | $CH_3$ | CH | |

| Nr. | $R^1$ | $R^4$ | $R^5$ | $R^7$ | X | Y | Z | Fp. (°C) |
|---|---|---|---|---|---|---|---|---|
| 263 | $CH_3$ | H | 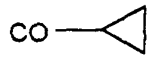 | H | $CH_3$ | $CH_3$ | CH | |
| 264 | $CH_3$ | H | 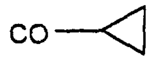 | H | Cl | $OCH_3$ | CH | |
| 265 | $CH_3$ | H | 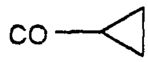 | H | $OCH_3$ | $OCH_3$ | N | |
| 266 | $CH_3$ | H | 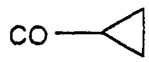 | H | $OCH_3$ | $CH_3$ | N | |
| 267 | $CH_3$ | H | 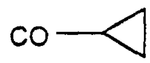 | H | $OCH_3$ | $CF_3$ | N | |
| 268 | $CH_3$ | H | 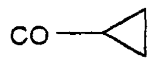 | $CH_3$ | $OCH_3$ | $OCH_3$ | CH | |
| 269 | $CH_3$ | H | 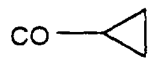 | $CH_3$ | $OCH_3$ | $CH_3$ | N | |
| 270 | $CH_3$ | H | 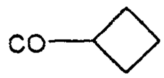 | H | $OCH_3$ | $OCH_3$ | CH | |
| 271 | $CH_3$ | H | 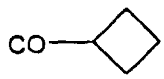 | H | $OCH_3$ | $CH_3$ | CH | |
| 272 | $CH_3$ | H | 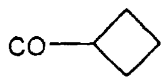 | H | $CH_3$ | $CH_3$ | CH | |
| 273 | $CH_3$ | H | 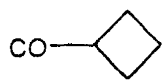 | H | Cl | $OCH_3$ | CH | |
| 274 | $CH_3$ | H | 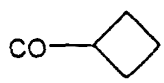 | H | $OCH_3$ | $OCH_3$ | N | |
| 275 | $CH_3$ | H | 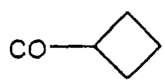 | H | $OCH_3$ | $CH_3$ | N | |
| 276 | $CH_3$ | H | 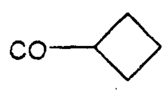 | H | $OCH_3$ | $CF_3$ | N | |
| 277 | $CH_3$ | H | 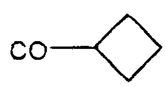 | $CH_3$ | $OCH_3$ | $OCH_3$ | CH | |

| Nr. | $R^1$ | $R^4$ | $R^5$ | $R^7$ | X | Y | Z | Fp. (°C) |
|---|---|---|---|---|---|---|---|---|
| 278 | $CH_3$ | H | 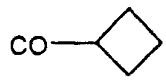 | $CH_3$ | $OCH_3$ | $CH_3$ | N | |
| 279 | $CH_3$ | H | 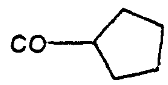 | H | $OCH_3$ | $OCH_3$ | CH | |
| 280 | $CH_3$ | H | 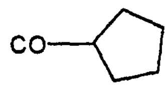 | H | $OCH_3$ | $CH_3$ | CH | |
| 281 | $CH_3$ | H | 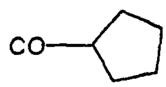 | H | $CH_3$ | $CH_3$ | CH | |
| 282 | $CH_3$ | H | 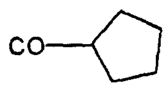 | H | Cl | $OCH_3$ | CH | |
| 283 | $CH_3$ | H | 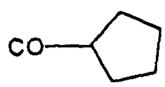 | H | $OCH_3$ | $OCH_3$ | N | |
| 284 | $CH_3$ | H | 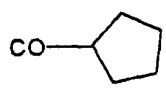 | H | $OCH_3$ | $CH_3$ | N | |
| 285 | $CH_3$ | H | 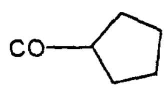 | H | $OCH_3$ | $CF_3$ | N | |
| 286 | $CH_3$ | H | 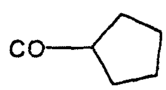 | $CH_3$ | $OCH_3$ | $OCH_3$ | CH | |
| 287 | $CH_3$ | H | 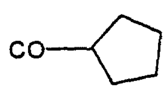 | $CH_3$ | $OCH_3$ | $CH_3$ | N | |
| 288 | $CH_3$ | H | 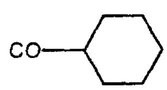 | H | $OCH_3$ | $OCH_3$ | CH | |
| 289 | $CH_3$ | H | 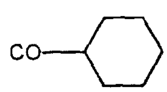 | H | $OCH_3$ | $CH_3$ | CH | |
| 290 | $CH_3$ | H | 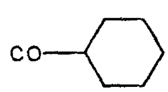 | H | $CH_3$ | $CH_3$ | CH | |
| 291 | $CH_3$ | H | 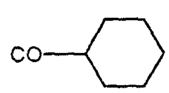 | H | Cl | $OCH_3$ | CH | |

| Nr. | $R^1$ | $R^4$ | $R^5$ | $R^7$ | X | Y | Z | Fp. (°C) |
|---|---|---|---|---|---|---|---|---|
| 292 | $CH_3$ | H | CO 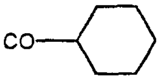 | H | $OCH_3$ | $OCH_3$ | N | |
| 293 | $CH_3$ | H | CO 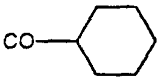 | H | $OCH_3$ | $CH_3$ | N | |
| 294 | $CH_3$ | H | CO 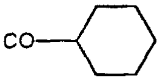 | H | $OCH_3$ | $CF_3$ | N | |
| 295 | $CH_3$ | H | CO 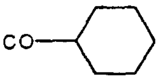 | $CH_3$ | $OCH_3$ | $OCH_3$ | CH | |
| 296 | $CH_3$ | H | CO 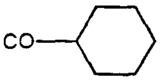 | $CH_3$ | $OCH_3$ | $CH_3$ | N | |
| 297 | $CH_3$ | H | $CF_3SO_2$ | H | $OCH_3$ | $OCH_3$ | CH | |
| 298 | $CH_3$ | H | $CF_3SO_2$ | H | $OCH_3$ | $CH_3$ | CH | |
| 299 | $CH_3$ | H | $CF_3SO_2$ | H | $CH_3$ | $CH_3$ | CH | |
| 300 | $CH_3$ | H | $CF_3SO_2$ | H | Cl | $OCH_3$ | CH | |
| 301 | $CH_3$ | H | $CF_3SO_2$ | H | $OCH_3$ | $OCH_3$ | N | |
| 302 | $CH_3$ | H | $CF_3SO_2$ | H | $OCH_3$ | $CH_3$ | N | |
| 303 | $CH_3$ | H | $CF_3SO_2$ | H | $OCH_3$ | $CF_3$ | N | |
| 304 | $CH_3$ | H | $CF_3SO_2$ | $CH_3$ | $OCH_3$ | $OCH_3$ | CH | |
| 305 | $CH_3$ | H | $CF_3SO_2$ | $CH_3$ | $OCH_3$ | $CH_3$ | N | |
| 306 | $CH_3$ | H | $FCH_2SO_2$ | H | $OCH_3$ | $OCH_3$ | CH | |
| 307 | $CH_3$ | H | $FCH_2SO_2$ | H | $OCH_3$ | $CH_3$ | CH | |
| 308 | $CH_3$ | H | $FCH_2SO_2$ | H | $CH_3$ | $CH_3$ | CH | |
| 309 | $CH_3$ | H | $FCH_2SO_2$ | H | Cl | $OCH_3$ | CH | |
| 310 | $CH_3$ | H | $FCH_2SO_2$ | H | $OCH_3$ | $OCH_3$ | N | |
| 311 | $CH_3$ | H | $FCH_2SO_2$ | H | $OCH_3$ | $CH_3$ | N | |
| 312 | $CH_3$ | H | $FCH_2SO_2$ | H | $OCH_3$ | $CF_3$ | N | |
| 313 | $CH_3$ | H | $FCH_2SO_2$ | $CH_3$ | $OCH_3$ | $OCH_3$ | CH | |
| 314 | $CH_3$ | H | $FCH_2SO_2$ | $CH_3$ | $OCH_3$ | $CH_3$ | N | |
| 315 | $CH_3$ | H | $ClCH_2SO_2$ | H | $OCH_3$ | $OCH_3$ | CH | |
| 316 | $CH_3$ | H | $ClCH_2SO_2$ | H | $OCH_3$ | $CH_3$ | CH | |
| 317 | $CH_3$ | H | $ClCH_2SO_2$ | H | $CH_3$ | $CH_3$ | CH | |
| 318 | $CH_3$ | H | $ClCH_2SO_2$ | H | Cl | $OCH_3$ | CH | |
| 319 | $CH_3$ | H | $ClCH_2SO_2$ | H | $OCH_3$ | $OCH_3$ | N | |

| Nr. | $R^1$ | $R^4$ | $R^5$ | $R^7$ | X | Y | Z | Fp. (°C) |
|---|---|---|---|---|---|---|---|---|
| 320 | $CH_3$ | H | $ClCH_2SO_2$ | H | $OCH_3$ | $CH_3$ | N | |
| 321 | $CH_3$ | H | $ClCH_2SO_2$ | H | $OCH_3$ | $CF_3$ | N | |
| 322 | $CH_3$ | H | $ClCH_2SO_2$ | $CH_3$ | $OCH_3$ | $OCH_3$ | CH | |
| 323 | $CH_3$ | H | $ClCH_2SO_2$ | $CH_3$ | $OCH_3$ | $CH_3$ | N | |
| 324 | $CH_3$ | H | $Cl_2CHSO_2$ | H | $OCH_3$ | $OCH_3$ | CH | |
| 325 | $CH_3$ | H | $Cl_2CHSO_2$ | H | $OCH_3$ | $CH_3$ | CH | |
| 326 | $CH_3$ | H | $Cl_2CHSO_2$ | H | $CH_3$ | $CH_3$ | CH | |
| 327 | $CH_3$ | H | $Cl_2CHSO_2$ | H | Cl | $OCH_3$ | CH | |
| 328 | $CH_3$ | H | $Cl_2CHSO_2$ | H | $OCH_3$ | $OCH_3$ | N | |
| 329 | $CH_3$ | H | $Cl_2CHSO_2$ | H | $OCH_3$ | $CH_3$ | N | |
| 330 | $CH_3$ | H | $Cl_2CHSO_2$ | H | $OCH_3$ | $CF_3$ | N | |
| 331 | $CH_3$ | H | $Cl_2CHSO_2$ | $CH_3$ | $OCH_3$ | $OCH_3$ | CH | |
| 332 | $CH_3$ | H | $Cl_2CHSO_2$ | $CH_3$ | $OCH_3$ | $CH_3$ | N | |
| 333 | $CH_3$ | H | $Cl_3CSO_2$ | H | $OCH_3$ | $OCH_3$ | CH | |
| 334 | $CH_3$ | H | $Cl_3CSO_2$ | H | $OCH_3$ | $CH_3$ | CH | |
| 335 | $CH_3$ | H | $Cl_3CSO_2$ | H | $CH_3$ | $CH_3$ | CH | |
| 336 | $CH_3$ | H | $Cl_3CSO_2$ | H | Cl | $OCH_3$ | CH | |
| 337 | $CH_3$ | H | $Cl_3CSO_2$ | H | $OCH_3$ | $OCH_3$ | N | |
| 338 | $CH_3$ | H | $Cl_3CSO_2$ | H | $OCH_3$ | $CH_3$ | N | |
| 339 | $CH_3$ | H | $Cl_3CSO_2$ | H | $OCH_3$ | $CF_3$ | N | |
| 340 | $CH_3$ | H | $Cl_3CSO_2$ | $CH_3$ | $OCH_3$ | $OCH_3$ | CH | |
| 341 | $CH_3$ | H | $Cl_3CSO_2$ | $CH_3$ | $OCH_3$ | $CH_3$ | N | |
| 342 | $CH_3$ | H | $nC_4H_9SO_2$ | H | $OCH_3$ | $OCH_3$ | CH | |
| 343 | $CH_3$ | H | $nC_4H_9SO_2$ | H | $OCH_3$ | $CH_3$ | CH | |
| 344 | $CH_3$ | H | $nC_4H_9SO_2$ | H | $CH_3$ | $CH_3$ | CH | |
| 345 | $CH_3$ | H | $nC_4H_9SO_2$ | H | Cl | $OCH_3$ | CH | |
| 346 | $CH_3$ | H | $nC_4H_9SO_2$ | H | $OCH_3$ | $OCH_3$ | N | |
| 347 | $CH_3$ | H | $nC_4H_9SO_2$ | H | $OCH_3$ | $CH_3$ | N | |
| 348 | $CH_3$ | H | $nC_4H_9SO_2$ | H | $OCH_3$ | $CF_3$ | N | |
| 349 | $CH_3$ | H | $nC_4H_9SO_2$ | $CH_3$ | $OCH_3$ | $OCH_3$ | CH | |
| 350 | $CH_3$ | H | $nC_4H_9SO_2$ | $CH_3$ | $OCH_3$ | $CH_3$ | N | |
| 351 | $CH_3$ | H | $CF_3CH_2SO_2$ | H | $OCH_3$ | $OCH_3$ | CH | |
| 352 | $CH_3$ | H | $CF_3CH_2SO_2$ | H | $OCH_3$ | $CH_3$ | CH | |
| 353 | $CH_3$ | H | $CF_3CH_2SO_2$ | H | $CH_3$ | $CH_3$ | CH | |

| Nr. | $R^1$ | $R^4$ | $R^5$ | $R^7$ | X | Y | Z | Fp. (°C) |
|---|---|---|---|---|---|---|---|---|
| 354 | $CH_3$ | H | $CF_3CH_2SO_2$ | H | Cl | $OCH_3$ | CH | |
| 355 | $CH_3$ | H | $CF_3CH_2SO_2$ | H | $OCH_3$ | $OCH_3$ | N | |
| 356 | $CH_3$ | H | $CF_3CH_2SO_2$ | H | $OCH_3$ | $CH_3$ | N | |
| 357 | $CH_3$ | H | $CF_3CH_2SO_2$ | H | $OCH_3$ | $CF_3$ | N | |
| 358 | $CH_3$ | H | $CF_3CH_2SO_2$ | $CH_3$ | $OCH_3$ | $OCH_3$ | CH | |
| 359 | $CH_3$ | H | $CF_3CH_2SO_2$ | $CH_3$ | $OCH_3$ | $CH_3$ | N | |
| 360 | $CH_3$ | H | $CH_3NHSO_2$ | H | $OCH_3$ | $OCH_3$ | CH | |
| 361 | $CH_3$ | H | $CH_3NHSO_2$ | H | $OCH_3$ | $CH_3$ | CH | |
| 362 | $CH_3$ | H | $CH_3NHSO_2$ | H | $CH_3$ | $CH_3$ | CH | |
| 363 | $CH_3$ | H | $CH_3NHSO_2$ | H | Cl | $OCH_3$ | CH | |
| 364 | $CH_3$ | H | $CH_3NHSO_2$ | H | $OCH_3$ | $OCH_3$ | N | |
| 365 | $CH_3$ | H | $CH_3NHSO_2$ | H | $OCH_3$ | $CH_3$ | N | |
| 366 | $CH_3$ | H | $CH_3NHSO_2$ | H | $OCH_3$ | $CF_3$ | N | |
| 367 | $CH_3$ | H | $CH_3NHSO_2$ | $CH_3$ | $OCH_3$ | $OCH_3$ | CH | |
| 368 | $CH_3$ | H | $CH_3NHSO_2$ | $CH_3$ | $OCH_3$ | $CH_3$ | N | |
| 369 | $CH_3$ | H | $Me_2NSO_2$ | H | $OCH_3$ | $OCH_3$ | CH | |
| 370 | $CH_3$ | H | $Me_2NSO_2$ | H | $OCH_3$ | $CH_3$ | CH | |
| 371 | $CH_3$ | H | $Me_2NSO_2$ | H | $CH_3$ | $CH_3$ | CH | |
| 372 | $CH_3$ | H | $Me_2NSO_2$ | H | Cl | $OCH_3$ | CH | |
| 373 | $CH_3$ | H | $Me_2NSO_2$ | H | $OCH_3$ | $OCH_3$ | N | |
| 374 | $CH_3$ | H | $Me_2NSO_2$ | H | $OCH_3$ | $CH_3$ | N | |
| 375 | $CH_3$ | H | $Me_2NSO_2$ | H | $OCH_3$ | $CF_3$ | N | |
| 376 | $CH_3$ | H | $Me_2NSO_2$ | $CH_3$ | $OCH_3$ | $OCH_3$ | CH | |
| 377 | $CH_3$ | H | $Me_2NSO_2$ | $CH_3$ | $OCH_3$ | $CH_3$ | N | |
| 378 | $CH_3$ | H | $Me_3C$-OCO | H | $OCH_3$ | $OCH_3$ | CH | |
| 379 | $CH_3$ | H | $Me_3C$-OCO | H | $OCH_3$ | $CH_3$ | CH | |
| 380 | $CH_3$ | H | $Me_3C$-OCO | H | $CH_3$ | $CH_3$ | CH | |
| 381 | $CH_3$ | H | $Me_3C$-OCO | H | Cl | $OCH_3$ | CH | |
| 382 | $CH_3$ | H | $Me_3C$-OCO | H | $OCH_3$ | $OCH_3$ | N | |
| 383 | $CH_3$ | H | $Me_3C$-OCO | H | $OCH_3$ | $CH_3$ | N | |
| 384 | $CH_3$ | H | $Me_3C$-OCO | H | $OCH_3$ | $CF_3$ | N | |
| 385 | $CH_3$ | H | $Me_3C$-OCO | $CH_3$ | $OCH_3$ | $OCH_3$ | CH | |
| 386 | $CH_3$ | H | $Me_3C$-OCO | $CH_3$ | $OCH_3$ | $CH_3$ | N | |
| 387 | $CH_3$ | H | PhCO | H | $OCH_3$ | $OCH_3$ | CH | |

| Nr. | $R^1$ | $R^4$ | $R^5$ | $R^7$ | X | Y | Z | Fp. (°C) |
|---|---|---|---|---|---|---|---|---|
| 388 | $CH_3$ | H | PhCO | H | $OCH_3$ | $CH_3$ | CH | |
| 389 | $CH_3$ | H | PhCO | H | $CH_3$ | $CH_3$ | CH | |
| 390 | $CH_3$ | H | PhCO | H | Cl | $OCH_3$ | CH | |
| 391 | $CH_3$ | H | PhCO | H | $OCH_3$ | $OCH_3$ | N | |
| 392 | $CH_3$ | H | PhCO | H | $OCH_3$ | $CH_3$ | N | |
| 393 | $CH_3$ | H | PhCO | H | $OCH_3$ | $CF_3$ | N | |
| 394 | $CH_3$ | H | PhCO | $CH_3$ | $OCH_3$ | $OCH_3$ | CH | |
| 395 | $CH_3$ | H | PhCO | $CH_3$ | $OCH_3$ | $CH_3$ | N | |
| 396 | $CH_3$ | H | $PhSO_2$ | H | $OCH_3$ | $OCH_3$ | CH | |
| 397 | $CH_3$ | H | $PhSO_2$ | H | $OCH_3$ | $CH_3$ | CH | |
| 398 | $CH_3$ | H | $PhSO_2$ | H | $CH_3$ | $CH_3$ | CH | |
| 399 | $CH_3$ | H | $PhSO_2$ | H | Cl | $OCH_3$ | CH | |
| 400 | $CH_3$ | H | $PhSO_2$ | H | $OCH_3$ | $OCH_3$ | N | |
| 401 | $CH_3$ | H | $PhSO_2$ | H | $OCH_3$ | $CH_3$ | N | |
| 402 | $CH_3$ | H | $PhSO_2$ | H | $OCH_3$ | $CF_3$ | N | |
| 403 | $CH_3$ | H | $PhSO_2$ | $CH_3$ | $OCH_3$ | $OCH_3$ | CH | |
| 404 | $CH_3$ | H | $PhSO_2$ | $CH_3$ | $OCH_3$ | $CH_3$ | N | |
| 405 | $CH_3$ | H | MeNHCO | H | $OCH_3$ | $OCH_3$ | CH | |
| 406 | $CH_3$ | H | MeNHCO | H | $OCH_3$ | $CH_3$ | CH | |
| 407 | $CH_3$ | H | MeNHCO | H | $CH_3$ | $CH_3$ | CH | |
| 408 | $CH_3$ | H | MeNHCO | H | Cl | $OCH_3$ | CH | |
| 409 | $CH_3$ | H | MeNHCO | H | $OCH_3$ | $OCH_3$ | N | |
| 410 | $CH_3$ | H | MeNHCO | H | $OCH_3$ | $CH_3$ | N | |
| 411 | $CH_3$ | H | MeNHCO | H | $OCH_3$ | $CF_3$ | N | |
| 412 | $CH_3$ | H | MeNHCO | $CH_3$ | $OCH_3$ | $OCH_3$ | CH | |
| 413 | $CH_3$ | H | MeNHCO | $CH_3$ | $OCH_3$ | $CH_3$ | N | |
| 414 | $CH_3$ | H | EtNHCO | H | $OCH_3$ | $OCH_3$ | CH | |
| 415 | $CH_3$ | H | EtNHCO | H | $OCH_3$ | $CH_3$ | CH | |
| 416 | $CH_3$ | H | EtNHCO | H | $CH_3$ | $CH_3$ | CH | |
| 417 | $CH_3$ | H | EtNHCO | H | Cl | $OCH_3$ | CH | |
| 418 | $CH_3$ | H | EtNHCO | H | $OCH_3$ | $OCH_3$ | N | |
| 419 | $CH_3$ | H | EtNHCO | H | $OCH_3$ | $CH_3$ | N | |
| 420 | $CH_3$ | H | EtNHCO | H | $OCH_3$ | $CF_3$ | N | |
| 421 | $CH_3$ | H | EtNHCO | $CH_3$ | $OCH_3$ | $OCH_3$ | CH | |

| Nr. | $R^1$ | $R^4$ | $R^5$ | $R^7$ | X | Y | Z | Fp. (°C) |
|---|---|---|---|---|---|---|---|---|
| 422 | $CH_3$ | H | EtNHCO | $CH_3$ | $OCH_3$ | $CH_3$ | N | |
| 423 | $CH_3$ | H | MeNHCS | H | $OCH_3$ | $OCH_3$ | CH | |
| 424 | $CH_3$ | H | MeNHCS | H | $OCH_3$ | $CH_3$ | CH | |
| 425 | $CH_3$ | H | MeNHCS | H | $CH_3$ | $CH_3$ | CH | |
| 426 | $CH_3$ | H | MeNHCS | H | Cl | $OCH_3$ | CH | |
| 427 | $CH_3$ | H | MeNHCS | H | $OCH_3$ | $OCH_3$ | N | |
| 428 | $CH_3$ | H | MeNHCS | H | $OCH_3$ | $CH_3$ | N | |
| 429 | $CH_3$ | H | MeNHCS | H | $OCH_3$ | $CF_3$ | N | |
| 430 | $CH_3$ | H | MeNHCS | $CH_3$ | $OCH_3$ | $OCH_3$ | CH | |
| 431 | $CH_3$ | H | MeNHCS | $CH_3$ | $OCH_3$ | $CH_3$ | N | |
| 432 | $CH_3$ | H | EtNHCS | H | $OCH_3$ | $OCH_3$ | CH | |
| 433 | $CH_3$ | H | EtNHCS | H | $OCH_3$ | $CH_3$ | CH | |
| 434 | $CH_3$ | H | EtNHCS | H | $CH_3$ | $CH_3$ | CH | |
| 435 | $CH_3$ | H | EtNHCS | H | Cl | $OCH_3$ | CH | |
| 436 | $CH_3$ | H | EtNHCS | H | $OCH_3$ | $OCH_3$ | N | |
| 437 | $CH_3$ | H | EtNHCS | H | $OCH_3$ | $CH_3$ | N | |
| 438 | $CH_3$ | H | EtNHCS | H | $OCH_3$ | $CF_3$ | N | |
| 439 | $CH_3$ | H | EtNHCS | $CH_3$ | $OCH_3$ | $OCH_3$ | CH | |
| 440 | $CH_3$ | H | EtNHCS | $CH_3$ | $OCH_3$ | $CH_3$ | N | |
| 441 | $CH_3$ | 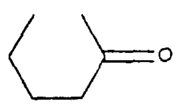 | | H | $OCH_3$ | $OCH_3$ | CH | |
| 442 | $CH_3$ | 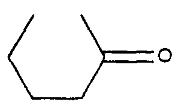 | | H | $OCH_3$ | $CH_3$ | CH | |
| 443 | $CH_3$ | 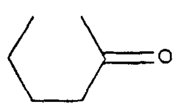 | | H | $CH_3$ | $CH_3$ | CH | |
| 444 | $CH_3$ | 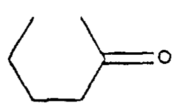 | | H | Cl | $OCH_3$ | CH | |

| Nr. | $R^1$ | $R^4$ | $R^5$ | $R^7$ | X | Y | Z | Fp. (°C) |
|---|---|---|---|---|---|---|---|---|
| 445 | $CH_3$ | 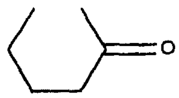 | | H | $OCH_3$ | $OCH_3$ | N | |
| 446 | $CH_3$ | 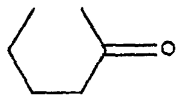 | | H | $OCH_3$ | $CH_3$ | N | |
| 447 | $CH_3$ | 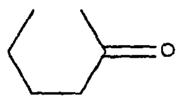 | | H | $OCH_3$ | $CF_3$ | N | |
| 448 | $CH_3$ | 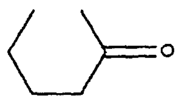 | | $CH_3$ | $OCH_3$ | $OCH_3$ | CH | |
| 449 | $CH_3$ | 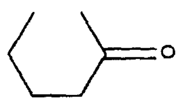 | | $CH_3$ | $OCH_3$ | $CH_3$ | N | |
| 450 | $CH_3$ | | 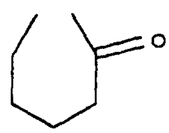 | H | $OCH_3$ | $OCH_3$ | CH | |
| 451 | $CH_3$ | | 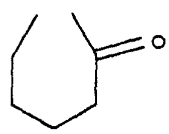 | H | $OCH_3$ | $CH_3$ | CH | |
| 452 | $CH_3$ | | 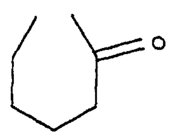 | H | $CH_3$ | $CH_3$ | CH | |
| 453 | $CH_3$ | | 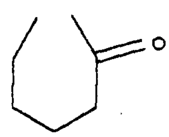 | H | Cl | $OCH_3$ | CH | |
| 454 | $CH_3$ | | 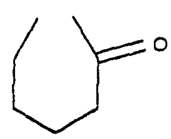 | H | $OCH_3$ | $OCH_3$ | N | |

| Nr. | $R^1$ | $R^4$ | $R^5$ | $R^7$ | X | Y | Z | Fp. (°C) |
|---|---|---|---|---|---|---|---|---|
| 455 | $CH_3$ | | 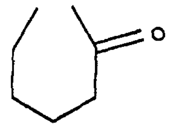 | H | $OCH_3$ | $CH_3$ | N | |
| 456 | $CH_3$ | | 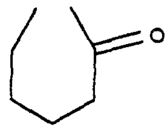 | H | $OCH_3$ | $CF_3$ | N | |
| 457 | $CH_3$ | | 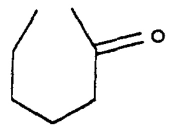 | $CH_3$ | $OCH_3$ | $OCH_3$ | CH | |
| 458 | $CH_3$ | | 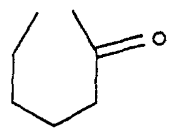 | $CH_3$ | $OCH_3$ | $CH_3$ | N | |
| 459 | $CH_3$ | | 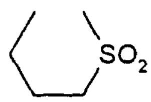 | H | $OCH_3$ | $OCH_3$ | CH | |
| 460 | $CH_3$ | | 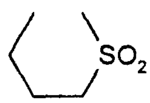 | H | $OCH_3$ | $CH_3$ | CH | |
| 461 | $CH_3$ | | 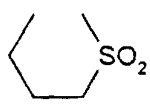 | H | $CH_3$ | $CH_3$ | CH | |
| 462 | $CH_3$ | | 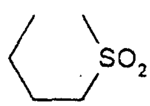 | H | Cl | $OCH_3$ | CH | |
| 463 | $CH_3$ | | 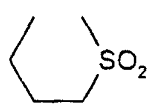 | H | $OCH_3$ | $OCH_3$ | N | |
| 464 | $CH_3$ | | 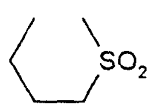 | H | $OCH_3$ | $CH_3$ | N | |
| 465 | $CH_3$ | | 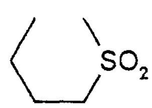 | H | $OCH_3$ | $CF_3$ | N | |

| Nr. | $R^1$ | $R^4$ | $R^5$ | $R^7$ | X | Y | Z | Fp. (°C) |
|---|---|---|---|---|---|---|---|---|
| 466 | $CH_3$ | | 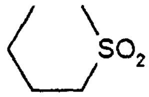 | $CH_3$ | $OCH_3$ | $OCH_3$ | CH | |
| 467 | $CH_3$ | | 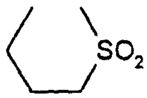 | $CH_3$ | $OCH_3$ | $CH_3$ | N | |
| 468 | $CH_3$ | | 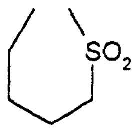 | H | $OCH_3$ | $OCH_3$ | CH | |
| 469 | $CH_3$ | | 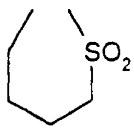 | H | $OCH_3$ | $CH_3$ | CH | |
| 470 | $CH_3$ | | 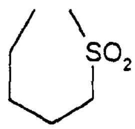 | H | $CH_3$ | $CH_3$ | CH | |
| 471 | $CH_3$ | | 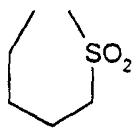 | H | Cl | $OCH_3$ | CH | |
| 472 | $CH_3$ | | 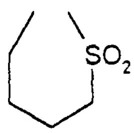 | H | $OCH_3$ | $OCH_3$ | N | |
| 473 | $CH_3$ | | 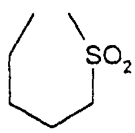 | H | $OCH_3$ | $CH_3$ | N | |
| 474 | $CH_3$ | | 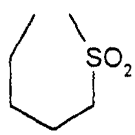 | H | $OCH_3$ | $CF_3$ | N | |
| 475 | $CH_3$ | | 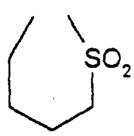 | $CH_3$ | $OCH_3$ | $OCH_3$ | CH | |

| Nr. | $R^1$ | $R^4$ | $R^5$ | $R^7$ | X | Y | Z | Fp. (°C) |
|---|---|---|---|---|---|---|---|---|
| 476 | $CH_3$ | | 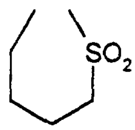 | $CH_3$ | $OCH_3$ | $CH_3$ | N | |
| 477 | $CH_3$ | | 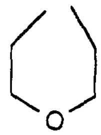 | H | $OCH_3$ | $OCH_3$ | CH | |
| 478 | $CH_3$ | | 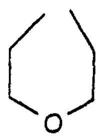 | H | $OCH_3$ | $CH_3$ | CH | |
| 479 | $CH_3$ | | 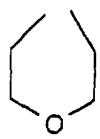 | H | $CH_3$ | $CH_3$ | CH | |
| 480 | $CH_3$ | | 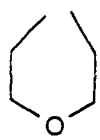 | H | Cl | $OCH_3$ | CH | |
| 481 | $CH_3$ | | 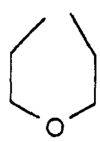 | H | $OCH_3$ | $OCH_3$ | N | |
| 482 | $CH_3$ | | 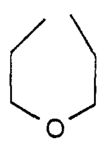 | H | $OCH_3$ | $CH_3$ | N | |
| 483 | $CH_3$ | | 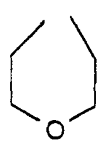 | H | $OCH_3$ | $CF_3$ | N | |
| 484 | $CH_3$ | | 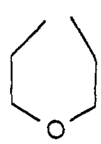 | $CH_3$ | $OCH_3$ | $OCH_3$ | CH | |
| 485 | $CH_3$ | | 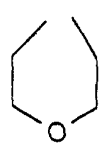 | $CH_3$ | $OCH_3$ | $CH_3$ | N | |

| Nr. | $R^1$ | $R^4$ | $R^5$ | $R^7$ | X | Y | Z | Fp. (°C) |
|---|---|---|---|---|---|---|---|---|
| 486 | $CH_3$ | H | $COSCH_3$ | H | $OCH_3$ | $OCH_3$ | CH | |
| 487 | $CH_3$ | H | $COSCH_3$ | H | $OCH_3$ | $CH_3$ | CH | |
| 488 | $CH_3$ | H | $COSCH_3$ | H | $CH_3$ | $CH_3$ | CH | |
| 489 | $CH_3$ | H | $COSCH_3$ | H | Cl | $OCH_3$ | CH | |
| 490 | $CH_3$ | H | $COSCH_3$ | H | $OCH_3$ | $OCH_3$ | N | |
| 491 | $CH_3$ | H | $COSCH_3$ | H | $OCH_3$ | $CH_3$ | N | |
| 492 | $CH_3$ | H | $COSCH_3$ | H | $OCH_3$ | $CF_3$ | N | |
| 493 | $CH_3$ | H | $COSCH_3$ | $CH_3$ | $OCH_3$ | $OCH_3$ | CH | |
| 494 | $CH_3$ | H | $COSCH_3$ | $CH_3$ | $OCH_3$ | $CH_3$ | N | |
| 495 | $CH_3$ | H | $CSOCH_3$ | H | $OCH_3$ | $OCH_3$ | CH | |
| 496 | $CH_3$ | H | $CSOCH_3$ | H | $OCH_3$ | $CH_3$ | CH | |
| 497 | $CH_3$ | H | $CSOCH_3$ | H | $CH_3$ | $CH_3$ | CH | |
| 498 | $CH_3$ | H | $CSOCH_3$ | H | Cl | $OCH_3$ | CH | |
| 499 | $CH_3$ | H | $CSOCH_3$ | H | $OCH_3$ | $OCH_3$ | N | |
| 500 | $CH_3$ | H | $CSOCH_3$ | H | $OCH_3$ | $CH_3$ | N | |
| 501 | $CH_3$ | H | $CSOCH_3$ | H | $OCH_3$ | $CF_3$ | N | |
| 502 | $CH_3$ | H | $CSOCH_3$ | $CH_3$ | $OCH_3$ | $OCH_3$ | CH | |
| 503 | $CH_3$ | H | $CSOCH_3$ | $CH_3$ | $OCH_3$ | $CH_3$ | N | |
| 504 | $CH_3$ | H | $CSSCH_3$ | H | $OCH_3$ | $OCH_3$ | CH | |
| 505 | $CH_3$ | H | $CSSCH_3$ | H | $OCH_3$ | $CH_3$ | CH | |
| 506 | $CH_3$ | H | $CSSCH_3$ | H | $CH_3$ | $CH_3$ | CH | |
| 507 | $CH_3$ | H | $CSSCH_3$ | H | Cl | $OCH_3$ | CH | |
| 508 | $CH_3$ | H | $CSSCH_3$ | H | $OCH_3$ | $OCH_3$ | N | |
| 509 | $CH_3$ | H | $CSSCH_3$ | H | $OCH_3$ | $CH_3$ | N | |
| 510 | $CH_3$ | H | $CSSCH_3$ | H | $OCH_3$ | $CF_3$ | N | |
| 511 | $CH_3$ | H | $CSSCH_3$ | $CH_3$ | $OCH_3$ | $OCH_3$ | CH | |
| 512 | $CH_3$ | H | $CSSCH_3$ | $CH_3$ | $OCH_3$ | $CH_3$ | N | |
| 513 | $CH_3$ | H | $COCOOCH_3$ | H | $OCH_3$ | $OCH_3$ | CH | |
| 514 | $CH_3$ | H | $COCOOCH_3$ | H | $OCH_3$ | $CH_3$ | CH | |
| 515 | $CH_3$ | H | $COCOOCH_3$ | H | $CH_3$ | $CH_3$ | CH | |
| 516 | $CH_3$ | H | $COCOOCH_3$ | H | Cl | $OCH_3$ | CH | |
| 517 | $CH_3$ | H | $COCOOCH_3$ | H | $OCH_3$ | $OCH_3$ | N | |
| 518 | $CH_3$ | H | $COCOOCH_3$ | H | $OCH_3$ | $CH_3$ | N | |
| 519 | $CH_3$ | H | $COCOOCH_3$ | H | $OCH_3$ | $CF_3$ | N | |

| Nr. | $R^1$ | $R^4$ | $R^5$ | $R^7$ | X | Y | Z | Fp. (°C) |
|---|---|---|---|---|---|---|---|---|
| 520 | $CH_3$ | H | $COCOOCH_3$ | $CH_3$ | $OCH_3$ | $OCH_3$ | CH | |
| 521 | $CH_3$ | H | $COCOOCH_3$ | $CH_3$ | $OCH_3$ | $CH_3$ | N | |
| 522 | $CH_3$ | H | $i\text{-}C_3H_7OCO$ | H | $OCH_3$ | $OCH_3$ | CH | |
| 523 | $CH_3$ | H | $i\text{-}C_3H_7OCO$ | H | $OCH_3$ | $CH_3$ | CH | |
| 524 | $CH_3$ | H | $i\text{-}C_3H_7OCO$ | H | $CH_3$ | $CH_3$ | CH | |
| 525 | $CH_3$ | H | $i\text{-}C_3H_7OCO$ | H | Cl | $OCH_3$ | CH | |
| 526 | $CH_3$ | H | $i\text{-}C_3H_7OCO$ | H | $OCH_3$ | $OCH_3$ | N | |
| 527 | $CH_3$ | H | $i\text{-}C_3H_7OCO$ | H | $OCH_3$ | $CH_3$ | N | |
| 528 | $CH_3$ | H | $i\text{-}C_3H_7OCO$ | H | $OCH_3$ | $CF_3$ | N | |
| 529 | $CH_3$ | H | $i\text{-}C_3H_7OCO$ | $CH_3$ | $OCH_3$ | $OCH_3$ | CH | |
| 530 | $CH_3$ | H | $i\text{-}C_3H_7OCO$ | $CH_3$ | $OCH_3$ | $CH_3$ | N | |
| 531 | $CH_3$ | $CH_3$ | CHO | H | $OCH_3$ | $OCH_3$ | CH | 180-182 |
| 532 | $CH_3$ | $CH_3$ | CHO | H | $OCH_3$ | $CH_3$ | CH | |
| 533 | $CH_3$ | $CH_3$ | CHO | H | $CH_3$ | $CH_3$ | CH | |
| 534 | $CH_3$ | $CH_3$ | CHO | H | $CH_3$ | $OC_2H_5$ | CH | |
| 535 | $CH_3$ | $CH_3$ | CHO | H | $C_2H_5$ | $OCH_3$ | CH | |
| 536 | $CH_3$ | $CH_3$ | CHO | H | $OC_2H_5$ | $OC_2H_5$ | CH | |
| 537 | $CH_3$ | $CH_3$ | CHO | H | $OCHF_2$ | $OCHF_2$ | CH | |
| 538 | $CH_3$ | $CH_3$ | CHO | H | $CH_3$ | $OCHF_2$ | CH | |
| 539 | $CH_3$ | $CH_3$ | CHO | H | Cl | $OCH_3$ | CH | |
| 540 | $CH_3$ | $CH_3$ | CHO | H | $OCH_3$ | $OCH_3$ | N | |
| 541 | $CH_3$ | $CH_3$ | CHO | H | $OCH_3$ | $CH_3$ | N | |
| 542 | $CH_3$ | $CH_3$ | CHO | H | $CH_3$ | $CH_3$ | N | |
| 543 | $CH_3$ | $CH_3$ | CHO | H | $N(CH_3)_2$ | $OCH_2CF_3$ | N | |
| 544 | $CH_3$ | $CH_3$ | CHO | H | $OCH_3$ | $CF_3$ | N | |
| 545 | $CH_3$ | $CH_3$ | CHO | H | $OC_2H_5$ | $NHCH_3$ | N | |
| 546 | $CH_3$ | $CH_3$ | CHO | $CH_3$ | $OCH_3$ | $OCH_3$ | CH | |
| 547 | $CH_3$ | $CH_3$ | CHO | $CH_3$ | $OCH_3$ | $CH_3$ | N | |
| 548 | $CH_3$ | $CH_3$ | $COCH_3$ | H | $OCH_3$ | $OCH_3$ | CH | 133-136 |
| 549 | $CH_3$ | $CH_3$ | $COCH_3$ | H | $OCH_3$ | $CH_3$ | CH | |
| 550 | $CH_3$ | $CH_3$ | $COCH_3$ | H | $CH_3$ | $CH_3$ | CH | |
| 551 | $CH_3$ | $CH_3$ | $COCH_3$ | H | $CH_3$ | $OC_2H_5$ | CH | |
| 552 | $CH_3$ | $CH_3$ | $COCH_3$ | H | $C_2H_5$ | $OCH_3$ | CH | |
| 553 | $CH_3$ | $CH_3$ | $COCH_3$ | H | $OC_2H_5$ | $OC_2H_5$ | CH | |

| Nr. | $R^1$ | $R^4$ | $R^5$ | $R^7$ | X | Y | Z | Fp. (°C) |
|---|---|---|---|---|---|---|---|---|
| 554 | $CH_3$ | $CH_3$ | $COCH_3$ | H | $OCHF_2$ | $OCHF_2$ | CH | |
| 555 | $CH_3$ | $CH_3$ | $COCH_3$ | H | $CH_3$ | $OCHF_2$ | CH | |
| 556 | $CH_3$ | $CH_3$ | $COCH_3$ | H | Cl | $OCH_3$ | CH | |
| 557 | $CH_3$ | $CH_3$ | $COCH_3$ | H | $OCH_3$ | $OCH_3$ | N | |
| 558 | $CH_3$ | $CH_3$ | $COCH_3$ | H | $OCH_3$ | $CH_3$ | N | |
| 559 | $CH_3$ | $CH_3$ | $COCH_3$ | H | $CH_3$ | $CH_3$ | N | |
| 560 | $CH_3$ | $CH_3$ | $COCH_3$ | H | $N(CH_3)_2$ | $OCH_2CF_3$ | N | |
| 561 | $CH_3$ | $CH_3$ | $COCH_3$ | H | $OCH_3$ | $CF_3$ | N | |
| 562 | $CH_3$ | $CH_3$ | $COCH_3$ | H | $OC_2H_5$ | $NHCH_3$ | N | |
| 563 | $CH_3$ | $CH_3$ | $COCH_3$ | $CH_3$ | $OCH_3$ | $OCH_3$ | CH | |
| 564 | $CH_3$ | $CH_3$ | $COCH_3$ | $CH_3$ | $OCH_3$ | $CH_3$ | N | |
| 565 | $CH_3$ | $CH_3$ | $COC_2H_5$ | H | $OCH_3$ | $OCH_3$ | CH | 182-185 |
| 566 | $CH_3$ | $CH_3$ | $COC_2H_5$ | H | $OCH_3$ | $CH_3$ | CH | |
| 567 | $CH_3$ | $CH_3$ | $COC_2H_5$ | H | $CH_3$ | $CH_3$ | CH | |
| 568 | $CH_3$ | $CH_3$ | $COC_2H_5$ | H | $CH_3$ | $OC_2H_5$ | CH | |
| 569 | $CH_3$ | $CH_3$ | $COC_2H_5$ | H | $C_2H_5$ | $OCH_3$ | CH | |
| 570 | $CH_3$ | $CH_3$ | $COC_2H_5$ | H | $OC_2H_5$ | $OC_2H_5$ | CH | |
| 571 | $CH_3$ | $CH_3$ | $COC_2H_5$ | H | $OCHF_2$ | $OCHF_2$ | CH | |
| 572 | $CH_3$ | $CH_3$ | $COC_2H_5$ | H | $CH_3$ | $OCHF_2$ | CH | |
| 573 | $CH_3$ | $CH_3$ | $COC_2H_5$ | H | Cl | $OCH_3$ | CH | |
| 574 | $CH_3$ | $CH_3$ | $COC_2H_5$ | H | $OCH_3$ | $OCH_3$ | N | |
| 575 | $CH_3$ | $CH_3$ | $COC_2H_5$ | H | $OCH_3$ | $CH_3$ | N | |
| 576 | $CH_3$ | $CH_3$ | $COC_2H_5$ | H | $CH_3$ | $CH_3$ | N | |
| 577 | $CH_3$ | $CH_3$ | $COC_2H_5$ | H | $N(CH_3)_2$ | $OCH_2CF_3$ | N | |
| 578 | $CH_3$ | $CH_3$ | $COC_2H_5$ | H | $OCH_3$ | $CF_3$ | N | |
| 579 | $CH_3$ | $CH_3$ | $COC_2H_5$ | H | $OC_2H_5$ | $NHCH_3$ | N | |
| 580 | $CH_3$ | $CH_3$ | $COC_2H_5$ | $CH_3$ | $OCH_3$ | $OCH_3$ | CH | |
| 581 | $CH_3$ | $CH_3$ | $COC_2H_5$ | $CH_3$ | $OCH_3$ | $CH_3$ | N | |
| 582 | $CH_3$ | $CH_3$ | $CO\text{-}n\text{-}C_3H_7$ | H | $OCH_3$ | $OCH_3$ | CH | |
| 583 | $CH_3$ | $CH_3$ | $CO\text{-}n\text{-}C_3H_7$ | H | $OCH_3$ | $CH_3$ | CH | |
| 584 | $CH_3$ | $CH_3$ | $CO\text{-}n\text{-}C_3H_7$ | H | $CH_3$ | $CH_3$ | CH | |
| 585 | $CH_3$ | $CH_3$ | $CO\text{-}n\text{-}C_3H_7$ | H | $CH_3$ | $OC_2H_5$ | CH | |
| 586 | $CH_3$ | $CH_3$ | $CO\text{-}n\text{-}C_3H_7$ | H | $C_2H_5$ | $OCH_3$ | CH | |
| 587 | $CH_3$ | $CH_3$ | $CO\text{-}n\text{-}C_3H_7$ | H | $OC_2H_5$ | $OC_2H_5$ | CH | |

| Nr. | $R^1$ | $R^4$ | $R^5$ | $R^7$ | X | Y | Z | Fp. (°C) |
|---|---|---|---|---|---|---|---|---|
| 588 | $CH_3$ | $CH_3$ | CO-n-$C_3H_7$ | H | $OCHF_2$ | $OCHF_2$ | CH | |
| 589 | $CH_3$ | $CH_3$ | CO-n-$C_3H_7$ | H | $CH_3$ | $OCHF_2$ | CH | |
| 590 | $CH_3$ | $CH_3$ | CO-n-$C_3H_7$ | H | Cl | $OCH_3$ | CH | |
| 591 | $CH_3$ | $CH_3$ | CO-n-$C_3H_7$ | H | $OCH_3$ | $OCH_3$ | N | |
| 592 | $CH_3$ | $CH_3$ | CO-n-$C_3H_7$ | H | $OCH_3$ | $CH_3$ | N | |
| 593 | $CH_3$ | $CH_3$ | CO-n-$C_3H_7$ | H | $CH_3$ | $CH_3$ | N | |
| 594 | $CH_3$ | $CH_3$ | CO-n-$C_3H_7$ | H | $N(CH_3)_2$ | $OCH_2CF_3$ | N | |
| 595 | $CH_3$ | $CH_3$ | CO-n-$C_3H_7$ | H | $OCH_3$ | $CF_3$ | N | |
| 596 | $CH_3$ | $CH_3$ | CO-n-$C_3H_7$ | H | $OC_2H_5$ | $NHCH_3$ | N | |
| 597 | $CH_3$ | $CH_3$ | CO-n-$C_3H_7$ | $CH_3$ | $OCH_3$ | $OCH_3$ | CH | |
| 598 | $CH_3$ | $CH_3$ | CO-n-$C_3H_7$ | $CH_3$ | $OCH_3$ | $CH_3$ | N | |
| 599 | $CH_3$ | $CH_3$ | CO-i-$C_3H_7$ | H | $OCH_3$ | $OCH_3$ | CH | 186-188 |
| 600 | $CH_3$ | $CH_3$ | CO-i-$C_3H_7$ | H | $OCH_3$ | $CH_3$ | CH | |
| 601 | $CH_3$ | $CH_3$ | CO-i-$C_3H_7$ | H | $CH_3$ | $CH_3$ | CH | |
| 602 | $CH_3$ | $CH_3$ | CO-i-$C_3H_7$ | H | $CH_3$ | $OC_2H_5$ | CH | |
| 603 | $CH_3$ | $CH_3$ | CO-i-$C_3H_7$ | H | $C_2H_5$ | $OCH_3$ | CH | |
| 604 | $CH_3$ | $CH_3$ | CO-i-$C_3H_7$ | H | $OC_2H_5$ | $OC_2H_5$ | CH | |
| 605 | $CH_3$ | $CH_3$ | CO-i-$C_3H_7$ | H | $OCHF_2$ | $OCHF_2$ | CH | |
| 606 | $CH_3$ | $CH_3$ | CO-i-$C_3H_7$ | H | $CH_3$ | $OCHF_2$ | CH | |
| 607 | $CH_3$ | $CH_3$ | CO-i-$C_3H_7$ | H | Cl | $OCH_3$ | CH | |
| 608 | $CH_3$ | $CH_3$ | CO-i-$C_3H_7$ | H | $OCH_3$ | $OCH_3$ | N | |
| 609 | $CH_3$ | $CH_3$ | CO-i-$C_3H_7$ | H | $OCH_3$ | $CH_3$ | N | |
| 610 | $CH_3$ | $CH_3$ | CO-i-$C_3H_7$ | H | $CH_3$ | $CH_3$ | N | |
| 611 | $CH_3$ | $CH_3$ | CO-i-$C_3H_7$ | H | $N(CH_3)_2$ | $OCH_2CF_3$ | N | |
| 612 | $CH_3$ | $CH_3$ | CO-i-$C_3H_7$ | H | $OCH_3$ | $CF_3$ | N | |
| 613 | $CH_3$ | $CH_3$ | CO-i-$C_3H_7$ | H | $OC_2H_5$ | $NHCH_3$ | N | |
| 614 | $CH_3$ | $CH_3$ | CO-i-$C_3H_7$ | $CH_3$ | $OCH_3$ | $OCH_3$ | CH | |
| 615 | $CH_3$ | $CH_3$ | CO-i-$C_3H_7$ | $CH_3$ | $OCH_3$ | $CH_3$ | N | |
| 616 | $CH_3$ | $CH_3$ | $COCF_3$ | H | $OCH_3$ | $OCH_3$ | CH | 133-136 |
| 617 | $CH_3$ | $CH_3$ | $COCF_3$ | H | $OCH_3$ | $CH_3$ | CH | |
| 618 | $CH_3$ | $CH_3$ | $COCF_3$ | H | $CH_3$ | $CH_3$ | CH | |
| 619 | $CH_3$ | $CH_3$ | $COCF_3$ | H | $CH_3$ | $OC_2H_5$ | CH | |
| 620 | $CH_3$ | $CH_3$ | $COCF_3$ | H | $C_2H_5$ | $OCH_3$ | CH | |
| 621 | $CH_3$ | $CH_3$ | $COCF_3$ | H | $OC_2H_5$ | $OC_2H_5$ | CH | |

| Nr. | $R^1$ | $R^4$ | $R^5$ | $R^7$ | X | Y | Z | Fp. (°C) |
|---|---|---|---|---|---|---|---|---|
| 622 | $CH_3$ | $CH_3$ | $COCF_3$ | H | $OCHF_2$ | $OCHF_2$ | CH | |
| 623 | $CH_3$ | $CH_3$ | $COCF_3$ | H | $CH_3$ | $OCHF_2$ | CH | |
| 624 | $CH_3$ | $CH_3$ | $COCF_3$ | H | Cl | $OCH_3$ | CH | |
| 625 | $CH_3$ | $CH_3$ | $COCF_3$ | H | $OCH_3$ | $OCH_3$ | N | |
| 626 | $CH_3$ | $CH_3$ | $COCF_3$ | H | $OCH_3$ | $CH_3$ | N | |
| 627 | $CH_3$ | $CH_3$ | $COCF_3$ | H | $CH_3$ | $CH_3$ | N | |
| 628 | $CH_3$ | $CH_3$ | $COCF_3$ | H | $N(CH_3)_2$ | $OCH_2CF_3$ | N | |
| 629 | $CH_3$ | $CH_3$ | $COCF_3$ | H | $OCH_3$ | $CF_3$ | N | |
| 630 | $CH_3$ | $CH_3$ | $COCF_3$ | H | $OC_2H_5$ | $NHCH_3$ | N | |
| 631 | $CH_3$ | $CH_3$ | $COCF_3$ | $CH_3$ | $OCH_3$ | $OCH_3$ | CH | |
| 632 | $CH_3$ | $CH_3$ | $COCF_3$ | $CH_3$ | $OCH_3$ | $CH_3$ | N | |
| 633 | $CH_3$ | $CH_3$ | $COOCH_3$ | H | $OCH_3$ | $OCH_3$ | CH | 105-111 |
| 634 | $CH_3$ | $CH_3$ | $COOCH_3$ | H | $OCH_3$ | $CH_3$ | CH | |
| 635 | $CH_3$ | $CH_3$ | $COOCH_3$ | H | $CH_3$ | $CH_3$ | CH | |
| 636 | $CH_3$ | $CH_3$ | $COOCH_3$ | H | $CH_3$ | $OC_2H_5$ | CH | |
| 637 | $CH_3$ | $CH_3$ | $COOCH_3$ | H | $C_2H_5$ | $OCH_3$ | CH | |
| 638 | $CH_3$ | $CH_3$ | $COOCH_3$ | H | $OC_2H_5$ | $OC_2H_5$ | CH | |
| 639 | $CH_3$ | $CH_3$ | $COOCH_3$ | H | $OCHF_2$ | $OCHF_2$ | CH | |
| 640 | $CH_3$ | $CH_3$ | $COOCH_3$ | H | $CH_3$ | $OCHF_2$ | CH | |
| 641 | $CH_3$ | $CH_3$ | $COOCH_3$ | H | Cl | $OCH_3$ | CH | |
| 642 | $CH_3$ | $CH_3$ | $COOCH_3$ | H | $OCH_3$ | $OCH_3$ | N | |
| 643 | $CH_3$ | $CH_3$ | $COOCH_3$ | H | $OCH_3$ | $CH_3$ | N | |
| 644 | $CH_3$ | $CH_3$ | $COOCH_3$ | H | $CH_3$ | $CH_3$ | N | |
| 645 | $CH_3$ | $CH_3$ | $COOCH_3$ | H | $N(CH_3)_2$ | $OCH_2CF_3$ | N | |
| 646 | $CH_3$ | $CH_3$ | $COOCH_3$ | H | $OCH_3$ | $CF_3$ | N | |
| 647 | $CH_3$ | $CH_3$ | $COOCH_3$ | H | $OC_2H_5$ | $NHCH_3$ | N | |
| 648 | $CH_3$ | $CH_3$ | $COOCH_3$ | $CH_3$ | $OCH_3$ | $OCH_3$ | CH | |
| 649 | $CH_3$ | $CH_3$ | $COOCH_3$ | $CH_3$ | $OCH_3$ | $CH_3$ | N | |
| 650 | $CH_3$ | $CH_3$ | $COOC_2H_5$ | H | $OCH_3$ | $OCH_3$ | CH | 227-229 |
| 651 | $CH_3$ | $CH_3$ | $COOC_2H_5$ | H | $OCH_3$ | $CH_3$ | CH | |
| 652 | $CH_3$ | $CH_3$ | $COOC_2H_5$ | H | $CH_3$ | $CH_3$ | CH | |
| 653 | $CH_3$ | $CH_3$ | $COOC_2H_5$ | H | $CH_3$ | $OC_2H_5$ | CH | |
| 654 | $CH_3$ | $CH_3$ | $COOC_2H_5$ | H | $C_2H_5$ | $OCH_3$ | CH | |
| 655 | $CH_3$ | $CH_3$ | $COOC_2H_5$ | H | $OC_2H_5$ | $OC_2H_5$ | CH | |

| Nr. | $R^1$ | $R^4$ | $R^5$ | $R^7$ | X | Y | Z | Fp. (°C) |
|---|---|---|---|---|---|---|---|---|
| 656 | $CH_3$ | $CH_3$ | $COOC_2H_5$ | H | $OCHF_2$ | $OCHF_2$ | CH | |
| 657 | $CH_3$ | $CH_3$ | $COOC_2H_5$ | H | $CH_3$ | $OCHF_2$ | CH | |
| 658 | $CH_3$ | $CH_3$ | $COOC_2H_5$ | H | Cl | $OCH_3$ | CH | |
| 659 | $CH_3$ | $CH_3$ | $COOC_2H_5$ | H | $OCH_3$ | $OCH_3$ | N | |
| 660 | $CH_3$ | $CH_3$ | $COOC_2H_5$ | H | $OCH_3$ | $CH_3$ | N | |
| 661 | $CH_3$ | $CH_3$ | $COOC_2H_5$ | H | $CH_3$ | $CH_3$ | N | |
| 662 | $CH_3$ | $CH_3$ | $COOC_2H_5$ | H | $N(CH_3)_2$ | $OCH_2CF_3$ | N | |
| 663 | $CH_3$ | $CH_3$ | $COOC_2H_5$ | H | $OCH_3$ | $CF_3$ | N | |
| 664 | $CH_3$ | $CH_3$ | $COOC_2H_5$ | H | $OC_2H_5$ | $NHCH_3$ | N | |
| 665 | $CH_3$ | $CH_3$ | $COOC_2H_5$ | $CH_3$ | $OCH_3$ | $OCH_3$ | CH | |
| 666 | $CH_3$ | $CH_3$ | $COOC_2H_5$ | $CH_3$ | $OCH_3$ | $CH_3$ | N | |
| 667 | $CH_3$ | $CH_3$ | $SO_2CH_3$ | H | $OCH_3$ | $OCH_3$ | CH | 205-210 |
| 668 | $CH_3$ | $CH_3$ | $SO_2CH_3$ | H | $OCH_3$ | $CH_3$ | CH | |
| 669 | $CH_3$ | $CH_3$ | $SO_2CH_3$ | H | $CH_3$ | $CH_3$ | CH | |
| 670 | $CH_3$ | $CH_3$ | $SO_2CH_3$ | H | $CH_3$ | $OC_2H_5$ | CH | |
| 671 | $CH_3$ | $CH_3$ | $SO_2CH_3$ | H | $C_2H_5$ | $OCH_3$ | CH | |
| 672 | $CH_3$ | $CH_3$ | $SO_2CH_3$ | H | $OC_2H_5$ | $OC_2H_5$ | CH | |
| 673 | $CH_3$ | $CH_3$ | $SO_2CH_3$ | H | $OCHF_2$ | $OCHF_2$ | CH | |
| 674 | $CH_3$ | $CH_3$ | $SO_2CH_3$ | H | $CH_3$ | $OCHF_2$ | CH | |
| 675 | $CH_3$ | $CH_3$ | $SO_2CH_3$ | H | Cl | $OCH_3$ | CH | |
| 676 | $CH_3$ | $CH_3$ | $SO_2CH_3$ | H | $OCH_3$ | $OCH_3$ | N | |
| 677 | $CH_3$ | $CH_3$ | $SO_2CH_3$ | H | $OCH_3$ | $CH_3$ | N | |
| 678 | $CH_3$ | $CH_3$ | $SO_2CH_3$ | H | $CH_3$ | $CH_3$ | N | |
| 679 | $CH_3$ | $CH_3$ | $SO_2CH_3$ | H | $N(CH_3)_2$ | $OCH_2CF_3$ | N | |
| 680 | $CH_3$ | $CH_3$ | $SO_2CH_3$ | H | $OCH_3$ | $CF_3$ | N | |
| 681 | $CH_3$ | $CH_3$ | $SO_2CH_3$ | H | $OC_2H_5$ | $NHCH_3$ | N | |
| 682 | $CH_3$ | $CH_3$ | $SO_2CH_3$ | $CH_3$ | $OCH_3$ | $OCH_3$ | CH | |
| 683 | $CH_3$ | $CH_3$ | $SO_2CH_3$ | $CH_3$ | $OCH_3$ | $CH_3$ | N | |
| 684 | $CH_3$ | $CH_3$ | $SO_2C_2H_5$ | H | $OCH_3$ | $OCH_3$ | CH | 206-208 |
| 685 | $CH_3$ | $CH_3$ | $SO_2C_2H_5$ | H | $OCH_3$ | $CH_3$ | CH | |
| 686 | $CH_3$ | $CH_3$ | $SO_2C_2H_5$ | H | $CH_3$ | $CH_3$ | CH | |
| 687 | $CH_3$ | $CH_3$ | $SO_2C_2H_5$ | H | $CH_3$ | $OC_2H_5$ | CH | |
| 688 | $CH_3$ | $CH_3$ | $SO_2C_2H_5$ | H | $C_2H_5$ | $OCH_3$ | CH | |
| 689 | $CH_3$ | $CH_3$ | $SO_2C_2H_5$ | H | $OC_2H_5$ | $OC_2H_5$ | CH | |

| Nr. | $R^1$ | $R^4$ | $R^5$ | $R^7$ | X | Y | Z | Fp. (°C) |
|---|---|---|---|---|---|---|---|---|
| 690 | $CH_3$ | $CH_3$ | $SO_2C_2H_5$ | H | $OCHF_2$ | $OCHF_2$ | CH | |
| 691 | $CH_3$ | $CH_3$ | $SO_2C_2H_5$ | H | $CH_3$ | $OCHF_2$ | CH | |
| 692 | $CH_3$ | $CH_3$ | $SO_2C_2H_5$ | H | Cl | $OCH_3$ | CH | |
| 693 | $CH_3$ | $CH_3$ | $SO_2C_2H_5$ | H | $OCH_3$ | $OCH_3$ | N | |
| 694 | $CH_3$ | $CH_3$ | $SO_2C_2H_5$ | H | $OCH_3$ | $CH_3$ | N | |
| 695 | $CH_3$ | $CH_3$ | $SO_2C_2H_5$ | H | $CH_3$ | $CH_3$ | N | |
| 696 | $CH_3$ | $CH_3$ | $SO_2C_2H_5$ | H | $N(CH_3)_2$ | $OCH_2CF_3$ | N | |
| 697 | $CH_3$ | $CH_3$ | $SO_2C_2H_5$ | H | $OCH_3$ | $CF_3$ | N | |
| 698 | $CH_3$ | $CH_3$ | $SO_2C_2H_5$ | H | $OC_2H_5$ | $NHCH_3$ | N | |
| 699 | $CH_3$ | $CH_3$ | $SO_2C_2H_5$ | $CH_3$ | $OCH_3$ | $OCH_3$ | CH | |
| 700 | $CH_3$ | $CH_3$ | $SO_2C_2H_5$ | $CH_3$ | $OCH_3$ | $CH_3$ | N | |
| 701 | $CH_3$ | $CH_3$ | $SO_2$-n-$C_3H_7$ | H | $OCH_3$ | $OCH_3$ | CH | |
| 702 | $CH_3$ | $CH_3$ | $SO_2$-n-$C_3H_7$ | H | $OCH_3$ | $CH_3$ | CH | |
| 703 | $CH_3$ | $CH_3$ | $SO_2$-n-$C_3H_7$ | H | $CH_3$ | $CH_3$ | CH | |
| 704 | $CH_3$ | $CH_3$ | $SO_2$-n-$C_3H_7$ | H | $CH_3$ | $OC_2H_5$ | CH | |
| 705 | $CH_3$ | $CH_3$ | $SO_2$-n-$C_3H_7$ | H | $C_2H_5$ | $OCH_3$ | CH | |
| 706 | $CH_3$ | $CH_3$ | $SO_2$-n-$C_3H_7$ | H | $OC_2H_5$ | $OC_2H_5$ | CH | |
| 707 | $CH_3$ | $CH_3$ | $SO_2$-n-$C_3H_7$ | H | $OCHF_2$ | $OCHF_2$ | CH | |
| 708 | $CH_3$ | $CH_3$ | $SO_2$-n-$C_3H_7$ | H | $CH_3$ | $OCHF_2$ | CH | |
| 709 | $CH_3$ | $CH_3$ | $SO_2$-n-$C_3H_7$ | H | Cl | $OCH_3$ | CH | |
| 710 | $CH_3$ | $CH_3$ | $SO_2$-n-$C_3H_7$ | H | $OCH_3$ | $OCH_3$ | N | |
| 711 | $CH_3$ | $CH_3$ | $SO_2$-n-$C_3H_7$ | H | $OCH_3$ | $CH_3$ | N | |
| 712 | $CH_3$ | $CH_3$ | $SO_2$-n-$C_3H_7$ | H | $CH_3$ | $CH_3$ | N | |
| 713 | $CH_3$ | $CH_3$ | $SO_2$-n-$C_3H_7$ | H | $N(CH_3)_2$ | $OCH_2CF_3$ | N | |
| 714 | $CH_3$ | $CH_3$ | $SO_2$-n-$C_3H_7$ | H | $OCH_3$ | $CF_3$ | N | |
| 715 | $CH_3$ | $CH_3$ | $SO_2$-n-$C_3H_7$ | H | $OC_2H_5$ | $NHCH_3$ | N | |
| 716 | $CH_3$ | $CH_3$ | $SO_2$-n-$C_3H_7$ | $CH_3$ | $OCH_3$ | $OCH_3$ | CH | |
| 717 | $CH_3$ | $CH_3$ | $SO_2$-n-$C_3H_7$ | $CH_3$ | $OCH_3$ | $CH_3$ | N | |
| 718 | $CH_3$ | $CH_3$ | $SO_2$-i-$C_3H_7$ | H | $OCH_3$ | $OCH_3$ | CH | |
| 719 | $CH_3$ | $CH_3$ | $SO_2$-i-$C_3H_7$ | H | $OCH_3$ | $CH_3$ | CH | |
| 720 | $CH_3$ | $CH_3$ | $SO_2$-i-$C_3H_7$ | H | $CH_3$ | $CH_3$ | CH | |
| 721 | $CH_3$ | $CH_3$ | $SO_2$-i-$C_3H_7$ | H | $CH_3$ | $OC_2H_5$ | CH | |
| 722 | $CH_3$ | $CH_3$ | $SO_2$-i-$C_3H_7$ | H | $C_2H_5$ | $OCH_3$ | CH | |
| 723 | $CH_3$ | $CH_3$ | $SO_2$-i-$C_3H_7$ | H | $OC_2H_5$ | $OC_2H_5$ | CH | |

| Nr. | $R^1$ | $R^4$ | $R^5$ | $R^7$ | X | Y | Z | Fp. (°C) |
|---|---|---|---|---|---|---|---|---|
| 724 | $CH_3$ | $CH_3$ | $SO_2$-i-$C_3H_7$ | H | $OCHF_2$ | $OCHF_2$ | CH | |
| 725 | $CH_3$ | $CH_3$ | $SO_2$-i-$C_3H_7$ | H | $CH_3$ | $OCHF_2$ | CH | |
| 726 | $CH_3$ | $CH_3$ | $SO_2$-i-$C_3H_7$ | H | Cl | $OCH_3$ | CH | |
| 727 | $CH_3$ | $CH_3$ | $SO_2$-i-$C_3H_7$ | H | $OCH_3$ | $OCH_3$ | N | |
| 728 | $CH_3$ | $CH_3$ | $SO_2$-i-$C_3H_7$ | H | $OCH_3$ | $CH_3$ | N | |
| 729 | $CH_3$ | $CH_3$ | $SO_2$-i-$C_3H_7$ | H | $CH_3$ | $CH_3$ | N | |
| 730 | $CH_3$ | $CH_3$ | $SO_2$-i-$C_3H_7$ | H | $N(CH_3)_2$ | $OCH_2CF_3$ | N | |
| 731 | $CH_3$ | $CH_3$ | $SO_2$-i-$C_3H_7$ | H | $OCH_3$ | $CF_3$ | N | |
| 732 | $CH_3$ | $CH_3$ | $SO_2$-i-$C_3H_7$ | H | $OC_2H_5$ | $NHCH_3$ | N | |
| 733 | $CH_3$ | $CH_3$ | $SO_2$-i-$C_3H_7$ | $CH_3$ | $OCH_3$ | $OCH_3$ | CH | |
| 734 | $CH_3$ | $CH_3$ | $SO_2$-i-$C_3H_7$ | $CH_3$ | $OCH_3$ | $CH_3$ | N | |
| 735 | $CH_3$ | $CH_3$ | $ClCH_2CO$ | H | $OCH_3$ | $OCH_3$ | CH | |
| 736 | $CH_3$ | $CH_3$ | $ClCH_2CO$ | H | $OCH_3$ | $CH_3$ | CH | |
| 737 | $CH_3$ | $CH_3$ | $ClCH_2CO$ | H | $CH_3$ | $CH_3$ | CH | |
| 738 | $CH_3$ | $CH_3$ | $ClCH_2CO$ | H | Cl | $OCH_3$ | CH | |
| 739 | $CH_3$ | $CH_3$ | $ClCH_2CO$ | H | $OCH_3$ | $OCH_3$ | N | |
| 740 | $CH_3$ | $CH_3$ | $ClCH_2CO$ | H | $OCH_3$ | $CH_3$ | N | |
| 741 | $CH_3$ | $CH_3$ | $ClCH_2CO$ | H | $OCH_3$ | $CF_3$ | N | |
| 742 | $CH_3$ | $CH_3$ | $ClCH_2CO$ | $CH_3$ | $OCH_3$ | $OCH_3$ | CH | |
| 743 | $CH_3$ | $CH_3$ | $ClCH_2CO$ | $CH_3$ | $OCH_3$ | $CH_3$ | N | |
| 744 | $CH_3$ | $CH_3$ | $Cl_2CHCO$ | H | $OCH_3$ | $OCH_3$ | CH | |
| 745 | $CH_3$ | $CH_3$ | $Cl_2CHCO$ | H | $OCH_3$ | $CH_3$ | CH | |
| 746 | $CH_3$ | $CH_3$ | $Cl_2CHCO$ | H | $CH_3$ | $CH_3$ | CH | |
| 747 | $CH_3$ | $CH_3$ | $Cl_2CHCO$ | H | Cl | $OCH_3$ | CH | |
| 748 | $CH_3$ | $CH_3$ | $Cl_2CHCO$ | H | $OCH_3$ | $OCH_3$ | N | |
| 749 | $CH_3$ | $CH_3$ | $Cl_2CHCO$ | H | $OCH_3$ | $CH_3$ | N | |
| 750 | $CH_3$ | $CH_3$ | $Cl_2CHCO$ | H | $OCH_3$ | $CF_3$ | N | |
| 751 | $CH_3$ | $CH_3$ | $Cl_2CHCO$ | $CH_3$ | $OCH_3$ | $OCH_3$ | CH | |
| 752 | $CH_3$ | $CH_3$ | $Cl_2CHCO$ | $CH_3$ | $OCH_3$ | $CH_3$ | N | |
| 753 | $CH_3$ | $CH_3$ | $Cl_3CCO$ | H | $OCH_3$ | $OCH_3$ | CH | 144-146 |
| 754 | $CH_3$ | $CH_3$ | $Cl_3CCO$ | H | $OCH_3$ | $CH_3$ | CH | |
| 755 | $CH_3$ | $CH_3$ | $Cl_3CCO$ | H | $CH_3$ | $CH_3$ | CH | |
| 756 | $CH_3$ | $CH_3$ | $Cl_3CCO$ | H | Cl | $OCH_3$ | CH | |
| 757 | $CH_3$ | $CH_3$ | $Cl_3CCO$ | H | $OCH_3$ | $OCH_3$ | N | |

| Nr. | $R^1$ | $R^4$ | $R^5$ | $R^7$ | X | Y | Z | Fp. (°C) |
|---|---|---|---|---|---|---|---|---|
| 758 | $CH_3$ | $CH_3$ | $Cl_3CCO$ | H | $OCH_3$ | $CH_3$ | N | |
| 759 | $CH_3$ | $CH_3$ | $Cl_3CCO$ | H | $OCH_3$ | $CF_3$ | N | |
| 760 | $CH_3$ | $CH_3$ | $Cl_3CCO$ | $CH_3$ | $OCH_3$ | $OCH_3$ | CH | |
| 761 | $CH_3$ | $CH_3$ | $Cl_3CCO$ | $CH_3$ | $OCH_3$ | $CH_3$ | N | |
| 762 | $CH_3$ | $CH_3$ | $CH_3OCH_2CO$ | H | $OCH_3$ | $OCH_3$ | CH | |
| 763 | $CH_3$ | $CH_3$ | $CH_3OCH_2CO$ | H | $OCH_3$ | $CH_3$ | CH | |
| 764 | $CH_3$ | $CH_3$ | $CH_3OCH_2CO$ | H | $CH_3$ | $CH_3$ | CH | |
| 765 | $CH_3$ | $CH_3$ | $CH_3OCH_2CO$ | H | Cl | $OCH_3$ | CH | |
| 766 | $CH_3$ | $CH_3$ | $CH_3OCH_2CO$ | H | $OCH_3$ | $OCH_3$ | N | |
| 767 | $CH_3$ | $CH_3$ | $CH_3OCH_2CO$ | H | $OCH_3$ | $CH_3$ | N | |
| 768 | $CH_3$ | $CH_3$ | $CH_3OCH_2CO$ | H | $OCH_3$ | $CF_3$ | N | |
| 769 | $CH_3$ | $CH_3$ | $CH_3OCH_2CO$ | $CH_3$ | $OCH_3$ | $OCH_3$ | CH | |
| 770 | $CH_3$ | $CH_3$ | $CH_3OCH_2CO$ | $CH_3$ | $OCH_3$ | $CH_3$ | N | |
| 771 | $CH_3$ | $CH_3$ | $CH_2{=}CHCO$ | H | $OCH_3$ | $OCH_3$ | CH | |
| 772 | $CH_3$ | $CH_3$ | $CH_2{=}CHCO$ | H | $OCH_3$ | $CH_3$ | CH | |
| 773 | $CH_3$ | $CH_3$ | $CH_2{=}CHCO$ | H | $CH_3$ | $CH_3$ | CH | |
| 774 | $CH_3$ | $CH_3$ | $CH_2{=}CHCO$ | H | Cl | $OCH_3$ | CH | |
| 775 | $CH_3$ | $CH_3$ | $CH_2{=}CHCO$ | H | $OCH_3$ | $OCH_3$ | N | |
| 776 | $CH_3$ | $CH_3$ | $CH_2{=}CHCO$ | H | $OCH_3$ | $CH_3$ | N | |
| 777 | $CH_3$ | $CH_3$ | $CH_2{=}CHCO$ | H | $OCH_3$ | $CF_3$ | N | |
| 778 | $CH_3$ | $CH_3$ | $CH_2{=}CHCO$ | $CH_3$ | $OCH_3$ | $OCH_3$ | CH | |
| 779 | $CH_3$ | $CH_3$ | $CH_2{=}CHCO$ | $CH_3$ | $OCH_3$ | $CH_3$ | N | |
| 780 | $CH_3$ | $CH_3$ | $CH{\equiv}CCO$ | H | $OCH_3$ | $OCH_3$ | CH | |
| 781 | $CH_3$ | $CH_3$ | $CH{\equiv}CCO$ | H | $OCH_3$ | $CH_3$ | CH | |
| 782 | $CH_3$ | $CH_3$ | $CH{\equiv}CCO$ | H | $CH_3$ | $CH_3$ | CH | |
| 783 | $CH_3$ | $CH_3$ | $CH{\equiv}CCO$ | H | Cl | $OCH_3$ | CH | |
| 784 | $CH_3$ | $CH_3$ | $CH{\equiv}CCO$ | H | $OCH_3$ | $OCH_3$ | N | |
| 785 | $CH_3$ | $CH_3$ | $CH{\equiv}CCO$ | H | $OCH_3$ | $CH_3$ | N | |
| 786 | $CH_3$ | $CH_3$ | $CH{\equiv}CCO$ | H | $OCH_3$ | $CF_3$ | N | |
| 787 | $CH_3$ | $CH_3$ | $CH{\equiv}CCO$ | $CH_3$ | $OCH_3$ | $OCH_3$ | CH | |
| 788 | $CH_3$ | $CH_3$ | $CH{\equiv}CCO$ | $CH_3$ | $OCH_3$ | $CH_3$ | N | |
| 789 | $CH_3$ | $CH_3$ | CO 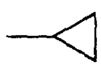 | H | $OCH_3$ | $OCH_3$ | CH | |

| Nr. | $R^1$ | $R^4$ | $R^5$ | $R^7$ | X | Y | Z | Fp. (°C) |
|---|---|---|---|---|---|---|---|---|
| 790 | $CH_3$ | $CH_3$ | CO 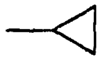 | H | $OCH_3$ | $CH_3$ | CH | |
| 791 | $CH_3$ | $CH_3$ | CO 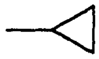 | H | $CH_3$ | $CH_3$ | CH | |
| 792 | $CH_3$ | $CH_3$ | CO 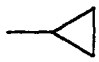 | H | Cl | $OCH_3$ | CH | |
| 793 | $CH_3$ | $CH_3$ | CO 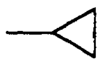 | H | $OCH_3$ | $OCH_3$ | N | |
| 794 | $CH_3$ | $CH_3$ | CO 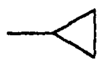 | H | $OCH_3$ | $CH_3$ | N | |
| 795 | $CH_3$ | $CH_3$ | CO 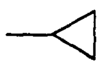 | H | $OCH_3$ | $CF_3$ | N | |
| 796 | $CH_3$ | $CH_3$ | CO 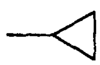 | $CH_3$ | $OCH_3$ | $OCH_3$ | CH | |
| 797 | $CH_3$ | $CH_3$ | CO 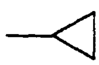 | $CH_3$ | $OCH_3$ | $CH_3$ | N | |
| 798 | $CH_3$ | $CH_3$ | CO 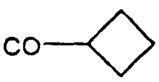 | H | $OCH_3$ | $OCH_3$ | CH | |
| 799 | $CH_3$ | $CH_3$ | CO 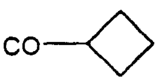 | H | $OCH_3$ | $CH_3$ | CH | |
| 800 | $CH_3$ | $CH_3$ | CO 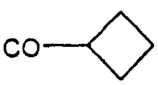 | H | $CH_3$ | $CH_3$ | CH | |
| 801 | $CH_3$ | $CH_3$ | CO 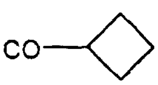 | H | Cl | $OCH_3$ | CH | |
| 802 | $CH_3$ | $CH_3$ | CO 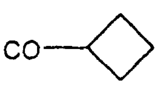 | H | $OCH_3$ | $OCH_3$ | N | |
| 803 | $CH_3$ | $CH_3$ | CO 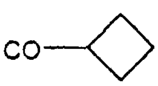 | H | $OCH_3$ | $CH_3$ | N | |
| 804 | $CH_3$ | $CH_3$ | CO 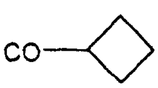 | H | $OCH_3$ | $CF_3$ | N | |
| 805 | $CH_3$ | $CH_3$ | CO 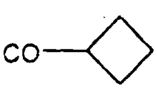 | $CH_3$ | $OCH_3$ | $OCH_3$ | CH | |

| Nr. | $R^1$ | $R^4$ | $R^5$ | $R^7$ | X | Y | Z | Fp. (°C) |
|---|---|---|---|---|---|---|---|---|
| 806 | $CH_3$ | $CH_3$ | 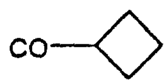 | $CH_3$ | $OCH_3$ | $CH_3$ | N | |
| 807 | $CH_3$ | $CH_3$ | 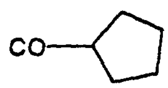 | H | $OCH_3$ | $OCH_3$ | CH | |
| 808 | $CH_3$ | $CH_3$ | 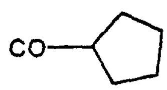 | H | $OCH_3$ | $CH_3$ | CH | |
| 809 | $CH_3$ | $CH_3$ | 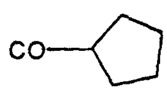 | H | $CH_3$ | $CH_3$ | CH | |
| 810 | $CH_3$ | $CH_3$ | 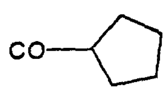 | H | Cl | $OCH_3$ | CH | |
| 811 | $CH_3$ | $CH_3$ | 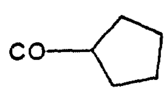 | H | $OCH_3$ | $OCH_3$ | N | |
| 812 | $CH_3$ | $CH_3$ | 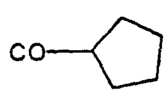 | H | $OCH_3$ | $CH_3$ | N | |
| 813 | $CH_3$ | $CH_3$ | 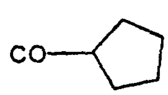 | H | $OCH_3$ | $CF_3$ | N | |
| 814 | $CH_3$ | $CH_3$ | 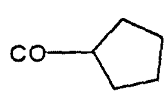 | $CH_3$ | $OCH_3$ | $OCH_3$ | CH | |
| 815 | $CH_3$ | $CH_3$ | 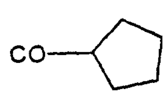 | $CH_3$ | $OCH_3$ | $CH_3$ | N | |
| 816 | $CH_3$ | $CH_3$ | 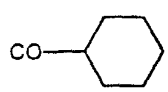 | H | $OCH_3$ | $OCH_3$ | CH | |
| 817 | $CH_3$ | $CH_3$ | 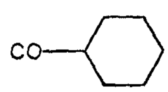 | H | $OCH_3$ | $CH_3$ | CH | |
| 818 | $CH_3$ | $CH_3$ | 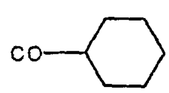 | H | $CH_3$ | $CH_3$ | CH | |
| 819 | $CH_3$ | $CH_3$ | 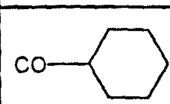 | H | Cl | $OCH_3$ | CH | |

| Nr. | $R^1$ | $R^4$ | $R^5$ | $R^7$ | X | Y | Z | Fp. (°C) |
|---|---|---|---|---|---|---|---|---|
| 820 | $CH_3$ | $CH_3$ | 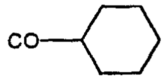 | H | $OCH_3$ | $OCH_3$ | N | |
| 821 | $CH_3$ | $CH_3$ | 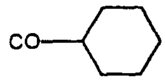 | H | $OCH_3$ | $CH_3$ | N | |
| 822 | $CH_3$ | $CH_3$ | 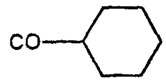 | H | $OCH_3$ | $CF_3$ | N | |
| 823 | $CH_3$ | $CH_3$ | 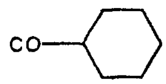 | $CH_3$ | $OCH_3$ | $OCH_3$ | CH | |
| 824 | $CH_3$ | $CH_3$ | 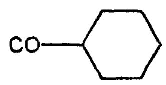 | $CH_3$ | $OCH_3$ | $CH_3$ | N | |
| 825 | $CH_3$ | $CH_3$ | $CF_3SO_2$ | H | $OCH_3$ | $OCH_3$ | CH | |
| 826 | $CH_3$ | $CH_3$ | $CF_3SO_2$ | H | $OCH_3$ | $CH_3$ | CH | |
| 827 | $CH_3$ | $CH_3$ | $CF_3SO_2$ | H | $CH_3$ | $CH_3$ | CH | |
| 828 | $CH_3$ | $CH_3$ | $CF_3SO_2$ | H | Cl | $OCH_3$ | CH | |
| 829 | $CH_3$ | $CH_3$ | $CF_3SO_2$ | H | $OCH_3$ | $OCH_3$ | N | |
| 830 | $CH_3$ | $CH_3$ | $CF_3SO_2$ | H | $OCH_3$ | $CH_3$ | N | |
| 831 | $CH_3$ | $CH_3$ | $CF_3SO_2$ | H | $OCH_3$ | $CF_3$ | N | |
| 832 | $CH_3$ | $CH_3$ | $CF_3SO_2$ | $CH_3$ | $OCH_3$ | $OCH_3$ | CH | |
| 833 | $CH_3$ | $CH_3$ | $CF_3SO_2$ | $CH_3$ | $OCH_3$ | $CH_3$ | N | |
| 834 | $CH_3$ | $CH_3$ | $FCH_2SO_2$ | H | $OCH_3$ | $OCH_3$ | CH | |
| 835 | $CH_3$ | $CH_3$ | $FCH_2SO_2$ | H | $OCH_3$ | $CH_3$ | CH | |
| 836 | $CH_3$ | $CH_3$ | $FCH_2SO_2$ | H | $CH_3$ | $CH_3$ | CH | |
| 837 | $CH_3$ | $CH_3$ | $FCH_2SO_2$ | H | Cl | $OCH_3$ | CH | |
| 838 | $CH_3$ | $CH_3$ | $FCH_2SO_2$ | H | $OCH_3$ | $OCH_3$ | N | |
| 839 | $CH_3$ | $CH_3$ | $FCH_2SO_2$ | H | $OCH_3$ | $CH_3$ | N | |
| 840 | $CH_3$ | $CH_3$ | $FCH_2SO_2$ | H | $OCH_3$ | $CF_3$ | N | |
| 841 | $CH_3$ | $CH_3$ | $FCH_2SO_2$ | $CH_3$ | $OCH_3$ | $OCH_3$ | CH | |
| 842 | $CH_3$ | $CH_3$ | $FCH_2SO_2$ | $CH_3$ | $OCH_3$ | $CH_3$ | N | |
| 843 | $CH_3$ | $CH_3$ | $ClCH_2SO_2$ | H | $OCH_3$ | $OCH_3$ | CH | |
| 844 | $CH_3$ | $CH_3$ | $ClCH_2SO_2$ | H | $OCH_3$ | $CH_3$ | CH | |
| 845 | $CH_3$ | $CH_3$ | $ClCH_2SO_2$ | H | $CH_3$ | $CH_3$ | CH | |
| 846 | $CH_3$ | $CH_3$ | $ClCH_2SO_2$ | H | Cl | $OCH_3$ | CH | |
| 847 | $CH_3$ | $CH_3$ | $ClCH_2SO_2$ | H | $OCH_3$ | $OCH_3$ | N | |

| Nr. | $R^1$ | $R^4$ | $R^5$ | $R^7$ | X | Y | Z | Fp. (°C) |
|---|---|---|---|---|---|---|---|---|
| 848 | $CH_3$ | $CH_3$ | $ClCH_2SO_2$ | H | $OCH_3$ | $CH_3$ | N | |
| 849 | $CH_3$ | $CH_3$ | $ClCH_2SO_2$ | H | $OCH_3$ | $CF_3$ | N | |
| 850 | $CH_3$ | $CH_3$ | $ClCH_2SO_2$ | $CH_3$ | $OCH_3$ | $OCH_3$ | CH | |
| 851 | $CH_3$ | $CH_3$ | $ClCH_2SO_2$ | $CH_3$ | $OCH_3$ | $CH_3$ | N | |
| 852 | $CH_3$ | $CH_3$ | $Cl_2CHSO_2$ | H | $OCH_3$ | $OCH_3$ | CH | |
| 853 | $CH_3$ | $CH_3$ | $Cl_2CHSO_2$ | H | $OCH_3$ | $CH_3$ | CH | |
| 854 | $CH_3$ | $CH_3$ | $Cl_2CHSO_2$ | H | $CH_3$ | $CH_3$ | CH | |
| 855 | $CH_3$ | $CH_3$ | $Cl_2CHSO_2$ | H | Cl | $OCH_3$ | CH | |
| 856 | $CH_3$ | $CH_3$ | $Cl_2CHSO_2$ | H | $OCH_3$ | $OCH_3$ | N | |
| 857 | $CH_3$ | $CH_3$ | $Cl_2CHSO_2$ | H | $OCH_3$ | $CH_3$ | N | |
| 858 | $CH_3$ | $CH_3$ | $Cl_2CHSO_2$ | H | $OCH_3$ | $CF_3$ | N | |
| 859 | $CH_3$ | $CH_3$ | $Cl_2CHSO_2$ | $CH_3$ | $OCH_3$ | $OCH_3$ | CH | |
| 860 | $CH_3$ | $CH_3$ | $Cl_2CHSO_2$ | $CH_3$ | $OCH_3$ | $CH_3$ | N | |
| 861 | $CH_3$ | $CH_3$ | $Cl_3CSO_2$ | H | $OCH_3$ | $OCH_3$ | CH | |
| 862 | $CH_3$ | $CH_3$ | $Cl_3CSO_2$ | H | $OCH_3$ | $CH_3$ | CH | |
| 863 | $CH_3$ | $CH_3$ | $Cl_3CSO_2$ | H | $CH_3$ | $CH_3$ | CH | |
| 864 | $CH_3$ | $CH_3$ | $Cl_3CSO_2$ | H | Cl | $OCH_3$ | CH | |
| 865 | $CH_3$ | $CH_3$ | $Cl_3CSO_2$ | H | $OCH_3$ | $OCH_3$ | N | |
| 866 | $CH_3$ | $CH_3$ | $Cl_3CSO_2$ | H | $OCH_3$ | $CH_3$ | N | |
| 867 | $CH_3$ | $CH_3$ | $Cl_3CSO_2$ | H | $OCH_3$ | $CF_3$ | N | |
| 868 | $CH_3$ | $CH_3$ | $Cl_3CSO_2$ | $CH_3$ | $OCH_3$ | $OCH_3$ | CH | |
| 869 | $CH_3$ | $CH_3$ | $Cl_3CSO_2$ | $CH_3$ | $OCH_3$ | $CH_3$ | N | |
| 870 | $CH_3$ | $CH_3$ | $n\text{-}C_4H_9SO_2$ | H | $OCH_3$ | $OCH_3$ | CH | |
| 871 | $CH_3$ | $CH_3$ | $n\text{-}C_4H_9SO_2$ | H | $OCH_3$ | $CH_3$ | CH | |
| 872 | $CH_3$ | $CH_3$ | $n\text{-}C_4H_9SO_2$ | H | $CH_3$ | $CH_3$ | CH | |
| 873 | $CH_3$ | $CH_3$ | $n\text{-}C_4H_9SO_2$ | H | Cl | $OCH_3$ | CH | |
| 874 | $CH_3$ | $CH_3$ | $n\text{-}C_4H_9SO_2$ | H | $OCH_3$ | $OCH_3$ | N | |
| 875 | $CH_3$ | $CH_3$ | $n\text{-}C_4H_9SO_2$ | H | $OCH_3$ | $CH_3$ | N | |
| 876 | $CH_3$ | $CH_3$ | $n\text{-}C_4H_9SO_2$ | H | $OCH_3$ | $CF_3$ | N | |
| 877 | $CH_3$ | $CH_3$ | $n\text{-}C_4H_9SO_2$ | $CH_3$ | $OCH_3$ | $OCH_3$ | CH | |
| 878 | $CH_3$ | $CH_3$ | $n\text{-}C_4H_9SO_2$ | $CH_3$ | $OCH_3$ | $CH_3$ | N | |
| 879 | $CH_3$ | $CH_3$ | $CF_3CH_2SO_2$ | H | $OCH_3$ | $OCH_3$ | CH | |
| 880 | $CH_3$ | $CH_3$ | $CF_3CH_2SO_2$ | H | $OCH_3$ | $CH_3$ | CH | |
| 881 | $CH_3$ | $CH_3$ | $CF_3CH_2SO_2$ | H | $CH_3$ | $CH_3$ | CH | |

| Nr. | $R^1$ | $R^4$ | $R^5$ | $R^7$ | X | Y | Z | Fp. (°C) |
|---|---|---|---|---|---|---|---|---|
| 882 | $CH_3$ | $CH_3$ | $CF_3CH_2SO_2$ | H | Cl | $OCH_3$ | CH | |
| 883 | $CH_3$ | $CH_3$ | $CF_3CH_2SO_2$ | H | $OCH_3$ | $OCH_3$ | N | |
| 884 | $CH_3$ | $CH_3$ | $CF_3CH_2SO_2$ | H | $OCH_3$ | $CH_3$ | N | |
| 885 | $CH_3$ | $CH_3$ | $CF_3CH_2SO_2$ | H | $OCH_3$ | $CF_3$ | N | |
| 886 | $CH_3$ | $CH_3$ | $CF_3CH_2SO_2$ | $CH_3$ | $OCH_3$ | $OCH_3$ | CH | |
| 887 | $CH_3$ | $CH_3$ | $CF_3CH_2SO_2$ | $CH_3$ | $OCH_3$ | $CH_3$ | N | |
| 888 | $CH_3$ | $CH_3$ | $CH_3NHSO_2$ | H | $OCH_3$ | $OCH_3$ | CH | |
| 889 | $CH_3$ | $CH_3$ | $CH_3NHSO_2$ | H | $OCH_3$ | $CH_3$ | CH | |
| 890 | $CH_3$ | $CH_3$ | $CH_3NHSO_2$ | H | $CH_3$ | $CH_3$ | CH | |
| 891 | $CH_3$ | $CH_3$ | $CH_3NHSO_2$ | H | Cl | $OCH_3$ | CH | |
| 892 | $CH_3$ | $CH_3$ | $CH_3NHSO_2$ | H | $OCH_3$ | $OCH_3$ | N | |
| 893 | $CH_3$ | $CH_3$ | $CH_3NHSO_2$ | H | $OCH_3$ | $CH_3$ | N | |
| 894 | $CH_3$ | $CH_3$ | $CH_3NHSO_2$ | H | $OCH_3$ | $CF_3$ | N | |
| 895 | $CH_3$ | $CH_3$ | $CH_3NHSO_2$ | $CH_3$ | $OCH_3$ | $OCH_3$ | CH | |
| 896 | $CH_3$ | $CH_3$ | $CH_3NHSO_2$ | $CH_3$ | $OCH_3$ | $CH_3$ | N | |
| 897 | $CH_3$ | $CH_3$ | $(CH_3)_2NSO_2$ | H | $OCH_3$ | $OCH_3$ | CH | |
| 898 | $CH_3$ | $CH_3$ | $(CH_3)_2NSO_2$ | H | $OCH_3$ | $CH_3$ | CH | |
| 899 | $CH_3$ | $CH_3$ | $(CH_3)_2NSO_2$ | H | $CH_3$ | $CH_3$ | CH | |
| 900 | $CH_3$ | $CH_3$ | $(CH_3)_2NSO_2$ | H | Cl | $OCH_3$ | CH | |
| 901 | $CH_3$ | $CH_3$ | $(CH_3)_2NSO_2$ | H | $OCH_3$ | $OCH_3$ | N | |
| 902 | $CH_3$ | $CH_3$ | $(CH_3)_2NSO_2$ | H | $OCH_3$ | $CH_3$ | N | |
| 903 | $CH_3$ | $CH_3$ | $(CH_3)_2NSO_2$ | H | $OCH_3$ | $CF_3$ | N | |
| 904 | $CH_3$ | $CH_3$ | $(CH_3)_2NSO_2$ | $CH_3$ | $OCH_3$ | $OCH_3$ | CH | |
| 905 | $CH_3$ | $CH_3$ | $(CH_3)_2NSO_2$ | $CH_3$ | $OCH_3$ | $CH_3$ | N | |
| 906 | $CH_3$ | $CH_3$ | $(CH_3)_3COCO$ | H | $OCH_3$ | $OCH_3$ | CH | |
| 907 | $CH_3$ | $CH_3$ | $(CH_3)_3COCO$ | H | $OCH_3$ | $CH_3$ | CH | |
| 908 | $CH_3$ | $CH_3$ | $(CH_3)_3COCO$ | H | $CH_3$ | $CH_3$ | CH | |
| 909 | $CH_3$ | $CH_3$ | $(CH_3)_3COCO$ | H | Cl | $OCH_3$ | CH | |
| 910 | $CH_3$ | $CH_3$ | $(CH_3)_3COCO$ | H | $OCH_3$ | $OCH_3$ | N | |
| 911 | $CH_3$ | $CH_3$ | $(CH_3)_3COCO$ | H | $OCH_3$ | $CH_3$ | N | |
| 912 | $CH_3$ | $CH_3$ | $(CH_3)_3COCO$ | H | $OCH_3$ | $CF_3$ | N | |
| 913 | $CH_3$ | $CH_3$ | $(CH_3)_3COCO$ | $CH_3$ | $OCH_3$ | $OCH_3$ | CH | |
| 914 | $CH_3$ | $CH_3$ | $(CH_3)_3COCO$ | $CH_3$ | $OCH_3$ | $CH_3$ | N | |
| 915 | $CH_3$ | $CH_3$ | PhCO | H | $OCH_3$ | $OCH_3$ | CH | |

| Nr. | $R^1$ | $R^4$ | $R^5$ | $R^7$ | X | Y | Z | Fp. (°C) |
|---|---|---|---|---|---|---|---|---|
| 916 | $CH_3$ | $CH_3$ | PhCO | H | $OCH_3$ | $CH_3$ | CH | |
| 917 | $CH_3$ | $CH_3$ | PhCO | H | $CH_3$ | $CH_3$ | CH | |
| 918 | $CH_3$ | $CH_3$ | PhCO | H | Cl | $OCH_3$ | CH | |
| 919 | $CH_3$ | $CH_3$ | PhCO | H | $OCH_3$ | $OCH_3$ | N | |
| 920 | $CH_3$ | $CH_3$ | PhCO | H | $OCH_3$ | $CH_3$ | N | |
| 921 | $CH_3$ | $CH_3$ | PhCO | H | $OCH_3$ | $CF_3$ | N | |
| 922 | $CH_3$ | $CH_3$ | PhCO | $CH_3$ | $OCH_3$ | $OCH_3$ | CH | |
| 923 | $CH_3$ | $CH_3$ | PhCO | $CH_3$ | $OCH_3$ | $CH_3$ | N | |
| 924 | $CH_3$ | $CH_3$ | $PhSO_2$ | H | $OCH_3$ | $OCH_3$ | CH | |
| 925 | $CH_3$ | $CH_3$ | $PhSO_2$ | H | $OCH_3$ | $CH_3$ | CH | |
| 926 | $CH_3$ | $CH_3$ | $PhSO_2$ | H | $CH_3$ | $CH_3$ | CH | |
| 927 | $CH_3$ | $CH_3$ | $PhSO_2$ | H | Cl | $OCH_3$ | CH | |
| 928 | $CH_3$ | $CH_3$ | $PhSO_2$ | H | $OCH_3$ | $OCH_3$ | N | |
| 929 | $CH_3$ | $CH_3$ | $PhSO_2$ | H | $OCH_3$ | $CH_3$ | N | |
| 930 | $CH_3$ | $CH_3$ | $PhSO_2$ | H | $OCH_3$ | $CF_3$ | N | |
| 931 | $CH_3$ | $CH_3$ | $PhSO_2$ | $CH_3$ | $OCH_3$ | $OCH_3$ | CH | |
| 932 | $CH_3$ | $CH_3$ | $PhSO_2$ | $CH_3$ | $OCH_3$ | $CH_3$ | N | |
| 933 | $CH_3$ | $CH_3$ | $CH_3NHCO$ | H | $OCH_3$ | $OCH_3$ | CH | |
| 934 | $CH_3$ | $CH_3$ | $CH_3NHCO$ | H | $OCH_3$ | $CH_3$ | CH | |
| 935 | $CH_3$ | $CH_3$ | $CH_3NHCO$ | H | $CH_3$ | $CH_3$ | CH | |
| 936 | $CH_3$ | $CH_3$ | $CH_3NHCO$ | H | Cl | $OCH_3$ | CH | |
| 937 | $CH_3$ | $CH_3$ | $CH_3NHCO$ | H | $OCH_3$ | $OCH_3$ | N | |
| 938 | $CH_3$ | $CH_3$ | $CH_3NHCO$ | H | $OCH_3$ | $CH_3$ | N | |
| 939 | $CH_3$ | $CH_3$ | $CH_3NHCO$ | H | $OCH_3$ | $CF_3$ | N | |
| 940 | $CH_3$ | $CH_3$ | $CH_3NHCO$ | $CH_3$ | $OCH_3$ | $OCH_3$ | CH | |
| 941 | $CH_3$ | $CH_3$ | $CH_3NHCO$ | $CH_3$ | $OCH_3$ | $CH_3$ | N | |
| 942 | $CH_3$ | $CH_3$ | EtNHCO | H | $OCH_3$ | $OCH_3$ | CH | |
| 943 | $CH_3$ | $CH_3$ | EtNHCO | H | $OCH_3$ | $CH_3$ | CH | |
| 944 | $CH_3$ | $CH_3$ | EtNHCO | H | $CH_3$ | $CH_3$ | CH | |
| 945 | $CH_3$ | $CH_3$ | EtNHCO | H | Cl | $OCH_3$ | CH | |
| 946 | $CH_3$ | $CH_3$ | EtNHCO | H | $OCH_3$ | $OCH_3$ | N | |
| 947 | $CH_3$ | $CH_3$ | EtNHCO | H | $OCH_3$ | $CH_3$ | N | |
| 948 | $CH_3$ | $CH_3$ | EtNHCO | H | $OCH_3$ | $CF_3$ | N | |
| 949 | $CH_3$ | $CH_3$ | EtNHCO | $CH_3$ | $OCH_3$ | $OCH_3$ | CH | |

| Nr. | $R^1$ | $R^4$ | $R^5$ | $R^7$ | X | Y | Z | Fp. (°C) |
|---|---|---|---|---|---|---|---|---|
| 950 | $CH_3$ | $CH_3$ | EtNHCO | $CH_3$ | $OCH_3$ | $CH_3$ | N | |
| 951 | $CH_3$ | $CH_3$ | $CH_3NHCS$ | H | $OCH_3$ | $OCH_3$ | CH | |
| 952 | $CH_3$ | $CH_3$ | $CH_3NHCS$ | H | $OCH_3$ | $CH_3$ | CH | |
| 953 | $CH_3$ | $CH_3$ | $CH_3NHCS$ | H | $CH_3$ | $CH_3$ | CH | |
| 954 | $CH_3$ | $CH_3$ | $CH_3NHCS$ | H | Cl | $OCH_3$ | CH | |
| 955 | $CH_3$ | $CH_3$ | $CH_3NHCS$ | H | $OCH_3$ | $OCH_3$ | N | |
| 956 | $CH_3$ | $CH_3$ | $CH_3NHCS$ | H | $OCH_3$ | $CH_3$ | N | |
| 957 | $CH_3$ | $CH_3$ | $CH_3NHCS$ | H | $OCH_3$ | $CF_3$ | N | |
| 958 | $CH_3$ | $CH_3$ | $CH_3NHCS$ | $CH_3$ | $OCH_3$ | $OCH_3$ | CH | |
| 959 | $CH_3$ | $CH_3$ | $CH_3NHCS$ | $CH_3$ | $OCH_3$ | $CH_3$ | N | |
| 960 | $CH_3$ | $CH_3$ | EtNHCS | H | $OCH_3$ | $OCH_3$ | CH | |
| 961 | $CH_3$ | $CH_3$ | EtNHCS | H | $OCH_3$ | $CH_3$ | CH | |
| 962 | $CH_3$ | $CH_3$ | EtNHCS | H | $CH_3$ | $CH_3$ | CH | |
| 963 | $CH_3$ | $CH_3$ | EtNHCS | H | Cl | $OCH_3$ | CH | |
| 964 | $CH_3$ | $CH_3$ | EtNHCS | H | $OCH_3$ | $OCH_3$ | N | |
| 965 | $CH_3$ | $CH_3$ | EtNHCS | H | $OCH_3$ | $CH_3$ | N | |
| 966 | $CH_3$ | $CH_3$ | EtNHCS | H | $OCH_3$ | $CF_3$ | N | |
| 967 | $CH_3$ | $CH_3$ | EtNHCS | $CH_3$ | $OCH_3$ | $OCH_3$ | CH | |
| 968 | $CH_3$ | $CH_3$ | EtNHCS | $CH_3$ | $OCH_3$ | $CH_3$ | N | |
| 969 | $CH_3$ | $CH_3$ | $COSCH_3$ | H | $OCH_3$ | $OCH_3$ | CH | |
| 970 | $CH_3$ | $CH_3$ | $COSCH_3$ | H | $OCH_3$ | $CH_3$ | CH | |
| 971 | $CH_3$ | $CH_3$ | $COSCH_3$ | H | $CH_3$ | $CH_3$ | CH | |
| 972 | $CH_3$ | $CH_3$ | $COSCH_3$ | H | Cl | $OCH_3$ | CH | |
| 973 | $CH_3$ | $CH_3$ | $COSCH_3$ | H | $OCH_3$ | $OCH_3$ | N | |
| 974 | $CH_3$ | $CH_3$ | $COSCH_3$ | H | $OCH_3$ | $CH_3$ | N | |
| 975 | $CH_3$ | $CH_3$ | $COSCH_3$ | H | $OCH_3$ | $CF_3$ | N | |
| 976 | $CH_3$ | $CH_3$ | $COSCH_3$ | $CH_3$ | $OCH_3$ | $OCH_3$ | CH | |
| 977 | $CH_3$ | $CH_3$ | $COSCH_3$ | $CH_3$ | $OCH_3$ | $CH_3$ | N | |
| 978 | $CH_3$ | $CH_3$ | $CSOCH_3$ | H | $OCH_3$ | $OCH_3$ | CH | |
| 979 | $CH_3$ | $CH_3$ | $CSOCH_3$ | H | $OCH_3$ | $CH_3$ | CH | |
| 980 | $CH_3$ | $CH_3$ | $CSOCH_3$ | H | $CH_3$ | $CH_3$ | CH | |
| 981 | $CH_3$ | $CH_3$ | $CSOCH_3$ | H | Cl | $OCH_3$ | CH | |
| 982 | $CH_3$ | $CH_3$ | $CSOCH_3$ | H | $OCH_3$ | $OCH_3$ | N | |
| 983 | $CH_3$ | $CH_3$ | $CSOCH_3$ | H | $OCH_3$ | $CH_3$ | N | |

| Nr. | $R^1$ | $R^4$ | $R^5$ | $R^7$ | X | Y | Z | Fp. (°C) |
|---|---|---|---|---|---|---|---|---|
| 984 | $CH_3$ | $CH_3$ | $CSOCH_3$ | H | $OCH_3$ | $CF_3$ | N | |
| 985 | $CH_3$ | $CH_3$ | $CSOCH_3$ | $CH_3$ | $OCH_3$ | $OCH_3$ | CH | |
| 986 | $CH_3$ | $CH_3$ | $CSOCH_3$ | $CH_3$ | $OCH_3$ | $CH_3$ | N | |
| 987 | $CH_3$ | $CH_3$ | $CSSCH_2$ | H | $OCH_3$ | $OCH_3$ | CH | |
| 988 | $CH_3$ | $CH_3$ | $CSSCH_2$ | H | $OCH_3$ | $CH_3$ | CH | |
| 989 | $CH_3$ | $CH_3$ | $CSSCH_2$ | H | $CH_3$ | $CH_3$ | CH | |
| 990 | $CH_3$ | $CH_3$ | $CSSCH_2$ | H | Cl | $OCH_3$ | CH | |
| 991 | $CH_3$ | $CH_3$ | $CSSCH_2$ | H | $OCH_3$ | $OCH_3$ | N | |
| 992 | $CH_3$ | $CH_3$ | $CSSCH_2$ | H | $OCH_3$ | $CH_3$ | N | |
| 993 | $CH_3$ | $CH_3$ | $CSSCH_2$ | H | $OCH_3$ | $CF_3$ | N | |
| 994 | $CH_3$ | $CH_3$ | $CSSCH_2$ | $CH_3$ | $OCH_3$ | $OCH_3$ | CH | |
| 995 | $CH_3$ | $CH_3$ | $CSSCH_2$ | $CH_3$ | $OCH_3$ | $CH_3$ | N | |
| 996 | $CH_3$ | $CH_3$ | $COCOOCH_3$ | H | $OCH_3$ | $OCH_3$ | CH | |
| 997 | $CH_3$ | $CH_3$ | $COCOOCH_3$ | H | $OCH_3$ | $CH_3$ | CH | |
| 998 | $CH_3$ | $CH_3$ | $COCOOCH_3$ | H | $CH_3$ | $CH_3$ | CH | |
| 999 | $CH_3$ | $CH_3$ | $COCOOCH_3$ | H | Cl | $OCH_3$ | CH | |
| 1000 | $CH_3$ | $CH_3$ | $COCOOCH_3$ | H | $OCH_3$ | $OCH_3$ | N | |
| 1001 | $CH_3$ | $CH_3$ | $COCOOCH_3$ | H | $OCH_3$ | $CH_3$ | N | |
| 1002 | $CH_3$ | $CH_3$ | $COCOOCH_3$ | H | $OCH_3$ | $CF_3$ | N | |
| 1003 | $CH_3$ | $CH_3$ | $COCOOCH_3$ | $CH_3$ | $OCH_3$ | $OCH_3$ | CH | |
| 1004 | $CH_3$ | $CH_3$ | $COCOOCH_3$ | $CH_3$ | $OCH_3$ | $CH_3$ | N | |
| 1005 | $CH_3$ | $CH_3$ | $i\text{-}C_3H_7OCO$ | H | $OCH_3$ | $OCH_3$ | CH | |
| 1006 | $CH_3$ | $CH_3$ | $i\text{-}C_3H_7OCO$ | H | $OCH_3$ | $CH_3$ | CH | |
| 1007 | $CH_3$ | $CH_3$ | $i\text{-}C_3H_7OCO$ | H | $CH_3$ | $CH_3$ | CH | |
| 1008 | $CH_3$ | $CH_3$ | $i\text{-}C_3H_7OCO$ | H | Cl | $OCH_3$ | CH | |
| 1009 | $CH_3$ | $CH_3$ | $i\text{-}C_3H_7OCO$ | H | $OCH_3$ | $OCH_3$ | N | |
| 1010 | $CH_3$ | $CH_3$ | $i\text{-}C_3H_7OCO$ | H | $OCH_3$ | $CH_3$ | N | |
| 1011 | $CH_3$ | $CH_3$ | $i\text{-}C_3H_7OCO$ | H | $OCH_3$ | $CF_3$ | N | |
| 1012 | $CH_3$ | $CH_3$ | $i\text{-}C_3H_7OCO$ | $CH_3$ | $OCH_3$ | $OCH_3$ | CH | |
| 1013 | $CH_3$ | $CH_3$ | $i\text{-}C_3H_7OCO$ | $CH_3$ | $OCH_3$ | $CH_3$ | N | |
| 1014 | $CH_3$ | $C_2H_5$ | CHO | H | $OCH_3$ | $OCH_3$ | CH | |
| 1015 | $CH_3$ | $C_2H_5$ | CHO | H | $OCH_3$ | $CH_3$ | CH | |
| 1016 | $CH_3$ | $C_2H_5$ | CHO | H | $CH_3$ | $CH_3$ | CH | |
| 1017 | $CH_3$ | $C_2H_5$ | CHO | H | Cl | $OCH_3$ | CH | |

| Nr. | $R^1$ | $R^4$ | $R^5$ | $R^7$ | X | Y | Z | Fp. (°C) |
|---|---|---|---|---|---|---|---|---|
| 1018 | $CH_3$ | $C_2H_5$ | CHO | H | $OCH_3$ | $OCH_3$ | N | |
| 1019 | $CH_3$ | $C_2H_5$ | CHO | H | $OCH_3$ | $CH_3$ | N | |
| 1020 | $CH_3$ | $C_2H_5$ | CHO | H | $OCH_3$ | $CF_3$ | N | |
| 1021 | $CH_3$ | $C_2H_5$ | CHO | $CH_3$ | $OCH_3$ | $OCH_3$ | CH | |
| 1022 | $CH_3$ | $C_2H_5$ | CHO | $CH_3$ | $OCH_3$ | $CH_3$ | N | |
| 1023 | $CH_3$ | $C_2H_5$ | $COCH_3$ | H | $OCH_3$ | $OCH_3$ | CH | |
| 1024 | $CH_3$ | $C_2H_5$ | $COCH_3$ | H | $OCH_3$ | $CH_3$ | CH | |
| 1025 | $CH_3$ | $C_2H_5$ | $COCH_3$ | H | $CH_3$ | $CH_3$ | CH | |
| 1026 | $CH_3$ | $C_2H_5$ | $COCH_3$ | H | Cl | $OCH_3$ | CH | |
| 1027 | $CH_3$ | $C_2H_5$ | $COCH_3$ | H | $OCH_3$ | $OCH_3$ | N | |
| 1028 | $CH_3$ | $C_2H_5$ | $COCH_3$ | H | $OCH_3$ | $CH_3$ | N | |
| 1029 | $CH_3$ | $C_2H_5$ | $COCH_3$ | H | $OCH_3$ | $CF_3$ | N | |
| 1030 | $CH_3$ | $C_2H_5$ | $COCH_3$ | $CH_3$ | $OCH_3$ | $OCH_3$ | CH | |
| 1031 | $CH_3$ | $C_2H_5$ | $COCH_3$ | $CH_3$ | $OCH_3$ | $CH_3$ | N | |
| 1032 | $CH_3$ | $C_2H_5$ | $COOCH_3$ | H | $OCH_3$ | $OCH_3$ | CH | |
| 1033 | $CH_3$ | $C_2H_5$ | $COOCH_3$ | H | $OCH_3$ | $CH_3$ | CH | |
| 1034 | $CH_3$ | $C_2H_5$ | $COOCH_3$ | H | $CH_3$ | $CH_3$ | CH | |
| 1035 | $CH_3$ | $C_2H_5$ | $COOCH_3$ | H | Cl | $OCH_3$ | CH | |
| 1036 | $CH_3$ | $C_2H_5$ | $COOCH_3$ | H | $OCH_3$ | $OCH_3$ | N | |
| 1037 | $CH_3$ | $C_2H_5$ | $COOCH_3$ | H | $OCH_3$ | $CH_3$ | N | |
| 1038 | $CH_3$ | $C_2H_5$ | $COOCH_3$ | H | $OCH_3$ | $CF_3$ | N | |
| 1039 | $CH_3$ | $C_2H_5$ | $COOCH_3$ | $CH_3$ | $OCH_3$ | $OCH_3$ | CH | |
| 1040 | $CH_3$ | $C_2H_5$ | $COOCH_3$ | $CH_3$ | $OCH_3$ | $CH_3$ | N | |
| 1041 | $CH_3$ | $C_2H_5$ | $COOC_2H_5$ | H | $OCH_3$ | $OCH_3$ | CH | |
| 1042 | $CH_3$ | $C_2H_5$ | $COOC_2H_5$ | H | $OCH_3$ | $CH_3$ | CH | |
| 1043 | $CH_3$ | $C_2H_5$ | $COOC_2H_5$ | H | $CH_3$ | $CH_3$ | CH | |
| 1044 | $CH_3$ | $C_2H_5$ | $COOC_2H_5$ | H | Cl | $OCH_3$ | CH | |
| 1045 | $CH_3$ | $C_2H_5$ | $COOC_2H_5$ | H | $OCH_3$ | $OCH_3$ | N | |
| 1046 | $CH_3$ | $C_2H_5$ | $COOC_2H_5$ | H | $OCH_3$ | $CH_3$ | N | |
| 1047 | $CH_3$ | $C_2H_5$ | $COOC_2H_5$ | H | $OCH_3$ | $CF_3$ | N | |
| 1048 | $CH_3$ | $C_2H_5$ | $COOC_2H_5$ | $CH_3$ | $OCH_3$ | $OCH_3$ | CH | |
| 1049 | $CH_3$ | $C_2H_5$ | $COOC_2H_5$ | $CH_3$ | $OCH_3$ | $CH_3$ | N | |
| 1050 | $CH_3$ | $C_2H_5$ | $SO_2CH_3$ | H | $OCH_3$ | $OCH_3$ | CH | |
| 1051 | $CH_3$ | $C_2H_5$ | $SO_2CH_3$ | H | $OCH_3$ | $CH_3$ | CH | |

| Nr. | $R^1$ | $R^4$ | $R^5$ | $R^7$ | X | Y | Z | Fp. (°C) |
|---|---|---|---|---|---|---|---|---|
| 1052 | $CH_3$ | $C_2H_5$ | $SO_2CH_3$ | H | $CH_3$ | $CH_3$ | CH | |
| 1053 | $CH_3$ | $C_2H_5$ | $SO_2CH_3$ | H | Cl | $OCH_3$ | CH | |
| 1054 | $CH_3$ | $C_2H_5$ | $SO_2CH_3$ | H | $OCH_3$ | $OCH_3$ | N | |
| 1055 | $CH_3$ | $C_2H_5$ | $SO_2CH_3$ | H | $OCH_3$ | $CH_3$ | N | |
| 1056 | $CH_3$ | $C_2H_5$ | $SO_2CH_3$ | H | $OCH_3$ | $CF_3$ | N | |
| 1057 | $CH_3$ | $C_2H_5$ | $SO_2CH_3$ | $CH_3$ | $OCH_3$ | $OCH_3$ | CH | |
| 1058 | $CH_3$ | $C_2H_5$ | $SO_2CH_3$ | $CH_3$ | $OCH_3$ | $CH_3$ | N | |
| 1059 | $CH_3$ | $C_2H_5$ | $SO_2C_2H_5$ | H | $OCH_3$ | $OCH_3$ | CH | |
| 1060 | $CH_3$ | $C_2H_5$ | $SO_2C_2H_5$ | H | $OCH_3$ | $CH_3$ | CH | |
| 1061 | $CH_3$ | $C_2H_5$ | $SO_2C_2H_5$ | H | $CH_3$ | $CH_3$ | CH | |
| 1062 | $CH_3$ | $C_2H_5$ | $SO_2C_2H_5$ | H | Cl | $OCH_3$ | CH | |
| 1063 | $CH_3$ | $C_2H_5$ | $SO_2C_2H_5$ | H | $OCH_3$ | $OCH_3$ | N | |
| 1064 | $CH_3$ | $C_2H_5$ | $SO_2C_2H_5$ | H | $OCH_3$ | $CH_3$ | N | |
| 1065 | $CH_3$ | $C_2H_5$ | $SO_2C_2H_5$ | H | $OCH_3$ | $CF_3$ | N | |
| 1066 | $CH_3$ | $C_2H_5$ | $SO_2C_2H_5$ | $CH_3$ | $OCH_3$ | $OCH_3$ | CH | |
| 1067 | $CH_3$ | $C_2H_5$ | $SO_2C_2H_5$ | $CH_3$ | $OCH_3$ | $CH_3$ | N | |
| 1068 | $C_2H_5$ | H | CHO | H | $OCH_3$ | $OCH_3$ | CH | 146-147 |
| 1069 | $C_2H_5$ | H | CHO | H | $OCH_3$ | $CH_3$ | CH | |
| 1070 | $C_2H_5$ | H | CHO | H | $CH_3$ | $CH_3$ | CH | |
| 1071 | $C_2H_5$ | H | CHO | H | $CH_3$ | $OC_2H_5$ | CH | |
| 1072 | $C_2H_5$ | H | CHO | H | $C_2H_5$ | $OCH_3$ | CH | |
| 1073 | $C_2H_5$ | H | CHO | H | $OC_2H_5$ | $OC_2H_5$ | CH | |
| 1074 | $C_2H_5$ | H | CHO | H | $OCHF_2$ | $OCHF_2$ | CH | |
| 1075 | $C_2H_5$ | H | CHO | H | $CH_3$ | $OCHF_2$ | CH | |
| 1076 | $C_2H_5$ | H | CHO | H | Cl | $OCH_3$ | CH | |
| 1077 | $C_2H_5$ | H | CHO | H | $OCH_3$ | $OCH_3$ | N | |
| 1078 | $C_2H_5$ | H | CHO | H | $OCH_3$ | $CH_3$ | N | |
| 1079 | $C_2H_5$ | H | CHO | H | $CH_3$ | $CH_3$ | N | |
| 1080 | $C_2H_5$ | H | CHO | H | $N(CH_3)_2$ | $OCH_2CF_3$ | N | |
| 1081 | $C_2H_5$ | H | CHO | H | $OCH_3$ | $CF_3$ | N | |
| 1082 | $C_2H_5$ | H | CHO | H | $OC_2H_5$ | $NHCH_3$ | N | |
| 1083 | $C_2H_5$ | H | CHO | $CH_3$ | $OCH_3$ | $OCH_3$ | CH | |
| 1084 | $C_2H_5$ | H | CHO | $CH_3$ | $OCH_3$ | $CH_3$ | N | |
| 1085 | $C_2H_5$ | H | $COCH_3$ | H | $OCH_3$ | $OCH_3$ | CH | 126-128 |

| Nr. | $R^1$ | $R^4$ | $R^5$ | $R^7$ | X | Y | Z | Fp. (°C) |
|---|---|---|---|---|---|---|---|---|
| 1086 | $C_2H_5$ | H | $COCH_3$ | H | $OCH_3$ | $CH_3$ | CH | |
| 1087 | $C_2H_5$ | H | $COCH_3$ | H | $CH_3$ | $CH_3$ | CH | |
| 1088 | $C_2H_5$ | H | $COCH_3$ | H | $CH_3$ | $OC_2H_5$ | CH | |
| 1089 | $C_2H_5$ | H | $COCH_3$ | H | $C_2H_5$ | $OCH_3$ | CH | |
| 1090 | $C_2H_5$ | H | $COCH_3$ | H | $OC_2H_5$ | $OC_2H_5$ | CH | |
| 1091 | $C_2H_5$ | H | $COCH_3$ | H | $OCHF_2$ | $OCHF_2$ | CH | |
| 1092 | $C_2H_5$ | H | $COCH_3$ | H | $CH_3$ | $OCHF_2$ | CH | |
| 1093 | $C_2H_5$ | H | $COCH_3$ | H | Cl | $OCH_3$ | CH | |
| 1094 | $C_2H_5$ | H | $COCH_3$ | H | $OCH_3$ | $OCH_3$ | N | |
| 1095 | $C_2H_5$ | H | $COCH_3$ | H | $OCH_3$ | $CH_3$ | N | |
| 1096 | $C_2H_5$ | H | $COCH_3$ | H | $CH_3$ | $CH_3$ | N | |
| 1097 | $C_2H_5$ | H | $COCH_3$ | H | $N(CH_3)_2$ | $OCH_2CF_3$ | N | |
| 1098 | $C_2H_5$ | H | $COCH_3$ | H | $OCH_3$ | $CF_3$ | N | |
| 1099 | $C_2H_5$ | H | $COCH_3$ | H | $OC_2H_5$ | $NHCH_3$ | N | |
| 1100 | $C_2H_5$ | H | $COCH_3$ | $CH_3$ | $OCH_3$ | $OCH_3$ | CH | |
| 1101 | $C_2H_5$ | H | $COCH_3$ | $CH_3$ | $OCH_3$ | $CH_3$ | N | |
| 1102 | $C_2H_5$ | H | $COC_2H_5$ | H | $OCH_3$ | $OCH_3$ | CH | |
| 1103 | $C_2H_5$ | H | $COC_2H_5$ | H | $OCH_3$ | $CH_3$ | CH | |
| 1104 | $C_2H_5$ | H | $COC_2H_5$ | H | $CH_3$ | $CH_3$ | CH | |
| 1105 | $C_2H_5$ | H | $COC_2H_5$ | H | $CH_3$ | $OC_2H_5$ | CH | |
| 1106 | $C_2H_5$ | H | $COC_2H_5$ | H | $C_2H_5$ | $OCH_3$ | CH | |
| 1107 | $C_2H_5$ | H | $COC_2H_5$ | H | $OC_2H_5$ | $OC_2H_5$ | CH | |
| 1108 | $C_2H_5$ | H | $COC_2H_5$ | H | $OCHF_2$ | $OCHF_2$ | CH | |
| 1109 | $C_2H_5$ | H | $COC_2H_5$ | H | $CH_3$ | $OCHF_2$ | CH | |
| 1110 | $C_2H_5$ | H | $COC_2H_5$ | H | Cl | $OCH_3$ | CH | |
| 1111 | $C_2H_5$ | H | $COC_2H_5$ | H | $OCH_3$ | $OCH_3$ | N | |
| 1112 | $C_2H_5$ | H | $COC_2H_5$ | H | $OCH_3$ | $CH_3$ | N | |
| 1113 | $C_2H_5$ | H | $COC_2H_5$ | H | $CH_3$ | $CH_3$ | N | |
| 1114 | $C_2H_5$ | H | $COC_2H_5$ | H | $N(CH_3)_2$ | $OCH_2CF_3$ | N | |
| 1115 | $C_2H_5$ | H | $COC_2H_5$ | H | $OCH_3$ | $CF_3$ | N | |
| 1116 | $C_2H_5$ | H | $COC_2H_5$ | H | $OC_2H_5$ | $NHCH_3$ | N | |
| 1117 | $C_2H_5$ | H | $COC_2H_5$ | $CH_3$ | $OCH_3$ | $OCH_3$ | CH | |
| 1118 | $C_2H_5$ | H | $COC_2H_5$ | $CH_3$ | $OCH_3$ | $CH_3$ | N | |
| 1119 | $C_2H_5$ | H | $CO\text{-}n\text{-}C_3H_7$ | H | $OCH_3$ | $OCH_3$ | CH | |

| Nr. | $R^1$ | $R^4$ | $R^5$ | $R^7$ | X | Y | Z | Fp. (°C) |
|---|---|---|---|---|---|---|---|---|
| 1120 | $C_2H_5$ | H | CO-n-$C_3H_7$ | H | $OCH_3$ | $CH_3$ | CH | |
| 1121 | $C_2H_5$ | H | CO-n-$C_3H_7$ | H | $CH_3$ | $CH_3$ | CH | |
| 1122 | $C_2H_5$ | H | CO-n-$C_3H_7$ | H | $CH_3$ | $OC_2H_5$ | CH | |
| 1123 | $C_2H_5$ | H | CO-n-$C_3H_7$ | H | $C_2H_5$ | $OCH_3$ | CH | |
| 1124 | $C_2H_5$ | H | CO-n-$C_3H_7$ | H | $OC_2H_5$ | $OC_2H_5$ | CH | |
| 1125 | $C_2H_5$ | H | CO-n-$C_3H_7$ | H | $OCHF_2$ | $OCHF_2$ | CH | |
| 1126 | $C_2H_5$ | H | CO-n-$C_3H_7$ | H | $CH_3$ | $OCHF_2$ | CH | |
| 1127 | $C_2H_5$ | H | CO-n-$C_3H_7$ | H | Cl | $OCH_3$ | CH | |
| 1128 | $C_2H_5$ | H | CO-n-$C_3H_7$ | H | $OCH_3$ | $OCH_3$ | N | |
| 1129 | $C_2H_5$ | H | CO-n-$C_3H_7$ | H | $OCH_3$ | $CH_3$ | N | |
| 1130 | $C_2H_5$ | H | CO-n-$C_3H_7$ | H | $CH_3$ | $CH_3$ | N | |
| 1131 | $C_2H_5$ | H | CO-n-$C_3H_7$ | H | $N(CH_3)_2$ | $OCH_2CF_3$ | N | |
| 1132 | $C_2H_5$ | H | CO-n-$C_3H_7$ | H | $OCH_3$ | $CF_3$ | N | |
| 1133 | $C_2H_5$ | H | CO-n-$C_3H_7$ | H | $OC_2H_5$ | $NHCH_3$ | N | |
| 1134 | $C_2H_5$ | H | CO-n-$C_3H_7$ | $CH_3$ | $OCH_3$ | $OCH_3$ | CH | |
| 1135 | $C_2H_5$ | H | CO-n-$C_3H_7$ | $CH_3$ | $OCH_3$ | $CH_3$ | N | |
| 1136 | $C_2H_5$ | H | CO-i-$C_3H_7$ | H | $OCH_3$ | $OCH_3$ | CH | |
| 1137 | $C_2H_5$ | H | CO-i-$C_3H_7$ | H | $OCH_3$ | $CH_3$ | CH | |
| 1138 | $C_2H_5$ | H | CO-i-$C_3H_7$ | H | $CH_3$ | $CH_3$ | CH | |
| 1139 | $C_2H_5$ | H | CO-i-$C_3H_7$ | H | $CH_3$ | $OC_2H_5$ | CH | |
| 1140 | $C_2H_5$ | H | CO-i-$C_3H_7$ | H | $C_2H_5$ | $OCH_3$ | CH | |
| 1141 | $C_2H_5$ | H | CO-i-$C_3H_7$ | H | $OC_2H_5$ | $OC_2H_5$ | CH | |
| 1142 | $C_2H_5$ | H | CO-i-$C_3H_7$ | H | $OCHF_2$ | $OCHF_2$ | CH | |
| 1143 | $C_2H_5$ | H | CO-i-$C_3H_7$ | H | $CH_3$ | $OCHF_2$ | CH | |
| 1144 | $C_2H_5$ | H | CO-i-$C_3H_7$ | H | Cl | $OCH_3$ | CH | |
| 1145 | $C_2H_5$ | H | CO-i-$C_3H_7$ | H | $OCH_3$ | $OCH_3$ | N | |
| 1146 | $C_2H_5$ | H | CO-i-$C_3H_7$ | H | $OCH_3$ | $CH_3$ | N | |
| 1147 | $C_2H_5$ | H | CO-i-$C_3H_7$ | H | $CH_3$ | $CH_3$ | N | |
| 1148 | $C_2H_5$ | H | CO-i-$C_3H_7$ | H | $N(CH_3)_2$ | $OCH_2CF_3$ | N | |
| 1149 | $C_2H_5$ | H | CO-i-$C_3H_7$ | H | $OCH_3$ | $CF_3$ | N | |
| 1150 | $C_2H_5$ | H | CO-i-$C_3H_7$ | H | $OC_2H_5$ | $NHCH_3$ | N | |
| 1151 | $C_2H_5$ | H | CO-i-$C_3H_7$ | $CH_3$ | $OCH_3$ | $OCH_3$ | CH | |
| 1152 | $C_2H_5$ | H | CO-i-$C_3H_7$ | $CH_3$ | $OCH_3$ | $CH_3$ | N | |
| 1153 | $C_2H_5$ | H | $COCF_3$ | H | $OCH_3$ | $OCH_3$ | CH | |

| Nr. | $R^1$ | $R^4$ | $R^5$ | $R^7$ | X | Y | Z | Fp. (°C) |
|---|---|---|---|---|---|---|---|---|
| 1154 | $C_2H_5$ | H | $COCF_3$ | H | $OCH_3$ | $CH_3$ | CH | |
| 1155 | $C_2H_5$ | H | $COCF_3$ | H | $CH_3$ | $CH_3$ | CH | |
| 1156 | $C_2H_5$ | H | $COCF_3$ | H | $CH_3$ | $OC_2H_5$ | CH | |
| 1157 | $C_2H_5$ | H | $COCF_3$ | H | $C_2H_5$ | $OCH_3$ | CH | |
| 1158 | $C_2H_5$ | H | $COCF_3$ | H | $OC_2H_5$ | $OC_2H_5$ | CH | |
| 1159 | $C_2H_5$ | H | $COCF_3$ | H | $OCHF_2$ | $OCHF_2$ | CH | |
| 1160 | $C_2H_5$ | H | $COCF_3$ | H | $CH_3$ | $OCHF_2$ | CH | |
| 1161 | $C_2H_5$ | H | $COCF_3$ | H | Cl | $OCH_3$ | CH | |
| 1162 | $C_2H_5$ | H | $COCF_3$ | H | $OCH_3$ | $OCH_3$ | N | |
| 1163 | $C_2H_5$ | H | $COCF_3$ | H | $OCH_3$ | $CH_3$ | N | |
| 1164 | $C_2H_5$ | H | $COCF_3$ | H | $CH_3$ | $CH_3$ | N | |
| 1165 | $C_2H_5$ | H | $COCF_3$ | H | $N(CH_3)_2$ | $OCH_2CF_3$ | N | |
| 1166 | $C_2H_5$ | H | $COCF_3$ | H | $OCH_3$ | $CF_3$ | N | |
| 1167 | $C_2H_5$ | H | $COCF_3$ | H | $OC_2H_5$ | $NHCH_3$ | N | |
| 1168 | $C_2H_5$ | H | $COCF_3$ | $CH_3$ | $OCH_3$ | $OCH_3$ | CH | |
| 1169 | $C_2H_5$ | H | $COCF_3$ | $CH_3$ | $OCH_3$ | $CH_3$ | N | |
| 1170 | $C_2H_5$ | H | $COOCH_3$ | H | $OCH_3$ | $OCH_3$ | CH | 132-136 |
| 1170 a | $C_2H_5$ | H | $COOCH_3$ | H | $OCH_3$ | $CH_3$ | CH | |
| 1171 | $C_2H_5$ | H | $COOCH_3$ | H | $CH_3$ | $CH_3$ | CH | |
| 1172 | $C_2H_5$ | H | $COOCH_3$ | H | $CH_3$ | $OC_2H_5$ | CH | |
| 1173 | $C_2H_5$ | H | $COOCH_3$ | H | $C_2H_5$ | $OCH_3$ | CH | |
| 1174 | $C_2H_5$ | H | $COOCH_3$ | H | $OC_2H_5$ | $OC_2H_5$ | CH | |
| 1175 | $C_2H_5$ | H | $COOCH_3$ | H | $OCHF_2$ | $OCHF_2$ | CH | |
| 1176 | $C_2H_5$ | H | $COOCH_3$ | H | $CH_3$ | $OCHF_2$ | CH | |
| 1177 | $C_2H_5$ | H | $COOCH_3$ | H | Cl | $OCH_3$ | CH | |
| 1178 | $C_2H_5$ | H | $COOCH_3$ | H | $OCH_3$ | $OCH_3$ | N | |
| 1179 | $C_2H_5$ | H | $COOCH_3$ | H | $OCH_3$ | $CH_3$ | N | |
| 1180 | $C_2H_5$ | H | $COOCH_3$ | H | $CH_3$ | $CH_3$ | N | |
| 1181 | $C_2H_5$ | H | $COOCH_3$ | H | $N(CH_3)_2$ | $OCH_2CF_3$ | N | |
| 1182 | $C_2H_5$ | H | $COOCH_3$ | H | $OCH_3$ | $CF_3$ | N | |
| 1183 | $C_2H_5$ | H | $COOCH_3$ | H | $OC_2H_5$ | $NHCH_3$ | N | |
| 1184 | $C_2H_5$ | H | $COOCH_3$ | $CH_3$ | $OCH_3$ | $OCH_3$ | CH | |
| 1185 | $C_2H_5$ | H | $COOCH_3$ | $CH_3$ | $OCH_3$ | $CH_3$ | N | |

| Nr. | $R^1$ | $R^4$ | $R^5$ | $R^7$ | X | Y | Z | Fp. (°C) |
|---|---|---|---|---|---|---|---|---|
| 1186 | $C_2H_5$ | H | $COOC_2H_5$ | H | $OCH_3$ | $OCH_3$ | CH | |
| 1186 a | $C_2H_5$ | H | $COOC_2H_5$ | H | $OCH_3$ | $CH_3$ | CH | |
| 1187 | $C_2H_5$ | H | $COOC_2H_5$ | H | $CH_3$ | $CH_3$ | CH | |
| 1188 | $C_2H_5$ | H | $COOC_2H_5$ | H | $CH_3$ | $OC_2H_5$ | CH | |
| 1189 | $C_2H_5$ | H | $COOC_2H_5$ | H | $C_2H_5$ | $OCH_3$ | CH | |
| 1190 | $C_2H_5$ | H | $COOC_2H_5$ | H | $OC_2H_5$ | $OC_2H_5$ | CH | |
| 1191 | $C_2H_5$ | H | $COOC_2H_5$ | H | $OCHF_2$ | $OCHF_2$ | CH | |
| 1192 | $C_2H_5$ | H | $COOC_2H_5$ | H | $CH_3$ | $OCHF_2$ | CH | |
| 1193 | $C_2H_5$ | H | $COOC_2H_5$ | H | Cl | $OCH_3$ | CH | |
| 1194 | $C_2H_5$ | H | $COOC_2H_5$ | H | $OCH_3$ | $OCH_3$ | N | |
| 1195 | $C_2H_5$ | H | $COOC_2H_5$ | H | $OCH_3$ | $CH_3$ | N | |
| 1196 | $C_2H_5$ | H | $COOC_2H_5$ | H | $CH_3$ | $CH_3$ | N | |
| 1197 | $C_2H_5$ | H | $COOC_2H_5$ | H | $N(CH_3)_2$ | $OCH_2CF_3$ | N | |
| 1198 | $C_2H_5$ | H | $COOC_2H_5$ | H | $OCH_3$ | $CF_3$ | N | |
| 1199 | $C_2H_5$ | H | $COOC_2H_5$ | H | $OC_2H_5$ | $NHCH_3$ | N | |
| 1200 | $C_2H_5$ | H | $COOC_2H_5$ | $CH_3$ | $OCH_3$ | $OCH_3$ | CH | |
| 1201 | $C_2H_5$ | H | $COOC_2H_5$ | $CH_3$ | $OCH_3$ | $CH_3$ | N | |
| 1202 | $C_2H_5$ | H | $SO_2CH_3$ | H | $OCH_3$ | $OCH_3$ | CH | 130-132 |
| 1202 a | $C_2H_5$ | H | $SO_2CH_3$ | H | $OCH_3$ | $CH_3$ | CH | |
| 1203 | $C_2H_5$ | H | $SO_2CH_3$ | H | $CH_3$ | $CH_3$ | CH | |
| 1204 | $C_2H_5$ | H | $SO_2CH_3$ | H | $CH_3$ | $OC_2H_5$ | CH | |
| 1205 | $C_2H_5$ | H | $SO_2CH_3$ | H | $C_2H_5$ | $OCH_3$ | CH | |
| 1206 | $C_2H_5$ | H | $SO_2CH_3$ | H | $OC_2H_5$ | $OC_2H_5$ | CH | |
| 1207 | $C_2H_5$ | H | $SO_2CH_3$ | H | $OCHF_2$ | $OCHF_2$ | CH | |
| 1208 | $C_2H_5$ | H | $SO_2CH_3$ | H | $CH_3$ | $OCHF_2$ | CH | |
| 1209 | $C_2H_5$ | H | $SO_2CH_3$ | H | Cl | $OCH_3$ | CH | |
| 1210 | $C_2H_5$ | H | $SO_2CH_3$ | H | $OCH_3$ | $OCH_3$ | N | |
| 1211 | $C_2H_5$ | H | $SO_2CH_3$ | H | $OCH_3$ | $CH_3$ | N | |
| 1212 | $C_2H_5$ | H | $SO_2CH_3$ | H | $CH_3$ | $CH_3$ | N | |
| 1213 | $C_2H_5$ | H | $SO_2CH_3$ | H | $N(CH_3)_2$ | $OCH_2CF_3$ | N | |
| 1214 | $C_2H_5$ | H | $SO_2CH_3$ | H | $OCH_3$ | $CF_3$ | N | |
| 1215 | $C_2H_5$ | H | $SO_2CH_3$ | H | $OC_2H_5$ | $NHCH_3$ | N | |

| Nr. | $R^1$ | $R^4$ | $R^5$ | $R^7$ | X | Y | Z | Fp. (°C) |
|---|---|---|---|---|---|---|---|---|
| 1216 | $C_2H_5$ | H | $SO_2CH_3$ | $CH_3$ | $OCH_3$ | $OCH_3$ | CH | |
| 1217 | $C_2H_5$ | H | $SO_2CH_3$ | $CH_3$ | $OCH_3$ | $CH_3$ | N | |
| 1218 | $C_2H_5$ | H | $SO_2C_2H_5$ | H | $OCH_3$ | $OCH_3$ | CH | |
| 1218 a | $C_2H_5$ | H | $SO_2C_2H_5$ | H | $OCH_3$ | $CH_3$ | CH | |
| 1219 | $C_2H_5$ | H | $SO_2C_2H_5$ | H | $CH_3$ | $CH_3$ | CH | |
| 1220 | $C_2H_5$ | H | $SO_2C_2H_5$ | H | $CH_3$ | $OC_2H_5$ | CH | |
| 1221 | $C_2H_5$ | H | $SO_2C_2H_5$ | H | $C_2H_5$ | $OCH_3$ | CH | |
| 1222 | $C_2H_5$ | H | $SO_2C_2H_5$ | H | $OC_2H_5$ | $OC_2H_5$ | CH | |
| 1223 | $C_2H_5$ | H | $SO_2C_2H_5$ | H | $OCHF_2$ | $OCHF_2$ | CH | |
| 1224 | $C_2H_5$ | H | $SO_2C_2H_5$ | H | $CH_3$ | $OCHF_2$ | CH | |
| 1225 | $C_2H_5$ | H | $SO_2C_2H_5$ | H | Cl | $OCH_3$ | CH | |
| 1226 | $C_2H_5$ | H | $SO_2C_2H_5$ | H | $OCH_3$ | $OCH_3$ | N | |
| 1227 | $C_2H_5$ | H | $SO_2C_2H_5$ | H | $OCH_3$ | $CH_3$ | N | |
| 1228 | $C_2H_5$ | H | $SO_2C_2H_5$ | H | $CH_3$ | $CH_3$ | N | |
| 1229 | $C_2H_5$ | H | $SO_2C_2H_5$ | H | $N(CH_3)_2$ | $OCH_2CF_3$ | N | |
| 1230 | $C_2H_5$ | H | $SO_2C_2H_5$ | H | $OCH_3$ | $CF_3$ | N | |
| 1231 | $C_2H_5$ | H | $SO_2C_2H_5$ | H | $OC_2H_5$ | $NHCH_3$ | N | |
| 1232 | $C_2H_5$ | H | $SO_2C_2H_5$ | $CH_3$ | $OCH_3$ | $OCH_3$ | CH | |
| 1233 | $C_2H_5$ | H | $SO_2C_2H_5$ | $CH_3$ | $OCH_3$ | $CH_3$ | N | |
| 1234 | $C_2H_5$ | H | $SO_2$-n-$C_3H_7$ | H | $OCH_3$ | $OCH_3$ | CH | |
| 1234 a | $C_2H_5$ | H | $SO_2$-n-$C_3H_7$ | H | $OCH_3$ | $CH_3$ | CH | |
| 1235 | $C_2H_5$ | H | $SO_2$-n-$C_3H_7$ | H | $CH_3$ | $CH_3$ | CH | |
| 1236 | $C_2H_5$ | H | $SO_2$-n-$C_3H_7$ | H | $CH_3$ | $OC_2H_5$ | CH | |
| 1237 | $C_2H_5$ | H | $SO_2$-n-$C_3H_7$ | H | $C_2H_5$ | $OCH_3$ | CH | |
| 1238 | $C_2H_5$ | H | $SO_2$-n-$C_3H_7$ | H | $OC_2H_5$ | $OC_2H_5$ | CH | |
| 1239 | $C_2H_5$ | H | $SO_2$-n-$C_3H_7$ | H | $OCHF_2$ | $OCHF_2$ | CH | |
| 1240 | $C_2H_5$ | H | $SO_2$-n-$C_3H_7$ | H | $CH_3$ | $OCHF_2$ | CH | |
| 1241 | $C_2H_5$ | H | $SO_2$-n-$C_3H_7$ | H | Cl | $OCH_3$ | CH | |
| 1242 | $C_2H_5$ | H | $SO_2$-n-$C_3H_7$ | H | $OCH_3$ | $OCH_3$ | N | |
| 1243 | $C_2H_5$ | H | $SO_2$-n-$C_3H_7$ | H | $OCH_3$ | $CH_3$ | N | |
| 1244 | $C_2H_5$ | H | $SO_2$-n-$C_3H_7$ | H | $CH_3$ | $CH_3$ | N | |
| 1245 | $C_2H_5$ | H | $SO_2$-n-$C_3H_7$ | H | $N(CH_3)_2$ | $OCH_2CF_3$ | N | |

| Nr. | $R^1$ | $R^4$ | $R^5$ | $R^7$ | X | Y | Z | Fp. (°C) |
|---|---|---|---|---|---|---|---|---|
| 1246 | $C_2H_5$ | H | $SO_2$-n-$C_3H_7$ | H | $OCH_3$ | $CF_3$ | N | |
| 1247 | $C_2H_5$ | H | $SO_2$-n-$C_3H_7$ | H | $OC_2H_5$ | $NHCH_3$ | N | |
| 1248 | $C_2H_5$ | H | $SO_2$-n-$C_3H_7$ | $CH_3$ | $OCH_3$ | $OCH_3$ | CH | |
| 1249 | $C_2H_5$ | H | $SO_2$-n-$C_3H_7$ | $CH_3$ | $OCH_3$ | $CH_3$ | N | |
| 1250 | $C_2H_5$ | H | $SO_2$-i-$C_3H_7$ | H | $OCH_3$ | $OCH_3$ | CH | |
| 1250 a | $C_2H_5$ | H | $SO_2$-i-$C_3H_7$ | H | $OCH_3$ | $CH_3$ | CH | |
| 1251 | $C_2H_5$ | H | $SO_2$-i-$C_3H_7$ | H | $CH_3$ | $CH_3$ | CH | |
| 1252 | $C_2H_5$ | H | $SO_2$-i-$C_3H_7$ | H | $CH_3$ | $OC_2H_5$ | CH | |
| 1253 | $C_2H_5$ | H | $SO_2$-i-$C_3H_7$ | H | $C_2H_5$ | $OCH_3$ | CH | |
| 1254 | $C_2H_5$ | H | $SO_2$-i-$C_3H_7$ | H | $OC_2H_5$ | $OC_2H_5$ | CH | |
| 1255 | $C_2H_5$ | H | $SO_2$-i-$C_3H_7$ | H | $OCHF_2$ | $OCHF_2$ | CH | |
| 1256 | $C_2H_5$ | H | $SO_2$-i-$C_3H_7$ | H | $CH_3$ | $OCHF_2$ | CH | |
| 1257 | $C_2H_5$ | H | $SO_2$-i-$C_3H_7$ | H | Cl | $OCH_3$ | CH | |
| 1258 | $C_2H_5$ | H | $SO_2$-i-$C_3H_7$ | H | $OCH_3$ | $OCH_3$ | N | |
| 1259 | $C_2H_5$ | H | $SO_2$-i-$C_3H_7$ | H | $OCH_3$ | $CH_3$ | N | |
| 1260 | $C_2H_5$ | H | $SO_2$-i-$C_3H_7$ | H | $CH_3$ | $CH_3$ | N | |
| 1261 | $C_2H_5$ | H | $SO_2$-i-$C_3H_7$ | H | $N(CH_3)_2$ | $OCH_2CF_3$ | N | |
| 1262 | $C_2H_5$ | H | $SO_2$-i-$C_3H_7$ | H | $OCH_3$ | $CF_3$ | N | |
| 1263 | $C_2H_5$ | H | $SO_2$-i-$C_3H_7$ | H | $OC_2H_5$ | $NHCH_3$ | N | |
| 1264 | $C_2H_5$ | H | $SO_2$-i-$C_3H_7$ | $CH_3$ | $OCH_3$ | $OCH_3$ | CH | |
| 1265 | $C_2H_5$ | H | $SO_2$-i-$C_3H_7$ | $CH_3$ | $OCH_3$ | $CH_3$ | N | |
| 1266 | $C_2H_5$ | H | $ClCH_2CO$ | H | $OCH_3$ | $OCH_3$ | CH | |
| 1267 | $C_2H_5$ | H | $ClCH_2CO$ | H | $OCH_3$ | $CH_3$ | CH | |
| 1268 | $C_2H_5$ | H | $ClCH_2CO$ | H | $CH_3$ | $CH_3$ | CH | |
| 1269 | $C_2H_5$ | H | $ClCH_2CO$ | H | Cl | $OCH_3$ | CH | |
| 1270 | $C_2H_5$ | H | $ClCH_2CO$ | H | $OCH_3$ | $OCH_3$ | N | |
| 1271 | $C_2H_5$ | H | $ClCH_2CO$ | H | $OCH_3$ | $CH_3$ | N | |
| 1272 | $C_2H_5$ | H | $ClCH_2CO$ | H | $OCH_3$ | $CF_3$ | N | |
| 1273 | $C_2H_5$ | H | $ClCH_2CO$ | $CH_3$ | $OCH_3$ | $OCH_3$ | CH | |
| 1274 | $C_2H_5$ | H | $ClCH_2CO$ | $CH_3$ | $OCH_3$ | $CH_3$ | N | |
| 1275 | $C_2H_5$ | H | $Cl_2CHCO$ | H | $OCH_3$ | $OCH_3$ | CH | |
| 1276 | $C_2H_5$ | H | $Cl_2CHCO$ | H | $OCH_3$ | $CH_3$ | CH | |
| 1277 | $C_2H_5$ | H | $Cl_2CHCO$ | H | $CH_3$ | $CH_3$ | CH | |

| Nr. | $R^1$ | $R^4$ | $R^5$ | $R^7$ | X | Y | Z | Fp. (°C) |
|---|---|---|---|---|---|---|---|---|
| 1278 | $C_2H_5$ | H | $Cl_2CHCO$ | H | Cl | $OCH_3$ | CH | |
| 1279 | $C_2H_5$ | H | $Cl_2CHCO$ | H | $OCH_3$ | $OCH_3$ | N | |
| 1280 | $C_2H_5$ | H | $Cl_2CHCO$ | H | $OCH_3$ | $CH_3$ | N | |
| 1281 | $C_2H_5$ | H | $Cl_2CHCO$ | H | $OCH_3$ | $CF_3$ | N | |
| 1282 | $C_2H_5$ | H | $Cl_2CHCO$ | $CH_3$ | $OCH_3$ | $OCH_3$ | CH | |
| 1283 | $C_2H_5$ | H | $Cl_2CHCO$ | $CH_3$ | $OCH_3$ | $CH_3$ | N | |
| 1284 | $C_2H_5$ | H | $Cl_3CCO$ | H | $OCH_3$ | $OCH_3$ | CH | |
| 1285 | $C_2H_5$ | H | $Cl_3CCO$ | H | $OCH_3$ | $CH_3$ | CH | |
| 1286 | $C_2H_5$ | H | $Cl_3CCO$ | H | $CH_3$ | $CH_3$ | CH | |
| 1287 | $C_2H_5$ | H | $Cl_3CCO$ | H | Cl | $OCH_3$ | CH | |
| 1288 | $C_2H_5$ | H | $Cl_3CCO$ | H | $OCH_3$ | $OCH_3$ | N | |
| 1289 | $C_2H_5$ | H | $Cl_3CCO$ | H | $OCH_3$ | $CH_3$ | N | |
| 1290 | $C_2H_5$ | H | $Cl_3CCO$ | H | $OCH_3$ | $CF_3$ | N | |
| 1291 | $C_2H_5$ | H | $Cl_3CCO$ | $CH_3$ | $OCH_3$ | $OCH_3$ | CH | |
| 1292 | $C_2H_5$ | H | $Cl_3CCO$ | $CH_3$ | $OCH_3$ | $CH_3$ | N | |
| 1293 | $C_2H_5$ | H | $CH_3OCH_2CO$ | H | $OCH_3$ | $OCH_3$ | CH | |
| 1294 | $C_2H_5$ | H | $CH_3OCH_2CO$ | H | $OCH_3$ | $CH_3$ | CH | |
| 1295 | $C_2H_5$ | H | $CH_3OCH_2CO$ | H | $CH_3$ | $CH_3$ | CH | |
| 1296 | $C_2H_5$ | H | $CH_3OCH_2CO$ | H | Cl | $OCH_3$ | CH | |
| 1297 | $C_2H_5$ | H | $CH_3OCH_2CO$ | H | $OCH_3$ | $OCH_3$ | N | |
| 1298 | $C_2H_5$ | H | $CH_3OCH_2CO$ | H | $OCH_3$ | $CH_3$ | N | |
| 1299 | $C_2H_5$ | H | $CH_3OCH_2CO$ | H | $OCH_3$ | $CF_3$ | N | |
| 1300 | $C_2H_5$ | H | $CH_3OCH_2CO$ | $CH_3$ | $OCH_3$ | $OCH_3$ | CH | |
| 1301 | $C_2H_5$ | H | $CH_3OCH_2CO$ | $CH_3$ | $OCH_3$ | $CH_3$ | N | |
| 1302 | $C_2H_5$ | H | $CH_2$=CHCO | H | $OCH_3$ | $OCH_3$ | CH | |
| 1303 | $C_2H_5$ | H | $CH_2$=CHCO | H | $OCH_3$ | $CH_3$ | CH | |
| 1304 | $C_2H_5$ | H | $CH_2$=CHCO | H | $CH_3$ | $CH_3$ | CH | |
| 1305 | $C_2H_5$ | H | $CH_2$=CHCO | H | Cl | $OCH_3$ | CH | |
| 1306 | $C_2H_5$ | H | $CH_2$=CHCO | H | $OCH_3$ | $OCH_3$ | N | |
| 1307 | $C_2H_5$ | H | $CH_2$=CHCO | H | $OCH_3$ | $CH_3$ | N | |
| 1308 | $C_2H_5$ | H | $CH_2$=CHCO | H | $OCH_3$ | $CF_3$ | N | |
| 1309 | $C_2H_5$ | H | $CH_2$=CHCO | $CH_3$ | $OCH_3$ | $OCH_3$ | CH | |
| 1310 | $C_2H_5$ | H | $CH_2$=CHCO | $CH_3$ | $OCH_3$ | $CH_3$ | N | |
| 1311 | $C_2H_5$ | H | CH≡CCO | H | $OCH_3$ | $OCH_3$ | CH | |

| Nr. | $R^1$ | $R^4$ | $R^5$ | $R^7$ | X | Y | Z | Fp. (°C) |
|---|---|---|---|---|---|---|---|---|
| 1312 | $C_2H_5$ | H | CH≡CCO | H | $OC_2H_5$ | $OCH_3$ | CH | |
| 1313 | $C_2H_5$ | H | CH≡CCO | H | $OCH_3$ | $CH_3$ | CH | |
| 1314 | $C_2H_5$ | H | CH≡CCO | H | $CH_3$ | $CH_3$ | CH | |
| 1315 | $C_2H_5$ | H | CH≡CCO | H | Cl | $OCH_3$ | CH | |
| 1316 | $C_2H_5$ | H | CH≡CCO | H | $OCH_3$ | $OCH_3$ | N | |
| 1317 | $C_2H_5$ | H | CH≡CCO | H | $OCH_3$ | $CH_3$ | N | |
| 1318 | $C_2H_5$ | H | CH≡CCO | H | $OCH_3$ | $CF_3$ | N | |
| 1319 | $C_2H_5$ | H | CH≡CCO | $CH_3$ | $OCH_3$ | $OCH_3$ | CH | |
| 1320 | $C_2H_5$ | H | CH≡CCO | $CH_3$ | $OCH_3$ | $CH_3$ | N | |
| 1321 | $C_2H_5$ | H | CO 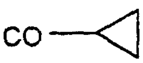 | H | $OCH_3$ | $OCH_3$ | CH | |
| 1322 | $C_2H_5$ | H | CO 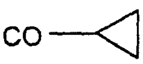 | H | $OCH_3$ | $CH_3$ | CH | |
| 1323 | $C_2H_5$ | H | CO 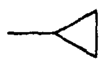 | H | $CH_3$ | $CH_3$ | CH | |
| 1324 | $C_2H_5$ | H | CO 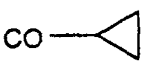 | H | Cl | $OCH_3$ | CH | |
| 1325 | $C_2H_5$ | H | CO 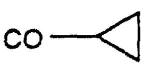 | H | $OCH_3$ | $OCH_3$ | N | |
| 1326 | $C_2H_5$ | H | CO 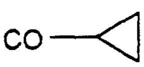 | H | $OCH_3$ | $CH_3$ | N | |
| 1327 | $C_2H_5$ | H | CO 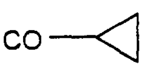 | H | $OCH_3$ | $CF_3$ | N | |
| 1328 | $C_2H_5$ | H | CO 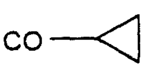 | $CH_3$ | $OCH_3$ | $OCH_3$ | CH | |
| 1329 | $C_2H_5$ | H | CO 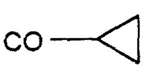 | $CH_3$ | $OCH_3$ | $CH_3$ | N | |
| 1330 | $C_2H_5$ | H | CO 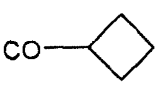 | H | $OCH_3$ | $OCH_3$ | CH | |
| 1331 | $C_2H_5$ | H | CO 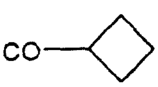 | H | $OCH_3$ | $CH_3$ | CH | |
| 1332 | $C_2H_5$ | H | CO 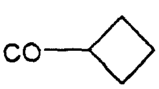 | H | $CH_3$ | $CH_3$ | CH | |

| Nr. | $R^1$ | $R^4$ | $R^5$ | $R^7$ | X | Y | Z | Fp. (°C) |
|---|---|---|---|---|---|---|---|---|
| 1333 | $C_2H_5$ | H | 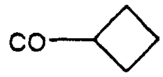 | H | Cl | $OCH_3$ | CH | |
| 1334 | $C_2H_5$ | H | 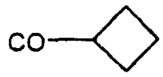 | H | $OCH_3$ | $OCH_3$ | N | |
| 1335 | $C_2H_5$ | H | 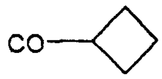 | H | $OCH_3$ | $CH_3$ | N | |
| 1336 | $C_2H_5$ | H | 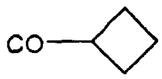 | H | $OCH_3$ | $CF_3$ | N | |
| 1337 | $C_2H_5$ | H | 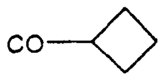 | $CH_3$ | $OCH_3$ | $OCH_3$ | CH | |
| 1338 | $C_2H_5$ | H | 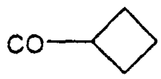 | $CH_3$ | $OCH_3$ | $CH_3$ | N | |
| 1339 | $C_2H_5$ | H | 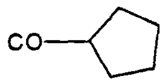 | H | $OCH_3$ | $OCH_3$ | CH | |
| 1340 | $C_2H_5$ | H | 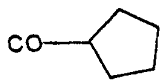 | H | $OCH_3$ | $CH_3$ | CH | |
| 1341 | $C_2H_5$ | H | 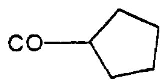 | H | $CH_3$ | $CH_3$ | CH | |
| 1342 | $C_2H_5$ | H | 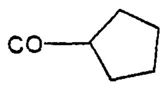 | H | Cl | $OCH_3$ | CH | |
| 1343 | $C_2H_5$ | H | 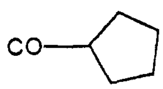 | H | $OCH_3$ | $OCH_3$ | N | |
| 1344 | $C_2H_5$ | H | 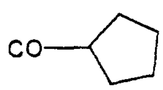 | H | $OCH_3$ | $CH_3$ | N | |
| 1345 | $C_2H_5$ | H | 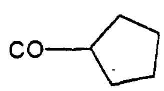 | H | $OCH_3$ | $CF_3$ | N | |
| 1346 | $C_2H_5$ | H | 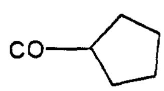 | $CH_3$ | $OCH_3$ | $OCH_3$ | CH | |

| Nr. | $R^1$ | $R^4$ | $R^5$ | $R^7$ | X | Y | Z | Fp. (°C) |
|---|---|---|---|---|---|---|---|---|
| 1347 | $C_2H_5$ | H | 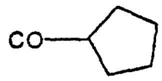 | $CH_3$ | $OCH_3$ | $CH_3$ | N | |
| 1348 | $C_2H_5$ | H | 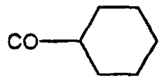 | H | $OCH_3$ | $OCH_3$ | CH | |
| 1349 | $C_2H_5$ | H | 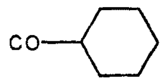 | H | $OCH_3$ | $CH_3$ | CH | |
| 1350 | $C_2H_5$ | H | 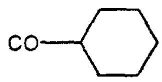 | H | $CH_3$ | $CH_3$ | CH | |
| 1351 | $C_2H_5$ | H | 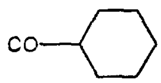 | H | Cl | $OCH_3$ | CH | |
| 1352 | $C_2H_5$ | H | 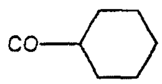 | H | $OCH_3$ | $OCH_3$ | N | |
| 1353 | $C_2H_5$ | H | 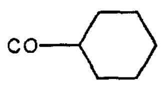 | H | $OCH_3$ | $CH_3$ | N | |
| 1354 | $C_2H_5$ | H | 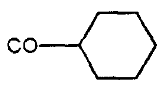 | H | $OCH_3$ | $CF_3$ | N | |
| 1355 | $C_2H_5$ | H | 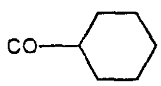 | $CH_3$ | $OCH_3$ | $OCH_3$ | CH | |
| 1356 | $C_2H_5$ | H | 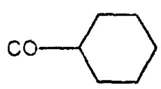 | $CH_3$ | $OCH_3$ | $CH_3$ | N | |
| 1357 | $C_2H_5$ | H | $CF_3SO_2$ | H | $OCH_3$ | $OCH_3$ | CH | |
| 1358 | $C_2H_5$ | H | $CF_3SO_2$ | H | $OCH_3$ | $CH_3$ | CH | |
| 1359 | $C_2H_5$ | H | $CF_3SO_2$ | H | $CH_3$ | $CH_3$ | CH | |
| 1360 | $C_2H_5$ | H | $CF_3SO_2$ | H | Cl | $OCH_3$ | CH | |
| 1361 | $C_2H_5$ | H | $CF_3SO_2$ | H | $OCH_3$ | $CH_3$ | N | |
| 1362 | $C_2H_5$ | H | $CF_3SO_2$ | H | $OCH_3$ | $CF_3$ | N | |
| 1363 | $C_2H_5$ | H | $CF_3SO_2$ | H | $OCH_3$ | $OCH_3$ | N | |
| 1364 | $C_2H_5$ | H | $CF_3SO_2$ | $CH_3$ | $OCH_3$ | $CH_3$ | CH | |
| 1365 | $C_2H_5$ | H | $CF_3SO_2$ | $CH_3$ | $OCH_3$ | $OCH_3$ | N | |
| 1366 | $C_2H_5$ | H | $FCH_2SO_2$ | H | $OCH_3$ | $OCH_3$ | CH | |
| 1367 | $C_2H_5$ | H | $FCH_2SO_2$ | H | $OCH_3$ | $CH_3$ | CH | |

| Nr. | $R^1$ | $R^4$ | $R^5$ | $R^7$ | X | Y | Z | Fp. (°C) |
|---|---|---|---|---|---|---|---|---|
| 1368 | $C_2H_5$ | H | $FCH_2SO_2$ | H | $CH_3$ | $CH_3$ | CH | |
| 1369 | $C_2H_5$ | H | $FCH_2SO_2$ | H | Cl | $OCH_3$ | CH | |
| 1370 | $C_2H_5$ | H | $FCH_2SO_2$ | H | $OCH_3$ | $OCH_3$ | N | |
| 1371 | $C_2H_5$ | H | $FCH_2SO_2$ | H | $OCH_3$ | $CH_3$ | N | |
| 1372 | $C_2H_5$ | H | $FCH_2SO_2$ | H | $OCH_3$ | $CF_3$ | N | |
| 1373 | $C_2H_5$ | H | $FCH_2SO_2$ | $CH_3$ | $OCH_3$ | $OCH_3$ | CH | |
| 1374 | $C_2H_5$ | H | $FCH_2SO_2$ | $CH_3$ | $OCH_3$ | $OCH_3$ | N | |
| 1375 | $C_2H_5$ | H | $ClCH_2SO_2$ | H | $OCH_3$ | $OCH_3$ | CH | |
| 1376 | $C_2H_5$ | H | $ClCH_2SO_2$ | H | $OCH_3$ | $CH_3$ | CH | |
| 1377 | $C_2H_5$ | H | $ClCH_2SO_2$ | H | $CH_3$ | $CH_3$ | CH | |
| 1378 | $C_2H_5$ | H | $ClCH_2SO_2$ | H | Cl | $OCH_3$ | CH | |
| 1379 | $C_2H_5$ | H | $ClCH_2SO_2$ | H | $OCH_3$ | $OCH_3$ | N | |
| 1380 | $C_2H_5$ | H | $ClCH_2SO_2$ | H | $OCH_3$ | $CH_3$ | N | |
| 1381 | $C_2H_5$ | H | $ClCH_2SO_2$ | H | $OCH_3$ | $CF_3$ | N | |
| 1382 | $C_2H_5$ | H | $ClCH_2SO_2$ | $CH_3$ | $OCH_3$ | $OCH_3$ | CH | |
| 1383 | $C_2H_5$ | H | $ClCH_2SO_2$ | $CH_3$ | $OCH_3$ | $CH_3$ | N | |
| 1384 | $C_2H_5$ | H | $Cl_2CHSO_2$ | H | $OCH_3$ | $OCH_3$ | CH | |
| 1385 | $C_2H_5$ | H | $Cl_2CHSO_2$ | H | $OCH_3$ | $CH_3$ | CH | |
| 1386 | $C_2H_5$ | H | $Cl_2CHSO_2$ | H | $CH_3$ | $CH_3$ | CH | |
| 1387 | $C_2H_5$ | H | $Cl_2CHSO_2$ | H | Cl | $OCH_3$ | CH | |
| 1388 | $C_2H_5$ | H | $Cl_2CHSO_2$ | H | $OCH_3$ | $OCH_3$ | N | |
| 1389 | $C_2H_5$ | H | $Cl_2CHSO_2$ | H | $OCH_3$ | $CH_3$ | N | |
| 1390 | $C_2H_5$ | H | $Cl_2CHSO_2$ | H | $OCH_3$ | $CF_3$ | N | |
| 1391 | $C_2H_5$ | H | $Cl_2CHSO_2$ | $CH_3$ | $OCH_3$ | $OCH_3$ | CH | |
| 1392 | $C_2H_5$ | H | $Cl_2CHSO_2$ | $CH_3$ | $OCH_3$ | $CH_3$ | N | |
| 1393 | $C_2H_5$ | H | $Cl_3CSO_2$ | H | $OCH_3$ | $OCH_3$ | CH | |
| 1394 | $C_2H_5$ | H | $Cl_3CSO_2$ | H | $OCH_3$ | $CH_3$ | CH | |
| 1395 | $C_2H_5$ | H | $Cl_3CSO_2$ | H | $CH_3$ | $CH_3$ | CH | |
| 1396 | $C_2H_5$ | H | $Cl_3CSO_2$ | H | Cl | $OCH_3$ | CH | |
| 1397 | $C_2H_5$ | H | $Cl_3CSO_2$ | H | $OCH_3$ | $OCH_3$ | N | |
| 1398 | $C_2H_5$ | H | $Cl_3CSO_2$ | H | $OCH_3$ | $CH_3$ | N | |
| 1399 | $C_2H_5$ | H | $Cl_3CSO_2$ | H | $OCH_3$ | $CF_3$ | N | |
| 1400 | $C_2H_5$ | H | $Cl_3CSO_2$ | $CH_3$ | $OCH_3$ | $OCH_3$ | CH | |
| 1401 | $C_2H_5$ | H | $Cl_3CSO_2$ | $CH_3$ | $OCH_3$ | $CH_3$ | N | |

| Nr. | $R^1$ | $R^4$ | $R^5$ | $R^7$ | X | Y | Z | Fp. (°C) |
|---|---|---|---|---|---|---|---|---|
| 1402 | $C_2H_5$ | H | n-$C_4H_9SO_2$ | H | $OCH_3$ | $OCH_3$ | CH | |
| 1403 | $C_2H_5$ | H | n-$C_4H_9SO_2$ | H | $OCH_3$ | $CH_3$ | CH | |
| 1404 | $C_2H_5$ | H | n-$C_4H_9SO_2$ | H | $CH_3$ | $CH_3$ | CH | |
| 1405 | $C_2H_5$ | H | n-$C_4H_9SO_2$ | H | Cl | $OCH_3$ | CH | |
| 1406 | $C_2H_5$ | H | n-$C_4H_9SO_2$ | H | $OCH_3$ | $CH_3$ | N | |
| 1407 | $C_2H_5$ | H | n-$C_4H_9SO_2$ | H | $OCH_3$ | $CF_3$ | N | |
| 1408 | $C_2H_5$ | H | n-$C_4H_9SO_2$ | H | $OCH_3$ | $OCH_3$ | N | |
| 1409 | $C_2H_5$ | H | n-$C_4H_9SO_2$ | $CH_3$ | $OCH_3$ | $CH_3$ | CH | |
| 1410 | $C_2H_5$ | H | n-$C_4H_9SO_2$ | $CH_3$ | $OCH_3$ | $OCH_3$ | N | |
| 1411 | $C_2H_5$ | H | $CF_3CH_2SO_2$ | H | $OCH_3$ | $OCH_3$ | CH | |
| 1412 | $C_2H_5$ | H | $CF_3CH_2SO_2$ | H | $OCH_3$ | $CH_3$ | CH | |
| 1413 | $C_2H_5$ | H | $CF_3CH_2SO_2$ | H | $CH_3$ | $CH_3$ | CH | |
| 1414 | $C_2H_5$ | H | $CF_3CH_2SO_2$ | H | Cl | $OCH_3$ | CH | |
| 1415 | $C_2H_5$ | H | $CF_3CH_2SO_2$ | H | $OCH_3$ | $OCH_3$ | N | |
| 1416 | $C_2H_5$ | H | $CF_3CH_2SO_2$ | H | $OCH_3$ | $CH_3$ | N | |
| 1417 | $C_2H_5$ | H | $CF_3CH_2SO_2$ | H | $OCH_3$ | $CF_3$ | N | |
| 1418 | $C_2H_5$ | H | $CF_3CH_2SO_2$ | $CH_3$ | $OCH_3$ | $OCH_3$ | CH | |
| 1419 | $C_2H_5$ | H | $CF_3CH_2SO_2$ | $CH_3$ | $OCH_3$ | $OCH_3$ | N | |
| 1420 | $C_2H_5$ | H | $CH_3NHSO_2$ | H | $OCH_3$ | $OCH_3$ | CH | |
| 1421 | $C_2H_5$ | H | $CH_3NHSO_2$ | H | $OCH_3$ | $CH_3$ | CH | |
| 1422 | $C_2H_5$ | H | $CH_3NHSO_2$ | H | $CH_3$ | $CH_3$ | CH | |
| 1423 | $C_2H_5$ | H | $CH_3NHSO_2$ | H | Cl | $OCH_3$ | CH | |
| 1424 | $C_2H_5$ | H | $CH_3NHSO_2$ | H | $OCH_3$ | $OCH_3$ | N | |
| 1425 | $C_2H_5$ | H | $CH_3NHSO_2$ | H | $OCH_3$ | $CH_3$ | N | |
| 1426 | $C_2H_5$ | H | $CH_3NHSO_2$ | H | $OCH_3$ | $CF_3$ | N | |
| 1427 | $C_2H_5$ | H | $CH_3NHSO_2$ | $CH_3$ | $OCH_3$ | $OCH_3$ | CH | |
| 1428 | $C_2H_5$ | H | $CH_3NHSO_2$ | $CH_3$ | $OCH_3$ | $CH_3$ | N | |
| 1429 | $C_2H_5$ | H | $(CH_3)_2NSO_2$ | H | $OCH_3$ | $OCH_3$ | CH | |
| 1430 | $C_2H_5$ | H | $(CH_3)_2NSO_2$ | H | $OCH_3$ | $CH_3$ | CH | |
| 1431 | $C_2H_5$ | H | $(CH_3)_2NSO_2$ | H | $CH_3$ | $CH_3$ | CH | |
| 1432 | $C_2H_5$ | H | $(CH_3)_2NSO_2$ | H | Cl | $OCH_3$ | CH | |
| 1433 | $C_2H_5$ | H | $(CH_3)_2NSO_2$ | H | $OCH_3$ | $OCH_3$ | N | |
| 1434 | $C_2H_5$ | H | $(CH_3)_2NSO_2$ | H | $OCH_3$ | $CH_3$ | N | |
| 1435 | $C_2H_5$ | H | $(CH_3)_2NSO_2$ | H | $OCH_3$ | $CF_3$ | N | |

| Nr. | $R^1$ | $R^4$ | $R^5$ | $R^7$ | X | Y | Z | Fp. (°C) |
|---|---|---|---|---|---|---|---|---|
| 1436 | $C_2H_5$ | H | $(CH_3)_2NSO_2$ | $CH_3$ | $OCH_3$ | $OCH_3$ | CH | |
| 1437 | $C_2H_5$ | H | $(CH_3)_2NSO_2$ | $CH_3$ | $OCH_3$ | $CH_3$ | N | |
| 1438 | $C_2H_5$ | H | $(CH_3)_3C\text{-}OCO$ | H | $OCH_3$ | $OCH_3$ | CH | |
| 1439 | $C_2H_5$ | H | $(CH_3)_3C\text{-}OCO$ | H | $OCH_3$ | $CH_3$ | CH | |
| 1440 | $C_2H_5$ | H | $(CH_3)_3C\text{-}OCO$ | H | $CH_3$ | $CH_3$ | CH | |
| 1441 | $C_2H_5$ | H | $(CH_3)_3C\text{-}OCO$ | H | Cl | $OCH_3$ | CH | |
| 1442 | $C_2H_5$ | H | $(CH_3)_3C\text{-}OCO$ | H | $OCH_3$ | $OCH_3$ | N | |
| 1443 | $C_2H_5$ | H | $(CH_3)_3C\text{-}OCO$ | H | $OCH_3$ | $CH_3$ | N | |
| 1444 | $C_2H_5$ | H | $(CH_3)_3C\text{-}OCO$ | H | $OCH_3$ | $CF_3$ | N | |
| 1445 | $C_2H_5$ | H | $(CH_3)_3C\text{-}OCO$ | $CH_3$ | $OCH_3$ | $OCH_3$ | CH | |
| 1446 | $C_2H_5$ | H | $(CH_3)_3C\text{-}OCO$ | $CH_3$ | $OCH_3$ | $CH_3$ | N | |
| 1447 | $C_2H_5$ | H | PhCO | H | $OCH_3$ | $OCH_3$ | CH | |
| 1448 | $C_2H_5$ | H | PhCO | H | $OCH_3$ | $CH_3$ | CH | |
| 1449 | $C_2H_5$ | H | PhCO | H | $CH_3$ | $CH_3$ | CH | |
| 1450 | $C_2H_5$ | H | PhCO | H | Cl | $OCH_3$ | CH | |
| 1451 | $C_2H_5$ | H | PhCO | H | $OCH_3$ | $OCH_3$ | N | |
| 1452 | $C_2H_5$ | H | PhCO | H | $OCH_3$ | $CH_3$ | N | |
| 1453 | $C_2H_5$ | H | PhCO | H | $OCH_3$ | $CF_3$ | N | |
| 1454 | $C_2H_5$ | H | PhCO | $CH_3$ | $OCH_3$ | $OCH_3$ | CH | |
| 1455 | $C_2H_5$ | H | PhCO | $CH_3$ | $OCH_3$ | $CH_3$ | N | |
| 1456 | $C_2H_5$ | H | $PhSO_2$ | H | $OCH_3$ | $OCH_3$ | CH | |
| 1457 | $C_2H_5$ | H | $PhSO_2$ | H | $OCH_3$ | $CH_3$ | CH | |
| 1458 | $C_2H_5$ | H | $PhSO_2$ | H | $CH_3$ | $CH_3$ | CH | |
| 1459 | $C_2H_5$ | H | $PhSO_2$ | H | Cl | $OCH_3$ | CH | |
| 1460 | $C_2H_5$ | H | $PhSO_2$ | H | $OCH_3$ | $OCH_3$ | N | |
| 1461 | $C_2H_5$ | H | $PhSO_2$ | H | $OCH_3$ | $CH_3$ | N | |
| 1462 | $C_2H_5$ | H | $PhSO_2$ | H | $OCH_3$ | $CF_3$ | N | |
| 1463 | $C_2H_5$ | H | $PhSO_2$ | $CH_3$ | $OCH_3$ | $OCH_3$ | CH | |
| 1464 | $C_2H_5$ | H | $PhSO_2$ | $CH_3$ | $OCH_3$ | $OCH_3$ | N | |
| 1465 | $C_2H_5$ | H | $CH_3NHCO$ | H | $OCH_3$ | $OCH_3$ | CH | |
| 1466 | $C_2H_5$ | H | $CH_3NHCO$ | H | $OCH_3$ | $CH_3$ | CH | |
| 1467 | $C_2H_5$ | H | $CH_3NHCO$ | H | $CH_3$ | $CH_3$ | CH | |
| 1468 | $C_2H_5$ | H | $CH_3NHCO$ | H | Cl | $OCH_3$ | CH | |
| 1469 | $C_2H_5$ | H | $CH_3NHCO$ | H | $OCH_3$ | $OCH_3$ | N | |

| Nr. | $R^1$ | $R^4$ | $R^5$ | $R^7$ | X | Y | Z | Fp. (°C) |
|---|---|---|---|---|---|---|---|---|
| 1470 | $C_2H_5$ | H | $CH_3NHCO$ | H | $OCH_3$ | $CH_3$ | N | |
| 1471 | $C_2H_5$ | H | $CH_3NHCO$ | H | $OCH_3$ | $CF_3$ | N | |
| 1472 | $C_2H_5$ | H | $CH_3NHCO$ | $CH_3$ | $OCH_3$ | $OCH_3$ | CH | |
| 1473 | $C_2H_5$ | H | $CH_3NHCO$ | $CH_3$ | $OCH_3$ | $CH_3$ | N | |
| 1474 | $C_2H_5$ | H | $C_2H_5NHCO$ | H | $OCH_3$ | $OCH_3$ | CH | |
| 1475 | $C_2H_5$ | H | $C_2H_5NHCO$ | H | $OCH_3$ | $CH_3$ | CH | |
| 1476 | $C_2H_5$ | H | $C_2H_5NHCO$ | H | $CH_3$ | $CH_3$ | CH | |
| 1477 | $C_2H_5$ | H | $C_2H_5NHCO$ | H | Cl | $OCH_3$ | CH | |
| 1478 | $C_2H_5$ | H | $C_2H_5NHCO$ | H | $OCH_3$ | $OCH_3$ | N | |
| 1479 | $C_2H_5$ | H | $C_2H_5NHCO$ | H | $OCH_3$ | $CH_3$ | N | |
| 1480 | $C_2H_5$ | H | $C_2H_5NHCO$ | H | $OCH_3$ | $CF_3$ | N | |
| 1481 | $C_2H_5$ | H | $C_2H_5NHCO$ | $CH_3$ | $OCH_3$ | $OCH_3$ | CH | |
| 1482 | $C_2H_5$ | H | $C_2H_5NHCO$ | $CH_3$ | $OCH_3$ | $CH_3$ | N | |
| 1483 | $C_2H_5$ | H | $CH_3NHCS$ | H | $OCH_3$ | $OCH_3$ | CH | |
| 1484 | $C_2H_5$ | H | $CH_3NHCS$ | H | $OCH_3$ | $CH_3$ | CH | |
| 1485 | $C_2H_5$ | H | $CH_3NHCS$ | H | $CH_3$ | $CH_3$ | CH | |
| 1486 | $C_2H_5$ | H | $CH_3NHCS$ | H | Cl | $OCH_3$ | CH | |
| 1487 | $C_2H_5$ | H | $CH_3NHCS$ | H | $OCH_3$ | $OCH_3$ | N | |
| 1488 | $C_2H_5$ | H | $CH_3NHCS$ | H | $OCH_3$ | $CH_3$ | N | |
| 1489 | $C_2H_5$ | H | $CH_3NHCS$ | H | $OCH_3$ | $CF_3$ | N | |
| 1490 | $C_2H_5$ | H | $CH_3NHCS$ | $CH_3$ | $OCH_3$ | $OCH_3$ | CH | |
| 1491 | $C_2H_5$ | H | $CH_3NHCS$ | $CH_3$ | $OCH_3$ | $CH_3$ | N | |
| 1492 | $C_2H_5$ | H | $C_2H_5NHCS$ | H | $OCH_3$ | $OCH_3$ | CH | |
| 1493 | $C_2H_5$ | H | $C_2H_5NHCS$ | H | $OCH_3$ | $CH_3$ | CH | |
| 1494 | $C_2H_5$ | H | $C_2H_5NHCS$ | H | $CH_3$ | $CH_3$ | CH | |
| 1495 | $C_2H_5$ | H | $C_2H_5NHCS$ | H | Cl | $OCH_3$ | CH | |
| 1496 | $C_2H_5$ | H | $C_2H_5NHCS$ | H | $OCH_3$ | $CH_3$ | N | |
| 1497 | $C_2H_5$ | H | $C_2H_5NHCS$ | H | $OCH_3$ | $CF_3$ | N | |
| 1498 | $C_2H_5$ | H | $C_2H_5NHCS$ | H | $OCH_3$ | $OCH_3$ | N | |
| 1499 | $C_2H_5$ | H | $C_2H_5NHCS$ | $CH_3$ | $OCH_3$ | $CH_3$ | CH | |
| 1500 | $C_2H_5$ | H | $C_2H_5NHCS$ | $CH_3$ | $OCH_3$ | $OCH_3$ | N | |

| Nr. | $R^1$ | $R^4$ | $R^5$ | $R^7$ | X | Y | Z | Fp. (°C) |
|---|---|---|---|---|---|---|---|---|
| 1501 | $C_2H_5$ | 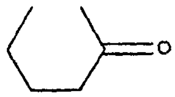 | | H | $OCH_3$ | $OCH_3$ | CH | |
| 1502 | $C_2H_5$ | 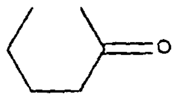 | | H | $OCH_3$ | $CH_3$ | CH | |
| 1503 | $C_2H_5$ | 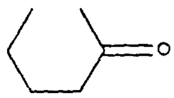 | | H | $CH_3$ | $CH_3$ | CH | |
| 1504 | $C_2H_5$ | 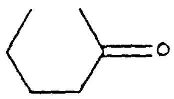 | | H | Cl | $OCH_3$ | CH | |
| 1505 | $C_2H_5$ | 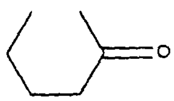 | | H | $OCH_3$ | $OCH_3$ | N | |
| 1506 | $C_2H_5$ | 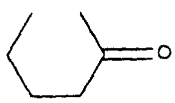 | | H | $OCH_3$ | $CH_3$ | N | |
| 1507 | $C_2H_5$ | 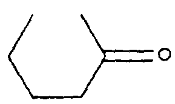 | | H | $OCH_3$ | $CF_3$ | N | |
| 1508 | $C_2H_5$ | 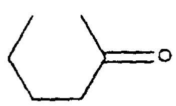 | | $CH_3$ | $OCH_3$ | $OCH_3$ | CH | |
| 1509 | $C_2H_5$ | 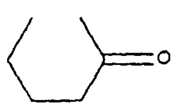 | | H | $OCH_3$ | $OCH_3$ | CH | |

| Nr. | $R^1$ | $R^4$ | $R^5$ | $R^7$ | X | Y | Z | Fp. (°C) |
|---|---|---|---|---|---|---|---|---|
| 1510 | $C_2H_5$ | | 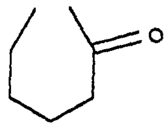 | H | $OCH_3$ | $OCH_3$ | CH | |
| 1511 | $C_2H_5$ | | 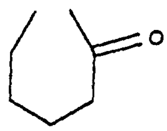 | H | $OCH_3$ | $CH_3$ | CH | |
| 1512 | $C_2H_5$ | | 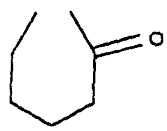 | H | $CH_3$ | $CH_3$ | CH | |
| 1513 | $C_2H_5$ | | 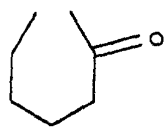 | H | Cl | $OCH_3$ | CH | |
| 1514 | $C_2H_5$ | | 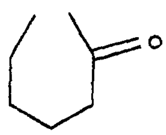 | H | $OCH_3$ | $OCH_3$ | N | |
| 1515 | $C_2H_5$ | | 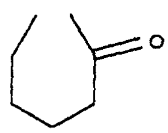 | H | $OCH_3$ | $CH_3$ | N | |
| 1516 | $C_2H_5$ | | 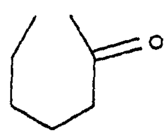 | H | $OCH_3$ | $CF_3$ | N | |
| 1517 | $C_2H_5$ | | 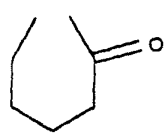 | $CH_3$ | $OCH_3$ | $OCH_3$ | CH | |
| 1518 | $C_2H_5$ | | 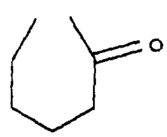 | $CH_3$ | $OCH_3$ | $CH_3$ | N | |
| 1519 | $C_2H_5$ | | 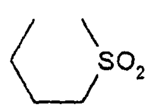 | H | $OCH_3$ | $OCH_3$ | CH | |

| Nr. | $R^1$ | $R^4$ | $R^5$ | $R^7$ | X | Y | Z | Fp. (°C) |
|---|---|---|---|---|---|---|---|---|
| 1520 | $C_2H_5$ | | 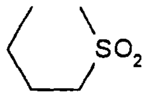 | H | $OCH_3$ | $CH_3$ | CH | |
| 1521 | $C_2H_5$ | | 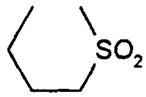 | H | $CH_3$ | $CH_3$ | CH | |
| 1522 | $C_2H_5$ | | 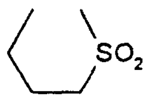 | H | Cl | $OCH_3$ | CH | |
| 1523 | $C_2H_5$ | | 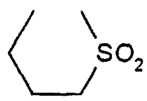 | H | $OCH_3$ | $OCH_3$ | N | |
| 1524 | $C_2H_5$ | | 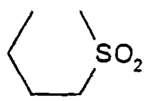 | H | $OCH_3$ | $CH_3$ | N | |
| 1525 | $C_2H_5$ | | 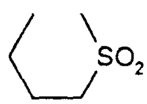 | H | $OCH_3$ | $CF_3$ | N | |
| 1526 | $C_2H_5$ | | 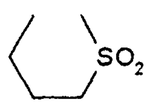 | $CH_3$ | $OCH_3$ | $OCH_3$ | CH | |
| 1527 | $C_2H_5$ | | 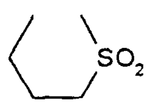 | $CH_3$ | $OCH_3$ | $CH_3$ | N | |
| 1528 | $C_2H_5$ | | 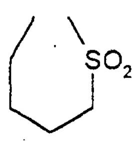 | H | $OCH_3$ | $OCH_3$ | CH | |
| 1529 | $C_2H_5$ | | 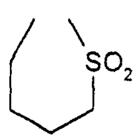 | H | $OCH_3$ | $CH_3$ | CH | |
| 1530 | $C_2H_5$ | | 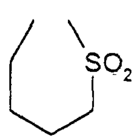 | H | $CH_3$ | $CH_3$ | CH | |

| Nr. | $R^1$ | $R^4$ | $R^5$ | $R^7$ | X | Y | Z | Fp. (°C) |
|---|---|---|---|---|---|---|---|---|
| 1531 | $C_2H_5$ | | 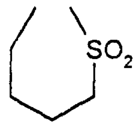 | H | Cl | $OCH_3$ | CH | |
| 1532 | $C_2H_5$ | | 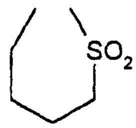 | H | $OCH_3$ | $OCH_3$ | N | |
| 1533 | $C_2H_5$ | | 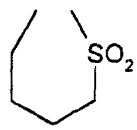 | H | $OCH_3$ | $CH_3$ | N | |
| 1534 | $C_2H_5$ | | 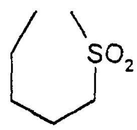 | H | $OCH_3$ | $CF_3$ | N | |
| 1535 | $C_2H_5$ | | 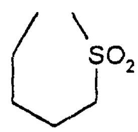 | $CH_3$ | $OCH_3$ | $OCH_3$ | CH | |
| 1536 | $C_2H_5$ | | 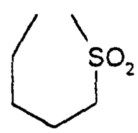 | $CH_3$ | $OCH_3$ | $CH_3$ | N | |
| 1537 | $C_2H_5$ | | 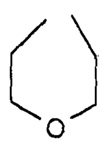 | H | $OCH_3$ | $OCH_3$ | CH | |
| 1538 | $C_2H_5$ | | 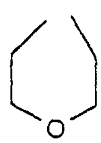 | H | $OCH_3$ | $CH_3$ | CH | |
| 1539 | $C_2H_5$ | | 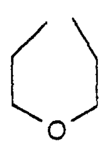 | H | $CH_3$ | $CH_3$ | CH | |
| 1540 | $C_2H_5$ | | 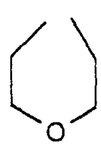 | H | Cl | $OCH_3$ | CH | |

| Nr. | $R^1$ | $R^4$ | $R^5$ | $R^7$ | X | Y | Z | Fp. (°C) |
|---|---|---|---|---|---|---|---|---|
| 1541 | $C_2H_5$ | 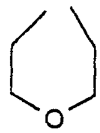 | | H | $OCH_3$ | $CH_3$ | N | |
| 1542 | $C_2H_5$ | 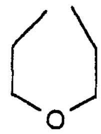 | | H | $OCH_3$ | $CF_3$ | N | |
| 1543 | $C_2H_5$ | 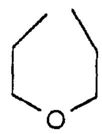 | | H | $OCH_3$ | $OCH_3$ | N | |
| 1544 | $C_2H_5$ | 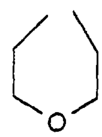 | | $CH_3$ | $OCH_3$ | $CH_3$ | CH | |
| 1545 | $C_2H_5$ | 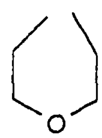 | | $CH_3$ | $OCH_3$ | $OCH_3$ | N | |
| 1546 | $C_2H_5$ | H | $COSCH_3$ | H | $OCH_3$ | $OCH_3$ | CH | |
| 1547 | $C_2H_5$ | H | $COSCH_3$ | H | $OCH_3$ | $CH_3$ | CH | |
| 1548 | $C_2H_5$ | H | $COSCH_3$ | H | $CH_3$ | $CH_3$ | CH | |
| 1549 | $C_2H_5$ | H | $COSCH_3$ | H | Cl | $OCH_3$ | CH | |
| 1550 | $C_2H_5$ | H | $COSCH_3$ | H | $OCH_3$ | $OCH_3$ | N | |
| 1551 | $C_2H_5$ | H | $COSCH_3$ | H | $OCH_3$ | $CH_3$ | N | |
| 1552 | $C_2H_5$ | H | $COSCH_3$ | H | $OCH_3$ | $CF_3$ | N | |
| 1553 | $C_2H_5$ | H | $COSCH_3$ | $CH_3$ | $OCH_3$ | $OCH_3$ | CH | |
| 1554 | $C_2H_5$ | H | $COSCH_3$ | $CH_3$ | $OCH_3$ | $CH_3$ | N | |
| 1555 | $C_2H_5$ | H | $CSOCH_3$ | H | $OCH_3$ | $OCH_3$ | CH | |
| 1556 | $C_2H_5$ | H | $CSOCH_3$ | H | $OCH_3$ | $CH_3$ | CH | |
| 1557 | $C_2H_5$ | H | $CSOCH_3$ | H | $CH_3$ | $CH_3$ | CH | |
| 1558 | $C_2H_5$ | H | $CSOCH_3$ | H | Cl | $OCH_3$ | CH | |
| 1559 | $C_2H_5$ | H | $CSOCH_3$ | H | $OCH_3$ | $OCH_3$ | N | |
| 1560 | $C_2H_5$ | H | $CSOCH_3$ | H | $OCH_3$ | $CH_3$ | N | |
| 1561 | $C_2H_5$ | H | $CSOCH_3$ | H | $OCH_3$ | $CF_3$ | N | |
| 1562 | $C_2H_5$ | H | $CSOCH_3$ | $CH_3$ | $OCH_3$ | $OCH_3$ | CH | |

| Nr. | $R^1$ | $R^4$ | $R^5$ | $R^7$ | X | Y | Z | Fp. (°C) |
|---|---|---|---|---|---|---|---|---|
| 1563 | $C_2H_5$ | H | $CSOCH_3$ | $CH_3$ | $OCH_3$ | $CH_3$ | N | |
| 1564 | $C_2H_5$ | H | $CSSCH_3$ | H | $OCH_3$ | $OCH_3$ | CH | |
| 1565 | $C_2H_5$ | H | $CSSCH_3$ | H | $OCH_3$ | $CH_3$ | CH | |
| 1566 | $C_2H_5$ | H | $CSSCH_3$ | H | $CH_3$ | $CH_3$ | CH | |
| 1567 | $C_2H_5$ | H | $CSSCH_3$ | H | Cl | $OCH_3$ | CH | |
| 1568 | $C_2H_5$ | H | $CSSCH_3$ | H | $OCH_3$ | $OCH_3$ | N | |
| 1569 | $C_2H_5$ | H | $CSSCH_3$ | H | $OCH_3$ | $CH_3$ | N | |
| 1570 | $C_2H_5$ | H | $CSSCH_3$ | H | $OCH_3$ | $CF_3$ | N | |
| 1571 | $C_2H_5$ | H | $CSSCH_3$ | $CH_3$ | $OCH_3$ | $OCH_3$ | CH | |
| 1572 | $C_2H_5$ | H | $CSSCH_3$ | $CH_3$ | $OCH_3$ | $CH_3$ | N | |
| 1573 | $C_2H_5$ | H | $COCOOCH_3$ | H | $OCH_3$ | $OCH_3$ | CH | |
| 1574 | $C_2H_5$ | H | $COCOOCH_3$ | H | $OCH_3$ | $CH_3$ | CH | |
| 1575 | $C_2H_5$ | H | $COCOOCH_3$ | H | $CH_3$ | $CH_3$ | CH | |
| 1576 | $C_2H_5$ | H | $COCOOCH_3$ | H | Cl | $OCH_3$ | CH | |
| 1577 | $C_2H_5$ | H | $COCOOCH_3$ | H | $OCH_3$ | $OCH_3$ | N | |
| 1578 | $C_2H_5$ | H | $COCOOCH_3$ | H | $OCH_3$ | $CH_3$ | N | |
| 1579 | $C_2H_5$ | H | $COCOOCH_3$ | H | $OCH_3$ | $CF_3$ | N | |
| 1580 | $C_2H_5$ | H | $COCOOCH_3$ | $CH_3$ | $OCH_3$ | $OCH_3$ | CH | |
| 1581 | $C_2H_5$ | H | $COCOOCH_3$ | $CH_3$ | $OCH_3$ | $CH_3$ | N | |
| 1582 | $C_2H_5$ | H | $i\text{-}C_3H_7OCO$ | H | $OCH_3$ | $OCH_3$ | CH | |
| 1583 | $C_2H_5$ | H | $i\text{-}C_3H_7OCO$ | H | $OCH_3$ | $CH_3$ | CH | |
| 1584 | $C_2H_5$ | H | $i\text{-}C_3H_7OCO$ | H | $CH_3$ | $CH_3$ | CH | |
| 1585 | $C_2H_5$ | H | $i\text{-}C_3H_7OCO$ | H | Cl | $OCH_3$ | CH | |
| 1586 | $C_2H_5$ | H | $i\text{-}C_3H_7OCO$ | H | $OCH_3$ | $CH_3$ | N | |
| 1587 | $C_2H_5$ | H | $i\text{-}C_3H_7OCO$ | H | $OCH_3$ | $CF_3$ | N | |
| 1588 | $C_2H_5$ | H | $i\text{-}C_3H_7OCO$ | H | $OCH_3$ | $OCH_3$ | N | |
| 1589 | $C_2H_5$ | H | $i\text{-}C_3H_7OCO$ | $CH_3$ | $OCH_3$ | $CH_3$ | CH | |
| 1590 | $C_2H_5$ | H | $i\text{-}C_3H_7OCO$ | $CH_3$ | $OCH_3$ | $OCH_3$ | N | |
| 1591 | $C_2H_5$ | $CH_3$ | CHO | H | $OCH_3$ | $OCH_3$ | CH | 212-213 |
| 1592 | $C_2H_5$ | $CH_3$ | CHO | H | $OCH_3$ | $CH_3$ | CH | |
| 1593 | $C_2H_5$ | $CH_3$ | CHO | H | $CH_3$ | $CH_3$ | CH | |
| 1594 | $C_2H_5$ | $CH_3$ | CHO | H | $CH_3$ | $OC_2H_5$ | CH | |
| 1595 | $C_2H_5$ | $CH_3$ | CHO | H | $C_2H_5$ | $OCH_3$ | CH | |
| 1596 | $C_2H_5$ | $CH_3$ | CHO | H | $OC_2H_5$ | $OC_2H_5$ | CH | |

| Nr. | $R^1$ | $R^4$ | $R^5$ | $R^7$ | X | Y | Z | Fp. (°C) |
|---|---|---|---|---|---|---|---|---|
| 1597 | $C_2H_5$ | $CH_3$ | CHO | H | $OCHF_2$ | $OCHF_2$ | CH | |
| 1598 | $C_2H_5$ | $CH_3$ | CHO | H | $CH_3$ | $OCHF_2$ | CH | |
| 1599 | $C_2H_5$ | $CH_3$ | CHO | H | Cl | $OCH_3$ | CH | |
| 1600 | $C_2H_5$ | $CH_3$ | CHO | H | $OCH_3$ | $OCH_3$ | N | |
| 1601 | $C_2H_5$ | $CH_3$ | CHO | H | $OCH_3$ | $CH_3$ | N | |
| 1602 | $C_2H_5$ | $CH_3$ | CHO | H | $CH_3$ | $CH_3$ | N | |
| 1603 | $C_2H_5$ | $CH_3$ | CHO | H | $N(CH_3)_2$ | $OCH_2CF_3$ | N | |
| 1604 | $C_2H_5$ | $CH_3$ | CHO | H | $OCH_3$ | $CF_3$ | N | |
| 1605 | $C_2H_5$ | $CH_3$ | CHO | H | $OC_2H_5$ | $NHCH_3$ | N | |
| 1606 | $C_2H_5$ | $CH_3$ | CHO | $CH_3$ | $OCH_3$ | $OCH_3$ | CH | |
| 1607 | $C_2H_5$ | $CH_3$ | CHO | $CH_3$ | $OCH_3$ | $CH_3$ | N | |
| 1608 | $C_2H_5$ | $CH_3$ | $COCH_3$ | H | $OCH_3$ | $OCH_3$ | CH | 174-176 |
| 1609 | $C_2H_5$ | $CH_3$ | $COCH_3$ | H | $OCH_3$ | $CH_3$ | CH | |
| 1610 | $C_2H_5$ | $CH_3$ | $COCH_3$ | H | $CH_3$ | $CH_3$ | CH | |
| 1611 | $C_2H_5$ | $CH_3$ | $COCH_3$ | H | $CH_3$ | $OC_2H_5$ | CH | |
| 1612 | $C_2H_5$ | $CH_3$ | $COCH_3$ | H | $C_2H_5$ | $OCH_3$ | CH | |
| 1613 | $C_2H_5$ | $CH_3$ | $COCH_3$ | H | $OC_2H_5$ | $OC_2H_5$ | CH | |
| 1614 | $C_2H_5$ | $CH_3$ | $COCH_3$ | H | $OCHF_2$ | $OCHF_2$ | CH | |
| 1615 | $C_2H_5$ | $CH_3$ | $COCH_3$ | H | $CH_3$ | $OCHF_2$ | CH | |
| 1616 | $C_2H_5$ | $CH_3$ | $COCH_3$ | H | Cl | $OCH_3$ | CH | |
| 1617 | $C_2H_5$ | $CH_3$ | $COCH_3$ | H | $OCH_3$ | $OCH_3$ | N | |
| 1618 | $C_2H_5$ | $CH_3$ | $COCH_3$ | H | $OCH_3$ | $CH_3$ | N | |
| 1619 | $C_2H_5$ | $CH_3$ | $COCH_3$ | H | $CH_3$ | $CH_3$ | N | |
| 1620 | $C_2H_5$ | $CH_3$ | $COCH_3$ | H | $N(CH_3)_2$ | $OCH_2CF_3$ | N | |
| 1621 | $C_2H_5$ | $CH_3$ | $COCH_3$ | H | $OCH_3$ | $CF_3$ | N | |
| 1622 | $C_2H_5$ | $CH_3$ | $COCH_3$ | H | $OC_2H_5$ | $NHCH_3$ | N | |
| 1623 | $C_2H_5$ | $CH_3$ | $COCH_3$ | $CH_3$ | $OCH_3$ | $OCH_3$ | CH | |
| 1624 | $C_2H_5$ | $CH_3$ | $COCH_3$ | $CH_3$ | $OCH_3$ | $CH_3$ | N | |
| 1625 | $C_2H_5$ | $CH_3$ | $COC_2H_5$ | H | $OCH_3$ | $OCH_3$ | CH | |
| 1626 | $C_2H_5$ | $CH_3$ | $COC_2H_5$ | H | $OCH_3$ | $CH_3$ | CH | |
| 1627 | $C_2H_5$ | $CH_3$ | $COC_2H_5$ | H | $CH_3$ | $CH_3$ | CH | |
| 1628 | $C_2H_5$ | $CH_3$ | $COC_2H_5$ | H | $CH_3$ | $OC_2H_5$ | CH | |
| 1629 | $C_2H_5$ | $CH_3$ | $COC_2H_5$ | H | $C_2H_5$ | $OCH_3$ | CH | |
| 1630 | $C_2H_5$ | $CH_3$ | $COC_2H_5$ | H | $OC_2H_5$ | $OC_2H_5$ | CH | |

| Nr. | $R^1$ | $R^4$ | $R^5$ | $R^7$ | X | Y | Z | Fp. (°C) |
|---|---|---|---|---|---|---|---|---|
| 1631 | $C_2H_5$ | $CH_3$ | $COC_2H_5$ | H | $OCHF_2$ | $OCHF_2$ | CH | |
| 1632 | $C_2H_5$ | $CH_3$ | $COC_2H_5$ | H | $CH_3$ | $OCHF_2$ | CH | |
| 1633 | $C_2H_5$ | $CH_3$ | $COC_2H_5$ | H | Cl | $OCH_3$ | CH | |
| 1634 | $C_2H_5$ | $CH_3$ | $COC_2H_5$ | H | $OCH_3$ | $OCH_3$ | N | |
| 1635 | $C_2H_5$ | $CH_3$ | $COC_2H_5$ | H | $OCH_3$ | $CH_3$ | N | |
| 1636 | $C_2H_5$ | $CH_3$ | $COC_2H_5$ | H | $CH_3$ | $CH_3$ | N | |
| 1637 | $C_2H_5$ | $CH_3$ | $COC_2H_5$ | H | $N(CH_3)_2$ | $OCH_2CF_3$ | N | |
| 1638 | $C_2H_5$ | $CH_3$ | $COC_2H_5$ | H | $OCH_3$ | $CF_3$ | N | |
| 1639 | $C_2H_5$ | $CH_3$ | $COC_2H_5$ | H | $OC_2H_5$ | $NHCH_3$ | N | |
| 1640 | $C_2H_5$ | $CH_3$ | $COC_2H_5$ | $CH_3$ | $OCH_3$ | $OCH_3$ | CH | |
| 1641 | $C_2H_5$ | $CH_3$ | $COC_2H_5$ | $CH_3$ | $OCH_3$ | $CH_3$ | N | |
| 1642 | $C_2H_5$ | $CH_3$ | $CO\text{-}n\text{-}C_3H_7$ | H | $OCH_3$ | $OCH_3$ | CH | |
| 1643 | $C_2H_5$ | $CH_3$ | $CO\text{-}n\text{-}C_3H_7$ | H | $OCH_3$ | $CH_3$ | CH | |
| 1644 | $C_2H_5$ | $CH_3$ | $CO\text{-}n\text{-}C_3H_7$ | H | $CH_3$ | $CH_3$ | CH | |
| 1645 | $C_2H_5$ | $CH_3$ | $CO\text{-}n\text{-}C_3H_7$ | H | $CH_3$ | $OC_2H_5$ | CH | |
| 1646 | $C_2H_5$ | $CH_3$ | $CO\text{-}n\text{-}C_3H_7$ | H | $C_2H_5$ | $OCH_3$ | CH | |
| 1647 | $C_2H_5$ | $CH_3$ | $CO\text{-}n\text{-}C_3H_7$ | H | $OC_2H_5$ | $OC_2H_5$ | CH | |
| 1648 | $C_2H_5$ | $CH_3$ | $CO\text{-}n\text{-}C_3H_7$ | H | $OCHF_2$ | $OCHF_2$ | CH | |
| 1649 | $C_2H_5$ | $CH_3$ | $CO\text{-}n\text{-}C_3H_7$ | H | $CH_3$ | $OCHF_2$ | CH | |
| 1650 | $C_2H_5$ | $CH_3$ | $CO\text{-}n\text{-}C_3H_7$ | H | Cl | $OCH_3$ | CH | |
| 1651 | $C_2H_5$ | $CH_3$ | $CO\text{-}n\text{-}C_3H_7$ | H | $OCH_3$ | $OCH_3$ | N | |
| 1652 | $C_2H_5$ | $CH_3$ | $CO\text{-}n\text{-}C_3H_7$ | H | $OCH_3$ | $CH_3$ | N | |
| 1653 | $C_2H_5$ | $CH_3$ | $CO\text{-}n\text{-}C_3H_7$ | H | $CH_3$ | $CH_3$ | N | |
| 1654 | $C_2H_5$ | $CH_3$ | $CO\text{-}n\text{-}C_3H_7$ | H | $N(CH_3)_2$ | $OCH_2CF_3$ | N | |
| 1655 | $C_2H_5$ | $CH_3$ | $CO\text{-}n\text{-}C_3H_7$ | H | $OCH_3$ | $CF_3$ | N | |
| 1656 | $C_2H_5$ | $CH_3$ | $CO\text{-}n\text{-}C_3H_7$ | H | $OC_2H_5$ | $NHCH_3$ | N | |
| 1657 | $C_2H_5$ | $CH_3$ | $CO\text{-}n\text{-}C_3H_7$ | $CH_3$ | $OCH_3$ | $OCH_3$ | CH | |
| 1658 | $C_2H_5$ | $CH_3$ | $CO\text{-}n\text{-}C_3H_7$ | $CH_3$ | $OCH_3$ | $CH_3$ | N | |
| 1659 | $C_2H_5$ | $CH_3$ | $CO\text{-}i\text{-}C_3H_7$ | H | $OCH_3$ | $OCH_3$ | CH | |
| 1660 | $C_2H_5$ | $CH_3$ | $CO\text{-}i\text{-}C_3H_7$ | H | $OCH_3$ | $CH_3$ | CH | |
| 1661 | $C_2H_5$ | $CH_3$ | $CO\text{-}i\text{-}C_3H_7$ | H | $CH_3$ | $CH_3$ | CH | |
| 1662 | $C_2H_5$ | $CH_3$ | $CO\text{-}i\text{-}C_3H_7$ | H | $CH_3$ | $OC_2H_5$ | CH | |
| 1663 | $C_2H_5$ | $CH_3$ | $CO\text{-}i\text{-}C_3H_7$ | H | $C_2H_5$ | $OCH_3$ | CH | |
| 1664 | $C_2H_5$ | $CH_3$ | $CO\text{-}i\text{-}C_3H_7$ | H | $OC_2H_5$ | $OC_2H_5$ | CH | |

| Nr. | $R^1$ | $R^4$ | $R^5$ | $R^7$ | X | Y | Z | Fp. (°C) |
|---|---|---|---|---|---|---|---|---|
| 1665 | $C_2H_5$ | $CH_3$ | CO-i-$C_3H_7$ | H | $OCHF_2$ | $OCHF_2$ | CH | |
| 1666 | $C_2H_5$ | $CH_3$ | CO-i-$C_3H_7$ | H | $CH_3$ | $OCHF_2$ | CH | |
| 1667 | $C_2H_5$ | $CH_3$ | CO-i-$C_3H_7$ | H | Cl | $OCH_3$ | CH | |
| 1668 | $C_2H_5$ | $CH_3$ | CO-i-$C_3H_7$ | H | $OCH_3$ | $OCH_3$ | N | |
| 1669 | $C_2H_5$ | $CH_3$ | CO-i-$C_3H_7$ | H | $OCH_3$ | $CH_3$ | N | |
| 1670 | $C_2H_5$ | $CH_3$ | CO-i-$C_3H_7$ | H | $CH_3$ | $CH_3$ | N | |
| 1671 | $C_2H_5$ | $CH_3$ | CO-i-$C_3H_7$ | H | $N(CH_3)_2$ | $OCH_2CF_3$ | N | |
| 1672 | $C_2H_5$ | $CH_3$ | CO-i-$C_3H_7$ | H | $OCH_3$ | $CF_3$ | N | |
| 1673 | $C_2H_5$ | $CH_3$ | CO-i-$C_3H_7$ | H | $OC_2H_5$ | $NHCH_3$ | N | |
| 1674 | $C_2H_5$ | $CH_3$ | CO-i-$C_3H_7$ | $CH_3$ | $OCH_3$ | $OCH_3$ | CH | |
| 1675 | $C_2H_5$ | $CH_3$ | CO-i-$C_3H_7$ | $CH_3$ | $OCH_3$ | $CH_3$ | N | |
| 1676 | $C_2H_5$ | $CH_3$ | $COCF_3$ | H | $OCH_3$ | $OCH_3$ | CH | 201-202 |
| 1677 | $C_2H_5$ | $CH_3$ | $COCF_3$ | H | $OCH_3$ | $CH_3$ | CH | |
| 1678 | $C_2H_5$ | $CH_3$ | $COCF_3$ | H | $CH_3$ | $CH_3$ | CH | |
| 1679 | $C_2H_5$ | $CH_3$ | $COCF_3$ | H | $CH_3$ | $OC_2H_5$ | CH | |
| 1680 | $C_2H_5$ | $CH_3$ | $COCF_3$ | H | $C_2H_5$ | $OCH_3$ | CH | |
| 1681 | $C_2H_5$ | $CH_3$ | $COCF_3$ | H | $OC_2H_5$ | $OC_2H_5$ | CH | |
| 1682 | $C_2H_5$ | $CH_3$ | $COCF_3$ | H | $OCHF_2$ | $OCHF_2$ | CH | |
| 1683 | $C_2H_5$ | $CH_3$ | $COCF_3$ | H | $CH_3$ | $OCHF_2$ | CH | |
| 1684 | $C_2H_5$ | $CH_3$ | $COCF_3$ | H | Cl | $OCH_3$ | CH | |
| 1685 | $C_2H_5$ | $CH_3$ | $COCF_3$ | H | $OCH_3$ | $OCH_3$ | N | |
| 1686 | $C_2H_5$ | $CH_3$ | $COCF_3$ | H | $OCH_3$ | $CH_3$ | N | |
| 1687 | $C_2H_5$ | $CH_3$ | $COCF_3$ | H | $CH_3$ | $CH_3$ | N | |
| 1688 | $C_2H_5$ | $CH_3$ | $COCF_3$ | H | $N(CH_3)_2$ | $OCH_2CF_3$ | N | |
| 1689 | $C_2H_5$ | $CH_3$ | $COCF_3$ | H | $OCH_3$ | $CF_3$ | N | |
| 1690 | $C_2H_5$ | $CH_3$ | $COCF_3$ | H | $OC_2H_5$ | $NHCH_3$ | N | |
| 1691 | $C_2H_5$ | $CH_3$ | $COCF_3$ | $CH_3$ | $OCH_3$ | $OCH_3$ | CH | |
| 1692 | $C_2H_5$ | $CH_3$ | $COCF_3$ | $CH_3$ | $OCH_3$ | $CH_3$ | N | |
| 1693 | $C_2H_5$ | $CH_3$ | $COOCH_3$ | H | $OCH_3$ | $OCH_3$ | CH | |
| 1694 | $C_2H_5$ | $CH_3$ | $COOCH_3$ | H | $OCH_3$ | $CH_3$ | CH | |
| 1695 | $C_2H_5$ | $CH_3$ | $COOCH_3$ | H | $CH_3$ | $CH_3$ | CH | |
| 1696 | $C_2H_5$ | $CH_3$ | $COOCH_3$ | H | $CH_3$ | $OC_2H_5$ | CH | |
| 1697 | $C_2H_5$ | $CH_3$ | $COOCH_3$ | H | $C_2H_5$ | $OCH_3$ | CH | |
| 1698 | $C_2H_5$ | $CH_3$ | $COOCH_3$ | H | $OC_2H_5$ | $OC_2H_5$ | CH | |

| Nr. | $R^1$ | $R^4$ | $R^5$ | $R^7$ | X | Y | Z | Fp. (°C) |
|---|---|---|---|---|---|---|---|---|
| 1699 | $C_2H_5$ | $CH_3$ | $COOCH_3$ | H | $OCHF_2$ | $OCHF_2$ | CH | |
| 1700 | $C_2H_5$ | $CH_3$ | $COOCH_3$ | H | $CH_3$ | $OCHF_2$ | CH | |
| 1701 | $C_2H_5$ | $CH_3$ | $COOCH_3$ | H | Cl | $OCH_3$ | CH | |
| 1702 | $C_2H_5$ | $CH_3$ | $COOCH_3$ | H | $OCH_3$ | $OCH_3$ | N | |
| 1703 | $C_2H_5$ | $CH_3$ | $COOCH_3$ | H | $OCH_3$ | $CH_3$ | N | |
| 1704 | $C_2H_5$ | $CH_3$ | $COOCH_3$ | H | $CH_3$ | $CH_3$ | N | |
| 1705 | $C_2H_5$ | $CH_3$ | $COOCH_3$ | H | $N(CH_3)_2$ | $OCH_2CF_3$ | N | |
| 1706 | $C_2H_5$ | $CH_3$ | $COOCH_3$ | H | $OCH_3$ | $CF_3$ | N | |
| 1707 | $C_2H_5$ | $CH_3$ | $COOCH_3$ | H | $OC_2H_5$ | $NHCH_3$ | N | |
| 1708 | $C_2H_5$ | $CH_3$ | $COOCH_3$ | $CH_3$ | $OCH_3$ | $OCH_3$ | CH | |
| 1709 | $C_2H_5$ | $CH_3$ | $COOCH_3$ | $CH_3$ | $OCH_3$ | $CH_3$ | N | |
| 1710 | $C_2H_5$ | $CH_3$ | $COOC_2H_5$ | H | $OCH_3$ | $OCH_3$ | CH | |
| 1711 | $C_2H_5$ | $CH_3$ | $COOC_2H_5$ | H | $OCH_3$ | $CH_3$ | CH | |
| 1712 | $C_2H_5$ | $CH_3$ | $COOC_2H_5$ | H | $CH_3$ | $CH_3$ | CH | |
| 1713 | $C_2H_5$ | $CH_3$ | $COOC_2H_5$ | H | $CH_3$ | $OC_2H_5$ | CH | |
| 1714 | $C_2H_5$ | $CH_3$ | $COOC_2H_5$ | H | $C_2H_5$ | $OCH_3$ | CH | |
| 1715 | $C_2H_5$ | $CH_3$ | $COOC_2H_5$ | H | $OC_2H_5$ | $OC_2H_5$ | CH | |
| 1716 | $C_2H_5$ | $CH_3$ | $COOC_2H_5$ | H | $OCHF_2$ | $OCHF_2$ | CH | |
| 1717 | $C_2H_5$ | $CH_3$ | $COOC_2H_5$ | H | $CH_3$ | $OCHF_2$ | CH | |
| 1718 | $C_2H_5$ | $CH_3$ | $COOC_2H_5$ | H | Cl | $OCH_3$ | CH | |
| 1719 | $C_2H_5$ | $CH_3$ | $COOC_2H_5$ | H | $OCH_3$ | $OCH_3$ | N | |
| 1720 | $C_2H_5$ | $CH_3$ | $COOC_2H_5$ | H | $OCH_3$ | $CH_3$ | N | |
| 1721 | $C_2H_5$ | $CH_3$ | $COOC_2H_5$ | H | $CH_3$ | $CH_3$ | N | |
| 1722 | $C_2H_5$ | $CH_3$ | $COOC_2H_5$ | H | $N(CH_3)_2$ | $OCH_2CF_3$ | N | |
| 1723 | $C_2H_5$ | $CH_3$ | $COOC_2H_5$ | H | $OCH_3$ | $CF_3$ | N | |
| 1724 | $C_2H_5$ | $CH_3$ | $COOC_2H_5$ | H | $OC_2H_5$ | $NHCH_3$ | N | |
| 1725 | $C_2H_5$ | $CH_3$ | $COOC_2H_5$ | $CH_3$ | $OCH_3$ | $OCH_3$ | CH | |
| 1726 | $C_2H_5$ | $CH_3$ | $COOC_2H_5$ | $CH_3$ | $OCH_3$ | $CH_3$ | N | |
| 1727 | $C_2H_5$ | $CH_3$ | $SO_2CH_3$ | H | $OCH_3$ | $OCH_3$ | CH | 195-196 |
| 1728 | $C_2H_5$ | $CH_3$ | $SO_2CH_3$ | H | $OCH_3$ | $CH_3$ | CH | |
| 1729 | $C_2H_5$ | $CH_3$ | $SO_2CH_3$ | H | $CH_3$ | $CH_3$ | CH | |
| 1730 | $C_2H_5$ | $CH_3$ | $SO_2CH_3$ | H | $CH_3$ | $OC_2H_5$ | CH | |
| 1731 | $C_2H_5$ | $CH_3$ | $SO_2CH_3$ | H | $C_2H_5$ | $OCH_3$ | CH | |
| 1732 | $C_2H_5$ | $CH_3$ | $SO_2CH_3$ | H | $OC_2H_5$ | $OC_2H_5$ | CH | |

| Nr. | $R^1$ | $R^4$ | $R^5$ | $R^7$ | X | Y | Z | Fp. (°C) |
|---|---|---|---|---|---|---|---|---|
| 1733 | $C_2H_5$ | $CH_3$ | $SO_2CH_3$ | H | $OCHF_2$ | $OCHF_2$ | CH | |
| 1734 | $C_2H_5$ | $CH_3$ | $SO_2CH_3$ | H | $CH_3$ | $OCHF_2$ | CH | |
| 1735 | $C_2H_5$ | $CH_3$ | $SO_2CH_3$ | H | Cl | $OCH_3$ | CH | |
| 1736 | $C_2H_5$ | $CH_3$ | $SO_2CH_3$ | H | $OCH_3$ | $OCH_3$ | N | |
| 1737 | $C_2H_5$ | $CH_3$ | $SO_2CH_3$ | H | $OCH_3$ | $CH_3$ | N | |
| 1738 | $C_2H_5$ | $CH_3$ | $SO_2CH_3$ | H | $CH_3$ | $CH_3$ | N | |
| 1739 | $C_2H_5$ | $CH_3$ | $SO_2CH_3$ | H | $N(CH_3)_2$ | $OCH_2CF_3$ | N | |
| 1740 | $C_2H_5$ | $CH_3$ | $SO_2CH_3$ | H | $OCH_3$ | $CF_3$ | N | |
| 1741 | $C_2H_5$ | $CH_3$ | $SO_2CH_3$ | H | $OC_2H_5$ | $NHCH_3$ | N | |
| 1742 | $C_2H_5$ | $CH_3$ | $SO_2CH_3$ | $CH_3$ | $OCH_3$ | $OCH_3$ | CH | |
| 1743 | $C_2H_5$ | $CH_3$ | $SO_2CH_3$ | $CH_3$ | $OCH_3$ | $CH_3$ | N | |
| 1744 | $C_2H_5$ | $CH_3$ | $SO_2C_2H_5$ | H | $OCH_3$ | $OCH_3$ | CH | |
| 1745 | $C_2H_5$ | $CH_3$ | $SO_2C_2H_5$ | H | $OCH_3$ | $CH_3$ | CH | |
| 1746 | $C_2H_5$ | $CH_3$ | $SO_2C_2H_5$ | H | $CH_3$ | $CH_3$ | CH | |
| 1747 | $C_2H_5$ | $CH_3$ | $SO_2C_2H_5$ | H | $CH_3$ | $OC_2H_5$ | CH | |
| 1748 | $C_2H_5$ | $CH_3$ | $SO_2C_2H_5$ | H | $C_2H_5$ | $OCH_3$ | CH | |
| 1749 | $C_2H_5$ | $CH_3$ | $SO_2C_2H_5$ | H | $OC_2H_5$ | $OC_2H_5$ | CH | |
| 1750 | $C_2H_5$ | $CH_3$ | $SO_2C_2H_5$ | H | $OCHF_2$ | $OCHF_2$ | CH | |
| 1751 | $C_2H_5$ | $CH_3$ | $SO_2C_2H_5$ | H | $CH_3$ | $OCHF_2$ | CH | |
| 1752 | $C_2H_5$ | $CH_3$ | $SO_2C_2H_5$ | H | Cl | $OCH_3$ | CH | |
| 1753 | $C_2H_5$ | $CH_3$ | $SO_2C_2H_5$ | H | $OCH_3$ | $OCH_3$ | N | |
| 1754 | $C_2H_5$ | $CH_3$ | $SO_2C_2H_5$ | H | $OCH_3$ | $CH_3$ | N | |
| 1755 | $C_2H_5$ | $CH_3$ | $SO_2C_2H_5$ | H | $CH_3$ | $CH_3$ | N | |
| 1756 | $C_2H_5$ | $CH_3$ | $SO_2C_2H_5$ | H | $N(CH_3)_2$ | $OCH_2CF_3$ | N | |
| 1757 | $C_2H_5$ | $CH_3$ | $SO_2C_2H_5$ | H | $OCH_3$ | $CF_3$ | N | |
| 1758 | $C_2H_5$ | $CH_3$ | $SO_2C_2H_5$ | H | $OC_2H_5$ | $NHCH_3$ | N | |
| 1759 | $C_2H_5$ | $CH_3$ | $SO_2C_2H_5$ | $CH_3$ | $OCH_3$ | $OCH_3$ | CH | |
| 1760 | $C_2H_5$ | $CH_3$ | $SO_2C_2H_5$ | $CH_3$ | $OCH_3$ | $CH_3$ | N | |
| 1761 | $C_2H_5$ | $CH_3$ | $SO_2$-n-$C_3H_7$ | H | $OCH_3$ | $OCH_3$ | CH | |
| 1762 | $C_2H_5$ | $CH_3$ | $SO_2$-n-$C_3H_7$ | H | $OCH_3$ | $CH_3$ | CH | |
| 1763 | $C_2H_5$ | $CH_3$ | $SO_2$-n-$C_3H_7$ | H | $CH_3$ | $CH_3$ | CH | |
| 1764 | $C_2H_5$ | $CH_3$ | $SO_2$-n-$C_3H_7$ | H | $CH_3$ | $OC_2H_5$ | CH | |
| 1765 | $C_2H_5$ | $CH_3$ | $SO_2$-n-$C_3H_7$ | H | $C_2H_5$ | $OCH_3$ | CH | |
| 1766 | $C_2H_5$ | $CH_3$ | $SO_2$-n-$C_3H_7$ | H | $OC_2H_5$ | $OC_2H_5$ | CH | |

| Nr. | $R^1$ | $R^4$ | $R^5$ | $R^7$ | X | Y | Z | Fp. (°C) |
|---|---|---|---|---|---|---|---|---|
| 1767 | $C_2H_5$ | $CH_3$ | $SO_2$-n-$C_3H_7$ | H | $OCHF_2$ | $OCHF_2$ | CH | |
| 1768 | $C_2H_5$ | $CH_3$ | $SO_2$-n-$C_3H_7$ | H | $CH_3$ | $OCHF_2$ | CH | |
| 1769 | $C_2H_5$ | $CH_3$ | $SO_2$-n-$C_3H_7$ | H | Cl | $OCH_3$ | CH | |
| 1770 | $C_2H_5$ | $CH_3$ | $SO_2$-n-$C_3H_7$ | H | $OCH_3$ | $OCH_3$ | N | |
| 1771 | $C_2H_5$ | $CH_3$ | $SO_2$-n-$C_3H_7$ | H | $OCH_3$ | $CH_3$ | N | |
| 1772 | $C_2H_5$ | $CH_3$ | $SO_2$-n-$C_3H_7$ | H | $CH_3$ | $CH_3$ | N | |
| 1773 | $C_2H_5$ | $CH_3$ | $SO_2$-n-$C_3H_7$ | H | $N(CH_3)_2$ | $OCH_2CF_3$ | N | |
| 1774 | $C_2H_5$ | $CH_3$ | $SO_2$-n-$C_3H_7$ | H | $OCH_3$ | $CF_3$ | N | |
| 1775 | $C_2H_5$ | $CH_3$ | $SO_2$-n-$C_3H_7$ | H | $OC_2H_5$ | $NHCH_3$ | N | |
| 1776 | $C_2H_5$ | $CH_3$ | $SO_2$-n-$C_3H_7$ | $CH_3$ | $OCH_3$ | $OCH_3$ | CH | |
| 1777 | $C_2H_5$ | $CH_3$ | $SO_2$-n-$C_3H_7$ | $CH_3$ | $OCH_3$ | $CH_3$ | N | |
| 1778 | $C_2H_5$ | $CH_3$ | $SO_2$-i-$C_3H_7$ | H | $OCH_3$ | $OCH_3$ | CH | |
| 1779 | $C_2H_5$ | $CH_3$ | $SO_2$-i-$C_3H_7$ | H | $OCH_3$ | $CH_3$ | CH | |
| 1780 | $C_2H_5$ | $CH_3$ | $SO_2$-i-$C_3H_7$ | H | $CH_3$ | $CH_3$ | CH | |
| 1781 | $C_2H_5$ | $CH_3$ | $SO_2$-i-$C_3H_7$ | H | $CH_3$ | $OC_2H_5$ | CH | |
| 1782 | $C_2H_5$ | $CH_3$ | $SO_2$-i-$C_3H_7$ | H | $C_2H_5$ | $OCH_3$ | CH | |
| 1783 | $C_2H_5$ | $CH_3$ | $SO_2$-i-$C_3H_7$ | H | $OC_2H_5$ | $OC_2H_5$ | CH | |
| 1784 | $C_2H_5$ | $CH_3$ | $SO_2$-i-$C_3H_7$ | H | $OCHF_2$ | $OCHF_2$ | CH | |
| 1785 | $C_2H_5$ | $CH_3$ | $SO_2$-i-$C_3H_7$ | H | $CH_3$ | $OCHF_2$ | CH | |
| 1786 | $C_2H_5$ | $CH_3$ | $SO_2$-i-$C_3H_7$ | H | Cl | $OCH_3$ | CH | |
| 1787 | $C_2H_5$ | $CH_3$ | $SO_2$-i-$C_3H_7$ | H | $OCH_3$ | $OCH_3$ | N | |
| 1788 | $C_2H_5$ | $CH_3$ | $SO_2$-i-$C_3H_7$ | H | $OCH_3$ | $CH_3$ | N | |
| 1789 | $C_2H_5$ | $CH_3$ | $SO_2$-i-$C_3H_7$ | H | $CH_3$ | $CH_3$ | N | |
| 1790 | $C_2H_5$ | $CH_3$ | $SO_2$-i-$C_3H_7$ | H | $N(CH_3)_2$ | $OCH_2CF_3$ | N | |
| 1791 | $C_2H_5$ | $CH_3$ | $SO_2$-i-$C_3H_7$ | H | $OCH_3$ | $CF_3$ | N | |
| 1792 | $C_2H_5$ | $CH_3$ | $SO_2$-i-$C_3H_7$ | H | $OC_2H_5$ | $NHCH_3$ | N | |
| 1793 | $C_2H_5$ | $CH_3$ | $SO_2$-i-$C_3H_7$ | $CH_3$ | $OCH_3$ | $OCH_3$ | CH | |
| 1794 | $C_2H_5$ | $CH_3$ | $SO_2$-i-$C_3H_7$ | $CH_3$ | $OCH_3$ | $CH_3$ | N | |
| 1795 | $C_2H_5$ | $CH_3$ | $ClCH_2CO$ | H | $OCH_3$ | $OCH_3$ | CH | |
| 1796 | $C_2H_5$ | $CH_3$ | $ClCH_2CO$ | H | $OCH_3$ | $CH_3$ | CH | |
| 1797 | $C_2H_5$ | $CH_3$ | $ClCH_2CO$ | H | $CH_3$ | $CH_3$ | CH | |
| 1798 | $C_2H_5$ | $CH_3$ | $ClCH_2CO$ | H | Cl | $OCH_3$ | CH | |
| 1799 | $C_2H_5$ | $CH_3$ | $ClCH_2CO$ | H | $OCH_3$ | $OCH_3$ | N | |
| 1800 | $C_2H_5$ | $CH_3$ | $ClCH_2CO$ | H | $OCH_3$ | $CH_3$ | N | |

| Nr. | $R^1$ | $R^4$ | $R^5$ | $R^7$ | X | Y | Z | Fp. (°C) |
|---|---|---|---|---|---|---|---|---|
| 1801 | $C_2H_5$ | $CH_3$ | $ClCH_2CO$ | H | $OCH_3$ | $CF_3$ | N | |
| 1802 | $C_2H_5$ | $CH_3$ | $ClCH_2CO$ | $CH_3$ | $OCH_3$ | $OCH_3$ | CH | |
| 1803 | $C_2H_5$ | $CH_3$ | $ClCH_2CO$ | $CH_3$ | $OCH_3$ | $CH_3$ | N | |
| 1804 | $C_2H_5$ | $CH_3$ | $Cl_2CHCO$ | H | $OCH_3$ | $OCH_3$ | CH | |
| 1805 | $C_2H_5$ | $CH_3$ | $Cl_2CHCO$ | H | $OCH_3$ | $CH_3$ | CH | |
| 1806 | $C_2H_5$ | $CH_3$ | $Cl_2CHCO$ | H | $CH_3$ | $CH_3$ | CH | |
| 1807 | $C_2H_5$ | $CH_3$ | $Cl_2CHCO$ | H | Cl | $OCH_3$ | CH | |
| 1808 | $C_2H_5$ | $CH_3$ | $Cl_2CHCO$ | H | $OCH_3$ | $OCH_3$ | N | |
| 1809 | $C_2H_5$ | $CH_3$ | $Cl_2CHCO$ | H | $OCH_3$ | $CH_3$ | N | |
| 1810 | $C_2H_5$ | $CH_3$ | $Cl_2CHCO$ | H | $OCH_3$ | $CF_3$ | N | |
| 1811 | $C_2H_5$ | $CH_3$ | $Cl_2CHCO$ | $CH_3$ | $OCH_3$ | $OCH_3$ | CH | |
| 1812 | $C_2H_5$ | $CH_3$ | $Cl_2CHCO$ | $CH_3$ | $OCH_3$ | $OCH_3$ | N | |
| 1813 | $C_2H_5$ | $CH_3$ | $Cl_3CCO$ | H | $OCH_3$ | $OCH_3$ | CH | |
| 1814 | $C_2H_5$ | $CH_3$ | $Cl_3CCO$ | H | $OCH_3$ | $CH_3$ | CH | |
| 1815 | $C_2H_5$ | $CH_3$ | $Cl_3CCO$ | H | $CH_3$ | $CH_3$ | CH | |
| 1816 | $C_2H_5$ | $CH_3$ | $Cl_3CCO$ | H | Cl | $OCH_3$ | CH | |
| 1817 | $C_2H_5$ | $CH_3$ | $Cl_3CCO$ | H | $OCH_3$ | $OCH_3$ | N | |
| 1818 | $C_2H_5$ | $CH_3$ | $Cl_3CCO$ | H | $OCH_3$ | $CH_3$ | N | |
| 1819 | $C_2H_5$ | $CH_3$ | $Cl_3CCO$ | H | $OCH_3$ | $CF_3$ | N | |
| 1820 | $C_2H_5$ | $CH_3$ | $Cl_3CCO$ | $CH_3$ | $OCH_3$ | $OCH_3$ | CH | |
| 1821 | $C_2H_5$ | $CH_3$ | $Cl_3CCO$ | $CH_3$ | $OCH_3$ | $CH_3$ | N | |
| 1822 | $C_2H_5$ | $CH_3$ | $CH_3OCH_2CO$ | H | $OCH_3$ | $OCH_3$ | CH | |
| 1823 | $C_2H_5$ | $CH_3$ | $CH_3OCH_2CO$ | H | $OCH_3$ | $CH_3$ | CH | |
| 1824 | $C_2H_5$ | $CH_3$ | $CH_3OCH_2CO$ | H | $CH_3$ | $CH_3$ | CH | |
| 1825 | $C_2H_5$ | $CH_3$ | $CH_3OCH_2CO$ | H | Cl | $OCH_3$ | CH | |
| 1826 | $C_2H_5$ | $CH_3$ | $CH_3OCH_2CO$ | H | $OCH_3$ | $OCH_3$ | N | |
| 1827 | $C_2H_5$ | $CH_3$ | $CH_3OCH_2CO$ | H | $OCH_3$ | $CH_3$ | N | |
| 1828 | $C_2H_5$ | $CH_3$ | $CH_3OCH_2CO$ | H | $OCH_3$ | $CF_3$ | N | |
| 1829 | $C_2H_5$ | $CH_3$ | $CH_3OCH_2CO$ | $CH_3$ | $OCH_3$ | $OCH_3$ | CH | |
| 1830 | $C_2H_5$ | $CH_3$ | $CH_3OCH_2CO$ | $CH_3$ | $OCH_3$ | $CH_3$ | N | |
| 1831 | $C_2H_5$ | $CH_3$ | $CH_2$=CHCO | H | $OCH_3$ | $OCH_3$ | CH | |
| 1832 | $C_2H_5$ | $CH_3$ | $CH_2$=CHCO | H | $OCH_3$ | $CH_3$ | CH | |
| 1833 | $C_2H_5$ | $CH_3$ | $CH_2$=CHCO | H | $CH_3$ | $CH_3$ | CH | |
| 1834 | $C_2H_5$ | $CH_3$ | $CH_2$=CHCO | H | Cl | $OCH_3$ | CH | |

| Nr. | $R^1$ | $R^4$ | $R^5$ | $R^7$ | X | Y | Z | Fp. (°C) |
|---|---|---|---|---|---|---|---|---|
| 1835 | $C_2H_5$ | $CH_3$ | $CH_2$=CHCO | H | $OCH_3$ | $OCH_3$ | N | |
| 1836 | $C_2H_5$ | $CH_3$ | $CH_2$=CHCO | H | $OCH_3$ | $CH_3$ | N | |
| 1837 | $C_2H_5$ | $CH_3$ | $CH_2$=CHCO | H | $OCH_3$ | $CF_3$ | N | |
| 1838 | $C_2H_5$ | $CH_3$ | $CH_2$=CHCO | $CH_3$ | $OCH_3$ | $OCH_3$ | CH | |
| 1839 | $C_2H_5$ | $CH_3$ | $CH_2$=CHCO | $CH_3$ | $OCH_3$ | $CH_3$ | N | |
| 1840 | $C_2H_5$ | $CH_3$ | CH≡CCO | H | $OCH_3$ | $OCH_3$ | CH | |
| 1841 | $C_2H_5$ | $CH_3$ | CH≡CCO | H | $OCH_3$ | $CH_3$ | CH | |
| 1842 | $C_2H_5$ | $CH_3$ | CH≡CCO | H | $CH_3$ | $CH_3$ | CH | |
| 1843 | $C_2H_5$ | $CH_3$ | CH≡CCO | H | Cl | $OCH_3$ | CH | |
| 1844 | $C_2H_5$ | $CH_3$ | CH≡CCO | H | $OCH_3$ | $CH_3$ | N | |
| 1845 | $C_2H_5$ | $CH_3$ | CH≡CCO | H | $OCH_3$ | $CF_3$ | N | |
| 1846 | $C_2H_5$ | $CH_3$ | CH≡CCO | H | $OCH_3$ | $OCH_3$ | N | |
| 1847 | $C_2H_5$ | $CH_3$ | CH≡CCO | $CH_3$ | $OCH_3$ | $OCH_3$ | CH | |
| 1848 | $C_2H_5$ | $CH_3$ | CH≡CCO | $CH_3$ | $OCH_3$ | $CH_3$ | N | |
| 1849 | $C_2H_5$ | $CH_3$ | CO 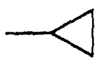 | H | $OCH_3$ | $OCH_3$ | CH | |
| 1850 | $C_2H_5$ | $CH_3$ | CO 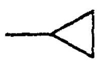 | H | $OCH_3$ | $CH_3$ | CH | |
| 1851 | $C_2H_5$ | $CH_3$ | CO 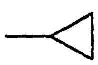 | H | $CH_3$ | $CH_3$ | CH | |
| 1852 | $C_2H_5$ | $CH_3$ | CO 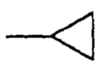 | H | Cl | $OCH_3$ | CH | |
| 1853 | $C_2H_5$ | $CH_3$ | CO 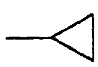 | H | $OCH_3$ | $OCH_3$ | N | |
| 1854 | $C_2H_5$ | $CH_3$ | CO 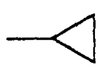 | H | $OCH_3$ | $CH_3$ | N | |
| 1855 | $C_2H_5$ | $CH_3$ | CO 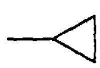 | H | $OCH_3$ | $CF_3$ | N | |
| 1856 | $C_2H_5$ | $CH_3$ | CO 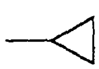 | $CH_3$ | $OCH_3$ | $OCH_3$ | CH | |
| 1857 | $C_2H_5$ | $CH_3$ | CO 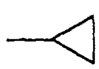 | $CH_3$ | $OCH_3$ | $OCH_3$ | CH | |

| Nr. | $R^1$ | $R^4$ | $R^5$ | $R^7$ | X | Y | Z | Fp. (°C) |
|---|---|---|---|---|---|---|---|---|
| 1858 | $C_2H_5$ | $CH_3$ | 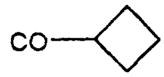 | H | $OCH_3$ | $OCH_3$ | CH | |
| 1859 | $C_2H_5$ | $CH_3$ | 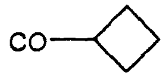 | H | $OCH_3$ | $CH_3$ | CH | |
| 1860 | $C_2H_5$ | $CH_3$ | 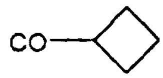 | H | $CH_3$ | $CH_3$ | CH | |
| 1861 | $C_2H_5$ | $CH_3$ | 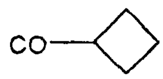 | H | Cl | $OCH_3$ | CH | |
| 1862 | $C_2H_5$ | $CH_3$ | 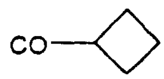 | H | $OCH_3$ | $OCH_3$ | N | |
| 1863 | $C_2H_5$ | $CH_3$ | 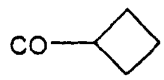 | H | $OCH_3$ | $CH_3$ | N | |
| 1864 | $C_2H_5$ | $CH_3$ | 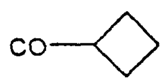 | H | $OCH_3$ | $CF_3$ | N | |
| 1865 | $C_2H_5$ | $CH_3$ | 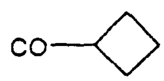 | $CH_3$ | $OCH_3$ | $OCH_3$ | CH | |
| 1866 | $C_2H_5$ | $CH_3$ | 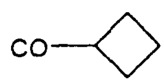 | $CH_3$ | $OCH_3$ | $CH_3$ | N | |
| 1867 | $C_2H_5$ | $CH_3$ | 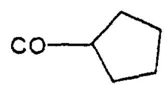 | H | $OCH_3$ | $OCH_3$ | CH | |
| 1868 | $C_2H_5$ | $CH_3$ | 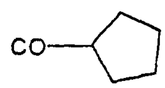 | H | $OCH_3$ | $CH_3$ | CH | |
| 1869 | $C_2H_5$ | $CH_3$ | 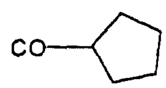 | H | $CH_3$ | $CH_3$ | CH | |
| 1870 | $C_2H_5$ | $CH_3$ | 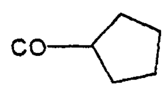 | H | Cl | $OCH_3$ | CH | |
| 1871 | $C_2H_5$ | $CH_3$ | 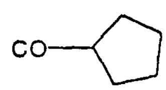 | H | $OCH_3$ | $OCH_3$ | N | |

| Nr. | $R^1$ | $R^4$ | $R^5$ | $R^7$ | X | Y | Z | Fp. (°C) |
|---|---|---|---|---|---|---|---|---|
| 1872 | $C_2H_5$ | $CH_3$ | 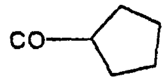 | H | $OCH_3$ | $CH_3$ | N | |
| 1873 | $C_2H_5$ | $CH_3$ | 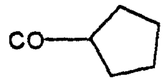 | H | $OCH_3$ | $CF_3$ | N | |
| 1874 | $C_2H_5$ | $CH_3$ | 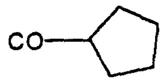 | $CH_3$ | $OCH_3$ | $OCH_3$ | CH | |
| 1875 | $C_2H_5$ | $CH_3$ | 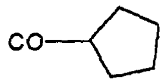 | $CH_3$ | $OCH_3$ | $CH_3$ | N | |
| 1876 | $C_2H_5$ | $CH_3$ | 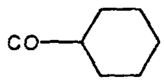 | H | $OCH_3$ | $OCH_3$ | CH | |
| 1877 | $C_2H_5$ | $CH_3$ | 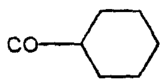 | H | $OCH_3$ | $CH_3$ | CH | |
| 1878 | $C_2H_5$ | $CH_3$ | 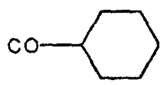 | H | $CH_3$ | $CH_3$ | CH | |
| 1879 | $C_2H_5$ | $CH_3$ | 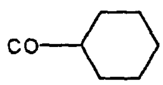 | H | Cl | $OCH_3$ | CH | |
| 1880 | $C_2H_5$ | $CH_3$ | 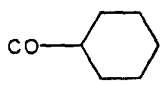 | H | $OCH_3$ | $OCH_3$ | N | |
| 1881 | $C_2H_5$ | $CH_3$ | 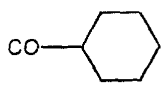 | H | $OCH_3$ | $CH_3$ | N | |
| 1882 | $C_2H_5$ | $CH_3$ | 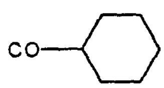 | H | $OCH_3$ | $CF_3$ | N | |
| 1883 | $C_2H_5$ | $CH_3$ | 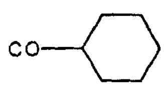 | $CH_3$ | $OCH_3$ | $OCH_3$ | CH | |
| 1884 | $C_2H_5$ | $CH_3$ | 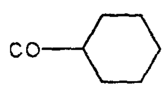 | $CH_3$ | $OCH_3$ | $CH_3$ | N | |
| 1885 | $C_2H_5$ | $CH_3$ | $CF_3SO_2$ | H | $OCH_3$ | $OCH_3$ | CH | |
| 1886 | $C_2H_5$ | $CH_3$ | $CF_3SO_2$ | H | $OCH_3$ | $CH_3$ | CH | |
| 1887 | $C_2H_5$ | $CH_3$ | $CF_3SO_2$ | H | $CH_3$ | $CH_3$ | CH | |
| 1888 | $C_2H_5$ | $CH_3$ | $CF_3SO_2$ | H | Cl | $OCH_3$ | CH | |

| Nr. | $R^1$ | $R^4$ | $R^5$ | $R^7$ | X | Y | Z | Fp. (°C) |
|---|---|---|---|---|---|---|---|---|
| 1889 | $C_2H_5$ | $CH_3$ | $CF_3SO_2$ | H | $OCH_3$ | $OCH_3$ | N | |
| 1890 | $C_2H_5$ | $CH_3$ | $CF_3SO_2$ | H | $OCH_3$ | $CH_3$ | N | |
| 1891 | $C_2H_5$ | $CH_3$ | $CF_3SO_2$ | H | $OCH_3$ | $CF_3$ | N | |
| 1892 | $C_2H_5$ | $CH_3$ | $CF_3SO_2$ | $CH_3$ | $OCH_3$ | $OCH_3$ | CH | |
| 1893 | $C_2H_5$ | $CH_3$ | $CF_3SO_2$ | $CH_3$ | $OCH_3$ | $CH_3$ | N | |
| 1894 | $C_2H_5$ | $CH_3$ | $FCH_2SO_2$ | H | $OCH_3$ | $OCH_3$ | CH | |
| 1895 | $C_2H_5$ | $CH_3$ | $FCH_2SO_2$ | H | $OCH_3$ | $CH_3$ | CH | |
| 1896 | $C_2H_5$ | $CH_3$ | $FCH_2SO_2$ | H | $CH_3$ | $CH_3$ | CH | |
| 1897 | $C_2H_5$ | $CH_3$ | $FCH_2SO_2$ | H | Cl | $OCH_3$ | CH | |
| 1898 | $C_2H_5$ | $CH_3$ | $FCH_2SO_2$ | H | $OCH_3$ | $OCH_3$ | N | |
| 1899 | $C_2H_5$ | $CH_3$ | $FCH_2SO_2$ | H | $OCH_3$ | $CH_3$ | N | |
| 1900 | $C_2H_5$ | $CH_3$ | $FCH_2SO_2$ | H | $OCH_3$ | $CF_3$ | N | |
| 1901 | $C_2H_5$ | $CH_3$ | $FCH_2SO_2$ | $CH_3$ | $OCH_3$ | $OCH_3$ | CH | |
| 1902 | $C_2H_5$ | $CH_3$ | $FCH_2SO_2$ | $CH_3$ | $OCH_3$ | $CH_3$ | N | |
| 1903 | $C_2H_5$ | $CH_3$ | $ClCH_2SO_2$ | H | $OCH_3$ | $OCH_3$ | CH | |
| 1904 | $C_2H_5$ | $CH_3$ | $ClCH_2SO_2$ | H | $OCH_3$ | $CH_3$ | CH | |
| 1905 | $C_2H_5$ | $CH_3$ | $ClCH_2SO_2$ | H | $CH_3$ | $CH_3$ | CH | |
| 1906 | $C_2H_5$ | $CH_3$ | $ClCH_2SO_2$ | H | Cl | $OCH_3$ | CH | |
| 1907 | $C_2H_5$ | $CH_3$ | $ClCH_2SO_2$ | H | $OCH_3$ | $OCH_3$ | N | |
| 1908 | $C_2H_5$ | $CH_3$ | $ClCH_2SO_2$ | H | $OCH_3$ | $CH_3$ | N | |
| 1909 | $C_2H_5$ | $CH_3$ | $ClCH_2SO_2$ | H | $OCH_3$ | $CF_3$ | N | |
| 1910 | $C_2H_5$ | $CH_3$ | $ClCH_2SO_2$ | $CH_3$ | $OCH_3$ | $OCH_3$ | CH | |
| 1911 | $C_2H_5$ | $CH_3$ | $ClCH_2SO_2$ | $CH_3$ | $OCH_3$ | $CH_3$ | N | |
| 1912 | $C_2H_5$ | $CH_3$ | $Cl_2CHSO_2$ | H | $OCH_3$ | $OCH_3$ | CH | |
| 1913 | $C_2H_5$ | $CH_3$ | $Cl_2CHSO_2$ | H | $OCH_3$ | $CH_3$ | CH | |
| 1914 | $C_2H_5$ | $CH_3$ | $Cl_2CHSO_2$ | H | $CH_3$ | $CH_3$ | CH | |
| 1915 | $C_2H_5$ | $CH_3$ | $Cl_2CHSO_2$ | H | Cl | $OCH_3$ | CH | |
| 1916 | $C_2H_5$ | $CH_3$ | $Cl_2CHSO_2$ | H | $OCH_3$ | $OCH_3$ | N | |
| 1917 | $C_2H_5$ | $CH_3$ | $Cl_2CHSO_2$ | H | $OCH_3$ | $CH_3$ | N | |
| 1918 | $C_2H_5$ | $CH_3$ | $Cl_2CHSO_2$ | H | $OCH_3$ | $CF_3$ | N | |
| 1919 | $C_2H_5$ | $CH_3$ | $Cl_2CHSO_2$ | $CH_3$ | $OCH_3$ | $OCH_3$ | CH | |
| 1920 | $C_2H_5$ | $CH_3$ | $Cl_2CHSO_2$ | $CH_3$ | $OCH_3$ | $CH_3$ | N | |
| 1921 | $C_2H_5$ | $CH_3$ | $Cl_3CSO_2$ | H | $OCH_3$ | $OCH_3$ | CH | |
| 1922 | $C_2H_5$ | $CH_3$ | $Cl_3CSO_2$ | H | $OCH_3$ | $CH_3$ | CH | |

| Nr. | $R^1$ | $R^4$ | $R^5$ | $R^7$ | X | Y | Z | Fp. (°C) |
|---|---|---|---|---|---|---|---|---|
| 1923 | $C_2H_5$ | $CH_3$ | $Cl_3CSO_2$ | H | $CH_3$ | $CH_3$ | CH | |
| 1924 | $C_2H_5$ | $CH_3$ | $Cl_3CSO_2$ | H | Cl | $OCH_3$ | CH | |
| 1925 | $C_2H_5$ | $CH_3$ | $Cl_3CSO_2$ | H | $OCH_3$ | $OCH_3$ | N | |
| 1926 | $C_2H_5$ | $CH_3$ | $Cl_3CSO_2$ | H | $OCH_3$ | $CH_3$ | N | |
| 1927 | $C_2H_5$ | $CH_3$ | $Cl_3CSO_2$ | H | $OCH_3$ | $CF_3$ | N | |
| 1928 | $C_2H_5$ | $CH_3$ | $Cl_3CSO_2$ | $CH_3$ | $OCH_3$ | $OCH_3$ | CH | |
| 1929 | $C_2H_5$ | $CH_3$ | $Cl_3CSO_2$ | $CH_3$ | $OCH_3$ | $CH_3$ | N | |
| 1930 | $C_2H_5$ | $CH_3$ | $nC_4H_9SO_2$ | H | $OCH_3$ | $OCH_3$ | CH | |
| 1931 | $C_2H_5$ | $CH_3$ | $nC_4H_9SO_2$ | H | $OCH_3$ | $CH_3$ | CH | |
| 1932 | $C_2H_5$ | $CH_3$ | $nC_4H_9SO_2$ | H | $CH_3$ | $CH_3$ | CH | |
| 1933 | $C_2H_5$ | $CH_3$ | $nC_4H_9SO_2$ | H | Cl | $OCH_3$ | CH | |
| 1934 | $C_2H_5$ | $CH_3$ | $nC_4H_9SO_2$ | H | $OCH_3$ | $CH_3$ | N | |
| 1935 | $C_2H_5$ | $CH_3$ | $nC_4H_9SO_2$ | H | $OCH_3$ | $CF_3$ | N | |
| 1936 | $C_2H_5$ | $CH_3$ | $nC_4H_9SO_2$ | H | $OCH_3$ | $OCH_3$ | N | |
| 1937 | $C_2H_5$ | $CH_3$ | $nC_4H_9SO_2$ | $CH_3$ | $OCH_3$ | $OCH_3$ | CH | |
| 1938 | $C_2H_5$ | $CH_3$ | $nC_4H_9SO_2$ | $CH_3$ | $OCH_3$ | $CH_3$ | N | |
| 1939 | $C_2H_5$ | $CH_3$ | $CF_3CH_2SO_2$ | H | $OCH_3$ | $OCH_3$ | CH | |
| 1940 | $C_2H_5$ | $CH_3$ | $CF_3CH_2SO_2$ | H | $OCH_3$ | $CH_3$ | CH | |
| 1941 | $C_2H_5$ | $CH_3$ | $CF_3CH_2SO_2$ | H | $CH_3$ | $CH_3$ | CH | |
| 1942 | $C_2H_5$ | $CH_3$ | $CF_3CH_2SO_2$ | H | Cl | $OCH_3$ | CH | |
| 1943 | $C_2H_5$ | $CH_3$ | $CF_3CH_2SO_2$ | H | $OCH_3$ | $CH_3$ | N | |
| 1944 | $C_2H_5$ | $CH_3$ | $CF_3CH_2SO_2$ | H | $OCH_3$ | $CF_3$ | N | |
| 1945 | $C_2H_5$ | $CH_3$ | $CF_3CH_2SO_2$ | H | $OCH_3$ | $OCH_3$ | N | |
| 1946 | $C_2H_5$ | $CH_3$ | $CF_3CH_2SO_2$ | $CH_3$ | $OCH_3$ | $OCH_3$ | CH | |
| 1947 | $C_2H_5$ | $CH_3$ | $CF_3CH_2SO_2$ | $CH_3$ | $OCH_3$ | $CH_3$ | N | |
| 1948 | $C_2H_5$ | $CH_3$ | $CH_3NHSO_2$ | H | $OCH_3$ | $OCH_3$ | CH | |
| 1949 | $C_2H_5$ | $CH_3$ | $CH_3NHSO_2$ | H | $OCH_3$ | $CH_3$ | CH | |
| 1950 | $C_2H_5$ | $CH_3$ | $CH_3NHSO_2$ | H | $CH_3$ | $CH_3$ | CH | |
| 1951 | $C_2H_5$ | $CH_3$ | $CH_3NHSO_2$ | H | Cl | $OCH_3$ | CH | |
| 1952 | $C_2H_5$ | $CH_3$ | $CH_3NHSO_2$ | H | $OCH_3$ | $OCH_3$ | N | |
| 1953 | $C_2H_5$ | $CH_3$ | $CH_3NHSO_2$ | H | $OCH_3$ | $CH_3$ | N | |
| 1954 | $C_2H_5$ | $CH_3$ | $CH_3NHSO_2$ | H | $OCH_3$ | $CF_3$ | N | |
| 1955 | $C_2H_5$ | $CH_3$ | $CH_3NHSO_2$ | $CH_3$ | $OCH_3$ | $OCH_3$ | CH | |
| 1956 | $C_2H_5$ | $CH_3$ | $CH_3NHSO_2$ | $CH_3$ | $OCH_3$ | $CH_3$ | N | |

| Nr. | $R^1$ | $R^4$ | $R^5$ | $R^7$ | X | Y | Z | Fp. (°C) |
|---|---|---|---|---|---|---|---|---|
| 1957 | $C_2H_5$ | $CH_3$ | $(CH_3)_2NSO_2$ | H | $OCH_3$ | $OCH_3$ | CH | |
| 1958 | $C_2H_5$ | $CH_3$ | $(CH_3)_2NSO_2$ | H | $OCH_3$ | $CH_3$ | CH | |
| 1959 | $C_2H_5$ | $CH_3$ | $(CH_3)_2NSO_2$ | H | $CH_3$ | $CH_3$ | CH | |
| 1960 | $C_2H_5$ | $CH_3$ | $(CH_3)_2NSO_2$ | H | Cl | $OCH_3$ | CH | |
| 1961 | $C_2H_5$ | $CH_3$ | $(CH_3)_2NSO_2$ | H | $OCH_3$ | $OCH_3$ | N | |
| 1962 | $C_2H_5$ | $CH_3$ | $(CH_3)_2NSO_2$ | H | $OCH_3$ | $CH_3$ | N | |
| 1963 | $C_2H_5$ | $CH_3$ | $(CH_3)_2NSO_2$ | H | $OCH_3$ | $CF_3$ | N | |
| 1964 | $C_2H_5$ | $CH_3$ | $(CH_3)_2NSO_2$ | $CH_3$ | $OCH_3$ | $OCH_3$ | CH | |
| 1965 | $C_2H_5$ | $CH_3$ | $(CH_3)_2NSO_2$ | $CH_3$ | $OCH_3$ | $CH_3$ | N | |
| 1966 | $C_2H_5$ | $CH_3$ | $(CH_3)_3C-OCO$ | H | $OCH_3$ | $OCH_3$ | CH | |
| 1967 | $C_2H_5$ | $CH_3$ | $(CH_3)_3C-OCO$ | H | $OCH_3$ | $CH_3$ | CH | |
| 1968 | $C_2H_5$ | $CH_3$ | $(CH_3)_3C-OCO$ | H | $CH_3$ | $CH_3$ | CH | |
| 1969 | $C_2H_5$ | $CH_3$ | $(CH_3)_3C-OCO$ | H | Cl | $OCH_3$ | CH | |
| 1970 | $C_2H_5$ | $CH_3$ | $(CH_3)_3C-OCO$ | H | $OCH_3$ | $OCH_3$ | N | |
| 1971 | $C_2H_5$ | $CH_3$ | $(CH_3)_3C-OCO$ | H | $OCH_3$ | $CH_3$ | N | |
| 1972 | $C_2H_5$ | $CH_3$ | $(CH_3)_3C-OCO$ | H | $OCH_3$ | $CF_3$ | N | |
| 1973 | $C_2H_5$ | $CH_3$ | $(CH_3)_3C-OCO$ | $CH_3$ | $OCH_3$ | $OCH_3$ | CH | |
| 1974 | $C_2H_5$ | $CH_3$ | $(CH_3)_3C-OCO$ | $CH_3$ | $OCH_3$ | $CH_3$ | N | |
| 1975 | $C_2H_5$ | $CH_3$ | PhCO | H | $OCH_3$ | $OCH_3$ | CH | |
| 1976 | $C_2H_5$ | $CH_3$ | PhCO | H | $OCH_3$ | $CH_3$ | CH | |
| 1977 | $C_2H_5$ | $CH_3$ | PhCO | H | $CH_3$ | $CH_3$ | CH | |
| 1978 | $C_2H_5$ | $CH_3$ | PhCO | H | Cl | $OCH_3$ | CH | |
| 1979 | $C_2H_5$ | $CH_3$ | PhCO | H | $OCH_3$ | $OCH_3$ | N | |
| 1980 | $C_2H_5$ | $CH_3$ | PhCO | H | $OCH_3$ | $CH_3$ | N | |
| 1981 | $C_2H_5$ | $CH_3$ | PhCO | H | $OCH_3$ | $CF_3$ | N | |
| 1982 | $C_2H_5$ | $CH_3$ | PhCO | $CH_3$ | $OCH_3$ | $OCH_3$ | CH | |
| 1983 | $C_2H_5$ | $CH_3$ | PhCO | $CH_3$ | $OCH_3$ | $CH_3$ | N | |
| 1984 | $C_2H_5$ | $CH_3$ | $PhSO_2$ | H | $OCH_3$ | $OCH_3$ | CH | |
| 1985 | $C_2H_5$ | $CH_3$ | $PhSO_2$ | H | $OCH_3$ | $CH_3$ | CH | |
| 1986 | $C_2H_5$ | $CH_3$ | $PhSO_2$ | H | $CH_3$ | $CH_3$ | CH | |
| 1987 | $C_2H_5$ | $CH_3$ | $PhSO_2$ | H | Cl | $OCH_3$ | CH | |
| 1988 | $C_2H_5$ | $CH_3$ | $PhSO_2$ | H | $OCH_3$ | $CH_3$ | N | |
| 1989 | $C_2H_5$ | $CH_3$ | $PhSO_2$ | H | $OCH_3$ | $CF_3$ | N | |
| 1990 | $C_2H_5$ | $CH_3$ | $PhSO_2$ | H | $OCH_3$ | $OCH_3$ | N | |

| Nr. | $R^1$ | $R^4$ | $R^5$ | $R^7$ | X | Y | Z | Fp. (°C) |
|---|---|---|---|---|---|---|---|---|
| 1991 | $C_2H_5$ | $CH_3$ | $PhSO_2$ | $CH_3$ | $OCH_3$ | $CH_3$ | CH | |
| 1992 | $C_2H_5$ | $CH_3$ | $PhSO_2$ | $CH_3$ | $OCH_3$ | $OCH_3$ | N | |
| 1993 | $C_2H_5$ | $CH_3$ | $CH_3NHCO$ | H | $OCH_3$ | $OCH_3$ | CH | |
| 1994 | $C_2H_5$ | $CH_3$ | $CH_3NHCO$ | H | $OCH_3$ | $CH_3$ | CH | |
| 1995 | $C_2H_5$ | $CH_3$ | $CH_3NHCO$ | H | $CH_3$ | $CH_3$ | CH | |
| 1996 | $C_2H_5$ | $CH_3$ | $CH_3NHCO$ | H | Cl | $OCH_3$ | CH | |
| 1997 | $C_2H_5$ | $CH_3$ | $CH_3NHCO$ | H | $OCH_3$ | $OCH_3$ | N | |
| 1998 | $C_2H_5$ | $CH_3$ | $CH_3NHCO$ | H | $OCH_3$ | $CH_3$ | N | |
| 1999 | $C_2H_5$ | $CH_3$ | $CH_3NHCO$ | H | $OCH_3$ | $CF_3$ | N | |
| 2000 | $C_2H_5$ | $CH_3$ | $CH_3NHCO$ | $CH_3$ | $OCH_3$ | $CH_3$ | N | |
| 2001 | $C_2H_5$ | $CH_3$ | $CH_3NHCO$ | $CH_3$ | $OCH_3$ | $OCH_3$ | CH | |
| 2002 | $C_2H_5$ | $CH_3$ | $C_2H_5NHCO$ | H | $OCH_3$ | $OCH_3$ | CH | |
| 2003 | $C_2H_5$ | $CH_3$ | $C_2H_5NHCO$ | H | $OCH_3$ | $CH_3$ | CH | |
| 2004 | $C_2H_5$ | $CH_3$ | $C_2H_5NHCO$ | H | $CH_3$ | $CH_3$ | CH | |
| 2005 | $C_2H_5$ | $CH_3$ | $C_2H_5NHCO$ | H | Cl | $OCH_3$ | CH | |
| 2006 | $C_2H_5$ | $CH_3$ | $C_2H_5NHCO$ | H | $OCH_3$ | $OCH_3$ | N | |
| 2007 | $C_2H_5$ | $CH_3$ | $C_2H_5NHCO$ | H | $OCH_3$ | $CH_3$ | N | |
| 2008 | $C_2H_5$ | $CH_3$ | $C_2H_5NHCO$ | H | $OCH_3$ | $CF_3$ | N | |
| 2009 | $C_2H_5$ | $CH_3$ | $C_2H_5NHCO$ | $CH_3$ | $OCH_3$ | $OCH_3$ | CH | |
| 2010 | $C_2H_5$ | $CH_3$ | $C_2H_5NHCO$ | $CH_3$ | $OCH_3$ | $CH_3$ | N | |
| 2011 | $C_2H_5$ | $CH_3$ | $CH_3NHCS$ | H | $OCH_3$ | $OCH_3$ | CH | |
| 2012 | $C_2H_5$ | $CH_3$ | $CH_3NHCS$ | H | $OCH_3$ | $CH_3$ | CH | |
| 2013 | $C_2H_5$ | $CH_3$ | $CH_3NHCS$ | H | $CH_3$ | $CH_3$ | CH | |
| 2014 | $C_2H_5$ | $CH_3$ | $CH_3NHCS$ | H | Cl | $OCH_3$ | CH | |
| 2015 | $C_2H_5$ | $CH_3$ | $CH_3NHCS$ | H | $OCH_3$ | $OCH_3$ | N | |
| 2016 | $C_2H_5$ | $CH_3$ | $CH_3NHCS$ | H | $OCH_3$ | $CH_3$ | N | |
| 2017 | $C_2H_5$ | $CH_3$ | $CH_3NHCS$ | H | $OCH_3$ | $CF_3$ | N | |
| 2018 | $C_2H_5$ | $CH_3$ | $CH_3NHCS$ | $CH_3$ | $OCH_3$ | $OCH_3$ | CH | |
| 2019 | $C_2H_5$ | $CH_3$ | $CH_3NHCS$ | $CH_3$ | $OCH_3$ | $CH_3$ | N | |
| 2020 | $C_2H_5$ | $CH_3$ | $C_2H_5NHCS$ | H | $OCH_3$ | $OCH_3$ | CH | |
| 2021 | $C_2H_5$ | $CH_3$ | $C_2H_5NHCS$ | H | $OCH_3$ | $CH_3$ | CH | |
| 2022 | $C_2H_5$ | $CH_3$ | $C_2H_5NHCS$ | H | $CH_3$ | $CH_3$ | CH | |
| 2023 | $C_2H_5$ | $CH_3$ | $C_2H_5NHCS$ | H | Cl | $OCH_3$ | CH | |
| 2024 | $C_2H_5$ | $CH_3$ | $C_2H_5NHCS$ | H | $OCH_3$ | $OCH_3$ | N | |

| Nr. | $R^1$ | $R^4$ | $R^5$ | $R^7$ | X | Y | Z | Fp. (°C) |
|---|---|---|---|---|---|---|---|---|
| 2025 | $C_2H_5$ | $CH_3$ | $C_2H_5NHCS$ | H | $OCH_3$ | $CH_3$ | N | |
| 2026 | $C_2H_5$ | $CH_3$ | $C_2H_5NHCS$ | H | $OCH_3$ | $CF_3$ | N | |
| 2027 | $C_2H_5$ | $CH_3$ | $C_2H_5NHCS$ | $CH_3$ | $OCH_3$ | $OCH_3$ | CH | |
| 2028 | $C_2H_5$ | $CH_3$ | $C_2H_5NHCS$ | $CH_3$ | $OCH_3$ | $CH_3$ | N | |
| 2029 | $C_2H_5$ | $CH_3$ | $COSCH_3$ | H | $OCH_3$ | $OCH_3$ | CH | |
| 2030 | $C_2H_5$ | $CH_3$ | $COSCH_3$ | H | $OCH_3$ | $CH_3$ | CH | |
| 2031 | $C_2H_5$ | $CH_3$ | $COSCH_3$ | H | $CH_3$ | $CH_3$ | CH | |
| 2032 | $C_2H_5$ | $CH_3$ | $COSCH_3$ | H | Cl | $OCH_3$ | CH | |
| 2033 | $C_2H_5$ | $CH_3$ | $COSCH_3$ | H | $OCH_3$ | $OCH_3$ | N | |
| 2034 | $C_2H_5$ | $CH_3$ | $COSCH_3$ | H | $OCH_3$ | $CH_3$ | N | |
| 2035 | $C_2H_5$ | $CH_3$ | $COSCH_3$ | H | $OCH_3$ | $CF_3$ | N | |
| 2036 | $C_2H_5$ | $CH_3$ | $COSCH_3$ | $CH_3$ | $OCH_3$ | $OCH_3$ | CN | |
| 2037 | $C_2H_5$ | $CH_3$ | $COSCH_3$ | $CH_3$ | $OCH_3$ | $CH_3$ | N | |
| 2038 | $C_2H_5$ | $CH_3$ | $CSOCH_3$ | H | $OCH_3$ | $OCH_3$ | CH | |
| 2039 | $C_2H_5$ | $CH_3$ | $CSOCH_3$ | H | $OCH_3$ | $CH_3$ | CH | |
| 2040 | $C_2H_5$ | $CH_3$ | $CSOCH_3$ | H | $CH_3$ | $CH_3$ | CH | |
| 2041 | $C_2H_5$ | $CH_3$ | $CSOCH_3$ | H | Cl | $OCH_3$ | CH | |
| 2042 | $C_2H_5$ | $CH_3$ | $CSOCH_3$ | H | $OCH_3$ | $OCH_3$ | N | |
| 2043 | $C_2H_5$ | $CH_3$ | $CSOCH_3$ | H | $OCH_3$ | $CH_3$ | N | |
| 2044 | $C_2H_5$ | $CH_3$ | $CSOCH_3$ | H | $OCH_3$ | $CF_3$ | N | |
| 2045 | $C_2H_5$ | $CH_3$ | $CSOCH_3$ | $CH_3$ | $OCH_3$ | $OCH_3$ | CH | |
| 2046 | $C_2H_5$ | $CH_3$ | $CSOCH_3$ | $CH_3$ | $OCH_3$ | $CH_3$ | N | |
| 2047 | $C_2H_5$ | $CH_3$ | $CSSCH_3$ | H | $OCH_3$ | $OCH_3$ | CH | |
| 2048 | $C_2H_5$ | $CH_3$ | $CSSCH_3$ | H | $OCH_3$ | $CH_3$ | CH | |
| 2049 | $C_2H_5$ | $CH_3$ | $CSSCH_3$ | H | $CH_3$ | $CH_3$ | CH | |
| 2050 | $C_2H_5$ | $CH_3$ | $CSSCH_3$ | H | Cl | $OCH_3$ | CH | |
| 2051 | $C_2H_5$ | $CH_3$ | $CSSCH_3$ | H | $OCH_3$ | $OCH_3$ | N | |
| 2052 | $C_2H_5$ | $CH_3$ | $CSSCH_3$ | H | $OCH_3$ | $CH_3$ | N | |
| 2053 | $C_2H_5$ | $CH_3$ | $CSSCH_3$ | H | $OCH_3$ | $CF_3$ | N | |
| 2054 | $C_2H_5$ | $CH_3$ | $CSSCH_3$ | $CH_3$ | $OCH_3$ | $OCH_3$ | CH | |
| 2055 | $C_2H_5$ | $CH_3$ | $CSSCH_3$ | $CH_3$ | $OCH_3$ | $CH_3$ | N | |
| 2056 | $C_2H_5$ | $CH_3$ | $COCOOCH_3$ | H | $OCH_3$ | $OCH_3$ | CH | |
| 2057 | $C_2H_5$ | $CH_3$ | $COCOOCH_3$ | H | $OCH_3$ | $CH_3$ | CH | |
| 2058 | $C_2H_5$ | $CH_3$ | $COCOOCH_3$ | H | $CH_3$ | $CH_3$ | CH | |

| Nr. | $R^1$ | $R^4$ | $R^5$ | $R^7$ | X | Y | Z | Fp. (°C) |
|---|---|---|---|---|---|---|---|---|
| 2059 | $C_2H_5$ | $CH_3$ | $COCOOCH_3$ | H | Cl | $OCH_3$ | CH | |
| 2060 | $C_2H_5$ | $CH_3$ | $COCOOCH_3$ | H | $OCH_3$ | $OCH_3$ | N | |
| 2061 | $C_2H_5$ | $CH_3$ | $COCOOCH_3$ | H | $OCH_3$ | $CH_3$ | N | |
| 2062 | $C_2H_5$ | $CH_3$ | $COCOOCH_3$ | H | $OCH_3$ | $CF_3$ | N | |
| 2063 | $C_2H_5$ | $CH_3$ | $COCOOCH_3$ | $CH_3$ | $OCH_3$ | $OCH_3$ | CH | |
| 2064 | $C_2H_5$ | $CH_3$ | $COCOOCH_3$ | $CH_3$ | $OCH_3$ | $CH_3$ | N | |
| 2065 | $C_2H_5$ | $CH_3$ | i-$C_3H_7OCO$ | H | $OCH_3$ | $OCH_3$ | CH | |
| 2066 | $C_2H_5$ | $CH_3$ | i-$C_3H_7OCO$ | H | $OCH_3$ | $CH_3$ | CH | |
| 2067 | $C_2H_5$ | $CH_3$ | i-$C_3H_7OCO$ | H | $CH_3$ | $CH_3$ | CH | |
| 2068 | $C_2H_5$ | $CH_3$ | i-$C_3H_7OCO$ | H | Cl | $OCH_3$ | CH | |
| 2069 | $C_2H_5$ | $CH_3$ | i-$C_3H_7OCO$ | H | $OCH_3$ | $OCH_3$ | N | |
| 2070 | $C_2H_5$ | $CH_3$ | i-$C_3H_7OCO$ | H | $OCH_3$ | $CH_3$ | N | |
| 2071 | $C_2H_5$ | $CH_3$ | i-$C_3H_7OCO$ | H | $OCH_3$ | $CF_3$ | N | |
| 2072 | $C_2H_5$ | $CH_3$ | i-$C_3H_7OCO$ | $CH_3$ | $OCH_3$ | $OCH_3$ | CH | |
| 2073 | $C_2H_5$ | $CH_3$ | i-$C_3H_7OCO$ | $CH_3$ | $OCH_3$ | $CH_3$ | N | |
| 2074 | $C_2H_5$ | $C_2H_5$ | CHO | H | $OCH_3$ | $OCH_3$ | CH | 161-165 |
| 2075 | $C_2H_5$ | $C_2H_5$ | CHO | H | $OCH_3$ | $CH_3$ | CH | |
| 2076 | $C_2H_5$ | $C_2H_5$ | CHO | H | $CH_3$ | $CH_3$ | CH | |
| 2077 | $C_2H_5$ | $C_2H_5$ | CHO | H | Cl | $OCH_3$ | CH | |
| 2078 | $C_2H_5$ | $C_2H_5$ | CHO | H | $OCH_3$ | $CH_3$ | N | |
| 2079 | $C_2H_5$ | $C_2H_5$ | CHO | H | $OCH_3$ | $CF_3$ | N | |
| 2080 | $C_2H_5$ | $C_2H_5$ | CHO | H | $OCH_3$ | $OCH_3$ | N | |
| 2081 | $C_2H_5$ | $C_2H_5$ | CHO | $CH_3$ | $OCH_3$ | $OCH_3$ | CH | |
| 2082 | $C_2H_5$ | $C_2H_5$ | CHO | $CH_3$ | $OCH_3$ | $CH_3$ | N | |
| 2083 | $C_2H_5$ | $C_2H_5$ | $COCH_3$ | H | $OCH_3$ | $OCH_3$ | CH | 173-179 |
| 2084 | $C_2H_5$ | $C_2H_5$ | $COCH_3$ | H | $OCH_3$ | $CH_3$ | CH | |
| 2085 | $C_2H_5$ | $C_2H_5$ | $COCH_3$ | H | $CH_3$ | $CH_3$ | CH | |
| 2086 | $C_2H_5$ | $C_2H_5$ | $COCH_3$ | H | Cl | $OCH_3$ | CH | |
| 2087 | $C_2H_5$ | $C_2H_5$ | $COCH_3$ | H | $OCH_3$ | $CH_3$ | N | |
| 2088 | $C_2H_5$ | $C_2H_5$ | $COCH_3$ | H | $OCH_3$ | $CF_3$ | N | |
| 2089 | $C_2H_5$ | $C_2H_5$ | $COCH_3$ | H | $OCH_3$ | $OCH_3$ | N | |
| 2090 | $C_2H_5$ | $C_2H_5$ | $COCH_3$ | $CH_3$ | $OCH_3$ | $CH_3$ | CH | |
| 2091 | $C_2H_5$ | $C_2H_5$ | $COCH_3$ | $CH_3$ | $OCH_3$ | $OCH_3$ | N | |
| 2092 | $C_2H_5$ | $C_2H_5$ | $COOCH_3$ | H | $OCH_3$ | $OCH_3$ | CH | 158-160 |

| Nr. | $R^1$ | $R^4$ | $R^5$ | $R^7$ | X | Y | Z | Fp. (°C) |
|---|---|---|---|---|---|---|---|---|
| 2093 | $C_2H_5$ | $C_2H_5$ | $COOCH_3$ | H | $OCH_3$ | $CH_3$ | CH | |
| 2094 | $C_2H_5$ | $C_2H_5$ | $COOCH_3$ | H | $CH_3$ | $CH_3$ | CH | |
| 2095 | $C_2H_5$ | $C_2H_5$ | $COOCH_3$ | H | Cl | $OCH_3$ | CH | |
| 2096 | $C_2H_5$ | $C_2H_5$ | $COOCH_3$ | H | $OCH_3$ | $CH_3$ | N | |
| 2097 | $C_2H_5$ | $C_2H_5$ | $COOCH_3$ | H | $OCH_3$ | $CF_3$ | N | |
| 2098 | $C_2H_5$ | $C_2H_5$ | $COOCH_3$ | H | $OCH_3$ | $OCH_3$ | N | |
| 2099 | $C_2H_5$ | $C_2H_5$ | $COOCH_3$ | $CH_3$ | $OCH_3$ | $OCH_3$ | CH | |
| 2100 | $C_2H_5$ | $C_2H_5$ | $COOCH_3$ | $CH_3$ | $OCH_3$ | $CH_3$ | N | |
| 2101 | $C_2H_5$ | $C_2H_5$ | $COOC_2H_5$ | H | $OCH_3$ | $OCH_3$ | CH | |
| 2102 | $C_2H_5$ | $C_2H_5$ | $COOC_2H_5$ | H | $OCH_3$ | $CH_3$ | CH | |
| 2103 | $C_2H_5$ | $C_2H_5$ | $COOC_2H_5$ | H | $CH_3$ | $CH_3$ | CH | |
| 2104 | $C_2H_5$ | $C_2H_5$ | $COOC_2H_5$ | H | Cl | $OCH_3$ | CH | |
| 2105 | $C_2H_5$ | $C_2H_5$ | $COOC_2H_5$ | H | $OCH_3$ | $CH_3$ | N | |
| 2106 | $C_2H_5$ | $C_2H_5$ | $COOC_2H_5$ | H | $OCH_3$ | $CF_3$ | N | |
| 2107 | $C_2H_5$ | $C_2H_5$ | $COOC_2H_5$ | H | $OCH_3$ | $OCH_3$ | N | |
| 2108 | $C_2H_5$ | $C_2H_5$ | $COOC_2H_5$ | $CH_3$ | $OCH_3$ | $OCH_3$ | CH | |
| 2109 | $C_2H_5$ | $C_2H_5$ | $COOC_2H_5$ | $CH_3$ | $OCH_3$ | $CH_3$ | N | |
| 2110 | $C_2H_5$ | $C_2H_5$ | $SO_2CH_3$ | H | $OCH_3$ | $OCH_3$ | CH | 98-101 |
| 2111 | $C_2H_5$ | $C_2H_5$ | $SO_2CH_3$ | H | $OCH_3$ | $CH_3$ | CH | |
| 2112 | $C_2H_5$ | $C_2H_5$ | $SO_2CH_3$ | H | $CH_3$ | $CH_3$ | CH | |
| 2113 | $C_2H_5$ | $C_2H_5$ | $SO_2CH_3$ | H | Cl | $OCH_3$ | CH | |
| 2114 | $C_2H_5$ | $C_2H_5$ | $SO_2CH_3$ | H | $OCH_3$ | $CH_3$ | N | |
| 2115 | $C_2H_5$ | $C_2H_5$ | $SO_2CH_3$ | H | $OCH_3$ | $CF_3$ | N | |
| 2116 | $C_2H_5$ | $C_2H_5$ | $SO_2CH_3$ | H | $OCH_3$ | $OCH_3$ | N | |
| 2117 | $C_2H_5$ | $C_2H_5$ | $SO_2CH_3$ | $CH_3$ | $OCH_3$ | $OCH_3$ | CH | |
| 2118 | $C_2H_5$ | $C_2H_5$ | $SO_2CH_3$ | $CH_3$ | $OCH_3$ | $CH_3$ | N | |
| 2119 | $C_2H_5$ | $C_2H_5$ | $SO_2C_2H_5$ | H | $OCH_3$ | $OCH_3$ | CH | |
| 2120 | $C_2H_5$ | $C_2H_5$ | $SO_2C_2H_5$ | H | $OCH_3$ | $CH_3$ | CH | |
| 2121 | $C_2H_5$ | $C_2H_5$ | $SO_2C_2H_5$ | H | $CH_3$ | $CH_3$ | CH | |
| 2122 | $C_2H_5$ | $C_2H_5$ | $SO_2C_2H_5$ | H | Cl | $OCH_3$ | CH | |
| 2123 | $C_2H_5$ | $C_2H_5$ | $SO_2C_2H_5$ | H | $OCH_3$ | $CH_3$ | N | |
| 2124 | $C_2H_5$ | $C_2H_5$ | $SO_2C_2H_5$ | H | $OCH_3$ | $CF_3$ | N | |
| 2125 | $C_2H_5$ | $C_2H_5$ | $SO_2C_2H_5$ | H | $OCH_3$ | $OCH_3$ | N | |
| 2126 | $C_2H_5$ | $C_2H_5$ | $SO_2C_2H_5$ | $CH_3$ | $OCH_3$ | $OCH_3$ | CH | |

| Nr. | $R^1$ | $R^4$ | $R^5$ | $R^7$ | X | Y | Z | Fp. (°C) |
|---|---|---|---|---|---|---|---|---|
| 2127 | $C_2H_5$ | $C_2H_5$ | $SO_2C_2H_5$ | $CH_3$ | $OCH_3$ | $CH_3$ | N | |
| 2128 | $C_2H_5$ | $C_2H_5$ | $COCF_3$ | H | $OCH_3$ | $OCH_3$ | CH | 143-146 |
| 2129 | $C_2H_5$ | $C_2H_5$ | $COCF_3$ | H | $OCH_3$ | $CH_3$ | CH | |
| 2130 | $C_2H_5$ | $C_2H_5$ | $COCF_3$ | H | $CH_3$ | $CH_3$ | CH | |
| 2131 | $C_2H_5$ | $C_2H_5$ | $COCF_3$ | H | Cl | $OCH_3$ | CH | |
| 2132 | $C_2H_5$ | $C_2H_5$ | $COCF_3$ | H | $OCH_3$ | $CH_3$ | N | |
| 2133 | $C_2H_5$ | $C_2H_5$ | $COCF_3$ | H | $OCH_3$ | $CF_3$ | N | |
| 2134 | $C_2H_5$ | $C_2H_5$ | $COCF_3$ | H | $OCH_3$ | $OCH_3$ | N | |
| 2135 | $C_2H_5$ | $C_2H_5$ | $COCF_3$ | $CH_3$ | $OCH_3$ | $OCH_3$ | CH | |
| 2136 | $C_2H_5$ | $C_2H_5$ | $COCF_3$ | $CH_3$ | $OCH_3$ | $CH_3$ | N | |
| 2137 | $nC_3H_7$ | H | CHO | H | $OCH_3$ | $OCH_3$ | CH | 148-154 |
| 2138 | $nC_3H_7$ | H | CHO | H | $OCH_3$ | $CH_3$ | N | |
| 2139 | $nC_3H_7$ | H | $COCH_3$ | H | $OCH_3$ | $OCH_3$ | CH | 106-110 |
| 2140 | $nC_3H_7$ | H | $COCH_3$ | H | $OCH_3$ | $CH_3$ | N | |
| 2141 | $nC_3H_7$ | H | $COC_2H_5$ | H | $OCH_3$ | $OCH_3$ | CH | |
| 2142 | $nC_3H_7$ | H | $COC_2H_5$ | H | $OCH_3$ | $CH_3$ | N | |
| 2143 | $nC_3H_7$ | H | $COCF_3$ | H | $OCH_3$ | $OCH_3$ | CH | 194-197 |
| 2144 | $nC_3H_7$ | H | $COCF_3$ | H | $OCH_3$ | $CH_3$ | N | |
| 2145 | $nC_3H_7$ | H | $COOCH_3$ | H | $OCH_3$ | $OCH_3$ | CH | 142-147 |
| 2146 | $nC_3H_7$ | H | $COOCH_3$ | H | $OCH_3$ | $CH_3$ | N | |
| 2147 | $nC_3H_7$ | H | $COOC_2H_5$ | H | $OCH_3$ | $OCH_3$ | CH | |
| 2148 | $nC_3H_7$ | H | $COOC_2H_5$ | H | $OCH_3$ | $CH_3$ | N | |
| 2149 | $nC_3H_7$ | H | $SO_2CH_3$ | H | $OCH_3$ | $OCH_3$ | CH | 199-205 |
| 2150 | $nC_3H_7$ | H | $SO_2CH_3$ | H | $OCH_3$ | $CH_3$ | N | |
| 2151 | $nC_3H_7$ | H | $SO_2C_2H_5$ | H | $OCH_3$ | $OCH_3$ | CH | 128-133 |
| 2152 | $nC_3H_7$ | H | $SO_2C_2H_5$ | H | $OCH_3$ | $CH_3$ | N | |
| 2153 | $nC_3H_7$ | $CH_3$ | CHO | H | $OCH_3$ | $OCH_3$ | CH | 139-145 |
| 2154 | $nC_3H_7$ | $CH_3$ | CHO | H | $OCH_3$ | $CH_3$ | N | |
| 2155 | $nC_3H_7$ | $CH_3$ | $COCH_3$ | H | $OCH_3$ | $OCH_3$ | CH | 138-141 |
| 2156 | $nC_3H_7$ | $CH_3$ | $COCH_3$ | H | $OCH_3$ | $CH_3$ | N | |
| 2157 | $nC_3H_7$ | $CH_3$ | $COC_2H_5$ | H | $OCH_3$ | $OCH_3$ | CH | |
| 2158 | $nC_3H_7$ | $CH_3$ | $COC_2H_5$ | H | $OCH_3$ | $CH_3$ | N | |
| 2159 | $nC_3H_7$ | $CH_3$ | $COCF_3$ | H | $OCH_3$ | $OCH_3$ | CH | 172-175 |
| 2160 | $nC_3H_7$ | $CH_3$ | $COCF_3$ | H | $OCH_3$ | $CH_3$ | N | |

| Nr. | $R^1$ | $R^4$ | $R^5$ | $R^7$ | X | Y | Z | Fp. (°C) |
|---|---|---|---|---|---|---|---|---|
| 2161 | $nC_3H_7$ | $CH_3$ | $COOCH_3$ | H | $OCH_3$ | $OCH_3$ | CH | 182-185 |
| 2162 | $nC_3H_7$ | $CH_3$ | $COOCH_3$ | H | $OCH_3$ | $CH_3$ | N | |
| 2163 | $nC_3H_7$ | $CH_3$ | $COOC_2H_5$ | H | $OCH_3$ | $OCH_3$ | CH | |
| 2164 | $nC_3H_7$ | $CH_3$ | $COOC_2H_5$ | H | $OCH_3$ | $CH_3$ | N | |
| 2165 | $nC_3H_7$ | $CH_3$ | $SO_2CH_3$ | H | $OCH_3$ | $OCH_3$ | CH | 168-176 |
| 2166 | $nC_3H_7$ | $CH_3$ | $SO_2CH_3$ | H | $OCH_3$ | $CH_3$ | N | |
| 2167 | $nC_3H_7$ | $CH_3$ | $SO_2C_2H_5$ | H | $OCH_3$ | $OCH_3$ | CH | 129-132 |
| 2168 | $nC_3H_7$ | $CH_3$ | $SO_2C_2H_5$ | H | $OCH_3$ | $CH_3$ | N | |
| 2169 | $nC_3H_7$ | $C_2H_5$ | CHO | H | $OCH_3$ | $OCH_3$ | CH | 142-145 |
| 2170 | $nC_3H_7$ | $C_2H_5$ | CHO | H | $OCH_3$ | $CH_3$ | N | |
| 2171 | $nC_3H_7$ | $C_2H_5$ | $COCH_3$ | H | $OCH_3$ | $OCH_3$ | CH | |
| 2172 | $nC_3H_7$ | $C_2H_5$ | $COCH_3$ | H | $OCH_3$ | $CH_3$ | N | |
| 2173 | $nC_3H_7$ | $C_2H_5$ | $COC_2H_5$ | H | $OCH_3$ | $OCH_3$ | CH | |
| 2174 | $nC_3H_7$ | $C_2H_5$ | $COC_2H_5$ | H | $OCH_3$ | $CH_3$ | N | |
| 2175 | $nC_3H_7$ | $C_2H_5$ | $COCF_3$ | H | $OCH_3$ | $OCH_3$ | CH | |
| 2176 | $nC_3H_7$ | $C_2H_5$ | $COCF_3$ | H | $OCH_3$ | $CH_3$ | N | |
| 2177 | $nC_3H_7$ | $C_2H_5$ | $COOCH_3$ | H | $OCH_3$ | $OCH_3$ | CH | 173-177 |
| 2178 | $nC_3H_7$ | $C_2H_5$ | $COOCH_3$ | H | $OCH_3$ | $CH_3$ | N | |
| 2179 | $nC_3H_7$ | $C_2H_5$ | $COOC_2H_5$ | H | $OCH_3$ | $OCH_3$ | CH | |
| 2180 | $nC_3H_7$ | $C_2H_5$ | $COOC_2H_5$ | H | $OCH_3$ | $CH_3$ | N | |
| 2181 | $nC_3H_7$ | $C_2H_5$ | $SO_2CH_3$ | H | $OCH_3$ | $OCH_3$ | CH | 173-179 |
| 2182 | $nC_3H_7$ | $C_2H_5$ | $SO_2CH_3$ | H | $OCH_3$ | $CH_3$ | N | |
| 2183 | $nC_3H_7$ | $C_2H_5$ | $SO_2C_2H_5$ | H | $OCH_3$ | $OCH_3$ | CH | |
| 2184 | $nC_3H_7$ | $C_2H_5$ | $SO_2C_2H_5$ | H | $OCH_3$ | $CH_3$ | N | |
| 2185 | $N(CH_3)_2$ | H | CHO | H | $OCH_3$ | $OCH_3$ | CH | 143-144 |
| 2186 | $N(CH_3)_2$ | H | CHO | H | $OCH_3$ | $CH_3$ | N | |
| 2187 | $N(CH_3)_2$ | H | $COCH_3$ | H | $OCH_3$ | $OCH_3$ | CH | |
| 2188 | $N(CH_3)_2$ | H | $COCH_3$ | H | $OCH_3$ | $CH_3$ | N | |
| 2189 | $N(CH_3)_2$ | H | $COC_2H_5$ | H | $OCH_3$ | $OCH_3$ | CH | |
| 2190 | $N(CH_3)_2$ | H | $COC_2H_5$ | H | $OCH_3$ | $CH_3$ | N | |
| 2191 | $N(CH_3)_2$ | H | $COCF_3$ | H | $OCH_3$ | $OCH_3$ | CH | |
| 2192 | $N(CH_3)_2$ | H | $COCF_3$ | H | $OCH_3$ | $CH_3$ | N | |
| 2193 | $N(CH_3)_2$ | H | $COOCH_3$ | H | $OCH_3$ | $OCH_3$ | CH | |
| 2194 | $N(CH_3)_2$ | H | $COOCH_3$ | H | $OCH_3$ | $CH_3$ | N | |

| Nr. | $R^1$ | $R^4$ | $R^5$ | $R^7$ | X | Y | Z | Fp. (°C) |
|---|---|---|---|---|---|---|---|---|
| 2195 | $N(CH_3)_2$ | H | $COOC_2H_5$ | H | $OCH_3$ | $OCH_3$ | CH | |
| 2196 | $N(CH_3)_2$ | H | $COOC_2H_5$ | H | $OCH_3$ | $CH_3$ | N | |
| 2197 | $N(CH_3)_2$ | H | $SO_2CH_3$ | H | $OCH_3$ | $OCH_3$ | CH | |
| 2198 | $N(CH_3)_2$ | H | $SO_2CH_3$ | H | $OCH_3$ | $CH_3$ | N | |
| 2199 | $N(CH_3)_2$ | H | $SO_2C_2H_5$ | H | $OCH_3$ | $OCH_3$ | CH | |
| 2200 | $N(CH_3)_2$ | H | $SO_2C_2H_5$ | H | $OCH_3$ | $CH_3$ | N | |
| 2201 | $N(CH_3)_2$ | $CH_3$ | CHO | H | $OCH_3$ | $OCH_3$ | CH | 201-203 |
| 2202 | $N(CH_3)_2$ | $CH_3$ | CHO | H | $OCH_3$ | $CH_3$ | N | |
| 2203 | $N(CH_3)_2$ | $CH_3$ | $COCH_3$ | H | $OCH_3$ | $OCH_3$ | CH | 176-179 |
| 2204 | $N(CH_3)_2$ | $CH_3$ | $COCH_3$ | H | $OCH_3$ | $CH_3$ | N | |
| 2205 | $N(CH_3)_2$ | $CH_3$ | $COC_2H_5$ | H | $OCH_3$ | $OCH_3$ | CH | |
| 2206 | $N(CH_3)_2$ | $CH_3$ | $COC_2H_5$ | H | $OCH_3$ | $CH_3$ | N | |
| 2207 | $N(CH_3)_2$ | $CH_3$ | $COCF_3$ | H | $OCH_3$ | $OCH_3$ | CH | |
| 2208 | $N(CH_3)_2$ | $CH_3$ | $COCF_3$ | H | $OCH_3$ | $CH_3$ | N | |
| 2209 | $N(CH_3)_2$ | $CH_3$ | $COOCH_3$ | H | $OCH_3$ | $OCH_3$ | CH | 192-194 |
| 2210 | $N(CH_3)_2$ | $CH_3$ | $COOCH_3$ | H | $OCH_3$ | $CH_3$ | N | |
| 2211 | $N(CH_3)_2$ | $CH_3$ | $COOC_2H_5$ | H | $OCH_3$ | $OCH_3$ | CH | |
| 2212 | $N(CH_3)_2$ | $CH_3$ | $COOC_2H_5$ | H | $OCH_3$ | $CH_3$ | N | |
| 2213 | $N(CH_3)_2$ | $CH_3$ | $SO_2CH_3$ | H | $OCH_3$ | $OCH_3$ | CH | 144-146 |
| 2214 | $N(CH_3)_2$ | $CH_3$ | $SO_2CH_3$ | H | $OCH_3$ | $CH_3$ | N | |
| 2215 | $N(CH_3)_2$ | $CH_3$ | $SO_2C_2H_5$ | H | $OCH_3$ | $OCH_3$ | CH | |
| 2216 | $N(CH_3)_2$ | $CH_3$ | $SO_2C_2H_5$ | H | $OCH_3$ | $CH_3$ | N | |
| 2217 | $N(CH_3)_2$ | $C_2H_5$ | CHO | H | $OCH_3$ | $OCH_3$ | CH | 143-146 |
| 2218 | $N(CH_3)_2$ | $C_2H_5$ | CHO | H | $OCH_3$ | $CH_3$ | N | |
| 2219 | $N(CH_3)_2$ | $C_2H_5$ | $COCH_3$ | H | $OCH_3$ | $OCH_3$ | CH | 116-118 |
| 2220 | $N(CH_3)_2$ | $C_2H_5$ | $COCH_3$ | H | $OCH_3$ | $CH_3$ | N | |
| 2221 | $N(CH_3)_2$ | $C_2H_5$ | $COC_2H_5$ | H | $OCH_3$ | $OCH_3$ | CH | |
| 2222 | $N(CH_3)_2$ | $C_2H_5$ | $COC_2H_5$ | H | $OCH_3$ | $CH_3$ | N | |
| 2223 | $N(CH_3)_2$ | $C_2H_5$ | $COCF_3$ | H | $OCH_3$ | $OCH_3$ | CH | |
| 2224 | $N(CH_3)_2$ | $C_2H_5$ | $COCF_3$ | H | $OCH_3$ | $CH_3$ | N | |
| 2225 | $N(CH_3)_2$ | $C_2H_5$ | $COOCH_3$ | H | $OCH_3$ | $OCH_3$ | CH | 201-203 |
| 2226 | $N(CH_3)_2$ | $C_2H_5$ | $COOCH_3$ | H | $OCH_3$ | $CH_3$ | N | |
| 2227 | $N(CH_3)_2$ | $C_2H_5$ | $COOC_2H_5$ | H | $OCH_3$ | $OCH_3$ | CH | |
| 2228 | $N(CH_3)_2$ | $C_2H_5$ | $COOC_2H_5$ | H | $OCH_3$ | $CH_3$ | N | |

| Nr. | $R^1$ | $R^4$ | $R^5$ | $R^7$ | X | Y | Z | Fp. (°C) |
|---|---|---|---|---|---|---|---|---|
| 2229 | $N(CH_3)_2$ | $C_2H_5$ | $SO_2CH_3$ | H | $OCH_3$ | $OCH_3$ | CH | 125-128 |
| 2230 | $N(CH_3)_2$ | $C_2H_5$ | $SO_2CH_3$ | H | $OCH_3$ | $CH_3$ | N | |
| 2231 | $N(CH_3)_2$ | $C_2H_5$ | $SO_2C_2H_5$ | H | $OCH_3$ | $OCH_3$ | CH | |
| 2232 | $N(CH_3)_2$ | $C_2H_5$ | $SO_2C_2H_5$ | H | $OCH_3$ | $CH_3$ | N | |

Tabelle 2:

Verbindungen der Formel (Ib)

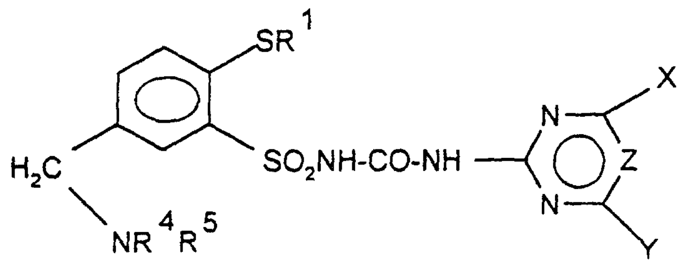

(Ib)

| Nr. | $R^1$ | $R^4$ | $R^5$ | X | Y | Z | Fp. (°C) |
|---|---|---|---|---|---|---|---|
| 2-1 | $CH_3$ | H | CHO | $OCH_3$ | $OCH_3$ | CH | |
| 2-2 | $CH_3$ | H | CHO | $OCH_3$ | $CH_3$ | N | |
| 2-3 | $CH_3$ | H | $COOCH_3$ | $OCH_3$ | $OCH_3$ | CH | |
| 2-4 | $CH_3$ | H | $COOCH_3$ | $OCH_3$ | $CH_3$ | N | |
| 2-5 | $CH_3$ | H | $SO_2CH_3$ | $OCH_3$ | $OCH_3$ | CH | |
| 2-6 | $CH_3$ | H | $SO_2CH_3$ | $OCH_3$ | $CH_3$ | N | |
| 2-7 | $CH_3$ | $CH_3$ | CHO | $OCH_3$ | $OCH_3$ | CH | |
| 2-8 | $CH_3$ | $CH_3$ | CHO | $OCH_3$ | $CH_3$ | N | |
| 2-9 | $CH_3$ | $CH_3$ | $COOCH_3$ | $OCH_3$ | $OCH_3$ | CH | |
| 2-10 | $CH_3$ | $CH_3$ | $COOCH_3$ | $OCH_3$ | $CH_3$ | N | |
| 2-11 | $CH_3$ | $CH_3$ | $SO_2CH_3$ | $OCH_3$ | $OCH_3$ | CH | |
| 2-12 | $CH_3$ | $CH_3$ | $SO_2CH_3$ | $OCH_3$ | $CH_3$ | N | |
| 2-13 | $C_2H_5$ | H | CHO | $OCH_3$ | $OCH_3$ | CH | |
| 2-14 | $C_2H_5$ | H | CHO | $OCH_3$ | $CH_3$ | N | |
| 2-15 | $C_2H_5$ | H | $COOCH_3$ | $OCH_3$ | $OCH_3$ | CH | |
| 2-16 | $C_2H_5$ | H | $COOCH_3$ | $OCH_3$ | $CH_3$ | N | |
| 2-17 | $C_2H_5$ | H | $SO_2CH_3$ | $OCH_3$ | $OCH_3$ | CH | |
| 2-18 | $C_2H_5$ | H | $SO_2CH_3$ | $OCH_3$ | $CH_3$ | N | |
| 2-19 | $C_2H_5$ | $CH_3$ | CHO | $OCH_3$ | $OCH_3$ | CH | |
| 2-20 | $C_2H_5$ | $CH_3$ | CHO | $OCH_3$ | $CH_3$ | N | |
| 2-21 | $C_2H_5$ | $CH_3$ | $COOCH_3$ | $OCH_3$ | $OCH_3$ | CH | |
| 2-22 | $C_2H_5$ | $CH_3$ | $COOCH_3$ | $OCH_3$ | $CH_3$ | N | |
| 2-23 | $C_2H_5$ | $CH_3$ | $SO_2CH_3$ | $OCH_3$ | $OCH_3$ | CH | |
| 2-24 | $C_2H_5$ | $CH_3$ | $SO_2CH_3$ | $OCH_3$ | $CH_3$ | N | |
| 2-25 | $C_2H_5$ | $C_2H_5$ | CHO | $OCH_3$ | $OCH_3$ | CH | |
| 2-26 | $C_2H_5$ | $C_2H_5$ | CHO | $OCH_3$ | $CH_3$ | N | |

| Nr. | $R^1$ | $R^4$ | $R^5$ | X | Y | Z | Fp. (°C) |
|---|---|---|---|---|---|---|---|
| 2-27 | $C_2H_5$ | $C_2H_5$ | $COOCH_3$ | $OCH_3$ | $OCH_3$ | CH | |
| 2-28 | $C_2H_5$ | $C_2H_5$ | $COOCH_3$ | $OCH_3$ | $CH_3$ | N | |
| 2-29 | $C_2H_5$ | $C_2H_5$ | $SO_2CH_3$ | $OCH_3$ | $OCH_3$ | CH | |
| 2-30 | $C_2H_5$ | $C_2H_5$ | $SO_2CH_3$ | $OCH_3$ | $CH_3$ | N | |

Tabelle 3: Verbindungen der Formel (Ic)

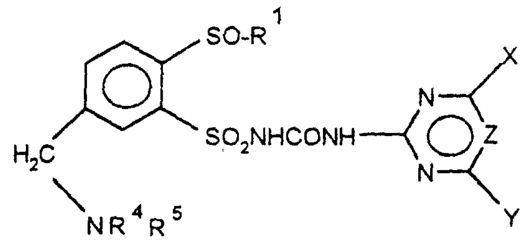

(Ic)

| Nr. | $R^1$ | $R^4$ | $R^5$ | X | Y | Z | Fp. (°C) |
|---|---|---|---|---|---|---|---|
| 3-1 | $CH_3$ | H | CHO | $OCH_3$ | $OCH_3$ | CH | |
| 3-2 | $CH_3$ | H | CHO | $OCH_3$ | $CH_3$ | N | |
| 3-3 | $CH_3$ | H | $COOCH_3$ | $OCH_3$ | $OCH_3$ | CH | |
| 3-4 | $CH_3$ | H | $COOCH_3$ | $OCH_3$ | $CH_3$ | N | |
| 3-5 | $CH_3$ | H | $SO_2CH_3$ | $OCH_3$ | $OCH_3$ | CH | |
| 3-6 | $CH_3$ | H | $SO_2CH_3$ | $OCH_3$ | $CH_3$ | N | |
| 3-7 | $CH_3$ | $CH_3$ | CHO | $OCH_3$ | $OCH_3$ | CH | |
| 3-8 | $CH_3$ | $CH_3$ | CHO | $OCH_3$ | $CH_3$ | N | |
| 3-9 | $CH_3$ | $CH_3$ | $COOCH_3$ | $OCH_3$ | $OCH_3$ | CH | |
| 3-10 | $CH_3$ | $CH_3$ | $COOCH_3$ | $OCH_3$ | $CH_3$ | N | |
| 3-11 | $CH_3$ | $CH_3$ | $SO_2CH_3$ | $OCH_3$ | $OCH_3$ | CH | |
| 3-12 | $CH_3$ | $CH_3$ | $SO_2CH_3$ | $OCH_3$ | $CH_3$ | N | |
| 3-13 | $C_2H_5$ | H | CHO | $OCH_3$ | $OCH_3$ | CH | 118-120 |
| 3-14 | $C_2H_5$ | H | CHO | $OCH_3$ | $CH_3$ | N | |
| 3-15 | $C_2H_5$ | H | $COOCH_3$ | $OCH_3$ | $OCH_3$ | CH | |
| 3-16 | $C_2H_5$ | H | $COOCH_3$ | $OCH_3$ | $CH_3$ | N | |
| 3-17 | $C_2H_5$ | H | $SO_2CH_3$ | $OCH_3$ | $OCH_3$ | CH | |
| 3-18 | $C_2H_5$ | H | $SO_2CH_3$ | $OCH_3$ | $CH_3$ | N | |
| 3-19 | $C_2H_5$ | $CH_3$ | CHO | $OCH_3$ | $OCH_3$ | CH | 138-140 |
| 3-20 | $C_2H_5$ | $CH_3$ | CHO | $OCH_3$ | $CH_3$ | N | |
| 3-21 | $C_2H_5$ | $CH_3$ | $COOCH_3$ | $OCH_3$ | $OCH_3$ | CH | 116-117 |
| 3-22 | $C_2H_5$ | $CH_3$ | $COOCH_3$ | $OCH_3$ | $CH_3$ | N | |
| 3-23 | $C_2H_5$ | $CH_3$ | $SO_2CH_3$ | $OCH_3$ | $OCH_3$ | CH | 179-180 |
| 3-24 | $C_2H_5$ | $CH_3$ | $SO_2CH_3$ | $OCH_3$ | $CH_3$ | N | |
| 3-25 | $C_2H_5$ | $C_2H_5$ | CHO | $OCH_3$ | $OCH_3$ | CH | 173-174 |
| 3-26 | $C_2H_5$ | $C_2H_5$ | CHO | $OCH_3$ | $CH_3$ | N | |
| 3-27 | $C_2H_5$ | $C_2H_5$ | $COOCH_3$ | $OCH_3$ | $OCH_3$ | CH | 130-131 |

| Nr. | $R^1$ | $R^4$ | $R^5$ | X | Y | Z | Fp. (°C) |
|---|---|---|---|---|---|---|---|
| 3-28 | $C_2H_5$ | $C_2H_5$ | $COOCH_3$ | $OCH_3$ | $CH_3$ | N | |
| 3-29 | $C_2H_5$ | $C_2H_5$ | $SO_2CH_3$ | $OCH_3$ | $OCH_3$ | CH | 130-131 |
| 3-30 | $C_2H_5$ | $C_2H_5$ | $SO_2CH_3$ | $OCH_3$ | $CH_3$ | N | |

Tabelle 4: $Na^+$-Salze

Verbindungen der Formel (Id)

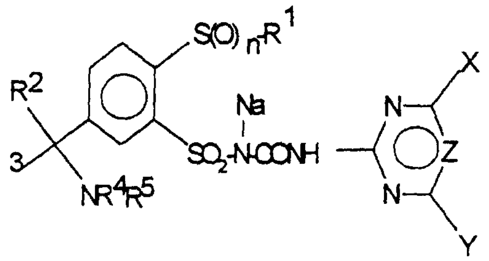

(Id)

| Nr. | n | $R^1$ | $R^4$ | $R^5$ | X | Y | Z | Fp. (°C) |
|---|---|---|---|---|---|---|---|---|
| 4-1 | 2 | $CH_3$ | H | CHO | $OCH_3$ | $OCH_3$ | CH | 204-209 |
| 4-2 | 2 | $CH_3$ | H | $COCH_3$ | $OCH_3$ | $OCH_3$ | CH | 190-193 |
| 4-3 | 2 | $CH_3$ | H | $COC_2H_5$ | $OCH_3$ | $OCH_3$ | CH | 125-135 |
| 4-4 | 2 | $CH_3$ | H | $CO\text{-}i\text{-}C_3H_7$ | $OCH_3$ | $OCH_3$ | CH | 198-201 |
| 4-5 | 2 | $CH_3$ | H | $COCCl_3$ | $OCH_3$ | $OCH_3$ | CH | 256-260 |
| 4-6 | 2 | $CH_3$ | H | $COOC_2H_5$ | $OCH_3$ | $OCH_3$ | CH | 189-193 |
| 4-7 | 2 | $CH_3$ | H | $SO_2CH_3$ | $OCH_3$ | $OCH_3$ | CH | 188-192 |
| 4-8 | 2 | $CH_3$ | H | $SO_2C_2H_5$ | $OCH_3$ | $OCH_3$ | CH | 194-198 |
| 4-9 | 2 | $CH_3$ | $CH_3$ | CHO | $OCH_3$ | $OCH_3$ | CH | 178-182 |
| 4-10 | 2 | $CH_3$ | $CH_3$ | $COCH_3$ | $OCH_3$ | $OCH_3$ | CH | 181-190 |
| 4-11 | 2 | $CH_3$ | $CH_3$ | $COC_2H_5$ | $OCH_3$ | $OCH_3$ | CH | 187-195 |
| 4-12 | 2 | $CH_3$ | $CH_3$ | $COCF_3$ | $OCH_3$ | $OCH_3$ | CH | 189-198 |
| 4-13 | 2 | $CH_3$ | $CH_3$ | $COCCl_3$ | $OCH_3$ | $OCH_3$ | CH | 181-188 |
| 4-14 | 2 | $CH_3$ | $CH_3$ | $CO\text{-}i\text{-}C_3H_7$ | $OCH_3$ | $OCH_3$ | CH | 175-180 |
| 4-15 | 2 | $CH_3$ | $CH_3$ | $COOCH_3$ | $OCH_3$ | $OCH_3$ | CH | 142-150 |
| 4-16 | 2 | $CH_3$ | $CH_3$ | $COOC_2H_5$ | $OCH_3$ | $OCH_3$ | CH | 138-145 |
| 4-17 | 2 | $CH_3$ | $CH_3$ | $SO_2CH_3$ | $OCH_3$ | $OCH_3$ | CH | 191-194 |
| 4-18 | 2 | $CH_3$ | $CH_3$ | $SO_2C_2H_5$ | $OCH_3$ | $OCH_3$ | CH | 187-191 |
| 4-19 | 2 | $C_2H_5$ | H | CHO | $OCH_3$ | $OCH_3$ | CH | 191-198 |
| 4-20 | 2 | $C_2H_5$ | H | $COCH_3$ | $OCH_3$ | $OCH_3$ | CH | 172-180 |
| 4-21 | 2 | $C_2H_5$ | H | $COOCH_3$ | $OCH_3$ | $OCH_3$ | CH | 184-189 |
| 4-22 | 2 | $C_2H_5$ | H | $SO_2CH_3$ | $OCH_3$ | $OCH_3$ | CH | 172-180 |
| 4-23 | 2 | $C_2H_5$ | $CH_3$ | CHO | $OCH_3$ | $OCH_3$ | CH | 182-185 |
| 4-24 | 2 | $C_2H_5$ | $CH_3$ | $COCH_3$ | $OCH_3$ | $OCH_3$ | CH | 184-187 |
| 4-25 | 2 | $C_2H_5$ | $CH_3$ | $SO_2CH_3$ | $OCH_3$ | $OCH_3$ | CH | 228-229 |
| 4-26 | 2 | $C_2H_5$ | $C_2H_5$ | CHO | $OCH_3$ | $OCH_3$ | CH | 184-189 |

| Nr. | n | $R^1$ | $R^4$ | $R^5$ | X | Y | Z | Fp. (°C) |
|---|---|---|---|---|---|---|---|---|
| 4-27 | 2 | $C_2H_5$ | $C_2H_5$ | $COCH_3$ | $OCH_3$ | $OCH_3$ | CH | 173-179 |
| 4-28 | 2 | $C_2H_5$ | $C_2H_5$ | $COCF_3$ | $OCH_3$ | $OCH_3$ | CH | 182-186 |
| 4-29 | 2 | $C_2H_5$ | $C_2H_5$ | $COOCH_3$ | $OCH_3$ | $OCH_3$ | CH | 155-162 |
| 4-30 | 2 | $C_2H_5$ | $C_2H_5$ | $SO_2CH_3$ | $OCH_3$ | $OCH_3$ | CH | 225-227 |
| 4-31 | 2 | $nC_3H_7$ | H | CHO | $OCH_3$ | $OCH_3$ | CH | 177-183 |
| 4-32 | 2 | $nC_3H_7$ | H | $COCH_3$ | $OCH_3$ | $OCH_3$ | CH | 178-182 |
| 4-33 | 2 | $nC_3H_7$ | H | $COCF_3$ | $OCH_3$ | $OCH_3$ | CH | 240-246 |
| 4-34 | 2 | $nC_3H_7$ | H | $COOCH_3$ | $OCH_3$ | $OCH_3$ | CH | 192-200 |
| 4-35 | 2 | $nC_3H_7$ | H | $SO_2Cl_3$ | $OCH_3$ | $OCH_3$ | CH | 247-250 |
| 4-36 | 2 | $nC_3H_7$ | H | $SO_2C_2H_5$ | $OCH_3$ | $OCH_3$ | CH | 183-187 |
| 4-37 | 2 | $nC_3H_7$ | $CH_3$ | CHO | $OCH_3$ | $OCH_3$ | CH | 194-202 |
| 4-38 | 2 | $nC_3H_7$ | $CH_3$ | $COCH_3$ | $OCH_3$ | $OCH_3$ | CH | 175-178 |
| 4-39 | 2 | $nC_3H_7$ | $CH_3$ | $COCF_3$ | $OCH_3$ | $OCH_3$ | CH | 155-161 |
| 4-40 | 2 | $nC_3H_7$ | $CH_3$ | $COOCH_3$ | $OCH_3$ | $OCH_3$ | CH | 213-216 |
| 4-41 | 2 | $nC_3H_7$ | $CH_3$ | $SO_2CH_3$ | $OCH_3$ | $OCH_3$ | CH | 213-216 |
| 4-42 | 2 | $nC_3H_7$ | $CH_3$ | $SO_2C_2H_5$ | $OCH_3$ | $OCH_3$ | CH | 168-174 |
| 4-43 | 2 | $nC_3H_7$ | $C_2H_5$ | CHO | $OCH_3$ | $OCH_3$ | CH | 170-176 |
| 4-44 | 2 | $nC_3H_7$ | $C_2H_5$ | $COOCH_3$ | $OCH_3$ | $OCH_3$ | CH | 150-155 |
| 4-45 | 2 | $nC_3H_7$ | $C_2H_5$ | $SO_2CH_3$ | $OCH_3$ | $OCH_3$ | CH | 167-173 |
| 4-46 | 2 | $N(CH_3)_2$ | H | CHO | $OCH_3$ | $OCH_3$ | CH | 188-192 |
| 4-47 | 2 | $N(CH_3)_2$ | H | $SO_2CH_3$ | $OCH_3$ | $OCH_3$ | CH | 192-197 |
| 4-48 | 2 | $N(CH_3)_2$ | $CH_3$ | CHO | $OCH_3$ | $OCH_3$ | CH | 180-184 |
| 4-49 | 2 | $N(CH_3)_2$ | $CH_3$ | $COCH_3$ | $OCH_3$ | $OCH_3$ | CH | 140-145 |
| 4-50 | 2 | $N(CH_3)_2$ | $CH_3$ | $COOCH_3$ | $OCH_3$ | $OCH_3$ | CH | 116 |
| 4-51 | 2 | $N(CH_3)_2$ | $CH_3$ | $SO_2CH_3$ | $OCH_3$ | $OCH_3$ | CH | 172-180 |
| 4-52 | 2 | $N(CH_3)_2$ | $C_2H_5$ | CHO | $OCH_3$ | $OCH_3$ | CH | 167-171 |
| 4-53 | 2 | $N(CH_3)_2$ | $C_2H_5$ | $COCH_3$ | $OCH_3$ | $OCH_3$ | CH | 128-133 |
| 4-54 | 2 | $N(CH_3)_2$ | $C_2H_5$ | $COOCH_3$ | $OCH_3$ | $OCH_3$ | CH | 163-168 |
| 4-55 | 2 | $N(CH_3)_2$ | $C_2H_5$ | $SO_2CH_3$ | $OCH_3$ | $OCH_3$ | CH | 178-182 |
| 4-56 | 1 | $C_2H_5$ | H | CHO | $OCH_3$ | $OCH_3$ | CH | 197-199 |
| 4-57 | 1 | $C_2H_5$ | $CH_3$ | CHO | $OCH_3$ | $OCH_3$ | CH | 112-115 |
| 4-58 | 1 | $C_2H_5$ | $CH_3$ | $COOCH_3$ | $OCH_3$ | $OCH_3$ | CH | 160-164 |
| 4-59 | 1 | $C_2H_5$ | $CH_3$ | $SO_2CH_3$ | $OCH_3$ | $OCH_3$ | CH | 180-183 |
| 4-60 | 1 | $C_2H_5$ | $C_2H_5$ | CHO | $OCH_3$ | $OCH_3$ | CH | 166-172 |

| Nr. | n | $R^1$ | $R^4$ | $R^5$ | X | Y | Z | Fp. (°C) |
|---|---|---|---|---|---|---|---|---|
| 4-61 | 1 | $C_2H_5$ | $C_2H_5$ | $SO_2CH_3$ | $OCH_3$ | $OCH_3$ | CH | 221-222 |

B. Formulierungsbeispiele

a) Ein Stäubemittel wird erhalten, indem man 10 Gew.-Teile einer Verbindung der Formel (I) und 90 Gew.-Teile Talkum als Inerstoff mischt und in einer Schlagmühle zerkleinert.

b) Ein in Wasser leicht dispergierbarer, benetzbares Pulver wird erhalten, indem man 25 Gewichtsteile einer Verbindung der Formel (I), 64 Gewichtsteile kaolinhaltigen Quarz als Inerstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.

c) Ein in Wasser leicht dispergierbares Dispersionskonzentrat wird erhalten, indem man 20 Gewichtsteile einer Verbindung der Formel (I) mit 6 Gew.-Teilen Alkylphenolpolyglykolether (®Triton X 207), 3 Gew.-Teilen Isotridecanolpolyglykolether (8 EO) und 71 Gew.-Teilen paraffinischem Mineralöl (Siedebereich z.B. ca. 255 bis über 277°C) mischt und in einer Reibkugelmühle auf eine Feinheit von unter 5 Mikron vermahlt.

d) Ein emulgierbares Konzentrat wird erhalten aus 15 Gew.-Teilen einer Verbindung der Formel (I), 75 Gew.-Teilen Cyclohexanon als Lösungsmittel und 10 Gew.-Teilen oxethyliertes Nonylphenol als Emulgator.

e) Ein in Wasser dispergierbares Granulat wird erhalten indem man

| | |
|---|---|
| 75 Gewichtsteile | einer Verbindung der Formel (I), |
| 10 " | ligninsulfonsaures Calcium, |
| 5 " | Natriumlaurylsulfat, |
| 3 " | Polyvinylalkohol und |
| 7 " | Kaolin |

mischt, auf einer Stiftsmühle mahlt und das Pulver in einem Wirbelbett durch Aufsprühen von Wasser als Granulierflüssigkeit granuliert.

f) Ein in Wasser dispergierbares Granulat wird auch erhalten, indem man

| | |
|---|---|
| 25 Gewichtsteile | einer Verbindung der Formel (I), |
| 5 " | 2,2'-dinaphthylmethan-6,6'-disulfonsaures Natrium |
| 2 " | oleoylmethyltaurinsaures Natrium, |
| 1 " | Polyvinylalkohol, |
| 17 " | Calciumcarbonat und |
| 50 " | Wasser |

auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

C. Biologische Beispiele

1. Unkrautwirkung im Vorauflauf

Samen bzw. Rhizomstücke von mono- und dikotylen Unkrautpflanzen werden in Plastiktöpfen in sandiger Lehmerde ausgelegt und mit Erde abgedeckt. Die in Form von benetzbaren Pulvern oder Emulsionskonzentraten formulierten erfindungsgemäßen Verbindungen der Formel (I) oder deren Salze werden dann als wäßrige Suspension

bzw. Emulsion mit einer Wasseraufwandmenge von umgerechnet 600 bis 800 l/ha in unterschiedlichen Dosierungen auf die Oberfläche der Abdeckerde appliziert.

Nach der Behandlung werden die Töpfe im Gewächshaus aufgestellt und unter guten Wachstumsbedingungen für die Unkräuter gehalten. Die optische Bonitur der Pflanzen- bzw. der Auflaufschäden erfolgt nach dem Auflaufen der Versuchspflanzen nach einer Versuchszeit von 3 bis 4 Wochen im Vergleich zu unbehandelten Kontrollen. Wie die Testergebnisse zeigen, weisen die erfindungsgemäßen Verbindungen eine gute herbizide Vorauflaufwirksamkeit gegen ein breites Spektrum von Ungräsern und Unkräutern auf. Beispielsweise haben die Verbindungen der Beispiele Nr. 1, 18, 35, 69, 86, 103, 120, 138, 155, 225, 531, 548, 565, 599, 616, 633, 650, 667, 684, 753, 1068, 1085, 1170, 1202, 1591, 1608, 1676, 1727, 2074, 2068, 2092, 2110, 2128, 2137, 2139, 2144, 2145, 2149, 2151, 2153, 2155, 2159, 2161, 2165, 2167, 2169, 2177, 2181, 2185, 2201, 2203, 2209, 2213, 2217, 2219, 2225, 2229, 3-13, 3-19, 3-21, 3-23, 3-25, 3-27, 3-29 (siehe Abschnitt A.) und deren Natriumsalze sehr gute herbizide Wirkung gegen Schadpflanzen wie Sinapis alba, Stellaria media, Chrysanthemum segetum und Lolium multiflorum im Vorauflaufverfahren bei einer Aufwendmenge von 0,3 kg bis 0,005 kg Aktivsubstanz pro Hektar.

2. Unkrautwirkung im Nachauflauf

Samen bzw. Rhizomstücke von mono- und dikotylen Unkräutern werden in Plastiktöpfen in sandigem Lehmboden ausgelegt, mit Erde abgedeckt und im Gewächshaus unter guten Wachstumsbedingungen angezogen. Drei Wochen nach der Aussaat werden die Versuchspflanzen im Dreiblattstadium behandelt. Die als Spritzpulver bzw. als Emulsionskonzentrate formulierten erfindungsgemäßen Verbindungen der Formel (I) oder deren Salze werden in verschiedenen Dosierungen mit einer Wasseraufwandmenge von umgerechnet 600 bis 800 l/ha auf die grünen Pflanzenteile gesprüht. Nach ca. 3 bis 4 Wochen Standzeit der Versuchspflanzen im Gewächshaus unter optimalen Wachstumsbedingungen wird die Wirkung der Präparate optisch im Vergleich zu unbehandelten Kontrollen bonitiert. Die erfindungsgemäßen Mittel weisen auch im Nachauflauf eine gute herbizide Wirksamkeit gegen ein breites Spektrum wirtschaftlich wichtiger Ungräser und Unkräuter auf. Beispielsweise haben die Beispiele Nr. 1, 18, 35, 69, 86, 103, 120, 138, 155, 225, 531, 548, 565, 599, 616, 633, 650, 667, 684, 753, 1068, 1085, 1170, 1202, 1591, 1608, 1676, 1727, 2074, 2068, 2092, 2110, 2128, 2137, 2139, 2144, 2145, 2149, 2151, 2153, 2155, 2159, 2161, 2165, 2167, 2169, 2177, 2181, 2185, 2201, 2203, 2209, 2213, 2217, 2219, 2225, 2229, 3-13, 3-19, 3-21, 3-23, 3-25, 3-27, 3-29 (siehe Abschnitt A.) und deren Natriumsalze sehr gute herbizide Wirkung gegen Schadpflanzen wie Sinapis alba, Stellaria media, Chrysanthemum segetum und Lolium multiflorum im Nachauflaufverfahren bei einer Aufwandmenge von 0,3 kg bis 0,005 kg Aktivsubstanz pro Hektar.

3. Kulturpflanzenverträglichkeit

In weiteren Versuchen im Gewächshaus wurden Samen einer größeren Anzahl von Kulturpflanzen und Unkräutern in sandigen Lehmboden ausgelegt und mit Erde abgedeckt.

Ein Teil der Töpfe wurde sofort wie unter 1. beschrieben behandelt, die übrigen im Gewächshaus aufgestellt, bis die Pflanzen zwei bis drei echte Blätter entwickelt hatten und dann mit den erfindungsgemäßen Substanzen der Formel (I) oder deren Salze in unterschiedlichen Dosierungen, wie unter 2. beschrieben besprüht.

Vier bis fünf Wochen nach der Applikation und Standzeit im Gewächshaus wurde mittels optischer Bonitur festgestellt, daß die erfindungsgemäßen Verbindungen zweikeimblättrigen Kulturen, wie z.B. Soja, Baumwolle, Raps, Zuckerrüben und Kartoffeln im Vor- und Nachauflaufverfahren selbst bei hohen Wirkstoffdosierungen ungeschädigt ließen. Einige Substanzen schonten darüber hinaus auch Gramineen-Kulturen wie z.B. Gerste, Weizen, Roggen, Sorghum-Hirsen, Mais oder Reis. Die Verbindungen der Formel (I) oder deren Salze weisen somit eine hohe Selektivität bei Anwendung zur Bekämpfung von unerwünschtem Pflanzenwuchs in landwirtschaftlichen Kulturen auf.

## Patentansprüche

**1.** Verbindungen der Formel (I) oder deren Salze,

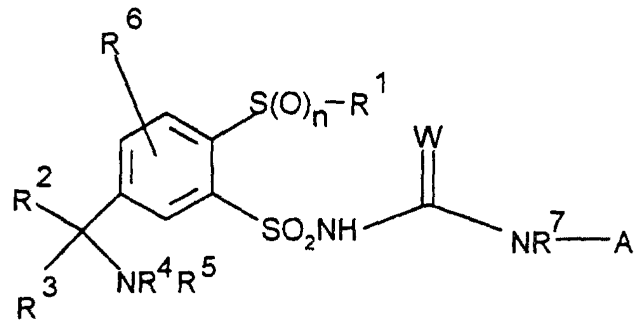

(I)

worin

| | |
|---|---|
| $R^1$ | $NR^8R^9$, einen Kohlenwasserstoffrest, der unsubstituiert oder substituiert ist, |
| n | 0, 1 oder 2, ausgenommen n = 0 oder 1, wenn $R^1 = NR^8R^9$ bedeutet, |
| $R^2$,$R^3$ | unabhängig voneinander H oder (1-4)Alkyl, |
| $R^4$ | H, OH, Formyl, einen Rest der Formel R, R-O-, R-CO- oder R-$SO_2$-, wobei R einen Kohlenwasserstoffrest bedeutet, der unsubstituiert oder substituiert ist, |
| $R^5$ | einen Acylrest oder |
| $NR^4R^5$ | gemeinsam einen heterocyclischen Rest, der unsubstituiert oder substituiert ist, |
| W | ein Sauerstoff- oder Schwefelatom, |
| $R^6$ | H, (1-4)Alkyl, (1-4)Alkoxy, (1-4)Alkylthio, [(1-4)Alkyl]-carbonyl, [(1-4)Alkoxy]carbonyl, wobei jeder der letztgenannten Reste unsubstituiert oder durch einen oder mehrere Halogenatome substituiert ist, oder Halogen, $NO_2$, CN oder mono- oder disubstituiertes Amino, |
| $R^7$ | H oder (1-4)Alkyl, |
| $R^8$,$R^9$ | unabhängig voneinander H, (1-4)Alkyl, (1-4)Alkoxy, [(1-4)Alkyl]carbonyl oder (1-4)Alkylsulfonyl, |
| A | einen Rest der Formel |

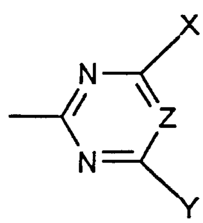 ,
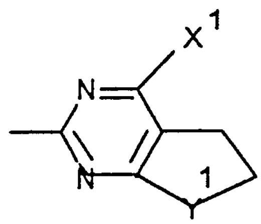 ,
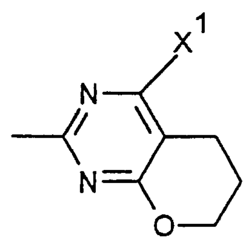 ,

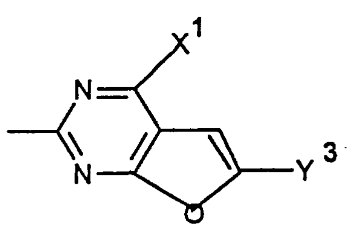 ,
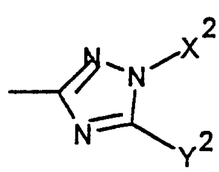 ,
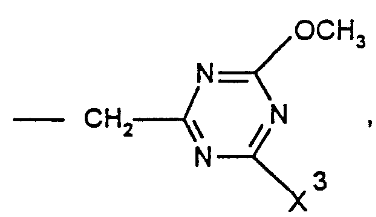 ,

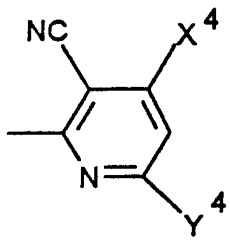

oder

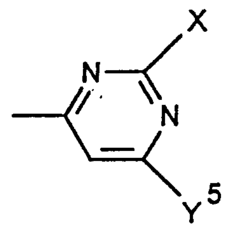

| | |
|---|---|
| einer der Reste X und Y | Wasserstoff, (1-3)Alkyl oder (1-3)Alkoxy, wobei die beiden letztgenannten Reste unsubstituiert oder ein- oder mehrfach durch Halogen oder einfach durch (1-3) Alkoxy substituiert sind, und |
| der andere der Reste X und Y | Wasserstoff, Halogen, (1-3)Alkyl, (1-3)Alkoxy oder (1-3)Alkylthio, wobei die drei letztgenannten alkylhaltigen Reste unsubstituiert oder ein- oder mehrfach durch Halogen oder ein- oder zweifach durch (1-3)Alkoxy oder (1-3)Alkylthio substituiert sind, oder einen Rest der Formel $NR^aR^b$, worin $R^a$, $R^b$ unabhängig voneinander H, (1-3)Alkyl oder Alkenyl mit bis zu 3 C-Atomen bedeuten, oder (3-6) Cycloalkyl, (2-4)Alkenyl, (2-4)Alkinyl, (3-4)-Alkenyloxy oder (3-4)Alkinyloxy, |
| Z | CH oder N, |
| $X^1$ | $CH_3$, $OCH_3$, $OC_2H_5$ oder $OCHF_2$, |
| $Y^1$ | -O- oder $-CH_2-$, |
| $X^2$ | $CH_3$, $C_2H_5$ oder $CH_2CF_3$, |
| $Y^2$ | $OCH_3$, $OC_2H_5$, $SCH_3$, $SCH_2CH_3$, $CH_3$ oder $C_2H_5$, |
| $X^3$ | $CH_3$ oder $OCH_3$, |
| $Y^3$ | H oder $CH_3$, |
| $X^4$ | $CH_3$, $OCH_3$, $OC_2H_5$, $CH_2OCH_3$ oder Cl, |
| $Y^4$ | $CH_3$, $OCH_3$, $OC_2H_5$ oder Cl und |
| $Y^5$ | $CH_3$, $C_2H_5$, $OCH_3$ oder Cl |

bedeuten.

2. Verbindungen oder deren Salze nach Anspruch 1, **dadurch gekennzeichnet, daß**

| | |
|---|---|
| $R^1$ | $NR^8R^9$, (1-6)Alkyl, (2-6)Alkenyl, (2-6)Alkinyl, (3-6)Cycloalkyl, (3-6)Cycloalkenyl oder Phenyl, wobei jeder der letztgenannten 6 Reste unsubstituiert oder durch einen oder mehrere Reste aus der Gruppe Halogen, CN, (1-4)Alkoxy, (1-4)Haloalkoxy, (1-4)Alkoxy(1-4)alkoxy, (1-4)Alkylthio, (1-4)Alkylsulfinyl, (1-4)-Alkylsulfonyl, Formyl, [(1-4)Alkyl]-carbonyl, [(1-4)Alkoxy]carbonyl, [(1-4)Alkyl]-carbonyloxy und im Falle cyclischer Reste auch (1-4)Alkyl, (1-4)Haloalkyl und (1-4)Alkoxy-(1-4)alkyl substituiert ist, |
| n | 0, 1 oder 2, ausgenommen n = 0 oder 1, wenn $R^1 = NR^8R^9$, |
| $R^2$, $R^3$ | unabhängig voneinander H oder (1-4)Alkyl, |
| $R^4$ | H, OH, Formyl, (1-6)Alkyl, (2-6)Alkenyl, (2-6)Alkinyl, (1-6)Alkoxy, (2-5)Alkenyloxy, (2-5)Alkinyloxy, [(1-6)Alkyl]-carbonyl, (1-6)Alkylsulfonyl, [(2-6)Alkenyl]-carbonyl, (2-6)Alkenylsulfonyl, [(2-6)Alkinyl]-carbonyl, (2-6)Alkinylsulfonyl, (3-6)Cycloalkyl, (3-6)Cycloalkenyl, [(3-6)Cycloalkyl]-carbonyl, (3-6) Cycloalkylsulfonyl, [(3-6)Cycloalkenyl]-carbonyl oder (3-6)Cycloalkenylsulfonyl, wobei jeder der 18 letztgenannten Reste unsubstituiert oder durch einen oder mehrere Reste aus der Gruppe Halogen, (1-4)Alkoxy, (1-4)Alkylthio, (1-4)Alkylsulfinyl, (1-4)Alkylsulfonyl, [(1-4)Alkoxy]-carbonyl, [(1-4)Alkyl]-carbonyl, [(1-4)Alkyl]-carbonyloxy und CN und im Falle cyclischer Reste auch (1-4)Alkyl und (1-4) Haloalkyl substituiert ist, oder Phenylcarbonyl oder Phenylsulfonyl, wobei in den beiden letztgenannten Resten der Phenylring unsubstituiert oder durch einen oder mehrere Reste aus der Gruppe Halogen, CN, $NO_2$, (1-4)Alkyl, (1-4)Haloalkyl, (1-4)Alkoxy und (1-4)Haloalkoxy substituiert ist, und |
| $R^5$ | CHO, [(1-6)Alkyl]-carbonyl, [(2-6)Alkenyl]-carbonyl, [(2-6)Alkinyl]-carbonyl, (1-6)Alkylsulfonyl, (2-6) Alkenylsulfonyl, (2-6)Alkinylsulfonyl, [(3-6)Cycloalkyl]-carbonyl, [(3-6)Cycloalkenyl]-carbonyl, (3-6) Cycloalkyl-sulfonyl oder (3-6)Cycloalkenylsulfonyl, wobei jeder der 10 letztgenannten Reste unsubstituiert oder durch einen oder mehrere Reste aus der Gruppe Halogen, (1-4)Alkoxy, (1-4)Alkylthio, |

(1-4)Alkylsulfinyl (1-4)Alkylsulfonyl, [(1-4)Alkyl]-carbonyl, [(1-4)Alkoxy]-carbonyl, [(1-4)Alkyl]-carbonyloxy und CN und im Falle cyclischer Reste auch (1-4)Alkyl und (1-4)Haloalkyl substituiert ist, oder Phenylcarbonyl oder Phenylsulfonyl, wobei jeder der beiden letztgenannten Reste im Phenylring unsubstituiert oder durch einen oder mehrere Reste aus der Gruppe Halogen, CN, $NO_2$, (1-4)Alkyl, (1-4)Haloalkyl, (1-4)Alkoxy und
(1-4)Haloalkoxy substituiert ist, oder Mono- oder Di-[(1-4)alkyl]aminosulfonyl, das im Alkylteil unsubstituiert oder durch einen oder mehrere Reste aus der Gruppe Halogen, (1-4)Alkoxy, (1-4)Alkylthio, (1-4)Alkylsulfinyl, [(1-4)Alkyl]-carbonyl, [(1-4)Alkyl]-carbonyloxy, [(1-4)Alkoxy]-carbonyl und CN substituiert ist oder eine Gruppe der Formel COCOR', worin R' = H, OH, (1-4)Alkoxy oder (1-4)Alkyl ist, oder eine Gruppe der Formel

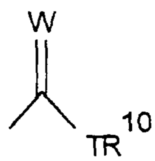 , 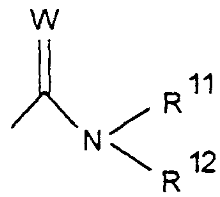 oder 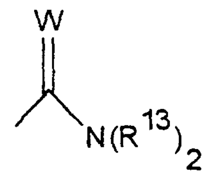 ,

oder

$R^4$ und $R^5$ gemeinsam eine Kette der Formel $(-CH_2)_{m1}$ $B^1$- oder $-B^1-(CH_2)_{m2}B^2$, wobei einzelne Gruppen $CH_2$ durch Sauerstoffatome ersetzt sein können und wobei die Kette unsubstituiert oder durch einen oder mehrere (1-3)Alkylreste oder Halogen substituiert ist und m1 = 3, 4 oder 5 bzw. m2 = 2, 3 oder 4 sind, sowie

W O oder S,

$B^1$, $B^2$ unabhängig voneinander $SO_2$ oder CO,

T O oder S,

$R^6$ H, (1-4)Alkyl, (1-4)Alkoxy, (1-4)Alkylthio, [(1-4)Alkyl]-carbonyl oder [(1-4)Alkoxy]-carbonyl, wobei jeder der letztgenannten 5 Reste unsubstituiert oder im Alkylteil durch ein oder mehrere Halogenatome substituiert ist, oder Halogen, $NO_2$, CN oder Mono- oder Di-[(1-4)alkyl]-amino,

$R^7$ H oder (1-4)Alkyl,

$R^8$ (1-4)Alkyl, (1-4)Alkoxy, (3-6)Cycloalkyl oder (3-6)Cycloalkenyl,

$R^9$ H oder (1-4)Alkyl,

$R^{10}$ (1-4)Alkyl, (3-4)Alkenyl oder (3-4)Alkinyl, wobei jeder der drei letztgenannten Reste unsubstituiert oder durch einen oder mehrere Reste aus der Gruppe Halogen, (1-4)Alkoxy, (1-4)Alkylthio, [(1-4) Alkyl]-carbonyl und [(1-4)Alkoxy]-carbonyl substituiert ist,

$R^{11}$, $R^{12}$ unabhängig voneinander H, (1-4)Alkyl, (3-4)Alkenyl oder (3-4)Alkinyl, wobei jeder der drei letztgenannten Reste unsubstituiert oder durch einen oder mehrere Reste aus der Gruppe Halogen, (1-4) Alkoxy, (1-4)Alkylthio, [(1-4)Alkyl]-carbonyl und [(1-4)Alkoxy]-carbonyl substituiert ist,

die Reste $R^{13}$ gemeinsam mit dem N-Atom einen heterocyclischen Ring mit 5 oder 6 Ringgliedern, der weitere Heteroatome aus der Gruppe N, O und S in den möglichen Oxidationsstufen enthalten kann und unsubstituiert oder durch (1-4)Alkyl oder die Oxogruppe substituiert ist oder benzokondensiert ist, bedeuten.

3. Verbindungen oder deren Salze nach Anspruch 1 oder 2, **dadurch gekennzeichnet, daß**

$R^1$ $NR^8R^9$, (1-4)Alkyl, (3-6)Cycloalkyl oder Phenyl, wobei jeder der letztgenannten 3 Reste unsubstituiert oder durch einen oder mehrere Reste aus der Gruppe Halogen, CN, (1-4)Alkoxy, (1-4)Haloalkoxy, (1-4)Alkylthio, (1-4)-Alkylsulfonyl, Formyl, [(1-4)Alkoxy]carbonyl, [(1-4)Alkyl]-carbonyloxy und im Falle cyclischer Reste auch (1-4)Alkyl, (1-4)Haloalkyl und (1-4)Alkoxy-(1-4)alkyl substituiert ist, oder (2-4)Alkenyl oder (2-4)Alkinyl,

n 0, 1 oder 2, ausgenommen n = 0 oder 1, wenn $R^1 = NR^8R^9$,

$R^2$, $R^3$ unabhängig voneinander H oder (1-4)Alkyl,

$R^4$ H, OH, Formyl, (1-4)Alkyl, (2-4)Alkenyl, (2-4)Alkinyl, (1-4)Alkoxy, [(1-4)Alkyl]-carbonyl, (1-4)Alkyl-sulfonyl, wobei jeder der 6 letztgenannten Reste unsubstituiert oder durch einen oder mehrere Reste aus der Gruppe Halogen und (1-4)

| | |
|---|---|
| | Alkoxy substituiert ist, oder Phenylcarbonyl oder Phenylsulfonyl, wobei in den beiden letztgenannten Resten der Phenylring unsubstituiert oder durch einen oder mehrere Reste aus der Gruppe Halogen, (1-4)Alkyl, (1-4)Haloalkyl, (1-4) Alkoxy und (1-4)Haloalkoxy substituiert ist, und |
| $R^5$ | CHO, [(1-4)Alkyl]-carbonyl, [(2-4)Alkenyl]-carbonyl, [(2-4)Alkinyl]-carbonyl, (1-4)Alkylsulfonyl, (2-4)Alkenylsulfonyl, (2-4)Alkinylsulfonyl, [(3-6)Cycloalkyl]-carbonyl, wobei jeder der 7 letztgenannten Reste unsubstituiert oder durch einen oder mehrere Reste aus der Gruppe Halogen, (1-4)Alkoxy, (1-4)Alkylthio, [(1-4) Alkoxy]-carbonyl, [(1-4)Alkyl]-carbonyloxy und CN und im Falle cyclischer Reste auch (1-4)Alkyl und (1-4)Haloalkyl substituiert ist, oder Phenylcarbonyl oder Phenylsulfonyl, wobei jeder der beiden letztgenannten Reste im Phenylring unsubstituiert oder durch einen oder mehrere Reste aus der Gruppe Halogen, CN, $NO_2$, (1-4)Alkyl, (1-4)Haloalkyl, (1-4)Alkoxy und (1-4)Haloalkoxy substituiert ist, oder Mono- oder Di-[(1-4)alkyl]amino-sulfonyl, das im Alkylteil unsubstituiert oder durch einen oder mehrere Halogenatome substituiert ist, oder eine Gruppe der Formel COCOR', worin R' = H, OH, (1-4)Alkoxy oder (1-4)Alkyl ist, oder eine Gruppe der Formel |

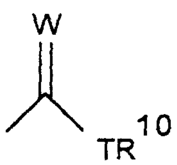 , 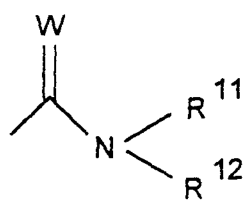 oder 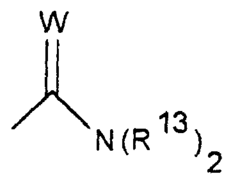

| | |
|---|---|
| | oder |
| $R^4$ und $R^5$ | gemeinsam eine Kette der Formel $(-CH_2)_{m1}$ $B^1$- oder $-B^1-(CH_2)_{m2}B^2-$, wobei einzelne Gruppen $CH_2$ durch Sauerstoffatome ersetzt sein können und wobei die Kette unsubstituiert oder durch einen oder mehrere (1-3)Alkylreste oder Halogen substituiert ist und m1 = 3, 4 oder 5 bzw. m2 = 2, 3 oder 4 sind, sowie |
| W | O oder S, |
| $B^1$, $B^2$ | unabhängig voneinander $SO_2$ oder CO, |
| T | O oder S, |
| $R^6$ | H, (1-4)Alkyl, (1-4)Alkoxy oder Halogen, |
| $R^7$ | H oder $CH_3$, |
| $R^8$ | (1-4)Alkyl, |
| $R^9$ | H oder (1-4)Alkyl, |
| $R^{10}$ | (1-4)Alkyl, (1-4)Haloalkyl, (3-4)Alkenyl oder (3-4)Alkinyl, |
| $R^{11}$, $R^{12}$ | unabhängig voneinander H oder (1-4)Alkyl, |
| die Reste $R^{13}$ | gemeinsam eine Alkylenkette mit 4 oder 5 C-Atomen, |
| A | einen Rest der Formel |

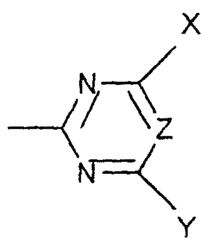 ,

| | |
|---|---|
| einer der Reste X und Y | (1-3)Alkyl, Halo(1-3)alkyl, (1-3)Alkoxy oder Halo(1-3)alkoxy und |
| der andere der Reste X und Y | (1-3)Alkyl, Halo(1-3)alkyl, (1-3)Alkoxy, Halo(1-3)alkoxy, Halogen, Mono- oder Di-[(1-3)Alkyl]-amino und |
| Z | CH oder N bedeuten. |

**4.** Verbindungen oder deren Salze nach einem der Ansprüche 1 bis 3, **dadurch gekennzeichnet, daß**

| | |
|---|---|
| $R^1$ | Mono- oder Di-[(1-4)alkyl]-amino oder (1-4)Alkyl, |
| n | die Zahl 2, |
| $R^2$, $R^3$ | jeweils Wasserstoff, |
| $R^4$ | H oder (1-4)Alkyl, |
| $R^5$ | CHO, ((1-4)Alkyl]-carbonyl, [(1-4)Haloalkyl]-carbonyl, (1-4)Alkylsulfonyl, (1-4)Haloalkylsulfonyl, [(1-4) Alkoxy]-carbonyl, Monooder Di-[(1-4)alkyl]-aminocarbonyl, Mono- oder Di-[(1-4)alkyl]-aminosulfonyl oder |
| $R^4$, $R^5$ | gemeinsam eine Kette der Formel $(-CH_2)_{m1}B^1$- oder $-B^1-(CH_2)_{m2}B^2$-, worin $B^1$ und $B^2$ unabhängig voneinander $SO_2$ oder CO bedeuten, |
| W, T | jeweils O, |
| $R^6$ | H und |
| $R^7$ | H oder $CH_3$ bedeuten. |

**5.** Verfahren zur Herstellung von Verbindungen der Formel (I) oder deren Salze, wie sie nach einem der Ansprüche 1 bis 4 definiert sind, **dadurch gekennzeichnet, daß** man

a) eine Verbindung der Formel (II)

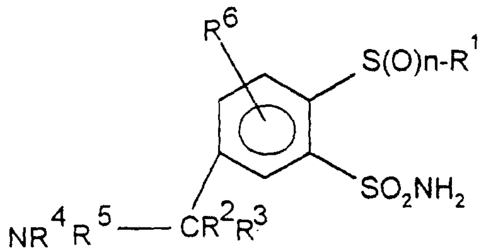

(II)

mit einem heterocyclischen Carbamat der Formel (III),

$$R^* - O - CO - NR^7 - A \qquad (III)$$

worin R* gegebenenfalls substituiertes Phenyl oder (1-4)Alkyl bedeutet, umsetzt oder

b) ein Arylsulfonylcarbamat der Formel (IV)

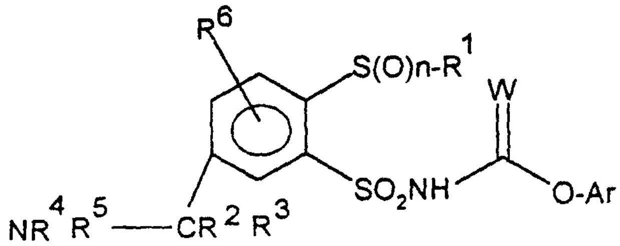

(IV) ,

worin Ar einen Arylrest bedeutet,
mit einem Aminoheterocyclus der Formel (V)

$$H - NR^7 - A \qquad (V)$$

umsetzt oder

c) ein Sulfonylisocyanat der Formel (VI)

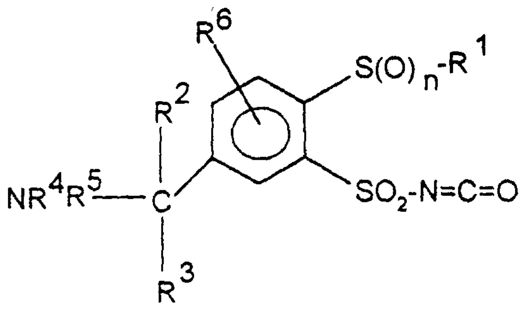

(VI)

mit einem Aminoheterocyclus der Formel H-$NR^7$-A (V) umsetzt oder

d) in einer Eintopfreaktion zunächst einen Aminoheterocyclus der Formel H-$NR^7$-A (V) in Gegenwart einer Base mit Phosgen umsetzt und das gebildete intermediat mit einem Phenylsulfonamid der Formel (II) umsetzt oder

e) ein Sulfonylchlorid der Formel (VII)

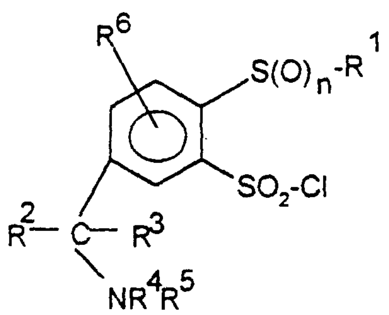

(VIi)

mit einem Cyanat M-OCN, worin M = ein Kation bedeutet, und mit einem Aminoheterocyclus der Formel H-$NR^7$-A (V) in Gegenwart einer Base umsetzt oder

f) ein Sulfonamid der genannten Formel (II) mit einem (Thio-)Isocyanat der Formel (V')

$$W = C = N - A \qquad (V')$$

in Gegenwart einer Base umsetzt,

wobei in den Formeln (II)-(VII) und (V') die Reste bzw. Symbole $R^1$ bis $R^7$, A, W und n wie in Formel (I) definiert sind und wobei in den Varianten a) und c)-e) zunächst Verbindungen der Formel (I) mit W = O erhalten werden.

6. Herbizides oder pflanzenwachstumsregulierendes Mittel, **dadurch gekennzeichnet, daß** es mindestens eine Verbindung der Formel (I) oder deren Salz nach einem der Ansprüche 1 bis 4 und im Pflanzenschutz übliche Formulierungshilfsmittel enthält.

7. Verfahren zur Bekämpfung von Schadpflanzen oder zur Wachstumsregulierung von Pflanzen, **dadurch gekennzeichnet, daß** man eine wirksame Menge von mindestens einer Verbindung der Formel (I) oder deren Salz nach einem der Ansprüche 1 bis 4 auf die Pflanzen, Pflanzensamen oder die Anbaufläche appliziert.

**8.** Verwendung von Verbindungen der Formel (I) oder deren Salzen nach einem der Ansprüche 1 bis 4 als Herbizide oder Pflanzenwachstumsregulatoren.

**9.** Verbindungen der Formel (II), (IV), (VI) und (VII), wie sie in Anspruch 5 definiert sind.

## Claims

**1.** A compound of the formula (I) or a salt thereof

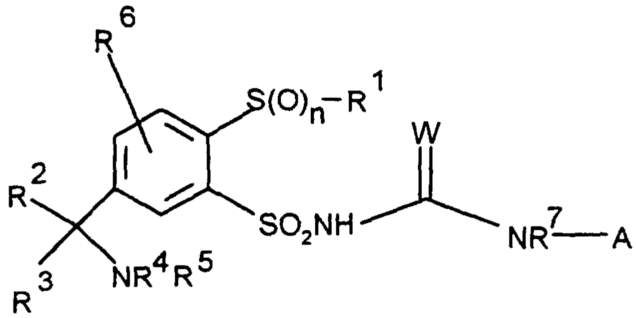

(I)

where

| | |
|---|---|
| $R^1$ | is $NR^8R^9$ or an unsubstituted or substituted hydrocarbon radical, |
| n | is 0, 1 or 2, excluding n = 0 or 1 if $R^1 = NR^8R^9$, |
| $R^2$ and $R^3$ | are each independently of the other H or (1-4)alkyl, |
| $R^4$ | is H, OH, formyl, or a radical of the formula R, R-O-, R-CO- or R-$SO_2$-, R being an unsubstituted or substituted hydrocarbon radical, |
| $R^5$ | is an acyl radical or |
| $NR^4R^5$ | together are an unsubstituted or substituted heterocyclic radical, |
| W | is an oxygen or sulfur atom, |
| $R^6$ | is H, (1-4)alkyl, (1-4)alkoxy, (1-4)alkylthio, [(1-4)alkyl]-carbonyl, or [(1-4) alkoxy]-carbonyl, each of the last radicals being unsubstituted or substituted by one or more halogen atoms, or halogen, $NO_2$, CN or mono- or disubstituted amino, |
| $R^7$ | is H or (1-4)alkyl, |
| $R^8$ and $R^9$ | are each independently of the other H, (1-4)alkyl, (1-4)alkoxy, [(1-4)alkyl]carbonyl or (1-4)alkylsulfonyl, |
| A | is a radical of the formula |

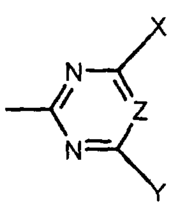

,

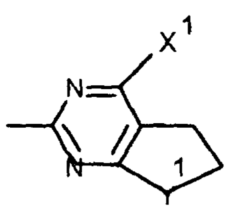

,

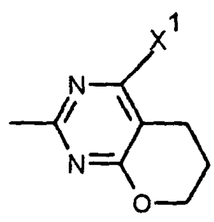

,

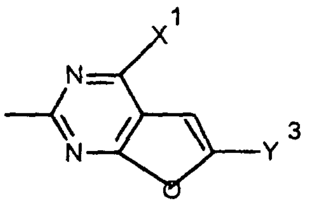 , 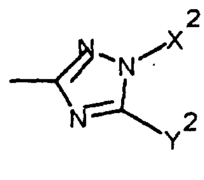 , 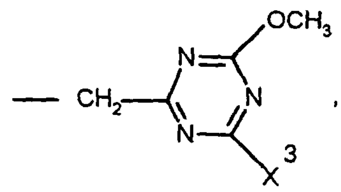 ,

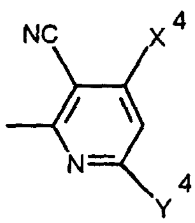 or 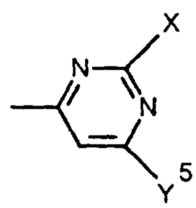

| | |
|---|---|
| one of the radicals X and Y | being hydrogen, (1-3)alkyl or (1-3)alkoxy, the last two radicals being unsubstituted or mono- or polysubstituted by halogen or monosubstituted by (1-3) alkoxy, and |
| the other of the radicals X and Y | being hydrogen, halogen, (1-3)alkyl, (1-3)alkoxy or (1-3)alkylthio, the last three alkyl-containing radicals being unsubstituted or mono- or polysubstituted by halogen or mono- or disubstituted by (1-3)alkoxy or (1-3)alkylthio, or a radical of the formula $NR^aR^b$, where $R^a$ and $R^b$, independently of each other, are H, (1-3)alkyl or alkenyl having up to 3 carbon atoms, or (3-6)cycloalkyl, (2-4) alkenyl, (2-4)alkynyl, (3-4)-alkenyloxy or (3-4)alkynyloxy, |
| Z | is CH or N, |
| $X^1$ | is $CH_3$, $OCH_3$, $OC_2H_5$ or $OCHF_2$, |
| $Y^1$ | is -O- or $-CH_2-$, |
| $X^2$ | is $CH_3$, $C_2H_5$ or $CH_2CF_3$, |
| $Y^2$ | is $OCH_3$, $OC_2H_5$, $SCH_3$, $SCH_2CH_3$, $CH_3$ or $C_2H_5$, |
| $X^3$ | is $CH_3$ or $OCH_3$, |
| $Y^3$ | is H or $CH_3$, |
| $X^4$ | is $CH_3$, $OCH_3$, $OC_2H_5$, $CH_2OCH_3$ or Cl, |
| $Y^4$ | is $CH_3$, $OCH_3$, $OC_2H_5$ or Cl and |
| $Y^5$ | is $CH_3$, $C_2H_5$, $OCH_3$ or Cl. |

**2.** A compound or a salt thereof as claimed in claim 1, wherein

| | |
|---|---|
| $R^1$ | is $NR^8R^9$, (1-6)alkyl, (2-6)alkenyl, (2-6)-alkynyl, (3-6)cycloalkyl, (3-6)cycloalkenyl or phenyl, each of the last 6 radicals being unsubstituted or substituted by one or more radicals from the group consisting of halogen, CN, (1-4)alkoxy, (1-4)haloalkoxy, (1-4)alkoxy(1-4)alkoxy, (1-4)alkylthio, (1-4)-alkylsulfinyl, (1-4)-alkylsulfonyl, formyl, [(1-4)alkyl]-carbonyl, [(1-4)alkoxy]carbonyl, [(1-4) alkyl]carbonyloxy and in the case of cyclic radicals also (1-4)alkyl, (1-4)haloalkyl and (1-4) alkoxy-(1-4)alkyl, |
| n | is 0, 1 or 2, excluding n = 0 or 1 if $R^1 = NR^8R^9$, |
| $R^2$ and $R^3$ | are each independently of the other H or (1-4)alkyl, |
| $R^4$ | is H, OH, formyl, (1-6)alkyl, (2-6)alkenyl, (2-6)alkynyl, (1-6)alkoxy, (2-5)alkenyloxy, (2-5)alkynyloxy, [(1-6)alkyl]-carbonyl, (1-6)-alkylsulfonyl, [(2-6)alkenyl]carbonyl, (2-6)-alkenylsulfonyl, [(2-6)alkynyl]carbonyl, (2-6)-alkynyl-sulfonyl, (3-6)cycloalkyl, (3-6)cycloalkenyl, [(3-6)cycloalkyl]-carbonyl, (3-6)-cycloalkylsulfonyl, [(3-6) cycloalkenyl] carbonyl or (3-6)cycloalkenylsulfonyl, each of the last 18 radicals being unsubstituted or substituted by one or more radicals from the group consisting |

of halogen, (1-4)alkoxy, (1-4)alkylthio, (1-4)alkylsulfinyl, (1-4)alkylsulfonyl, [(1-4)alkoxy]carbonyl, [(1-4)alkyl)carbonyl, [(1-4)alkyl]carbonyloxy and CN and in the case of cyclic radicals also (1-4) alkyl and (1-4)haloalkyl, or phenylcarbonyl or phenylsulfonyl, the phenyl ring in the last two radicals being unsubstituted or substituted by one or more radicals from the group consisting of halogen, CN, $NO_2$, (1-4)alkyl, (1-4)haloalkyl, (1-4)alkoxy and (1-4)haloalkoxy, and

$R^5$ is CHO, [(1-6)alkyl]carbonyl, [(2-6)alkenyl]-carbonyl, [(2-6)alkynyl]carbonyl, (1-6)alkylsulfonyl, (2-6)alkenylsulfonyl, (2-6)alkynylsulfonyl, [(3-6)cycloalkyl]carbonyl, [(3-6)-cycloalkenyl]carbonyl, (3-6)cycloalkylsulfonyl or (3-6)cycloalkenylsulfonyl, each of the last 10 radicals being unsubstituted or substituted by one or more radicals from the group consisting of halogen, (1-4)alkoxy, (1-4) alkylthio, (1-4)alkylsulfinyl, (1-4)alkylsulfonyl, [(1-4)alkyl]-carbonyl, [(1-4)alkoxy]carbonyl, [(1-4) alkyl]carbonyloxy and CN and in the case of cyclic radicals also (1-4)alkyl and (1-4)haloalkyl, or phenylcarbonyl or phenylsulfonyl, each of the last two radicals being unsubstituted in the phenyl ring or substituted by one or more radicals from the group consisting of halogen, CN, $NO_2$, (1-4) alkyl, (1-4)haloalkyl, (1-4)alkoxy and (1-4)haloalkoxy, or mono- or di-[(1-4)alkyl]aminosulfonyl which is unsubstituted in the alkyl moiety or substituted by one or more radicals from the group consisting of halogen, (1-4)alkoxy, (1-4)alkylthio, (1-4)alkylsulfinyl, [(1-4)-alkyl]-carbonyl, [(1-4) alkyi]carbonyloxy, [(1-4)alkoxy]carbonyl and CN or a group of the formula COCOR' where R' = H, OH, (1-4)alkoxy or (1-4)alkyl, or a group of the formula

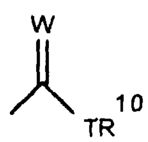 , 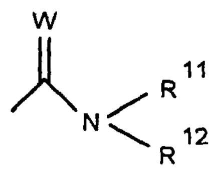 or 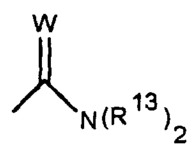 ,

or

$R^4$ and $R^5$ together are a chain of the formula $(-CH_2)_{m1}$ $B^1$- or $-B^1-(CH_2)_{m2}B^2$ where individual groups $CH_2$ may be replaced by oxygen atoms and where the chain is unsubstituted or substituted by one or more (1-3)alkyl radicals or halogen and m1 = 3, 4 or 5 or m2 = 2, 3 or 4, and

W is O or S,

$B^1$ and $B^2$ are each independently of the other $SO_2$ or CO,

T is O or S,

$R^6$ is H, (1-4)alkyl, (1-4)alkoxy, (1-4)alkylthio, [(1-4) alkyl]-carbonyl or [(1-4)alkoxy]carbonyl, each of the last 5 radicals being unsubstituted or substituted in the alkyl moiety by one or more halogen atoms, or halogen, $NO_2$, CN or mono- or di-[(1-4)alkyl]amino,

$R^7$ is H or (1-4)alkyl,

$R^8$ is (1-4)alkyl, (1-4)alkoxy, (3-6)cycloalkyl or (3-6)cycloalkenyl,

$R^9$ is H or (1-4)alkyl,

$R^{10}$ is (1-4)alkyl, (3-4)alkenyl or (3-4)alkynyl, each of the last three radicals being unsubstituted or substituted by one or more radicals from the group consisting of halogen, (1-4)alkoxy, (1-4)alkylthio, [(1-4)alkyl]-carbonyl and [(1-4)alkoxy]carbonyl,

$R^{11}$ and $R^{12}$ are each independently of the other H, (1-4)alkyl, (3-4)alkenyl or (3-4)alkynyl, each of the last three radicals being unsubstituted or substituted by one or more radicals from the group consisting of halogen, (1-4)alkoxy, (1-4)alkylthio, [(1-4)alkyl]carbonyl and [(1-4)alkoxy]carbonyl,

the radicals $R^{13}$ together with the nitrogen atom are a 5- to 6-membered heterocyclic ring which may contain further hetero atoms from the group consisting of N, O and S in the possible oxidation states and is unsubstituted or substituted by (1-4)alkyl or the oxo group or is benzo-fused.

**3.** A compound or a salt thereof as claimed in claim 1 or 2, wherein

$R^1$ is $NR^8R^9$, (1-4) alkyl, (3-6) cycloalkyl or phenyl, each of the last 3 radicals being unsubstituted or substituted by one or more radicals from the group consisting of halogen, CN, (1-4)alkoxy, (1-4) haloalkoxy, (1-4)alkylthio, (1-4)-alkylsulfonyl, formyl, [(1-4)alkoxy)carbonyl, [(1-4)-alkyl]carbonyloxy and in the case of cyclic radicals also (1-4)alkyl, (1-4)haloalkyl and (1-4)alkoxy-(1-4)alkyl, or (2-4) alkenyl or (2-4)alkynyl,

n is 0, 1 or 2, excluding n = 0 or 1 if $R^1 = NR^8R^9$,

$R^2$ and $R^3$ are each independently of the other H or (1-4)alkyl,

$R^4$ is H, OH, formyl, (1-4)alkyl, (2-4)alkenyl, (2-4)alkynyl, (1-4)alkoxy, [(1-4)alkyl]-carbonyl, (1-4)alkylsulfonyl, each of the last 6 radicals being unsubstituted or substituted by one or more radicals from the group consisting of halogen and (1-4)alkoxy,
or phenylcarbonyl or phenylsulfonyl, the phenyl ring in the last two radicals being unsubstituted or substituted by one or more radicals from the group consisting of halogen, (1-4)alkyl, (1-4)haloalkyl, (1-4)alkoxy and (1-4)haloalkoxy, and

$R^5$ is CHO, [(1-4)alkyl]carbonyl, [(2-4)alkenyl]-carbonyl, [(2-4)alkynyl]carbonyl, (1-4)alkylsulfonyl, (2-4) alkenylsulfonyl, (2-4)alkynylsulfonyl, [(3-6)cycloalkyl]carbonyl, each of the last 7 radicals being unsubstituted or substituted by one or more radicals from the group consisting of halogen, (1-4) alkoxy, (1-4)alkylthio, [(1-4)alkoxy]carbonyl, [(1-4)-alkyl]-carbonyloxy and CN and in the case of cyclic radicals also (1-4)alkyl and (1-4)haloalkyl, or phenylcarbonyl or phenylsulfonyl, each of the last two radicals being unsubstituted in the phenyl ring or substituted by one or more radicals from the group consisting of halogen, CN, $NO_2$, (1-4)alkyl, (1-4)haloalkyl, (1-4)alkoxy and (1-4)haloalkoxy, or mono- or di-[(1-4) alkyl) aminosulfonyl which is unsubstituted in the alkyl moiety or substituted by one or more halogen atoms, or a group of the formula COCOR' where
R' = H, OH, (1-4)alkoxy or (1-4)alkyl, or a group of the formula

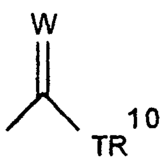
,
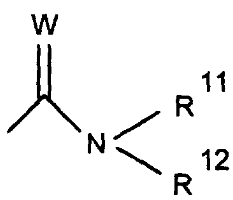
or
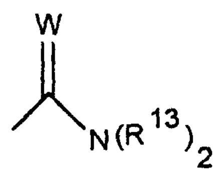

or

$R^4$ and $R^5$ together are a chain of the formula $(-CH_2)_{m1}B^1$- or $-B^1-(CH_2)_{m2}B^2$-, where individual groups $CH_2$ may be replaced by oxygen atoms and where the chain is unsubstituted or substituted by one or more (1-3)alkyl radicals or halogen and m1 = 3, 4 or 5 or m2 = 2, 3 or 4, and

W is O or S,

$B^1$ and $B^2$ are each independently of the other $SO_2$ or CO,

T is O or S,

$R^6$ is H, (1-4)alkyl, (1-4)alkoxy or halogen,

$R^7$ is H or $CH_3$,

$R^8$ is (1-4)alkyl,

$R^9$ is H or (1-4)alkyl,

$R^{10}$ is (1-4)alkyl, (1-4)haloalkyl, (3-4)alkenyl or (3-4)alkynyl,

$R^{11}$ and $R^{12}$ are each independently of the other H or (1-4)alkyl, the radicals $R^{13}$ together are an alkylene chain having 4 or 5 carbon atoms,

A is a radical of the formula

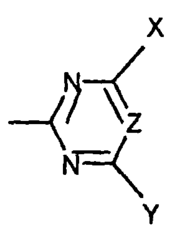
,

one of the radicals

| | |
|---|---|
| X and Y | being (1-3) alkyl, halo (1-3) alkyl, (1-3) alkoxy or halo(1-3)alkoxy and |
| the other of the radicals X and Y | being (1-3)alkyl, halo(1-3)alkyl, (1-3)alkoxy, halo(1-3)alkoxy, halogen, mono- or di-[(1-3)alkyl]amino and |
| Z | is CH or N. |

**4.** A compound or a salt thereof as claimed in any of claims 1 to 3, wherein

| | |
|---|---|
| $R^1$ | is mono- or di-[(1-4)alkyl]amino or (1-4)alkyl, |
| n | is 2, |
| $R^2$ and $R^3$ | are each hydrogen, |
| $R^4$ | is H or (1-4)alkyl, |
| $R^5$ | is CHO, (1-4)alkyl]carbonyl, [(1-4)haloalkyl]-carbonyl, (1-4)alkylsulfonyl, (1-4)haloalkylsulfonyl, [(1-4)alkoxy]-carbonyl, mono- or di-[(1-4)alkyl]aminocarbonyl, mono- or di-[(1-4)-alkyl) aminosulfonyl or |
| $R^4$ and $R^5$ | together are a chain of the formula $(-CH_2)_{m1}B^1$- or $-B^1-(CH_2)_{m2}B^2$- where $B^1$ and $B^2$ independently of each other are $SO_2$ or CO, |
| W and T | are each O, |
| $R^6$ | is H and |
| $R^7$ | is H or $CH_3$. |

**5.** A process for preparing compounds of the formula (I) or salts thereof as defined in any of claims 1 to 4, which comprises

a) reacting a compound of the formula (II)

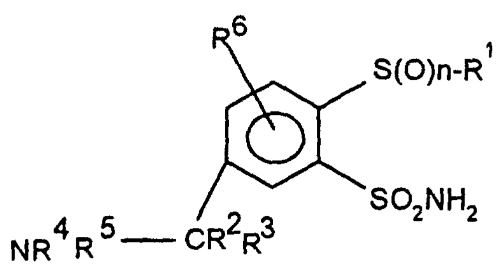

(II)

with a heterocyclic carbamate of the formula (III),

$$R^* - O - CO - NR^7 - A \qquad (III)$$

where R* is unsubstituted or substituted phenyl or (1-4)alkyl, or

b) reacting an arylsulfonylcarbamate of the formula (IV)

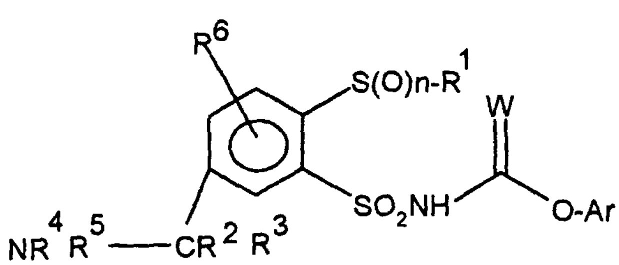

(IV) ,

where Ar is an aryl radical,
with an amino heterocycle of the formula (V)

$$H - NR^7 - A \quad (V),$$

or

c) reacting a sulfonyl isocyanate of the formula (VI)

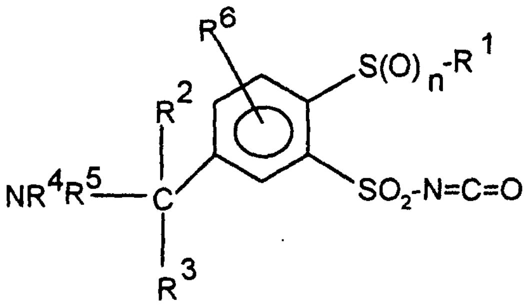

(VI)

with an amino heterocycle of the formula $H\text{-}NR^7\text{-}A$ (V), or

d) reacting in a one-pot reaction first an amino heterocycle of the formula $H\text{-}NR^7\text{-}A$ (V) in the presence of a base with phosgene and then the intermediate formed with a phenylsulfonamide of the formula (II), or

e) reacting a sulfonyl chloride of the formula (VII)

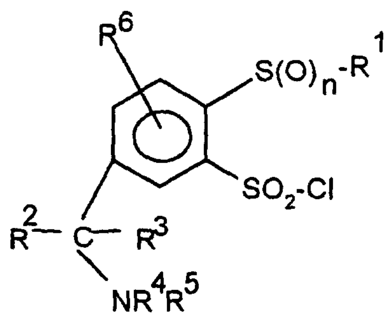

(VII)

with a cyanate M-OCN where M = a cation and with an amino heterocycle of the formula $H\text{-}NR^7\text{-}A$ (V) in the presence of a base, or

f) reacting a sulfonamide of the formula (II) mentioned with a (thio) isocyanate of the formula (V')

$$W = C = N - A \quad (V')$$

in the presence of a base,

where the radicals or symbols $R^1$ to $R^7$, A, W and n in the formulae (II)-(VII) and (V') are as defined in formula (I) and where the compounds initially obtained in variants a) and c)-e) are compounds of the formula (I) where W = O.

**6.** A herbicidal or plant growth regulating composition, which comprises at least one compound of the formula (I) or

a salt thereof as claimed in any of claims 1 to 4 and formulation auxiliaries customary in crop protection.

**7.** A method for controlling harmful plants or for regulating the growth of plants, which comprises applying an active amount of at least one compound of the formula (I) or a salt thereof as claimed in any of claims 1 to 4 to the plants, plant seeds or to the area cultivated.

**8.** Use of the compounds of the formula (I) or salts thereof as claimed in any of claims 1 to 4 as herbicides or plant growth regulators.

**9.** A compound of the formula (II), (IV), (VI) and (VII) as defined in claim 5.

## Revendications

**1.** Composés de formule (I) ou leurs sels,

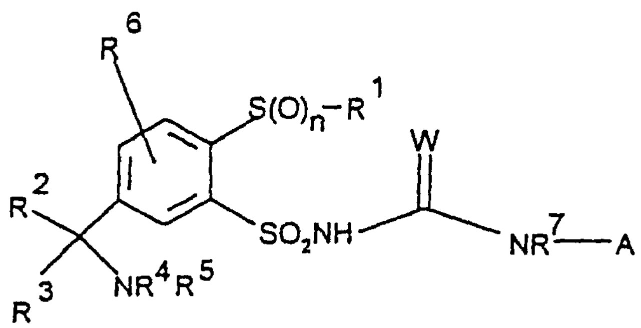

(I)

dans laquelle

| | |
|---|---|
| $R^1$ | signifie $NR^8R^9$, un résidu d'hydrocarbure, qui est substitué ou non, |
| n | signifie 0, 1 ou 2, excepté n=0 ou 1 si $R^1=NR^8R^9$, |
| $R^2$, $R^3$ | signifient indépendamment l'un de l'autre un atome d'hydrogêne ou un groupe (1-4) alkyle, |
| $R^4$ | signifie un atome d'hydrogène, un groupe OH, formyle, un résidu de formule R, R-O-, R-CO- ou $R-SO_2^-$, où R signifie un résidu d'hydrocarbure, qui est substitué ou non, |
| $R^5$ | signifie un résidu acyle ou |
| $NR^4R^5$ | signifie ensemble un résidu hétérocyclique qui est substitué ou non, |
| W | signifie un atome d'oxygène ou de soufre |
| $R^6$ | signifie un atome d'hydrogène, un groupe (1-4)alkyle, (1-4)alkoxy, (1-4)alkylthio, [(1-4)alkyl]carbonyle, [(1-4)aikoxy]carbonyle, où chacun des résidus cités en dernier lieu est non substitué ou substitué par un ou plusieurs atomes d'halogène, ou un atome d'halogène, un groupe NO2, CN ou un groupe amino mono- ou disubstitué, |
| $R^7$ | signifie un atome d'hydrogène ou un groupe (1-4)alkyle, |
| $R^8$, $R^9$ | signifient indépendamment l'un de l'autre un atome d'hydrogène, un groupe (1-4)alkyle, (1-4)alkoxy, [(1-4)alkyl]carbonyle ou (1-4)alkylsulfonyle, |
| A | signifie un résidu de formule |

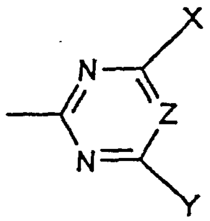 , 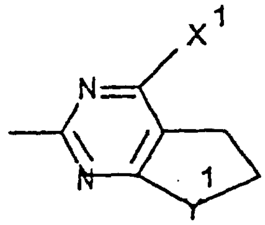 , 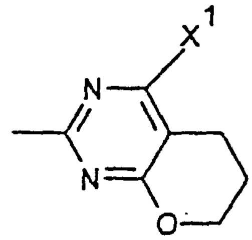

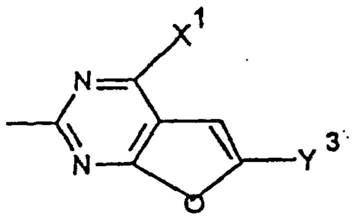 , 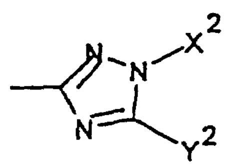 , 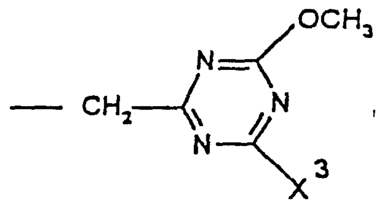 ,

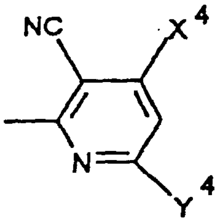 ou 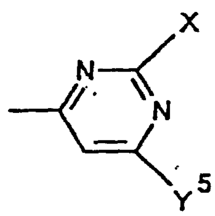

| | |
|---|---|
| un des résidus X et Y | signifie un atome d'hydrogène, un groupe (1-3)alkyle ou (1-3)alkoxy, où les deux résidus cités en dernier lieu sont non substitués ou substitués une ou plusieurs fois par un atome d'halogène ou une fois par un groupe (1-3)alkoxy, et |
| l'autre des résidus X et Y | signifie un atome d'hydrogène, d'halogène, un groupe (1-3)alkyle, (1-3)alkoxy ou (1-3)alkylthio, où les trois résidus cités en dernier lieu sont non substitués ou substitués une ou plusieurs fois par un atome d'halogène ou une ou deux fois par un groupe (1-3)alkoxy ou (1-3)alkylthio, ou un résidu de formule $NR^aR^b$ dans lequel $R^a$, $R^b$ signifient indépendamment l'un de l'autre un atome d'hydrogène, un groupe (1-3)alkyle ou alkényle avec jusqu'à 3 atomes de carbone, ou un groupe (3-6)cycloalkyle, (2-4)alkényle, (2-4)alkinyle, (3-4)-alkényloxy ou (3-4)alkinyloxy, |
| Z | signifie un groupe CH ou un atome d'azote, |
| $X^1$ | signifie un groupe $CH_3$, $OCH_3$, $OC_2H_5$ ou $OCHF_2$, |
| $Y^1$ | signifie -O- ou un groupe $-CH_2-$ |
| $X^2$ | signifie un groupe $CH_3$, $C_2H_5$ ou $CH_2CF_3$, |
| $Y^2$ | signifie un groupe $OCH_3$, $OC_2H_5$, $SCH_3$, $SCH_2CH_3$, $CH_3$, ou $C_2H_5$, |
| $X^3$ | signifie un groupe $CH_3$ ou $OCH_3$, |
| $Y^3$ | signifie un atome d'hydrogène ou un groupe $CH_3$, |
| $X^4$ | signifie un groupe $CH_3$, $OCH_3$, $OC_2H_5$, $CH_2OCH_3$ ou Cl, |
| $Y^4$ | signifie un groupe $CH_3$, $OCH_3$, $OC_2H_5$ ou Cl et |
| $Y^5$ | signifie un groupe $CH_3$, $C_2H_5$, $OCH_3$ ou Cl, |

**2.** Composés ou leurs sels selon la revendication 1, **caractérisés en ce que**,

$R^1$ signifie $NR^8R^9$, un groupe (1-6)alkyle, (2-6) alkényle, (2-6) alkinyle, (3-6)cycloalkyle, (3-6)cycloalkényle ou phényle, où chacun des 6 résidus cités en dernier lieu est non substitué ou substitué par un ou plusieurs résidus de l'ensemble halogène, un groupe CN, (1-4)alkoxy, (1-4)haloalkoxy, (1-4) alkoxy(1-4)alkoxy, (1-4)alkylthio, (1-4)alkyl-sulfinyle, (1-4)alkylsulfonyle, formyle, [(1-4)alkyl]-carbonyle, [(1-4)alkoxy]-carbonyle, [(1-4)alkyl]-carbonyloxy et dans les cas de résidus cycliques aussi un groupe (1-4)alkyle, (1-4)haloalkyle et (1-4)alkoxy-(1-4)alkyle,

n signifie 0, 1 ou 2, excepté n=0 ou 1 si $R^1=NR^8R^9$,

$R^2$, $R^3$ signifient indépendamment l'un de l'autre un atome d'hydrogène ou un groupe (1-4)alkyle,

$R^4$ signifie un atome d'hydrogène, un groupe OH, formyle, (1-6)alkyle, (2-6)alkényle, (2-6)alkinyle, (1-6) alkoxy, (2-5)alkényloxy, (2-5)alkinyloxy, [(1-6)alkyl]-carbonyle, (1-6)alkyl-sulfonyle, [(2-6)alkényl]-carbonyle, (2-6)alkényl-sulfonyle, [(2-6)alkinyl]-carbonyle, (2-6)alkinylsulfonyle, (3-6)cycloalkyle, (3-6)cycloalkényle, [(3-6)cycloalkyl]-carbonyle, (3-6)cycloalkylsulfonyle, [(3-6)cycloalkényl] -carbonyle ou (3-6)cycloalkénylsulfonyle, où chacun des 18 résidus cités en dernier lieu est non substitué ou substitué par un ou plusieurs résidus de l'ensemble halogène, groupe (1-4)alkoxy, (1-4)alkylthio, (1-4)alkylsulfinyle, (1-4)alkylsulfonyle, [(1-4)alkoxy]-carbonyle, [(1-4)alkyl]-carbonyle, [(1-4)alkyl)-carbonyloxy et CN et dans les cas de résidus cycliques aussi par un groupe (1-4)alkyle et (1-4) haloalkyle, ou phénylcarbonyle ou phénylsulfonyle, où dans les deux résidus cités en dernier lieu le cycle phényle est non substitué ou substitué par un ou plusieurs résidus de l'ensemble halogène, un groupe CN, $NO_2$, (1-4)alkyle, (1-4)haloalkyle, (1-4)alkoxy et (1-4)haloalkoxy,et

$R^5$ signifie un groupe CHO, [(1-6)alkyl]-carbonyle, [(2-6)alkényl]-carbonyle, [(2-6)alkinyl]-carbonyle, (1-6)alkyl-sulfonyle, (2-6)alkényl-sulfonyle, (2-6)alkinylsulfonyle, [(3-6)cycloalkyl]-carbonyle, [(3-6) cyclo-alkényl]-carbonyle, (3-6)cycloalkyl-sulfonyle ou (3-6)cycloalkénylsulfonyle où chacun des 10 résidus cités en dernier lieu est non substitué ou substitué par un ou plusieurs résidus de l'ensemble halogène, un groupe (1-4)alkoxy, (1-4)alkylthio, (1-4)alkylsulfinyle, (1-4)alkylsulfonyle, [(1-4)akyl]-carbonyle, [(1-4)alkoxy]-carbonyle, [(1-4)alkyl]-carbonyloxy et CN et dans les cas de résidus cycliques aussi par un groupe (1-4)alkyle et (1-4)haloalkyle, ou phénylcarbonyle ou phénylsulfonyle, où dans chacun des deux résidus cités en dernier lieu le cycle phényle est non substitué ou substitué par un ou plusieurs résidus du groupe halogène, un groupe CN, $NO_2$, (1-4)alkyl, (1-4)haloalkyle, (1-4)alkoxy et (1-4)haloalkoxy, ou un groupe mono-ou di-[(1-4)alkyl]aminosulfonyle qui dans la partie alkyle est non substitué ou substitué par un ou plusieurs résidus de l'ensemble halogène, un groupe (1-4)alkoxy, (1-4)alkylthio, (1-4)alkylsulfinyle, [(1-4)alkyl]-carbonyle, [(1-4)alkyl]-carbonyloxy, [(1-4) alkoxy]-carbonyle et CN ou un groupe de formule COCOR' dans laquelle R' = H, OH, (1-4)alkoxy ou (1-4)alkyle, ou un groupe de formule

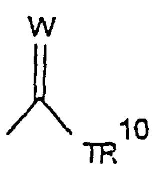 , 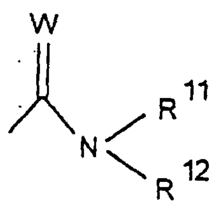 ou 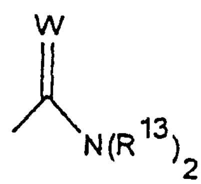

ou

$R^4$ et $R^5$ signifient ensemble une chaîne de formule $(-CH_2)_{m1}B^1$- ou $-B^1-(-CH_2)_{m2}B^2-$, où différents groupes $CH_2$ peuvent être remplacés par un atome d'oxygène et où la chaîne est non substituée ou substituée par un ou plusieurs résidus (1-3)alkyle ou un atome d'halogène et m1 = 3, 4 ou 5 respectivement m2 = 2, 3 ou 4, ainsi que

W signifie un atome d'oxygène ou de soufre

$B^1$, $B^2$ signifient indépendamment l'un de l'autre $SO_2$ ou CO,

T signifie un atome d'oxygène ou de soufre

$R^6$ signifie un atome d'hydrogène, un groupe (1-4)alkyle, (1-4)alkoxy, (1-4)alkylthio, [(1-4)alkyl]carbonyle ou [(1-4)alkoxy]carbonyle, où chacun des 5 résidus cités en dernier lieu est non substitué ou substitué dans la partie alkyle par un ou plusieurs atomes d'halogène, ou un atome d'halogène, un groupe NO2, CN ou un groupe mono- ou di-[(1-4)alkyl]-amino,

$R^7$ signifie un atome H ou un groupe (1-4)alkyle

$R^8$ signifie un groupe (1-4)alkyle, (1-4)alkoxy, (3-6)cycloalkyle ou (3-6)cycloalkényle,

$R^9$ signifie un atome d'hydrogène ou un groupe (1-4)alkyle

$R^{10}$ signifie un groupe (1-4) alkyle, (3-4)alkényle ou (3-4)alkinyle où chacun des trois résidus cités en dernier lieu est non substitué ou substitué par un ou plusieurs résidus de l'ensemble halogène, un groupe (1-4)alkoxy, (1-4)alkylthio, [(1-4)alkyl]carbonyle et [(1-4]alkoxy]carbonyle,

$R^{11}$ et $R^{12}$ signifient indépendamment l'un de l'autre un atome d'hydrogène, un groupe (1-4)alkyle, (3-4)alkényle ou (3-4)alkinyle, où chacun des trois résidus cités en dernier lieu est non substitué ou substitué par un ou plusieurs résidus de l'ensemble halogène, groupe (1-4)alkoxy, (1-4)alkylthio, [(1-4)alkyl] carbonyle et [(1-4)alkoxy]carbonyle,

les résidus $R^{13}$ signifient conjointement avec l'atome d'azote un composé hétérocyclique à 5 ou 6 chaînons qui peut contenir d'autres hétéroatomes du groupe N, O et S dans les degrés d'oxydation possibles et qui est non substitué ou substitué par un groupe (1-4)alkyl ou le groupe oxo ou benzocondensé.

3. Composés ou leurs sels selon la revendication 1 ou 2, **caractérisés en ce que**,

$R^1$ signifie $NR^8R^9$, un groupe (1-4)alkyle, (3-6)cycloalkyle ou phényle où chacun des 3 résidus cités en dernier lieu est non substitué ou substitué par un ou plusieurs résidus de l'ensemble halogène, groupe CN, (1-4)alkoxy, (1-4)haloalkoxy, (1-4)alkylthio, (1-4)alkylsulfonyle, formyle, [(1-4)alkoxy]-carbonyle, [(1-4)alkyl]-carbonyloxy et dans le cas de résidus cycliques aussi un groupe (1-4)alkyle, (1-4)haloalkyle et (1-4)alkoxy-(1-4)alkyle, ou un groupe (2-4)alkényle ou (2-4)alkinyle,

n signifie 0, 1 ou 2, excepté n=0 ou 1 si $R^1$=$NR^8R^9$,

$R^2$, $R^3$ signifient indépendamment l'un de l'autre un atome d'hydrogène ou un groupe (1-4)alkyle,

$R^4$ signifie un atome d'hydrogène, un groupe OH, formyle, (1-4)alkyle, (2-4)alkényle, (2-4)alkinyle, (1-4) alkoxy, [(1-4)alkyl]-carbonyle, (1-4)alkyl-sulfonyle, où chacun des 6 résidus cités en dernier lieu est non substitué ou substitué par un ou plusieurs résidus de l'ensemble halogène et groupe (1-4)alkoxy, ou un groupe phénylcarbonyle ou phénylsulfonyle, où dans les deux résidus cités en dernier lieu le cycle phényle est non substitué ou substitué par un ou plusieurs résidus de l'ensemble halogène, groupe (1-4) alkyle, (1-4)haloalkyle, (1-4)alkoxy et (1-4)haloalkoxy, et

$R^5$ signifie un groupe CHO, [(1-4)alkyl]-carbonyle, [(2-4)alkényl]-carbonyle, ((2-4)alkinyl]-carbonyle, (1-4) alkylsulfonyle, (2-4)alkényl-sulfonyle, (2-4)alkinylsulfonyle, [(3-6)cycloalkyl]-carbonyle, où chacun des 7 résidus cités en dernier lieu est non substitué ou substitué par un ou plusieurs résidus de l'ensemble halogène, groupe (1-4)alkoxy, (1-4)alkylthio, [(1-4)aikoxy]-carbonyle, [(1-4)alkyl]-carbonyloxy, et CN et dans les cas de résidus cycliques aussi par un groupe (1-4)alkyl ou (1-4)haloalkyle, ou phénylcarbonyle ou phénylsulfonyle, où dans chacun des deux résidus cités en dernier lieu le cycle phényle est non substitué ou substitué par un ou plusieurs résidus de l'ensemble halogène, un groupe CN, $NO_2$, (1-4) alkyle, (1-4)haloalkyle, (1-4)alkoxy et (1-4)haloalkoxy, ou un groupe mono-ou di-[(1-4)alkyl]aminosulfonyle qui est non substitué dans la partie alkyle ou substitué par un ou plusieurs atomes d'halogène, ou un groupe de formule COCOR', dans lequel R' = H, OH, (1-4)alkoxy ou (1-4) alkyle, ou un groupe de formule

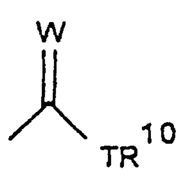 , 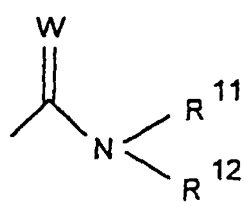 ou 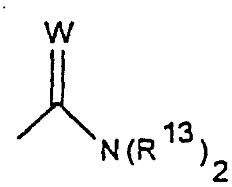

ou

$R^4$ et $R^5$ signifient ensemble une chaîne de formule $(-CH_2)_{m1}B^1$- ou $-B^1-(-CH_2)_{m2}B^2$-, où différents groupes $CH_2$ peuvent être remplacés par un atome d'oxygène et où la chaîne est non substituée ou substituée par un ou plusieurs résidus (1-3)alkyle ou un atome d'halogène et m1 = 3, 4 ou 5 respectivement m2 = 2, 3

W signifie un atome d'oxygène ou de soufre

$B^1$, $B^2$ signifient indépendamment l'un de l'autre $SO_2$ ou CO,

T signifie un atome d'oxygène ou de soufre

$R^6$ signifie un atome d'hydrogène, un groupe (1-4)alkyle, (1-4)alkoxy ou un atome d'halogène,

$R^7$ signifie un atome d'hydrogène ou un groupe $CH_3$,
$R^8$ signifie un groupe (1-4)alkyle,
$R^9$ signifie un atome d'hydrogène ou un groupe (1-4)alkyle
$R^{10}$ signifie un groupe (1-4)alkyle, (1-4)haloalkyle, (3-4)alkényle ou (3-4)alkinyle,
$R^{11}$, $R^{12}$ signifient indépendamment l'un de l'autre un atome d'hydrogène ou un groupe (1-4)alkyle,

les résidus $R^{13}$ signifient ensemble une chaîne alkylène avec 4 ou 5 atomes de carbone,

A signifie un résidu de formule

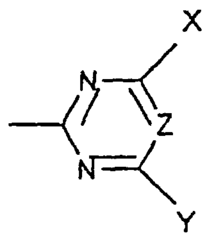

un des résidus X et Y signifie un groupe (1-3)alkyle, halo(1-3)alkyle, (1-3)alkoxy, ou halo(1-3)alkoxy et
l'autre des résidus X et Y signifie un groupe (1-3)alkyle, halo(1-3)alkyle, (1-3)alkoxy, halo(1-3)alkoxy, un atome d'halogène, un groupe mono ou di-[(1-3)alkyl]-amino et
Z signifie un groupe CH ou un atome d'azote,

**4.** Composés ou leurs sels selon une des revendications 1 à 3, **caractérisés en ce que**

$R^1$ signifie un groupe mono- ou di-[(1-4)alkyl]-amino ou (1-4)alkyle,
n signifie le chiffre 2,
$R^2$, $R^3$ signifient chacun un atome d'hydrogène,
$R^4$ signifie un atome d'hydrogène ou un groupe (1-4)alkyle,
$R^5$ signifie un groupe CHO, [(1-4)alkyl]-carbonyle, [(1-4)haloalkyl]-carbonyle, (1-4)alkyl-sulfonyle, (1-4) haloalkyl-sulfonyle, [(1-4)alkoxy]-carbonyle, mono- ou di-[(1-4)alkyl]aminocarbonyle, mono- ou di-[(1-4) alkyl]aminosulfonyle ou
$R^4$, $R^5$ signifient ensemble une chaîne de formule $(-CH_2)_{m1}B^1-$ ou $-B^1-(-CH_2)_{m2}B^2-$, où $B^1$ et $B^2$ signifient indépendamment l'un de l'autre $SO_2$ ou CO,
W, T signifient chacun un atome d'oxygène,
$R^6$ signifie un atome d'hydrogène et
$R^7$ signifie un atome d'hydrogène ou un groupe $CH_3$,

**5.** Procédé pour la préparation de composés de formule (I) ou de leurs sels, tels qu'ils sont définis selon l'une des revendications 1 à 4, **caractérisé par** la réaction

a) d'un composé de formule (II)

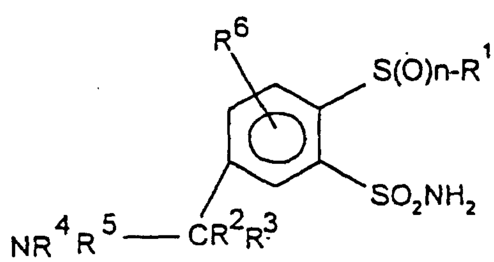

(II)

avec un carbamate hétérocyclique de formule (III),

R*-O-CO-NR7-A (III)

dans lequel R* signifie le cas échéant un groupe phényle ou (1-4)alkyle substitué.

b) ou d'un arylsulfonylcarbamate de formule (IV)

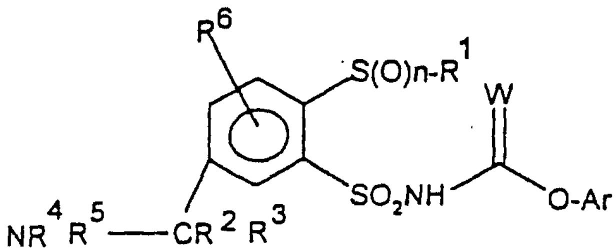

(IV)

dans lequel Ar signifie un résidu aryle,

avec un composé aminohétérocyclique de formule (V)

$$H\text{-}NR^7\text{-}A \qquad (V)$$

c) ou d'un sulfonylisocyanate de formule (VI)

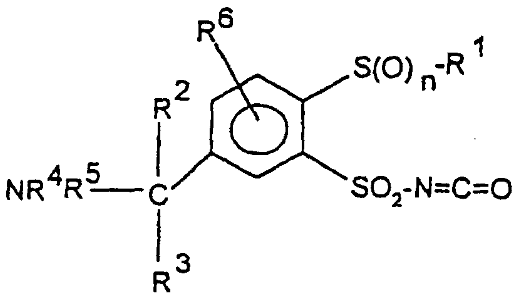

(VI)

avec un composé aminohétérocyclique de formule $H\text{-}NR^7\text{-}A$ (V)

d) ou, par une réaction unique, d'abord d'un composé aminohétérocyclique de formule $H\text{-}NR^7\text{-}A$ (V) en présence d'une base avec du phosgène puis de l'intermédiaire formé avec un phénylsulfonamide de formule (II)

e) ou d'un sulfonylchlorure de formule (VII)

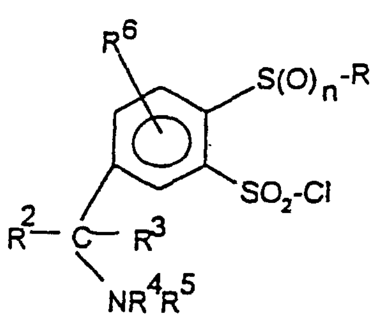

(VII)

avec un cyanate M-OCN, dans lequel M = un cation, avec un composé aminohétérocyclique de formule $H\text{-}NR^7\text{-}A$ (V) en présence d'une base

f) ou d'un sulfonamide de formule (II) citée avec un (thio-)isocyanate de formule (V')

$$W = C = N - A \qquad (V')$$

en présence d'une base,
où dans les formules (II)-(VII) et (V') les résidus respectivement les symboles $R^1$ jusqu'à $R^7$, A, W et n sont définis comme dans la formule (I) et où dans les variantes a) et c)-e) on obtient d'abord des composés de formule (I) dans lesquels W = O.

**6.** Agent herbicide ou régulant la croissance des plantes, **caractérisé en ce qu'**il contient au moins un composé de formule (I) ou un sel d'un tel composé selon l'une des revendications 1 à 4 et des additifs de formulation usuels dans la protection des plantes.

**7.** Procédé pour la lutte contre les plantes parasites ou pour la régulation de la croissance des plantes, **caractérisé en ce que** l'on applique une quantité efficace d'au moins un composé de formule (I) ou d'un sel d'un tel composé selon l'une des revendications 1 à 4 sur les plantes, les graines de plantes ou la surface cultivée.

**8.** Utilisation des composés de formule (I) ou de leurs sels selon l'une des revendications 1 à 4 comme herbicides ou régulateurs de la croissance des plantes.

**9.** Composés de formules (II), (IV), (VI) et (VII), tels qu'ils sont définis dans la revendication 5.